(19) Europäisches Patentamt
European Patent Office
Office européen des brevets

(11) EP 4 763 845 A1

(12) EUROPEAN PATENT APPLICATION
published in accordance with Art. 153(4) EPC

(43) Date of publication:
**24.06.2026 Bulletin 2026/26**

(21) Application number: **24853894.4**

(22) Date of filing: **16.08.2024**

(51) International Patent Classification (IPC):
***C07D 471/04*** (2006.01) ***C07D 471/12*** (2006.01)
***A61K 31/4985*** (2006.01) ***A61P 35/00*** (2006.01)

(52) Cooperative Patent Classification (CPC):
**A61K 31/4985; A61P 35/00; C07D 471/04; C07D 471/12**

(86) International application number:
**PCT/CN2024/112785**

(87) International publication number:
**WO 2025/036489 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**17.08.2023 CN 202311039226**
**15.09.2023 CN 202311193788**
**10.11.2023 CN 202311496667**
**06.02.2024 CN 202410169788**
**15.04.2024 CN 202410449334**

(71) Applicant: **Haisco Pharmaceutical Group Co., Ltd.**
**Shannan, Tibet 856000 (CN)**

(72) Inventors:
- **ZHANG, Chen**
  **Chengdu, Sichuan 611130 (CN)**
- **LIAO, Yuting**
  **Chengdu, Sichuan 611130 (CN)**
- **LI, Yupeng**
  **Chengdu, Sichuan 611130 (CN)**
- **LU, Yonghua**
  **Chengdu, Sichuan 611130 (CN)**
- **ZOU, Sijia**
  **Chengdu, Sichuan 611130 (CN)**
- **ZHAO, Junbin**
  **Chengdu, Sichuan 611130 (CN)**
- **TANG, Pingming**
  **Chengdu, Sichuan 611130 (CN)**
- **LI, Linli**
  **Chengdu, Sichuan 611130 (CN)**
- **GAO, Qiu**
  **Chengdu, Sichuan 611130 (CN)**
- **LI, Yao**
  **Chengdu, Sichuan 611130 (CN)**
- **YAN, Pangke**
  **Chengdu, Sichuan 611130 (CN)**

(74) Representative: **AWA Denmark A/S**
**Strandgade 56**
**1401 Copenhagen K (DK)**

# (54) CYANO AROMATIC RING DERIVATIVE AND PHARMACEUTICAL USE THEREOF

(57) A cyano aromatic ring derivative and the pharmaceutical use thereof. Specifically, the present invention relates to a compound of general formula (I) or a stereoisomer, a tautomer, a deuterated substance, a solvate, a prodrug, a metabolite, a pharmaceutically acceptable salt or cocrystal thereof, an intermediate thereof, and the use thereof in diseases associated with the inhibition or degradation of AR, such as cancers. B-L-K (I).



Processed by Luminess, 75001 PARIS (FR)

## Description

### Technical Field

The present invention relates to a compound of general formula (I) or a stereoisomer, tautomer, deuterated substance, solvate, prodrug, metabolite, pharmaceutically acceptable salt or co-crystal thereof, and an intermediate thereof and a preparation method therefor, as well as the use thereof in AR-related diseases such as cancer.

### Background Art

Prostate cancer is mostly found in early stages, and its pathogenesis is often related to genetic factors, high-fat diet and endocrine. Generally, prostate cancer is more prevalent in developed countries than in developing countries. In 2016, there were 120,000 new prostate cancer patients in China, and by 2030, it is expected that the number of new patients will reach 237,000, and the market share will reach 4.8 billion US dollars. Patients with early-stage prostate cancer may be treated with radical therapy and have a longer survival time, while advanced patients with metastasis may be treated by a castration procedure combined with anti-androgen therapy and may develop castration-resistant prostate cancer. Clinical studies have shown that in most patients with castration-resistant prostate cancer, androgen receptors (ARs) are overexpressed, and inhibition of androgen receptor (AR) signalling has significant efficacy in patients with hormone-refractory prostate cancer, so that inhibition of androgen receptors (ARs) is an effective means to directly block this pathway.

PROTAC (proteolysis targeting chimera) molecules are a class of bifunctional compounds that can simultaneously bind targeting proteins and E3 ubiquitin ligases. Such compounds can be recognised by proteasomes of cells, causing the degradation of targeting proteins, and can effectively reduce the content of targeting proteins in the cells. By introducing a ligand capable of binding to various targeting proteins into the PROTAC molecules, it is possible to apply the PROTAC technology to the treatment of various diseases, and this technology has attracted extensive attention in recent years.

Therefore, it is necessary to develop a novel PROTAC drug targeting androgen receptor (AR) for the treatment of androgen receptor-related tumour diseases.

### Summary of the Invention

An objective of the present invention is to provide a compound with a novel structure, good efficacy, high bioavailability and higher safety that can inhibit or degrade AR, for use in the treatment of AR-related diseases such as cancer.

The compounds of the present invention exhibit good inhibitory activity against the proliferation of prostate cancer cells (such as VCaP cells), good degradation of AR protein, good oral performance in *in vivo* pharmacokinetics, no significant inhibitory effect on hERG potassium channel current, good liver microsomal stability, no significant inhibitory activity against human liver microsomal cytochrome P450, and no significant inhibitory effect on the proliferation of CD34+ hematopoietic stem cells.

The present invention provides a compound or a stereoisomer, tautomer, deuterated substance, solvate, prodrug, metabolite, pharmaceutically acceptable salt or co-crystal thereof, wherein the compound is a compound represented by general formula (I),

B-L-K (I);

in some embodiments, L is selected from a bond or $-C_{1-50}$ hydrocarbyl-, wherein the hydrocarbyl has 1 to 20 methylene units optionally replaced by -Ak- or -Cy-;

in some embodiments, L is selected from a bond or $-C_{1-20}$ hydrocarbyl-, wherein the hydrocarbyl has 1 to 10 methylene units optionally replaced by -Ak- or -Cy-;

in some embodiments, each -Ak- is independently selected from $-(CH_2)_q-$, $-(CH_2)_q-O-$, $-O-(CH_2)_q-$, $-(CH_2)_q-S-$, $-S-(CH_2)_q-$, $-(CH_2)_q-NR^L-$, $-NR^L-(CH_2)_q-$, $-(CH_2)_q-NR^LC(=O)-$, $-NR^L(CH_2)_qC(=O)-$, $-(CH_2)_q-C(=O)NR^L-$, $-C(=O)-$, $-C(=O)-(CH_2)_q-NR^L-$, $-(C\equiv C)_q-$, $-CH=CH-$, $-Si(R^L)_2-$, $-Si(OH)(R^L)-$, $-Si(OH)_2-$, $-P(=O)(OR^L)-$, $-P(=O)(R^L)-$, $-S-$, $-S(=O)-$, $-S(=O)_2-$ or a bond, wherein the CH and $-CH_2-$ are optionally substituted with 1 to 2 substituents selected from deuterium, halogen, =O, OH, CN, $C_{1-4}$ alkyl or $C_{3-6}$ cycloalkyl;

in some embodiments, each q is independently selected from 0, 1, 2, 3, 4, 5 or 6;

in some embodiments, $R^L$ is selected from H, $C_{1-4}$ alkyl, $C_{3-7}$ carbocyclyl, and 4- to 10-membered heterocyclyl;

in some embodiments, each -Cy- is independently selected from a bond or one of the following groups optionally substituted with 1 to 4 $R^{L2}$: 4- to 8-membered mono-heterocyclyl, 4- to 12-membered fused heterocyclyl, 5- to 13-membered spiroheterocyclyl, 7- to 12-membered bridged heterocyclyl, $C_{3-7}$ monocycloalkyl, $C_{4-7}$ monocycloalkenyl, $C_{4-12}$ fused cycloalkyl, $C_{5-13}$ spirocycloalkyl, $C_{5-12}$ bridged cycloalkyl, 5- to 10-membered heteroaryl or $C_{6-10}$ aryl;
in some embodiments, Ak is selected from Ak1, Ak2, Ak3, Ak4, Ak5, Ak6, Ak7, Ak8 or Ak9;
in some embodiments, Ak is selected from Ak1, Ak2, Ak3, Ak4 or Ak5;
in some embodiments, -Cy- is selected from Cy1, Cy2, Cy3, Cy4 or Cy5;
in some embodiments, -Cy- is selected from Cy1, Cy2, Cy3 or Cy4;
in some embodiments, L is selected from -Cy1-Ak-Cy2-Ak2-Cy3-Ak3-Cy4-Ak4-Cy5-Ak5-, -Cy1-Cy2-Cy3-Cy4-Ak1-Ak2-Ak3-Ak4-Ak5-, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, -Cy1-Ak1-Ak2-Cy2-Cy3-Cy4-Ak3-Ak4-Ak5-, -Cy1-Cy2-Ak1-Ak2-Cy3-Cy4-Ak3-Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, - Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, -Akl-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy1-Cy2-Cy3-Ak2-Ak3-Ak4-Ak5-Cy4-, -Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, -Ak1-Ak2-Cyl-Cy2-Cy3-Ak3-Ak4-Ak5-Cy4-, -Ak1-Ak2-Ak3-Cy1-Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, - Ak1-Ak2-Ak3-Ak4-Cy1-Cy2-Cy3-Ak5-Cy4-, -Ak1-, -Ak1-Ak2-, -Ak1-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Ak1-Ak2-Ak3-Ak4-Ak5-Ak6-Ak7-, -Ak1-Ak2-Ak3-Ak4-Ak5-Ak6-Ak7-Ak8-, and -Ak1-Ak2-Ak3-Ak4-Ak5-Ak6-Ak7-Ak8-Ak9-;
in some embodiments, L is selected from a bond, -Ak1-, -Ak1-Ak2-, -Akl-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Cyl-, -Cy1-Ak1-, -Cy1-Ak1-Ak2-, -Cy1-Ak1-Ak2-Ak3-, -Cy1-Ak1-Ak2-Ak3-Ak4-, -Cy1-Cy2-, -Cy1-Ak1-Cy2-, -Cy1-Cy2-Ak2-, -Cy1-Ak1-Cy2-Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy1-Ak1-Cy2-Ak2-Ak3-Ak4-, -Cy1-Ak1-Ak2-Cy3-, -Cy1-Ak1-Ak2-Cy3-Ak3-, -Cy1-Cy2-Cy3-, -Cy1-Ak1-Cy2-Cy3-, -Cy1-Cy2-Ak2-Cy3-, -Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cy1-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cy1-Ak1-Cy2-Ak2-Cy3-Cy4-, -Akl-Cy2-, -Ak1-Cy2-Cy3-, -Ak1-Ak2-Cy3-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Cy1-Cy2-Cy3-Ak3-Ak4-Ak5-, -Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-Ak2-Ak3-Ak4-, and -Akl-Cy2-Ak2-Ak3-;
in some embodiments, L is -Ak1-Cy1-Ak2-Cy2-Ak3-Cy3-Ak4-Cy4-Ak5-;
in some embodiments, L is selected from -Cyl-, -Cy1-Ak2-, -Cy1-$CH_2$-, -Akl-Cy1-, -Cy1-Cy2-, -Cy1-$CH_2$-Cy2-, -Cy1-Cy2-Cy3-, -Cy1-$CH_2$-Cy2-Cy3-, and - Cy1-Cy2-$CH_2$-Cy3-;
in some embodiments, L is selected from -Cy1-Ak2-Cy2- and -Cy1-O-Cy2-;
in some embodiments, L is selected from -Cy1-, -Cy1-Ak2-, -Cy1-$CH_2$-, -Cy1-$C(CH_3)_2$-, -Ak1-Cy1-, and -C≡C-Cy1-;
in some embodiments, Ak1, Ak2, Ak3, Ak4, Ak5, Ak6, Ak7, Ak8, and Ak9 are each independently selected from $-(CH_2)_q-$, $-(CH_2)_q-O-$, $-O-(CH_2)_q-$, $-(CH_2)_q-S-$, $-S-(CH_2)_q-$, $-(CH_2)_q-NR^L-$, $-NR^L-(CH_2)_q-$, $-(CH_2)_q-NR^LC(=O)-$, $-(CH_2)_q-C(=O)NR^L-$, -C(=O)-, $-C(=O)-(CH_2)_q-NR^L-$, $-(C{\equiv}C)_q-$ or a bond, wherein the $-CH_2-$ is optionally substituted with 1 to 2 substituents selected from deuterium, halogen, =O, OH, CN, $C_{1-4}$ alkyl or $C_{3-6}$ cycloalkyl;
in some embodiments, q is selected from 0, 1, 2 or 3;
in some embodiments, Ak1, Ak2, Ak3, Ak4, Ak5, Ak6, Ak7, Ak8, and Ak9 are each independently selected from a bond, -O-, -S-, $-OCH_2-$, $-CH_2O-$, $-OCH_2CH_2-$, $-CH_2CH_2O-$, -C=C-, $-C(CH_3)_2-$, $-CH(CH_3)-$, $-CH_2-$, $-CH_2CH_2-$, $-CH_2CH_2CH_2-$, - $N(CH_3)$-, -NH-, $-CH_2N(CH_3)-$, $-CH_2NH-$, $-NHCH_2-$, $-CH_2CH_2N(CH_3)-$, - $CH_2CH_2NH$-, $-NHCH_2CH_2-$, -C(=O)-, $-C(=O)CH_2NH-$, $-CH_2C(=O)NH-$, - C(=O)NH- or -NHC(=O)-;
in some embodiments, Ak1, Ak2, Ak3, Ak4, and Ak5 are each independently selected from -C≡C-, $-C(CH_3)_2-$ or $-CH_2-$;
in some embodiments, $R^L$ is selected from H or $C_{1-4}$ alkyl;
in some embodiments, $R^L$ is selected from H, methyl or ethyl;
in some embodiments, each Cy1, Cy2, Cy3, Cy4 or Cy5 is independently selected from a bond or one of the following groups optionally substituted with 1 to 4 $R^{L2}$: 4- to 7-membered nitrogen-containing mono-heterocyclyl, 4- to 12-membered nitrogen-containing fused heterocyclyl, 5- to 13-membered nitrogen-containing spiroheterocyclyl, 7- to 12-membered nitrogen-containing bridged heterocyclyl, $C_{3-7}$ monocycloalkyl, $C_{4-7}$ monocycloalkenyl, $C_{4-12}$ fused cycloalkyl, $C_{5-13}$ spirocycloalkyl, $C_{7-12}$ bridged cycloalkyl, 5- to 10-membered heteroaryl or $C_{6-10}$ aryl;

in some embodiments, Cy5 is defined the same as Cy1;

in some embodiments, Cy1, Cy2, Cy3, and Cy4 are each independently selected from a bond or one of the following groups optionally substituted with 1 to 4 $R^{L2}$: phenyl, pyridyl, pyrimidyl, pyrazinyl, pyridazinyl, pyrazolyl, thiazolyl, oxazolyl, triazolyl,

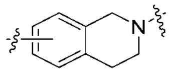, 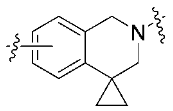, 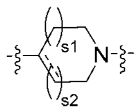, 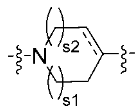, 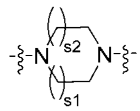,

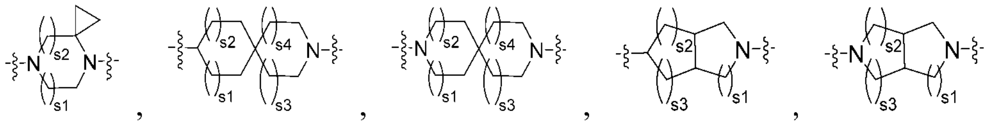

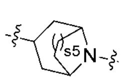, 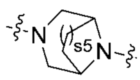, 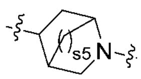, 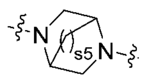, 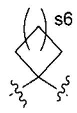, 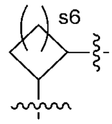, 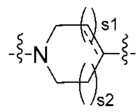,

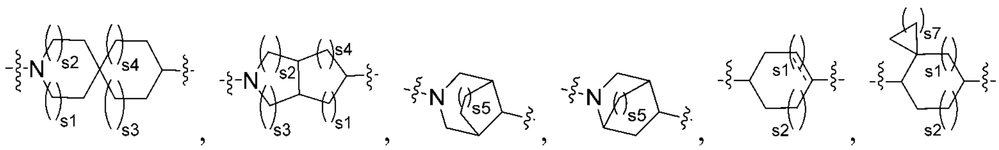

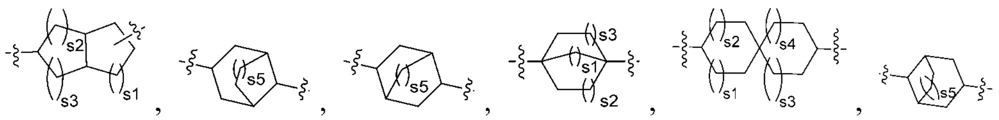

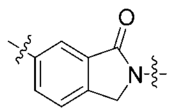, 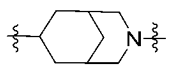, 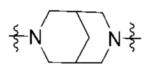, 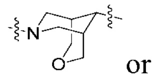 or 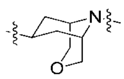,

wherein s1, s3, and s5 are each independently selected from 0, 1 or 2; s2 and s4 are each independently selected from 0 or 1; s6 is selected from 0, 1, 2 or 3; and s7 is selected from 1, 2 or 3;

in some embodiments, Cy1, Cy2, Cy3, and Cy4 are each independently selected from a bond or one of the following optionally substituted groups:

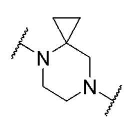,

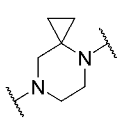, 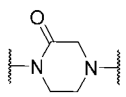, 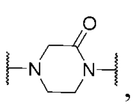, 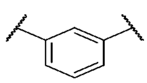, 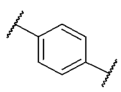, 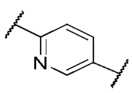, 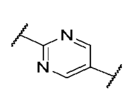,

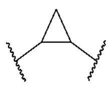, 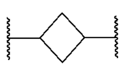, 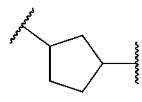, 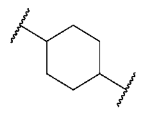, 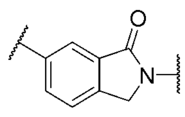, 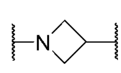,

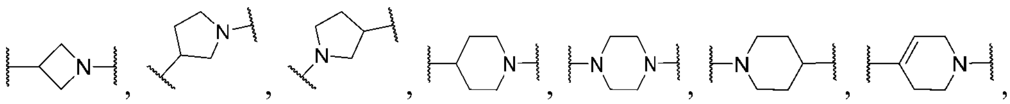

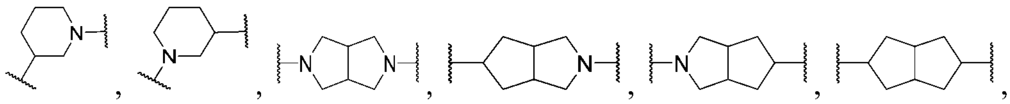

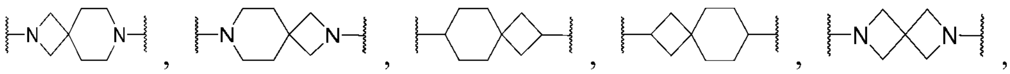

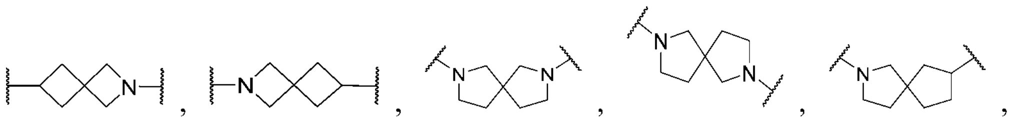

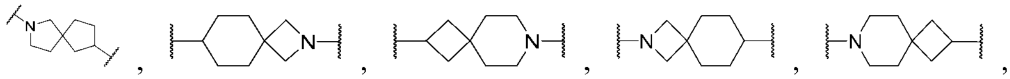

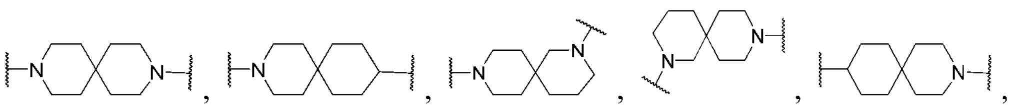

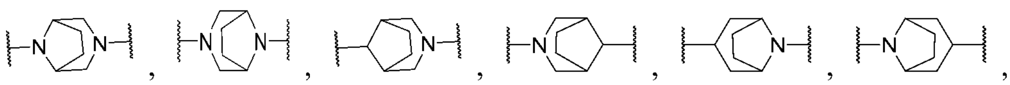

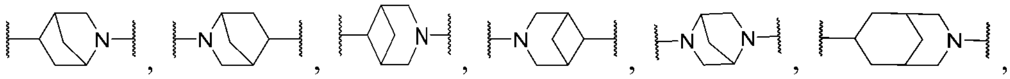

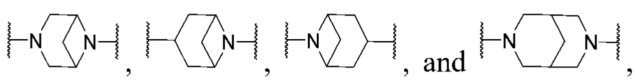

which, when substituted, are substituted with 1 to 4 substituents selected from deuterium, F, $CF_3$, OH, =O, COOH, CN, $NH_2$, hydroxymethyl, methyl, methoxy, and cyclopropyl;

in some embodiments, Cy1, Cy2, and Cy3 are each independently selected from one of the following optionally substituted groups:

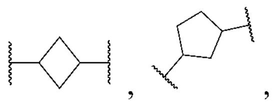

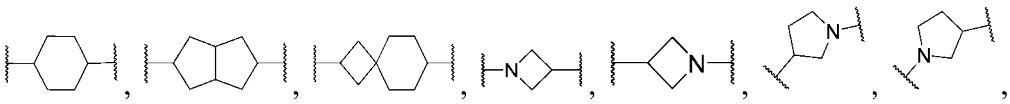

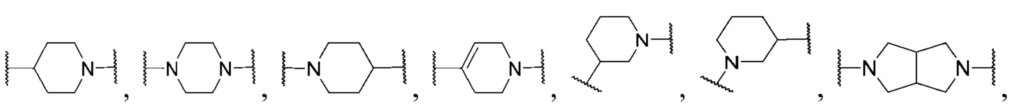

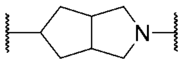, 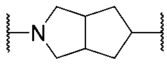, 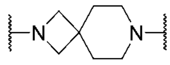, 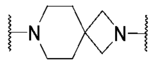, 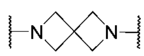,

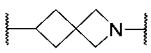, 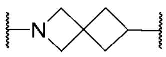, 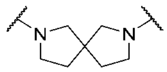, 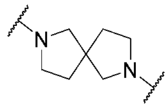, 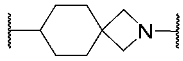,

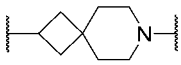, 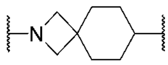, 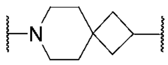, 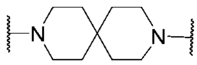, 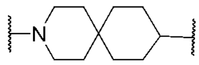,

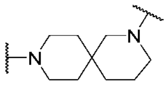, 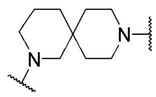, 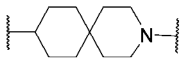, 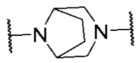, 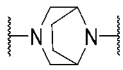, 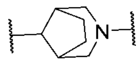,

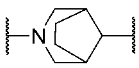, 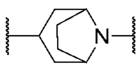, 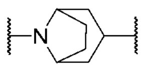, 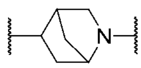, 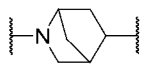, 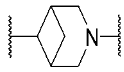,

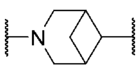, 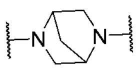, 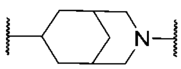, 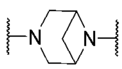, 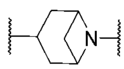, 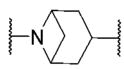,

and

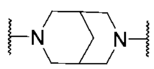,

which, when substituted, are substituted with 1 to 4 substituents selected from deuterium, F, $CF_3$, OH, =O, COOH, CN, $NH_2$, hydroxymethyl, methyl, methoxy, and cyclopropyl;
in some embodiments, L is selected from one of the structural fragments shown in Table L-1:

Table L-1

| | | | |
|---|---|---|---|
| 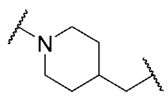 | 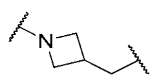 | 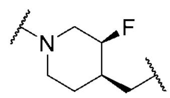 | 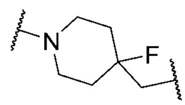 |

| | | | |
|---|---|---|---|
| 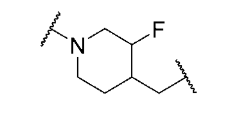 | 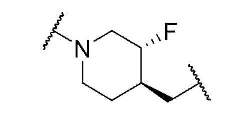 | 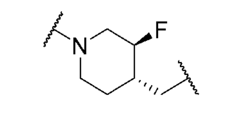 | 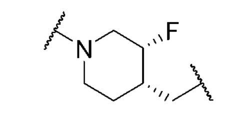 |
| 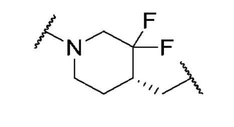 | 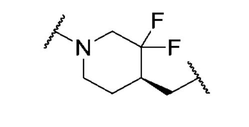 | 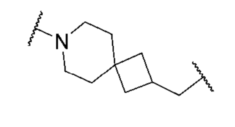 | 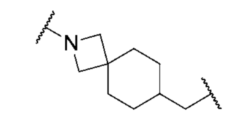 |
| 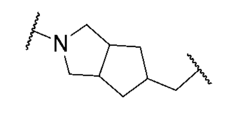 | 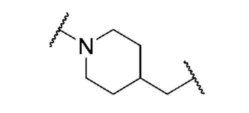 | 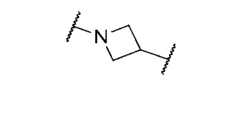 | 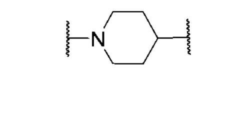 |
| 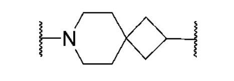 | 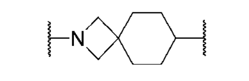 | 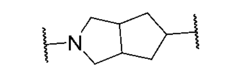 | 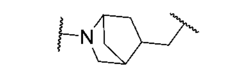 |
| 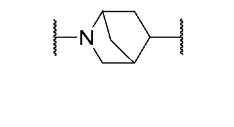 | 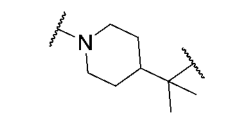 | 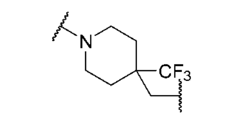 | 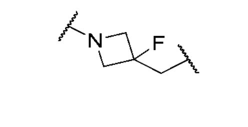 |
| 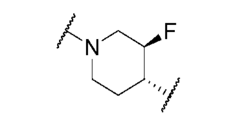 | 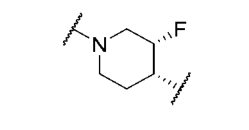 | 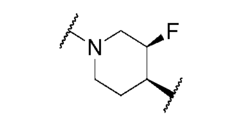 | 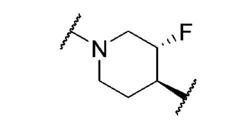 |
| 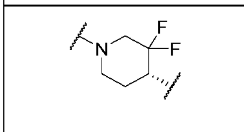 | 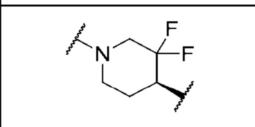 | 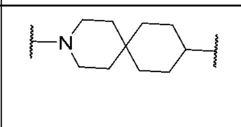 | 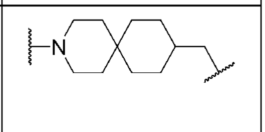 |
| 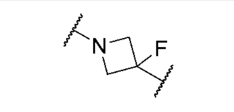 | 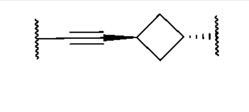 | 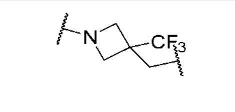 | 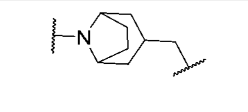 |
| 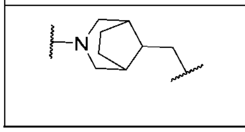 | 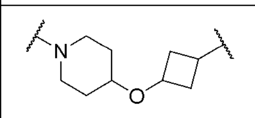 | 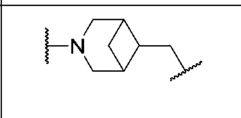 | |

;

in some embodiments, B is

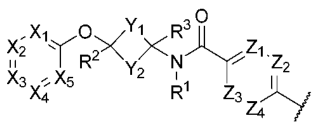 ;

in some embodiments, B is selected from

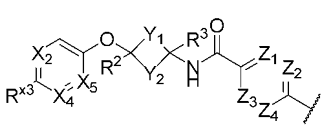 ,

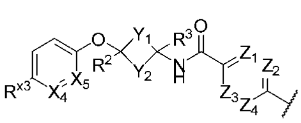 , 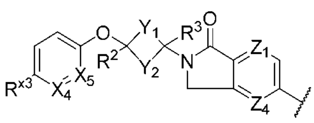 , and 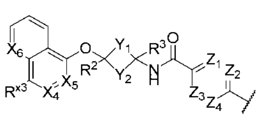 ;

in some embodiments, B is

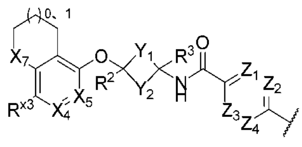;

in some embodiments, B is selected from

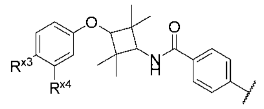,

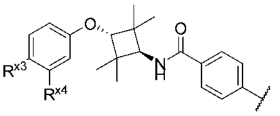, 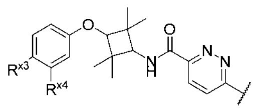, 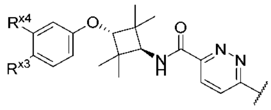,

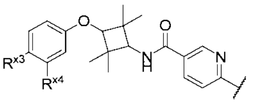, 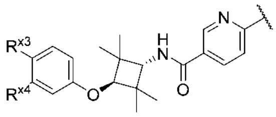, 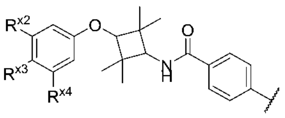,

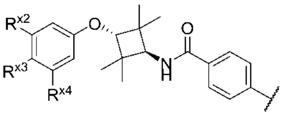, 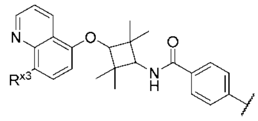, 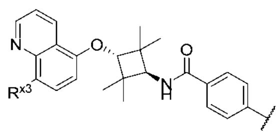,

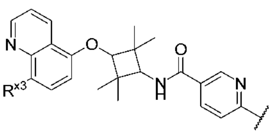, 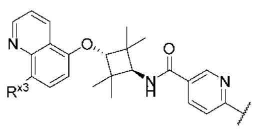, 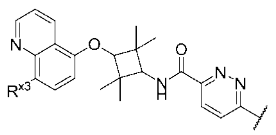,

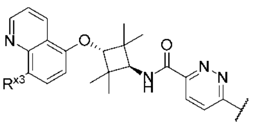, 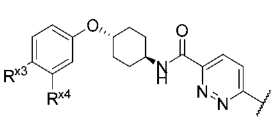, 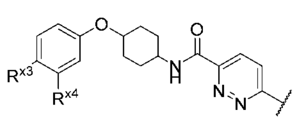,

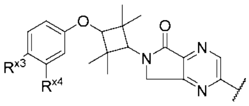, 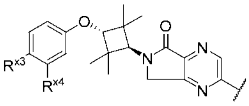, 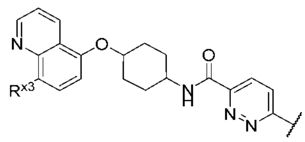,

and

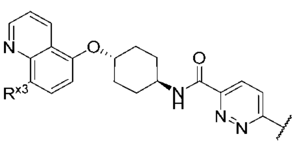;

in some embodiments, B is selected from

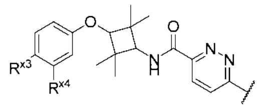,

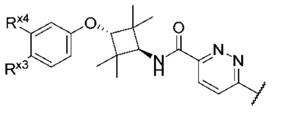, 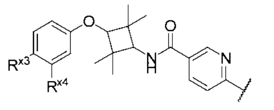, 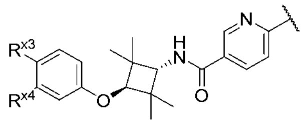,

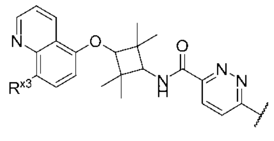, and 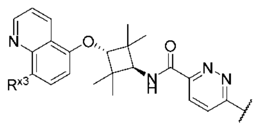;

in some embodiments, B is selected from

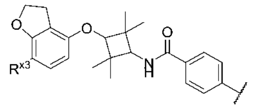, 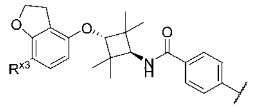, 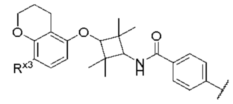,

and

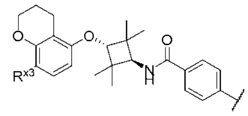;

in some embodiments, $Y_1$ and $Y_2$ are each independently selected from $-CR^{y1}R^{y2}-$, $-(CR^{y1}R^{y2})_2-$, and $-(CR^{y1}R^{y2})_3-$;
in some embodiments, $Y_1$ and $Y_2$ are each independently selected from - $CR^{y1}R^{y2}-$ and $-(CR^{y1}R^{y2})_2-$;
in some embodiments, $Y_1$ and $Y_2$ are each independently selected from $-CH_2-$, $-CH_2CH_2-$, and $-C(CH_3)_2-$;
in some embodiments, $X_1$ is selected from N or $CR^{x1}$; $X_2$ is selected from N or $CR^{x2}$; $X_3$ is selected from N or $CR^{x3}$; $X_4$ is selected from N or $CR^{x4}$; $X_5$ is selected from N or $CR^{x5}$; at most two of $X_1$, $X_2$, $X_3$, $X_4$, and $X_5$ are N; in some embodiments, $X_3$ is $CR^{x3}$; in some embodiments, one of $X_1$, $X_2$, $X_3$, $X_4$, and $X_5$ is N; in some embodiments, $X_1$ is $CR^{x1}$; $X_2$ is $CR^{x2}$; $X_3$ is $CR^{x3}$; $X_4$ is $CR^{x4}$; and $X_5$ is $CR^{x5}$;
in some embodiments, $X_6$ is selected from N or CH; in some embodiments, $X_7$ is selected from NH or O; in some embodiments, $X_7$ is O;
in some embodiments, $Z_1$ is selected from N or $CR^{z1}$; $Z_2$ is selected from N or $CR^{z2}$; $Z_3$ is selected from N or $CR^{z3}$; $Z_4$ is selected from N or $CR^{z4}$; at most three of $Z_1$, $Z_2$, $Z_3$, and $Z_4$ are N; in some embodiments, at most two of $Z_1$, $Z_2$, $Z_3$, and $Z_4$ are N; in some embodiments, one of $Z_1$, $Z_2$, $Z_3$, and $Z_4$ is N; in some embodiments, two of $Z_1$, $Z_2$, $Z_3$, and $Z_4$ are N; in some embodiments, $Z_1$ is $CR^{z1}$; $Z_2$ is $CR^{z2}$; $Z_3$ is $CR^{z3}$; and $Z_4$ is $CR^{z4}$;
in some embodiments, $R^{x1}$, $R^{x2}$, $R^{x3}$, $R^{x4}$, $R^{x5}$, $R^{z1}$, $R^{z2}$, $R^{z3}$, and $R^{z4}$ are each independently selected from H, deuterium, halogen, OH, $NH_2$, CN, $NO_2$, COOH, $CONH_2$, $NHC_{1-4}$ alkyl, $N(C_{1-4}$ alkyl$)_2$, $C_{1-4}$ alkyl, $C_{2-4}$ alkenyl, $C_{2-4}$ alkynyl, $-OC_{1-4}$ alkyl, $-SC_{1-4}$ alkyl, $-C_{0-4}$ alkylene-$C_{3-6}$ carbocyclyl, $-C_{0-4}$ alkylene-4- to 6-membered heterocyclyl, 5- to 6-membered heteroaryl, $-O-C_{3-6}$ carbocyclyl, and -O-4- to 6-membered heterocyclyl, wherein the alkylene, alkyl, alkenyl, alkynyl, carbocyclyl, heterocyclyl, and heteroaryl are optionally substituted with 1 to 4 $R^s$;
in some embodiments, $R^{x1}$, $R^{x2}$, $R^{x3}$, $R^{x4}$, $R^{x5}$, $R^{z1}$, $R^{z2}$, $R^{z3}$, and $R^{z4}$ are each independently selected from H, deuterium, halogen, OH, $NH_2$, CN, $NO_2$, COOH, $CONH_2$, $NHC_{1-4}$ alkyl, $N(C_{1-4}$ alkyl$)_2$, $C_{1-4}$ alkyl, $C_{2-4}$ alkenyl, $C_{2-4}$ alkynyl, $-OC_{1-4}$ alkyl, $-SC_{1-4}$ alkyl, $-C_{0-2}$ alkylene-$C_{3-6}$ cycloalkyl, $-C_{0-2}$ alkylene-4- to 6-membered heterocyclyl, 5- to 6-membered heteroaryl, $-O-C_{3-6}$ cycloalkyl, and -O-4- to 6-membered heterocyclyl, wherein the alkylene, alkyl, alkenyl, alkynyl, cycloalkyl, heterocyclyl, and heteroaryl are optionally substituted with 1 to 4 $R^s$;
in some embodiments, $R^{x1}$, $R^{x2}$, $R^{x3}$, $R^{x4}$, $R^{x5}$, $R^{z1}$, $R^{z2}$, $R^{z3}$, and $R^{z4}$ are each independently selected from H, deuterium, F, Cl, Br, I, OH, $NH_2$, CN, $NO_2$, COOH, $CONH_2$, $N(CH_3)_2$, $NHCH_3$, or one of the following groups optionally substituted with 1 to 4 $R^s$: methyl, ethyl, vinyl, ethynyl, methoxy, ethoxy, methylthio, cyclopropyl, cyclobutyl, cyclopentyl, $-CH_2$-cyclopropyl, $-CH_2$-cyclobutyl, -O-cyclopropyl, and pyrazolyl;
in some embodiments, $R^{x1}$, $R^{x2}$, $R^{x3}$, and $R^{x4}$ are each independently selected from $-S(=O)_2NH_2$ and $-S(=O)_2C_{1-4}$

alkyl;
in some embodiments, $R^{x1}$, $R^{x2}$, $R^{x3}$, and $R^{x4}$ are each independently selected from $-S(=O)_2NH_2$, $-S(=O)_2$ methyl, and $-S(=O)_2$ ethyl;
in some embodiments, $R^{x2}$, $R^{x3}$, and $R^{x4}$ are each independently selected from H, deuterium, F, Cl, Br, I, OH, $NH_2$, CN, $NO_2$, COOH, $CONH_2$, $N(CH_3)_2$, $NHCH_3$, $CF_3$, $CHF_2$, $CH_2F$, $OCF_3$, $OCH_2F$, $OCD_3$, $CH_2OH$, methyl, ethyl, vinyl, ethynyl, methoxy, ethoxy, methylthio, cyclopropyl, $-CH_2$-cyclopropyl, and -O-cyclopropyl;
in some embodiments, $R^{x3}$ and $R^{x4}$ are each independently selected from H, deuterium, F, Cl, Br, I, OH, $NH_2$, CN, $NO_2$, COOH, $CONH_2$, $N(CH_3)_2$, $NHCH_3$, $CF_3$, $CHF_2$, $CH_2F$, $OCF_3$, $OCH_2F$, $CH_2OH$, methyl, ethyl, vinyl, ethynyl, methoxy, ethoxy, methylthio, cyclopropyl, $-CH_2$-cyclopropyl, and -O-cyclopropyl;
in some embodiments, $R^{x2}$ and $R^{x4}$ are each independently selected from - $S(=O)_2CH_3$, $-O-CH_2$-propynyl, $-O-CH_2$-cyclopropyl, $-O-CH_2CH_2-OCH_3$, $-O-CH_2CH_2$-O-cyclopropyl,

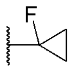, 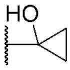, 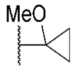, and 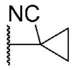;

in some embodiments, $R^2$, $R^3$, $R^{y1}$, and $R^{y2}$ are each independently selected from H, deuterium, halogen, OH, $NH_2$, CN, $NO_2$, COOH, $CONH_2$, $C_{1-4}$ alkyl, $C_{2-4}$ alkenyl, $C_{2-4}$ alkynyl, $-OC_{1-4}$ alkyl, and $-SC_{1-4}$ alkyl, wherein the alkyl, alkenyl, and alkynyl are optionally substituted with 1 to 4 substituents selected from deuterium, halogen, OH, $NH_2$, CN, $C_{1-4}$ alkyl, and $C_{1-4}$ alkoxy;
in some embodiments, $R^2$, $R^3$, $R^{y1}$, and $R^{y2}$ are each independently selected from H, deuterium, F, Cl, Br, I, OH, $NH_2$, CN, $NO_2$, COOH, $CONH_2$, methyl, ethyl, vinyl, ethynyl, methoxy, ethoxy, and methylthio, wherein the methyl, ethyl, vinyl, ethynyl, methoxy, ethoxy, and methylthio are substituted with 1 to 4 substituents selected from deuterium, F, Cl, Br, I, OH, $NH_2$, CN, $C_{1-4}$ alkyl, and $C_{1-4}$ alkoxy;
in some embodiments, $R^2$ and $R^3$ are each independently selected from H, deuterium, F, Cl, and Br;
in some embodiments, $R^{y1}$ and $R^{y2}$ are each independently selected from H, deuterium, F, Cl, Br, I, OH, $NH_2$, CN, $NO_2$, COOH, $CONH_2$, methyl, ethyl, vinyl, ethynyl, methoxy, ethoxy, and methylthio, wherein the methyl, ethyl, vinyl, ethynyl, methoxy, ethoxy, and methylthio are substituted with 1 to 4 substituents selected from deuterium, F, Cl, Br, I, OH, $NH_2$, CN, methyl, ethyl, and methoxy;
in some embodiments, $R^1$ is selected from H, deuterium, $C_{1-4}$ alkyl or $C_{3-6}$ cycloalkyl, wherein the alkyl or cycloalkyl is optionally substituted with 1 to 4 substituents selected from deuterium, halogen, OH, $NH_2$, CN, $C_{1-4}$ alkyl, and $C_{1-4}$ alkoxy;
in some embodiments, $R^1$ is selected from H, deuterium, methyl, ethyl, isopropyl, cyclopropyl, and cyclobutyl, wherein the methyl, ethyl, isopropyl, cyclopropyl, and cyclobutyl are optionally substituted with 1 to 4 substituents selected from deuterium, F, Cl, Br, I, OH, $NH_2$, CN, $C_{1-4}$ alkyl, and $C_{1-4}$ alkoxy;
in some embodiments, $R^1$ is selected from H, deuterium, $CF_3$, $CHF_2$, $CH_2F$, $CH_2OH$, methyl, ethyl, isopropyl, and cyclopropyl;
in some embodiments, $R^{x1}$ and $R^{x2}$, $R^{x2}$ and $R^{x3}$, $R^{x3}$ and $R^{x4}$, $R^{x4}$ and $R^{x5}$, $R^1$ and $R^{z3}$, and $R^1$ and $R^{z1}$ are directly connected to form $C_{4-6}$ carbocyclyl or 4- to 7-membered heterocyclyl, wherein the carbocyclyl or heterocyclyl is optionally substituted with 1 to 4 $R^s$;
in some embodiments, $R^{x1}$ and $R^{x2}$, $R^{x2}$ and $R^{x3}$, $R^{x3}$ and $R^{x4}$, and $R^{x4}$ and $R^{x5}$ are directly connected to form $C_{4-6}$ carbocyclyl, 5- to 6-membered heteroaryl or 5-to 7-membered heterocyclyl, wherein the carbocyclyl, heteroaryl or heterocyclyl is optionally substituted with 1 to 4 $R^s$;
in some embodiments, $R^1$ and $R^{z3}$, and $R^1$ and $R^{z1}$ are directly connected to form 5- to 7-membered heterocyclyl, wherein the heterocyclyl is optionally substituted with 1 to 4 $R^s$;
in some embodiments, $R^{x1}$ and $R^{x2}$, $R^{x2}$ and $R^{x3}$, $R^{x3}$ and $R^{x4}$, and $R^{x4}$ and $R^{x5}$ are directly connected to form one of the following groups optionally substituted with 1 to 3 $R^s$: piperidyl, cyclohexyl, phenyl, pyridyl, pyrimidyl, pyridazinyl, and pyrazinyl; in some embodiments, $R^{x1}$ and $R^{x2}$, $R^{x2}$ and $R^{x3}$, $R^{x3}$ and $R^{x4}$, and $R^{x4}$ and $R^{x5}$ are directly connected to form one of the following groups optionally substituted with 1 to 3 $R^s$: pyrrolidyl, oxolanyl, oxanyl, and 1,3-dioxolane;
in some embodiments, $R^1$ and $R^{z3}$, and $R^1$ and $R^{z1}$ are directly connected to form the following structure optionally substituted with 1 to 4 $R^s$:

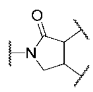,

or

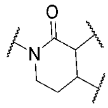;

in some embodiments, K is

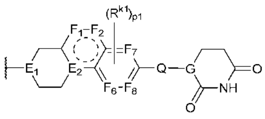; 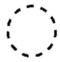

represents that the ring is an aromatic ring or a non-aromatic ring;
in some embodiments, K is selected from

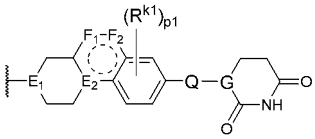,

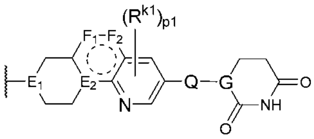, and 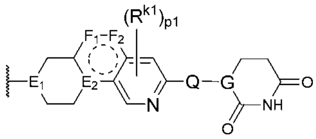;

in some embodiments, K is selected from

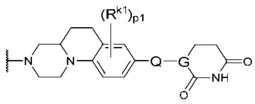,

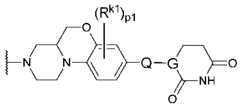, 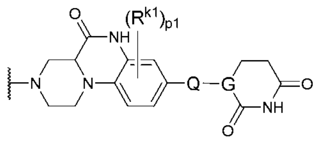, 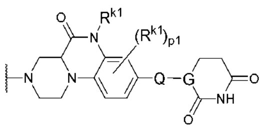,

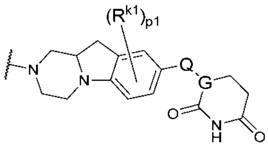, 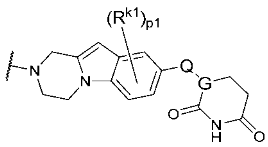, and 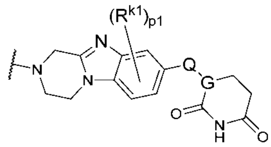;

in some embodiments, K is selected from

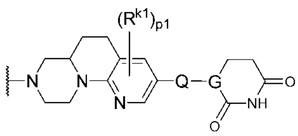

and

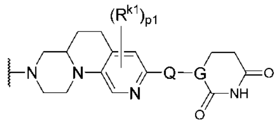;

in some embodiments, K is selected from

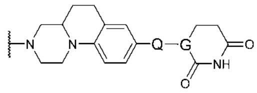,

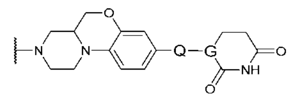, 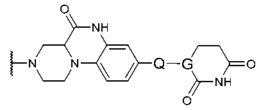, 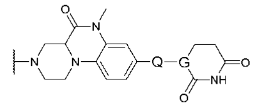,

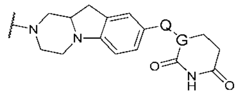, 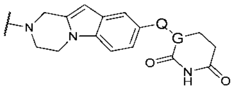, 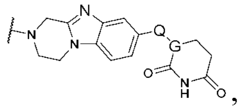, 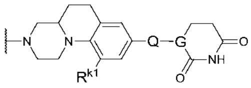,

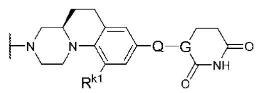, 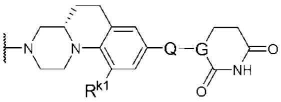, 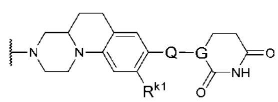,

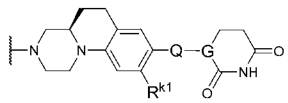, 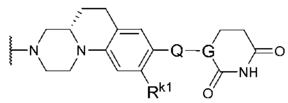, 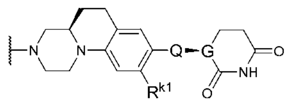,

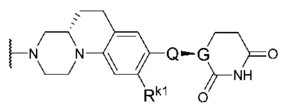, 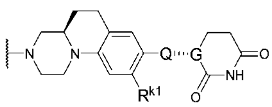, 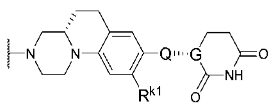,

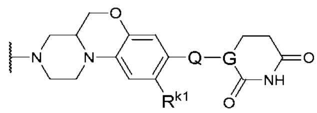, 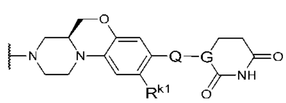, 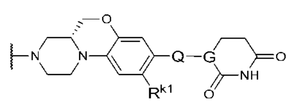,

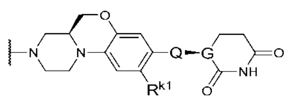, 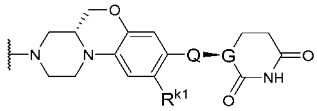, 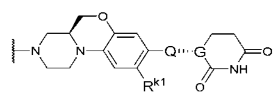,

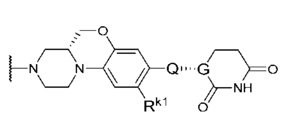, 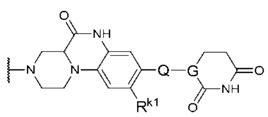, 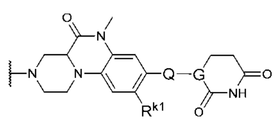,

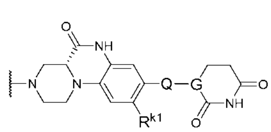, 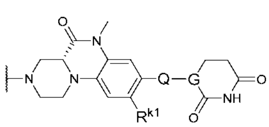, 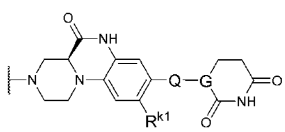,

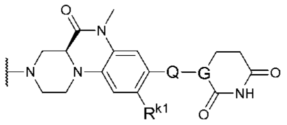, 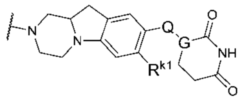, 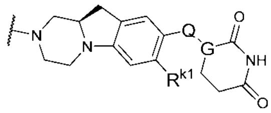,

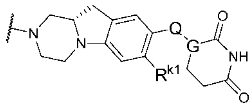, 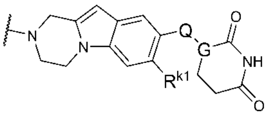, and 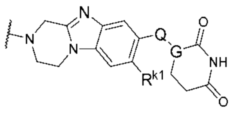;

in some embodiments, K is selected from

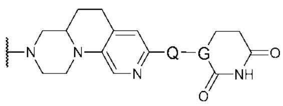,

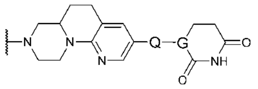, 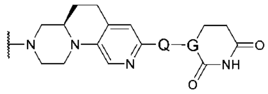, 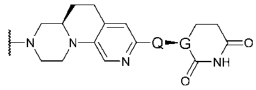,

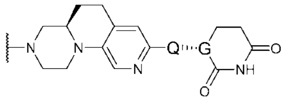, 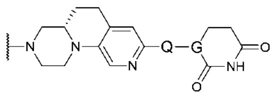, 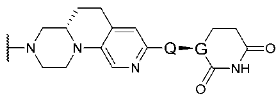,

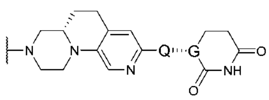, 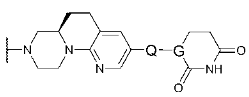, 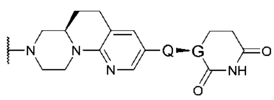,

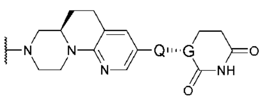, 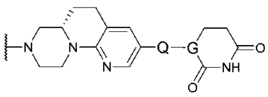, 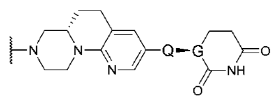,

and

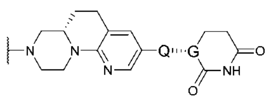;

in some embodiments, $F_1$ is selected from N, NH, CH, $CH_2$, $CHR^{k1}$, $NR^{k1}$, $CR^{k1}$, C(=O), and $C(R^{k1})_2$;
in some embodiments, $F_2$ is selected from a bond, O, N, NH, CH, $CH_2$, $CHR^{k1}$, $NR^{k1}$, $CR^{k1}$ or $C(R^{k1})_2$;
in some embodiments, when

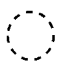

represents that the ring is a non-aromatic ring, $F_1$ is selected from NH, $CH_2$, $CHR^{k1}$, $NR^{k1}$, C(=O), and $C(R^{k1})_2$, and $F_2$ is selected from a bond, O, NH, $CH_2$, $CHR^{k1}$, $NR^{k1}$ or $C(R^{k1})_2$;
in some embodiments, when

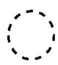

represents that the ring is an aromatic ring, $F_1$ is selected from N, CH, and $CR^{k1}$, and $F_2$ is selected from a bond, O, N, CH, and $CR^{k1}$;

in some embodiments, $F_6$, $F_7$, and Fs are each independently selected from N, C, CH or $CR^{k1}$, and at most two of $F_6$, $F_7$, and $F_8$ are N; in some embodiments, one of $F_6$, $F_7$, and $F_8$ is N, and the remaining two are selected from CH or $CR^{k1}$;

in some embodiments, G is selected from CH or N;

in some embodiments, $E_1$ is selected from N or CH, preferably N;

in some embodiments, $E_2$ is selected from C, N or CH, preferably N;

in some embodiments, each Q is independently selected from a bond, -O-, -S-, $-CH_2-$, $-NR^q-$, -CO-, $-NR^qCO-$, and $-CONR^q-$;

in some embodiments, each Q is independently selected from a bond, $CH_2$, NH, $N(CH_3)$, O, S, C(=O), NHC(=O), C(=O)NH, $N(CH_3)C(=O)$, and $C(=O)N(CH_3)$;

in some embodiments, each Q is independently selected from a bond, NH, $N(CH_3)$, O, S, NHC(=O), C(=O)NH, $N(CH_3)C(=O)$, and $C(=O)N(CH_3)$;

in some embodiments, each Q is independently selected from a bond, NH, and C(=O)NH;

in some embodiments, Q and G cannot be directly bonded to form an N-N or N-O bond;

in some embodiments,

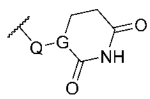

is selected from

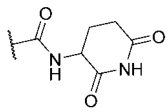

,

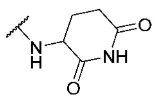

,

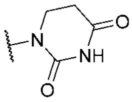

, and

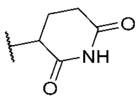

;

in some embodiments, each $R^q$ is independently selected from H or $C_{1-4}$ alkyl;

in some embodiments, each $R^q$ is independently selected from H, methyl, ethyl or isopropyl;

in some embodiments, $R^q$ is selected from H or methyl;

in some embodiments, each $R^{k1}$ is independently selected from deuterium, halogen, OH, $NH_2$, CN, COOH, $CONH_2$, $C_{1-4}$ alkyl, $C_{2-4}$ alkenyl, $C_{2-4}$ alkynyl, $C_{1-4}$ alkoxy, $C_{3-6}$ cycloalkyl, and 4- to 6-membered heterocycloalkyl, wherein the alkyl, alkenyl, alkynyl, alkoxy, cycloalkyl, and heterocycloalkyl are optionally substituted with 1 to 4 $R^s$;

in some embodiments, each $R^{k1}$ is independently selected from deuterium, halogen, OH, $NH_2$, CN, COOH, $CONH_2$, $C_{1-4}$ alkyl, $C_{1-4}$ alkoxy, $C_{3-6}$ cycloalkyl, and 4- to 6-membered heterocycloalkyl, wherein the alkyl, alkenyl, alkynyl, alkoxy, cycloalkyl, and heterocycloalkyl are optionally substituted with 1 to 4 substituents selected from deuterium, halogen, OH, $CF_3$, CN, $NH_2$, and $C_{1-4}$ alkyl;

in some embodiments, each $R^{k1}$ is independently selected from deuterium, F, Cl, Br, I, OH, $NH_2$, CN, COOH, $CONH_2$, methyl, ethyl, isopropyl, methoxy, ethoxy, isopropoxy, and cyclopropyl, wherein the methyl, ethyl, isopropyl, methoxy, ethoxy, isopropoxy, and cyclopropyl are optionally substituted with 1 to 4 substituents selected from deuterium, F, Cl, Br, I, OH, and $NH_2$;

in some embodiments, each $R^{k1}$ is independently selected from deuterium, F, Cl, Br, I, OH, $NH_2$, CN, COOH, $CONH_2$, $CF_3$, $CHF_2$, $CH_2F$, $OCF_3$, $OCH_2F$, $CH_2OH$, methyl, ethyl, isopropyl, methoxy, ethoxy, isopropoxy, and cyclopropyl;

in some embodiments, $R^{L2}$ and $R^s$ are each independently selected from deuterium, halogen, OH, CN, =O, $CF_3$, $SF_5$, $NO_2$, $NH_2$, $NHC_{1-4}$ alkyl, $N(C_{1-4}\ alkyl)_2$, COOH, $CONH_2$, $C_{1-4}$ alkyl, $C_{2-4}$ alkenyl, $C_{2-4}$ alkynyl, $C_{1-4}$ alkoxy, $-S-C_{1-4}$ alkyl, $-C_{0-4}$ alkylene-$C_{3-6}$ cycloalkyl, and $-C_{0-4}$ alkylene-4- to 6-membered heterocyclyl, wherein the alkyl, alkylene, alkoxy, alkenyl, alkynyl, and cycloalkyl are optionally substituted with 1 to 4 substituents selected from deuterium, F, Cl, Br, I, OH, CN, $C_{1-4}$ alkyl, and $C_{1-4}$ alkoxy;

in some embodiments, $R^{L2}$ and $R^s$ are each independently selected from deuterium, halogen, OH, CN, =O, $CF_3$, $SF_5$, $NO_2$, $NH_2$, $NHC_{1-4}$ alkyl, $N(C_{1-4}\ alkyl)_2$, COOH, $CONH_2$, $C_{1-4}$ alkyl, $C_{2-4}$ alkenyl, $C_{2-4}$ alkynyl, $C_{1-4}$ alkoxy, $-S-C_{1-4}$ alkyl, $-C_{0-2}$ alkylene-$C_{3-6}$ cycloalkyl, and $-C_{0-2}$ alkylene-4- to 6-membered heterocyclyl, wherein the alkyl, alkylene, alkoxy,

alkenyl, alkynyl, and cycloalkyl are optionally substituted with 1 to 4 substituents selected from deuterium, F, Cl, Br, I, OH, CN, $C_{1-4}$ alkyl, and $C_{1-4}$ alkoxy;

in some embodiments, $R^{L2}$ and $R^s$ are each independently selected from deuterium, F, Cl, Br, I, OH, =O, $CF_3$, $SF_5$, CN, $NH_2$, $NO_2$, COOH, $CONH_2$, $N(CH_3)_2$, $NHCH_3$, methyl, ethyl, vinyl, ethynyl, methoxy, ethoxy, methylthio, cyclopropyl, cyclobutyl, cyclopentyl, cyclohexyl, $-CH_2$-cyclopropyl, $-CH_2$-cyclobutyl, $-CH_2$-cyclopentyl, and $-CH_2$-cyclohexyl, wherein the methyl, ethyl, vinyl, ethynyl, methoxy, ethoxy, methylthio, cyclopropyl, cyclobutyl, cyclopentyl, and cyclohexyl are optionally substituted with 1 to 4 substituents selected from deuterium, F, Cl, Br, I, OH, CN, $C_{1-4}$ alkyl, and $C_{1-4}$ alkoxy;

in some embodiments, $R^{L2}$ and $R^s$ are each independently selected from deuterium, F, Cl, Br, I, OH, =O, $CF_3$, $SF_5$, CN, $NH_2$, $NO_2$, COOH, $CONH_2$, $N(CH_3)_2$, $NHCH_3$, $CHF_2$, $CH_2F$, $OCF_3$, $OCH_2F$, $OCHF_2$, $CH_2OH$, $OCH_2OH$, methyl, ethyl, vinyl, ethynyl, methoxy, ethoxy, methylthio, cyclopropyl, cyclobutyl, cyclopentyl, cyclohexyl, $-CH_2$-cyclopropyl, $-CH_2$-cyclobutyl, $-CH_2$-cyclopentyl, and $-CH_2$-cyclohexyl;

in some embodiments, each $R^s$ is independently $-O-C_{3-6}$ cycloalkyl;

in some embodiments, each $R^s$ is independently selected from propynyl and - O-cyclopropyl;

in some embodiments, each p1 is independently selected from 0, 1 or 2;

in some embodiments, each p1 is independently selected from 0 and 1.

A first embodiment of the present invention provides the above-mentioned compound represented by general formula (I) or the stereoisomer, tautomer, deuterated substance, solvate, prodrug, metabolite, pharmaceutically acceptable salt or co-crystal thereof, wherein

L is -Ak1-Cy1-Ak2-Cy2-Ak3-Cy3-Ak4-Cy4-Ak5-;

Ak1, Ak2, Ak3, Ak4, and Ak5 are each independently selected from $-(CH_2)_q-$, $-(CH_2)_q-O-$, $-O-(CH_2)_q-$, $-(CH_2)_q-S-$, $-S-(CH_2)_q-$, $-(CH_2)_q-NR^L-$, $-NR^L-(CH_2)_q-$, - $(CH_2)_q-NR^LC(=O)-$, $-(CH_2)_q-C(=O)NR^L-$, $-C(=O)-$, $-C(=O)-(CH_2)_q-NR^L-$, $-(C\equiv C)_q-$ or a bond, wherein the $-CH_2-$ is optionally substituted with 1 to 2 substituents selected from deuterium, halogen, =O, OH, CN, $C_{1-4}$ alkyl or $C_{3-6}$ cycloalkyl;

q is selected from 0, 1, 2 or 3;

each $R^L$ is independently selected from H or $C_{1-4}$ alkyl;

each Cy1, Cy2, Cy3 or Cy4 is independently selected from a bond or one of the following groups optionally substituted with 1 to 4 $R^{L2}$: 4- to 7-membered mono-heterocyclyl, 4- to 12-membered fused heterocyclyl, 5- to 13-membered spiroheterocyclyl, 7- to 12-membered bridged heterocyclyl, $C_{3-7}$ monocycloalkyl, $C_{4-7}$ monocycloalkenyl, $C_{4-12}$ fused cycloalkyl, $C_{5-13}$ spirocycloalkyl, $C_{5-12}$ bridged cycloalkyl, 5- to 10-membered heteroaryl or $C_{6-10}$ aryl;

B is

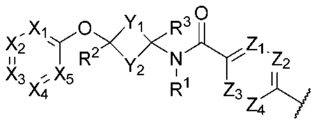;

$X_1$ is selected from N or $CR^{x1}$; $X_2$ is selected from N or $CR^{x2}$; $X_3$ is selected from N or $CR^{x3}$; $X_4$ is selected from N or $CR^{x4}$; $X_5$ is selected from N or $CR^{x5}$;

at most two of $X_1$, $X_2$, $X_3$, $X_4$, and $X_5$ are N;

$Z_1$ is selected from N or $CR^{z1}$; $Z_2$ is selected from N or $CR^{z2}$; $Z_3$ is selected from N or $CR^{z3}$; $Z_4$ is selected from N or $CR^{z4}$;

at most three of $Z_1$, $Z_2$, $Z_3$, and $Z_4$ are N;

$Y_1$ and $Y_2$ are each independently selected from $-CR^{y1}R^{y2}-$, $-(CR^{y1}R^{y2})_2-$, and - $(CR^{y1}R^{y2})_3-$;

$R^1$ is selected from H, deuterium, $C_{1-4}$ alkyl or $C_{3-6}$ cycloalkyl, wherein the alkyl or cycloalkyl is optionally substituted with 1 to 4 substituents selected from deuterium, halogen, OH, $NH_2$, CN, $C_{1-4}$ alkyl, and $C_{1-4}$ alkoxy;

$R^{x1}$, $R^{x2}$, $R^{x3}$, $R^{x4}$, $R^{x5}$, $R^{z1}$, $R^{z2}$, $R^{z3}$, and $R^{z4}$ are each independently selected from H, deuterium, halogen, OH, $NH_2$, CN, $NO_2$, COOH, $CONH_2$, $NHC_{1-4}$ alkyl, $N(C_{1-4}$ alkyl$)_2$, $C_{1-4}$ alkyl, $C_{2-4}$ alkenyl, $C_{2-4}$ alkynyl, $-OC_{1-4}$ alkyl, $-SC_{1-4}$ alkyl, $-C_{0-4}$ alkylene-$C_{3-6}$ carbocyclyl, $-C_{0-4}$ alkylene-4- to 6-membered heterocyclyl, 5- to 6-membered heteroaryl, $-O-C_{3-6}$ carbocyclyl, and -O-4- to 6-membered heterocyclyl, wherein the alkylene, alkyl, alkenyl, alkynyl, carbocyclyl, heterocyclyl, and heteroaryl are optionally substituted with 1 to 4 $R^s$;

or $R^{x1}$, $R^{x2}$, $R^{x3}$, and $R^{x4}$ are each independently selected from $-S(=O)_2NH_2$ and $-S(=O)_2C_{1-4}$ alkyl;

or $R^{x1}$ and $R^{x2}$, $R^{x2}$ and $R^{x3}$, $R^{x3}$ and $R^{x4}$, $R^{x4}$ and $R^{x5}$, $R^1$ and $R^{z3}$, and $R^1$ and $R^{z1}$ are directly connected to form $C_{4-6}$ carbocyclyl or 4- to 7-membered heterocyclyl, wherein the carbocyclyl or heterocyclyl is optionally substituted with 1 to 4 $R^s$;

$R^2$, $R^3$, $R^{y1}$, and $R^{y2}$ are each independently selected from H, deuterium, halogen, OH, $NH_2$, CN, $NO_2$, COOH,

$CONH_2$, $C_{1-4}$ alkyl, $C_{2-4}$ alkenyl, $C_{2-4}$ alkynyl, $-OC_{1-4}$ alkyl, and $-SC_{1-4}$ alkyl, wherein the alkyl, alkenyl, and alkynyl are optionally substituted with 1 to 4 substituents selected from deuterium, halogen, OH, $NH_2$, CN, $C_{1-4}$ alkyl, and $C_{1-4}$ alkoxy;

K is

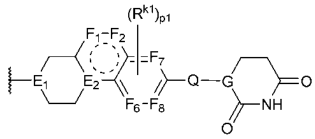

;

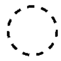

represents that the ring is an aromatic ring or a non-aromatic ring;

$F_1$ is selected from N, NH, CH, $CH_2$, $CHR^{k1}$, $NR^{k1}$, $CR^{k1}$, C(=O), and $C(R^{k1})_2$;

$F_2$ is selected from a bond, O, N, NH, CH, $CH_2$, $CHR^{k1}$, $NR^{k1}$, $CR^{k1}$ or $C(R^{k1})_2$;

$F_6$, $F_7$, and $F_8$ are each independently selected from N, C, CH or $CR^{k1}$, and at most two of $F_6$, $F_7$, and $F_8$ are N;

G is selected from CH or N;

$E_1$ is selected from N or CH;

$E_2$ is selected from C, N or CH;

each Q is independently selected from a bond, -O-, -S-, $-CH_2-$, $-NR^q-$, -C(=O)-, $-NR^qC(=O)-$, and $-C(=O)NR^q-$;

Q and G cannot be directly bonded to form an N-N or N-O bond;

each $R^q$ is independently selected from H or $C_{1-4}$ alkyl;

each $R^{k1}$ is independently selected from deuterium, halogen, OH, $NH_2$, CN, COOH, $CONH_2$, $C_{1-4}$ alkyl, $C_{2-4}$ alkenyl, $C_{2-4}$ alkynyl, $C_{1-4}$ alkoxy, $C_{3-6}$ cycloalkyl, and 4- to 6-membered heterocycloalkyl, wherein the alkyl, alkenyl, alkynyl, alkoxy, cycloalkyl, and heterocycloalkyl are optionally substituted with 1 to 4 $R^s$;

$R^{L2}$ and $R^s$ are each independently selected from deuterium, halogen, OH, CN, =O, $CF_3$, $SF_5$, $NO_2$, $NH_2$, $NHC_{1-4}$ alkyl, $N(C_{1-4}$ alkyl$)_2$, COOH, $CONH_2$, $C_{1-4}$ alkyl, $C_{2-4}$ alkenyl, $C_{2-4}$ alkynyl, $C_{1-4}$ alkoxy, $-S-C_{1-4}$ alkyl, $-C_{0-4}$ alkylene-$C_{3-6}$ cycloalkyl, and $-C_{0-4}$ alkylene-4- to 6-membered heterocyclyl, wherein the alkyl, alkylene, alkoxy, alkenyl, alkynyl, and cycloalkyl are optionally substituted with 1 to 4 substituents selected from deuterium, F, Cl, Br, I, OH, CN, $C_{1-4}$ alkyl, and $C_{1-4}$ alkoxy;

each p1 is independently selected from 0, 1 or 2;

or each $R^s$ is independently $-O-C_{3-6}$ cycloalkyl.

A second embodiment of the present invention provides the above-mentioned compound represented by general formula (I) or the stereoisomer, tautomer, deuterated substance, solvate, prodrug, metabolite, pharmaceutically acceptable salt or co-crystal thereof, wherein

$R^{x1}$, $R^{x2}$, $R^{x3}$, $R^{x4}$, $R^{x5}$, $R^{z1}$, $R^{z2}$, $R^{z3}$, and $R^{z4}$ are each independently selected from H, deuterium, halogen, OH, $NH_2$, CN, $NO_2$, COOH, $CONH_2$, $NHC_{1-4}$ alkyl, $N(C_{1-4}$ alkyl$)_2$, $C_{1-4}$ alkyl, $C_{2-4}$ alkenyl, $C_{2-4}$ alkynyl, $-OC_{1-4}$ alkyl, $-SC_{1-4}$ alkyl, $-C_{0-2}$ alkylene-$C_{3-6}$ cycloalkyl, $-C_{0-2}$ alkylene-4- to 6-membered heterocyclyl, 5- to 6-membered heteroaryl, $-O-C_{3-6}$ cycloalkyl, and -O-4- to 6-membered heterocyclyl, wherein the alkylene, alkyl, alkenyl, alkynyl, cycloalkyl, heterocyclyl, and heteroaryl are optionally substituted with 1 to 4 $R^s$;

or $R^{x1}$, $R^{x2}$, $R^{x3}$, and $R^{x4}$ are each independently selected from $-S(=O)_2NH_2$ and $-S(=O)_2C_{1-4}$ alkyl;

or $R^{x1}$ and $R^{x2}$, $R^{x2}$ and $R^{x3}$, $R^{x3}$ and $R^{x4}$, and $R^{x4}$ and $R^{x5}$ are directly connected to form $C_{4-6}$ carbocyclyl, 5- to 6-membered heteroaryl or 5- to 7-membered heterocyclyl, wherein the carbocyclyl, heteroaryl or heterocyclyl is optionally substituted with 1 to 4 $R^s$;

or $R^1$ and $R^{z3}$, and $R^1$ and $R^{z1}$ are directly connected to form 5- to 7-membered heterocyclyl, wherein the heterocyclyl is optionally substituted with 1 to 4 $R^s$;

$R^L$ is selected from H, methyl or ethyl;

each $R^q$ is independently selected from H, methyl, ethyl or isopropyl;

Cy1, Cy2, Cy3, and Cy4 are each independently selected from a bond or one of the following groups optionally substituted with 1 to 4 $R^{L2}$: phenyl, pyridyl, pyrimidyl, pyrazinyl, pyridazinyl, pyrazolyl, thiazolyl, oxazolyl, triazolyl,

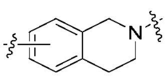, 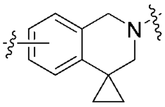, 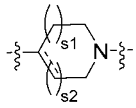, 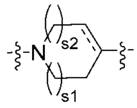, 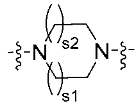, 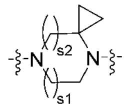,

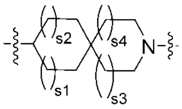, 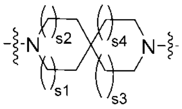, 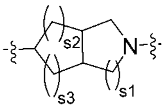, 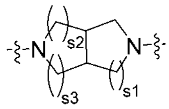, 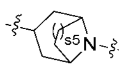,

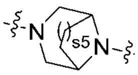, 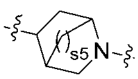, 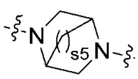, 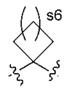, 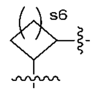, 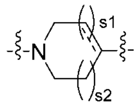, 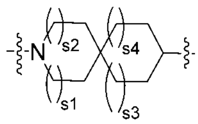,

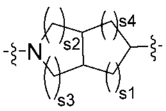, 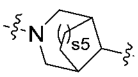, 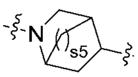, 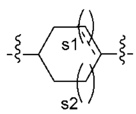, 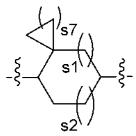, 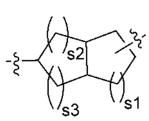,

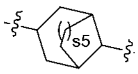, 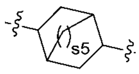, 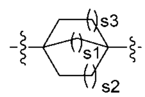, 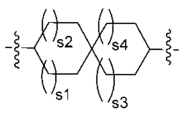, 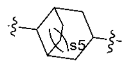, 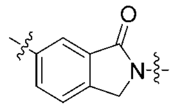,

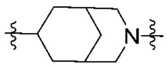, 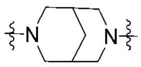, 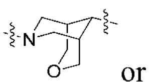 or 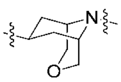;

s1, s3 and s5 are each independently selected from 0, 1 or 2;
s2 and s4 are each independently selected from 0 or 1;
s6 is selected from 0, 1, 2 or 3;
s7 is selected from 1, 2 or 3;
$R^{L2}$ and $R^s$ are each independently selected from deuterium, halogen, OH, CN, =O, $CF_3$, $SF_5$, $NO_2$, $NH_2$, $NHC_{1-4}$ alkyl, $N(C_{1-4}$ alkyl$)_2$, COOH, $CONH_2$, $C_{1-4}$ alkyl, $C_{2-4}$ alkenyl, $C_{2-4}$ alkynyl, $C_{1-4}$ alkoxy, -S-$C_{1-4}$ alkyl, -$C_{0-2}$ alkylene-$C_{3-6}$ cycloalkyl, and -$C_{0-2}$ alkylene-4- to 6-membered heterocyclyl, wherein the alkyl, alkylene, alkoxy, alkenyl, alkynyl, and cycloalkyl are optionally substituted with 1 to 4 substituents selected from deuterium, F, Cl, Br, I, OH, CN, $C_{1-4}$ alkyl, and $C_{1-4}$ alkoxy;
or each $R^s$ is independently -O-$C_{3-6}$ cycloalkyl;
the definitions of the remaining groups are the same as those in the first embodiment of the present invention.

A third embodiment of the present invention provides the above-mentioned compound represented by general formula (I) or the stereoisomer, tautomer, deuterated substance, solvate, prodrug, metabolite, pharmaceutically acceptable salt or co-crystal thereof, wherein

$X_3$ is $CR^{x3}$;
$Y_1$ and $Y_2$ are each independently selected from -$CR^{y1}R^{y2}$- and -$(CR^{y1}R^{y2})_2$-;
$R^1$ is selected from H, deuterium, methyl, ethyl, isopropyl, cyclopropyl, and cyclobutyl, wherein the methyl, ethyl, isopropyl, cyclopropyl, and cyclobutyl are optionally substituted with 1 to 4 substituents selected from deuterium, F, Cl, Br, I, OH, $NH_2$, CN, $C_{1-4}$ alkyl, and $C_{1-4}$ alkoxy;
$R^{x1}$, $R^{x2}$, $R^{x3}$, $R^{x4}$, $R^{x5}$, $R^{z1}$, $R^{z2}$, $R^{z3}$, and $R^{z4}$ are each independently selected from H, deuterium, F, Cl, Br, I, OH, $NH_2$, CN, $NO_2$, COOH, $CONH_2$, $N(CH_3)_2$, $NHCH_3$, or one of the following groups optionally substituted with 1 to 4 $R^s$: methyl, ethyl, vinyl, ethynyl, methoxy, ethoxy, methylthio, cyclopropyl, cyclobutyl, cyclopentyl, -$CH_2$-cyclopropyl, -$CH_2$-cyclobutyl, -O-cyclopropyl, and pyrazolyl;
or $R^{x1}$, $R^{x2}$, $R^{x3}$, and $R^{x4}$ are each independently selected from -$S(=O)_2NH_2$, - $S(=O)_2$ methyl, and -$S(=O)_2$ ethyl;

or $R^{x1}$ and $R^{x2}$, $R^{x2}$ and $R^{x3}$, $R^{x3}$ and $R^{x4}$, and $R^{x4}$ and $R^{x5}$ are directly connected to form one of the following groups optionally substituted with 1 to 3 $R^s$: piperidyl, cyclohexyl, phenyl, pyridyl, pyrimidyl, pyridazinyl, and pyrazinyl;
or $R^{x1}$ and $R^{x2}$, $R^{x2}$ and $R^{x3}$, $R^{x3}$ and $R^{x4}$, and $R^{x4}$ and $R^{x5}$ are directly connected to form one of the following groups optionally substituted with 1 to 3 $R^s$: pyrrolidyl, oxolanyl, oxanyl, and 1,3-dioxolane;
or $R^1$ and $R^{z3}$, and $R^1$ and $R^{z1}$ are directly connected to form the following structure optionally substituted with 1 to 4 $R^s$:

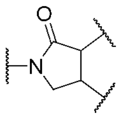
, or
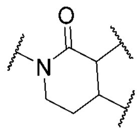
;

$R^2$, $R^3$, $R^{y1}$, and $R^{y2}$ are each independently selected from H, deuterium, F, Cl, Br, I, OH, $NH_2$, CN, $NO_2$, COOH, $CONH_2$, methyl, ethyl, vinyl, ethynyl, methoxy, ethoxy, and methylthio, wherein the methyl, ethyl, vinyl, ethynyl, methoxy, ethoxy, and methylthio are substituted with 1 to 4 substituents selected from deuterium, F, Cl, Br, I, OH, $NH_2$, CN, $C_{1-4}$ alkyl, and $C_{1-4}$ alkoxy;
Ak1, Ak2, Ak3, Ak4, and Ak5 are each independently selected from a bond, - O-, -S-, $-OCH_2-$, $-CH_2O-$, $-OCH_2CH_2-$, $-CH_2CH_2O-$, -C=C-, $-CH(CH_3)-$, $-CH_2-$,$-C(CH_3)_2-$, $-CH_2CH_2-$, $-CH_2CH_2CH_2-$, $-N(CH_3)-$, -NH-, $-CH_2N(CH_3)-$, $-CH_2NH-$, - $NHCH_2-$, $-CH_2CH_2N(CH_3)-$, $-CH_2CH_2NH-$, $-NHCH_2CH_2-$, -C(=O)-, - $C(=O)CH_2NH-$, $-CH_2C(=O)NH-$, -C(=O)NH- or -NHC(=O)-;
K is selected from

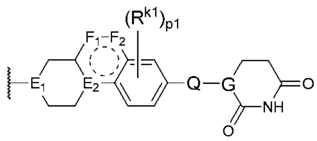
,
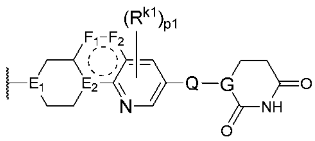
,

and

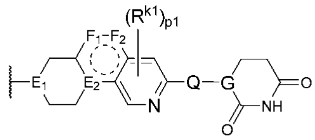
;

each Q is independently selected from a bond, $CH_2$, NH, $N(CH_3)$, O, S, C(=O), NHC(=O), C(=O)NH, $N(CH_3)C(=O)$, and $C(=O)N(CH_3)$;
each $R^{k1}$ is independently selected from deuterium, F, Cl, Br, I, OH, $NH_2$, CN, COOH, $CONH_2$, methyl, ethyl, isopropyl, methoxy, ethoxy, isopropoxy, and cyclopropyl, wherein the methyl, ethyl, isopropyl, methoxy, ethoxy, isopropoxy, and cyclopropyl are optionally substituted with 1 to 4 substituents selected from deuterium, F, Cl, Br, I, OH, and $NH_2$;
$R^{L2}$ and $R^s$ are each independently selected from deuterium, F, Cl, Br, I, OH, =O, $CF_3$, $SF_5$, CN, $NH_2$, $NO_2$, COOH, $CONH_2$, $N(CH_3)_2$, $NHCH_3$, methyl, ethyl, vinyl, ethynyl, methoxy, ethoxy, methylthio, cyclopropyl, cyclobutyl, cyclopentyl, cyclohexyl, $-CH_2$-cyclopropyl, $-CH_2$-cyclobutyl, $-CH_2$-cyclopentyl, and $-CH_2$-cyclohexyl, wherein the methyl, ethyl, vinyl, ethynyl, methoxy, ethoxy, methylthio, cyclopropyl, cyclobutyl, cyclopentyl, and cyclohexyl are optionally substituted with 1 to 4 substituents selected from deuterium, F, Cl, Br, I, OH, CN, $C_{1-4}$ alkyl, and $C_{1-4}$ alkoxy;
or each $R^s$ is independently selected from propynyl and -O-cyclopropyl;
the definitions of the remaining groups are the same as those in the first or second embodiment of the present invention.

A fourth embodiment of the present invention provides the above-mentioned compound represented by general formula (I) or the stereoisomer, tautomer, deuterated substance, solvate, prodrug, metabolite, pharmaceutically acceptable salt or co-crystal thereof, wherein

B is selected from

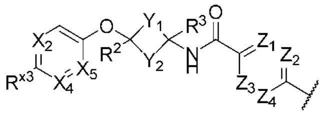, 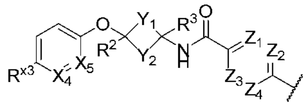,

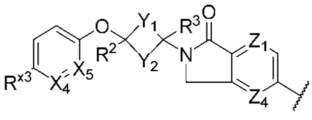, and 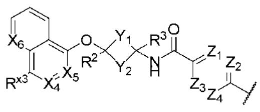;

or B is

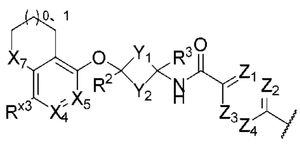;

$X_6$ is selected from N or CH;
$X_7$ is selected from NH or O;
at most two of $Z_1$, $Z_2$, $Z_3$, and $Z_4$ are N;
$R^2$ and $R^3$ are each independently selected from H, deuterium, F, Cl, and Br;
$R^{y1}$ and $R^{y2}$ are each independently selected from H, deuterium, F, Cl, Br, I, OH, $NH_2$, CN, $NO_2$, COOH, $CONH_2$, methyl, ethyl, vinyl, ethynyl, methoxy, ethoxy, and methylthio, wherein the methyl, ethyl, vinyl, ethynyl, methoxy, ethoxy, and methylthio are substituted with 1 to 4 substituents selected from deuterium, F, Cl, Br, I, OH, $NH_2$, CN, methyl, ethyl, and methoxy;
Cy1, Cy2, Cy3, and Cy4 are each independently selected from a bond or one of the following optionally substituted groups:

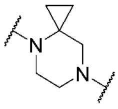, 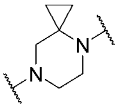, 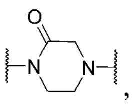,

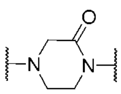, 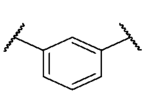, 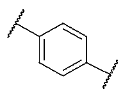, 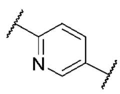, 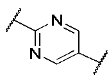, 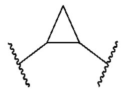, 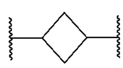,

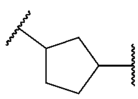, 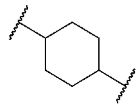, 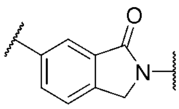, 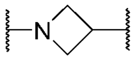, 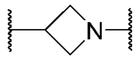, 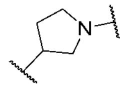,

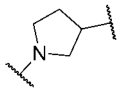, 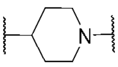, 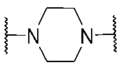, 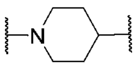, 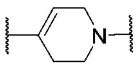, 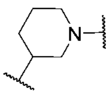, 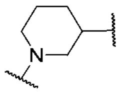,

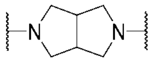, 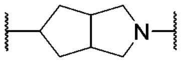, 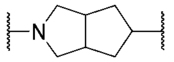, 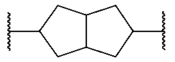, 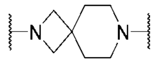,

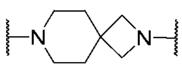, 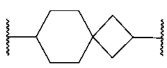, 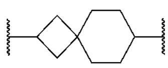, 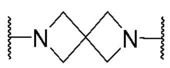, 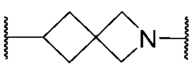,

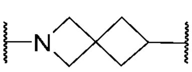, 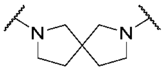, 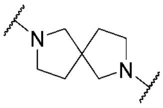, 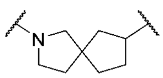, 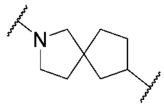,

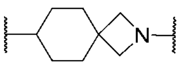, 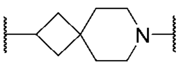, 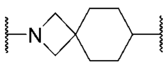, 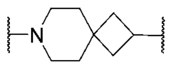, 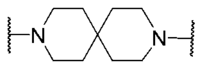,

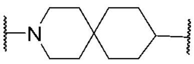, 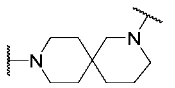, 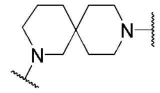, 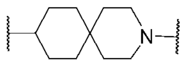, 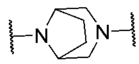,

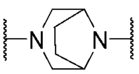, 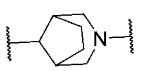, 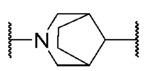, 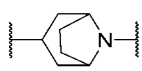, 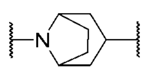, 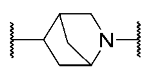,

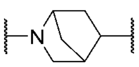, 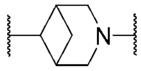, 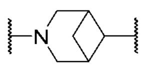, 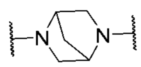, 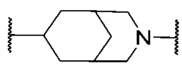, 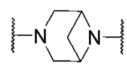,

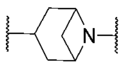, 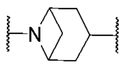, and 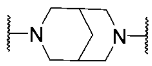,

which, when substituted, are substituted with 1 to 4 substituents selected from deuterium, F, $CF_3$, OH, =O, COOH, CN, $NH_2$, hydroxymethyl, methyl, methoxy, and cyclopropyl;
K is selected from

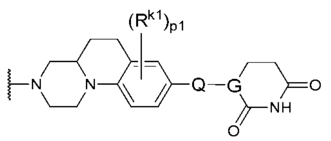, 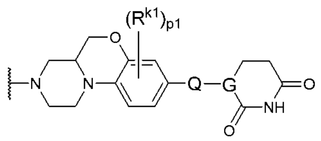,

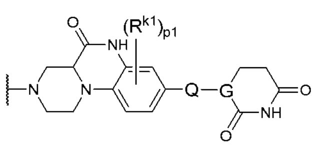, 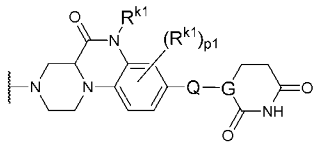, 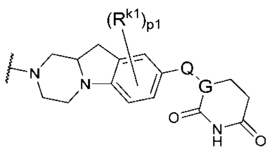,

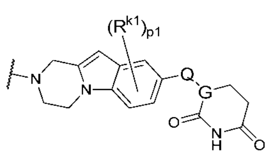, and 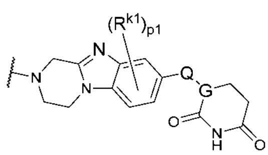;

or K is selected from

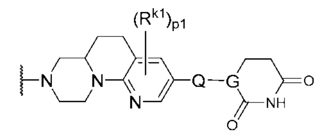 and 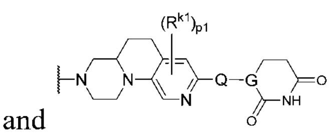;

the definitions of the remaining groups are the same as those in the first, second or third embodiment of the present invention.

A fifth embodiment of the present invention provides the above-mentioned compound represented by general formula (I) or the stereoisomer, tautomer, deuterated substance, solvate, prodrug, metabolite, pharmaceutically acceptable salt or co-crystal thereof, wherein

B is selected from

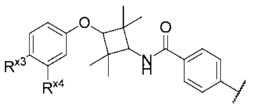 , 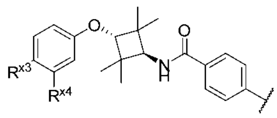 ,

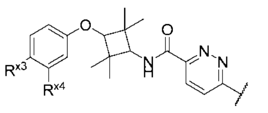 , 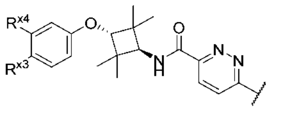 , 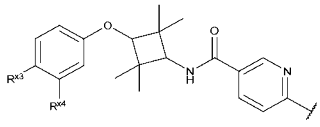 ,

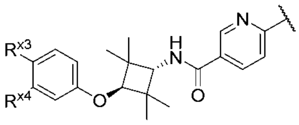 , 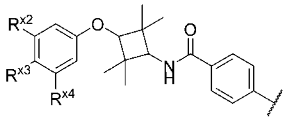 , 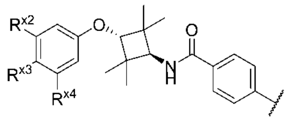 ,

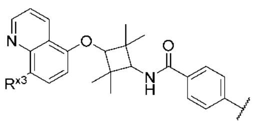 , 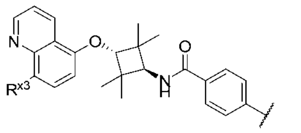 , 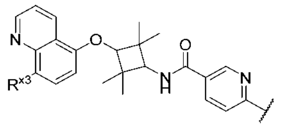 ,

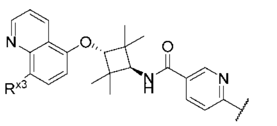 , 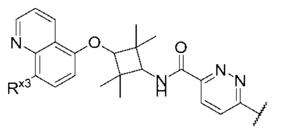 , 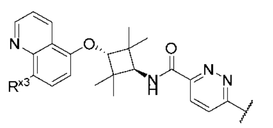 ,

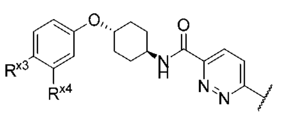 , 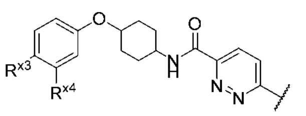 , 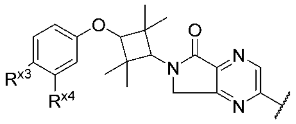 ,

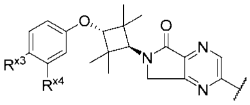 , 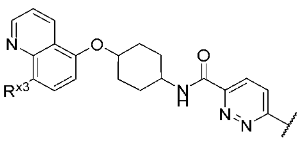 , and 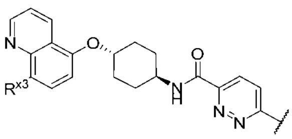 ;

or B is selected from

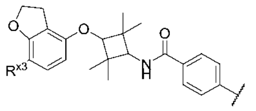 , 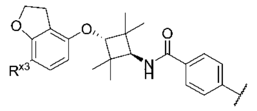 ,

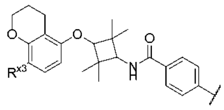 , and 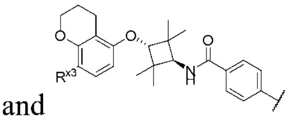 ;

$R^{x2}$, $R^{x3}$, and $R^{x4}$ are each independently selected from H, deuterium, F, Cl, Br, I, OH, $NH_2$, CN, $NO_2$, COOH, $CONH_2$, $N(CH_3)_2$, $NHCH_3$, $CF_3$, $CHF_2$, $CH_2F$, $OCF_3$, $OCH_2F$, $OCD_3$, $CH_2OH$, methyl, ethyl, vinyl, ethynyl, methoxy, ethoxy, methylthio, cyclopropyl, $-CH_2$-cyclopropyl, and -O-cyclopropyl;

or $R^{x2}$ and $R^{x4}$ are each independently selected from $-S(=O)_2CH_3$, $-O-CH_2$-propynyl, $-O-CH_2$-cyclopropyl, $-O-CH_2CH_2-OCH_3$, $-O-CH_2CH_2$-O-cyclopropyl,

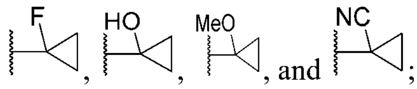
, and ;

each Q is independently selected from a bond, NH, $N(CH_3)$, O, S, NHC(=O), C(=O)NH, $N(CH_3)C(=O)$, and $C(=O)N(CH_3)$;

L is selected from -Cyl-, -Cy1-Ak2-, $-Cy1-CH_2-$, -Ak1-Cy1-, -Cy1-Cy2-, - $Cy1-CH_2-Cy2-$, -Cy1-Cy2-Cy3-, $-Cy1-CH_2-Cy2-Cy3-$, and $-Cy1-Cy2-CH_2-Cy3-$;

or L is selected from -Cy1-Ak2-Cy2- and -Cy1-O-Cy2-;

Cy1, Cy2, and Cy3 are each independently selected from one of the following optionally substituted groups:

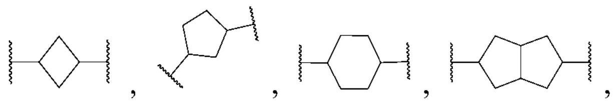

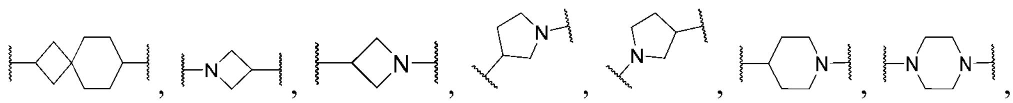

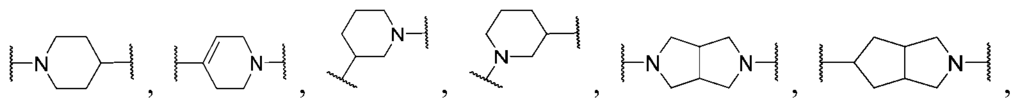

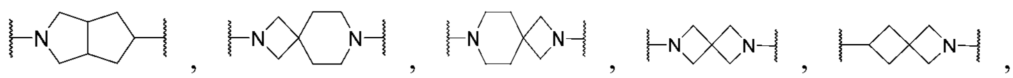

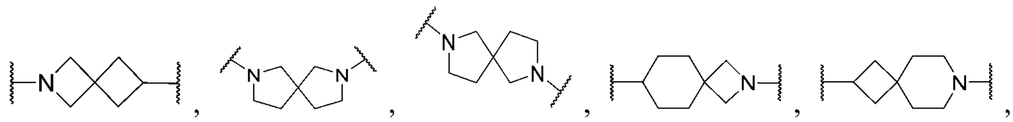

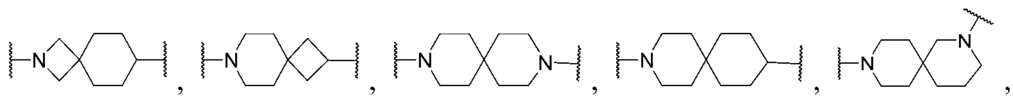

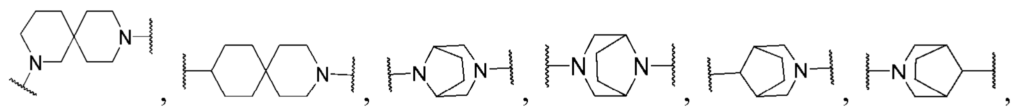

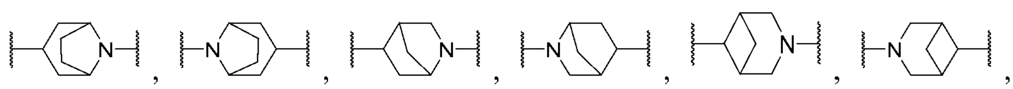

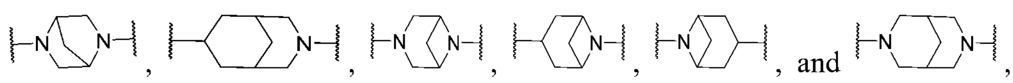
, and ,

which, when substituted, are substituted with 1 to 4 substituents selected from deuterium, F, $CF_3$, OH, =O, COOH, CN, $NH_2$, hydroxymethyl, methyl, methoxy, and cyclopropyl;

the definitions of the remaining groups are the same as those in the first, second, third or fourth embodiment of the present invention.

A sixth embodiment of the present invention provides the above-mentioned compound represented by general formula (I) or the stereoisomer, tautomer, deuterated substance, solvate, prodrug, metabolite, pharmaceutically acceptable salt or co-crystal thereof, wherein

K is selected from

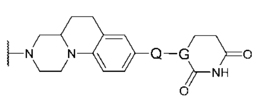, 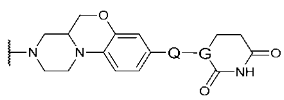,

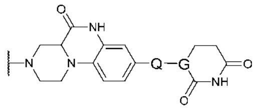, 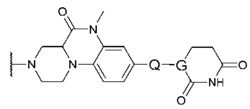, 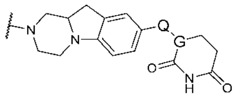, 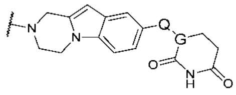,

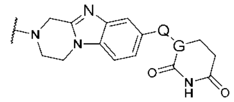, 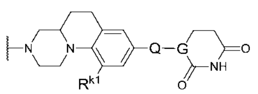, 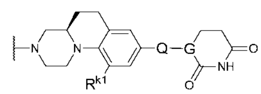,

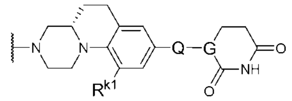, 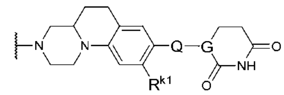, 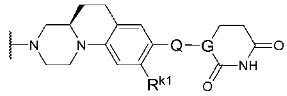,

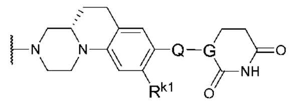, 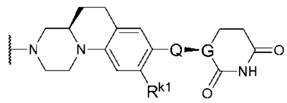, 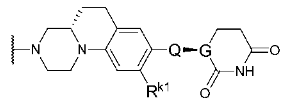,

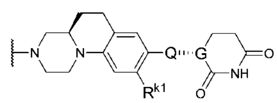, 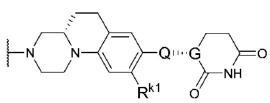, 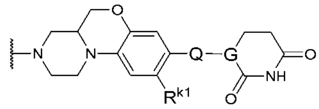,

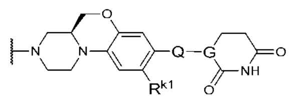, 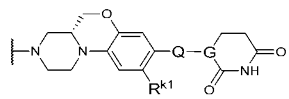, 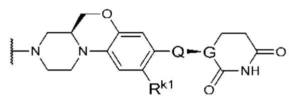,

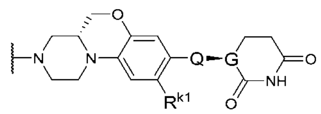, 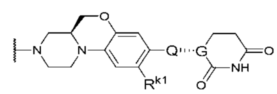, 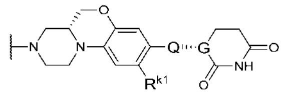,

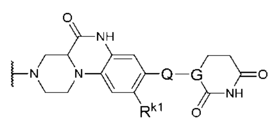, 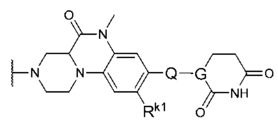, 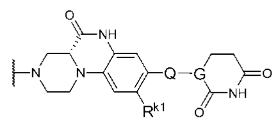,

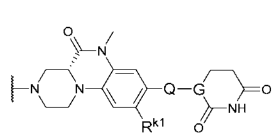, 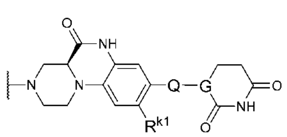, 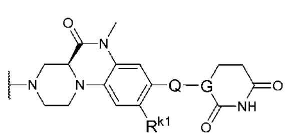,

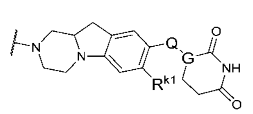, 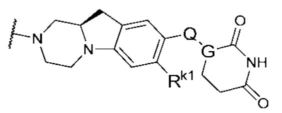, 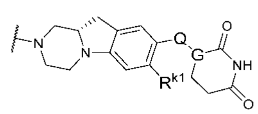,

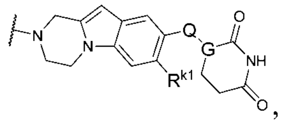, and 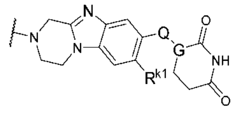;

or K is selected from

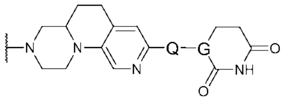, 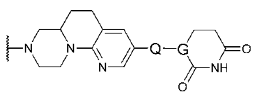,

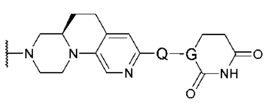, 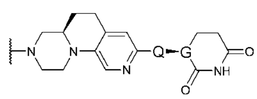, 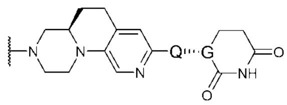,

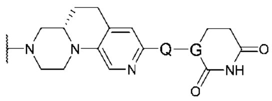, 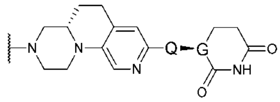, 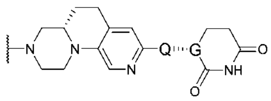,

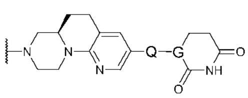, 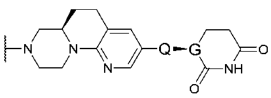, 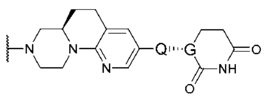,

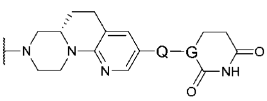, 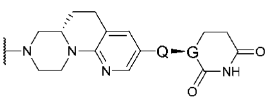, and 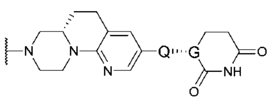;

each Q is independently selected from a bond, NH, and C(=O)NH;
each $R^{k1}$ is independently selected from deuterium, F, Cl, Br, I, OH, $NH_2$, CN, COOH, $CONH_2$, $CF_3$, $CHF_2$, $CH_2F$, $OCF_3$, $OCH_2F$, $CH_2OH$, methyl, ethyl, isopropyl, methoxy, ethoxy, isopropoxy, and cyclopropyl;
L is selected from one of the structural fragments shown in Table L-1;
the definitions of the remaining groups are the same as those in the first, second, third, fourth or fifth embodiment of the present invention.

A seventh embodiment of the present invention provides the above-mentioned compound represented by general formula (I) or the stereoisomer, tautomer, deuterated substance, solvate, prodrug, metabolite, pharmaceutically acceptable salt or co-crystal thereof, wherein

B is selected from

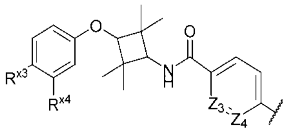 and 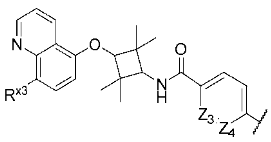;

$Z_3$ is selected from N or $CR^{z3}$; $Z_4$ is selected from N or $CR^{z4}$; preferably, B is selected from

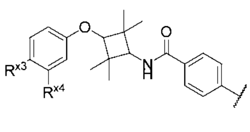, 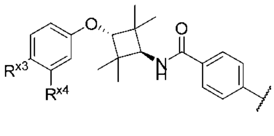, 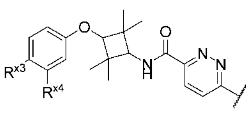,

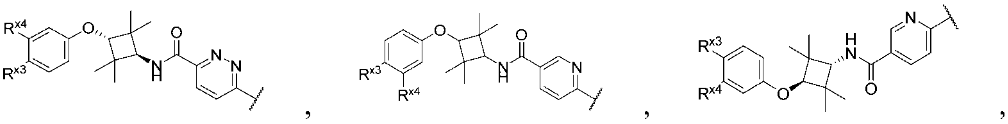 , , ,

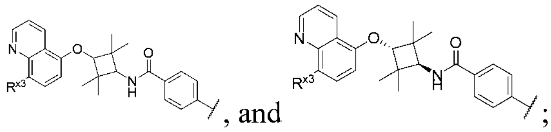 , and ;

L is selected from -Cyl-, -Cy1-Ak2-, and -Cy1-Ak2-Cy2-; preferably, L is selected from -Cyl-, -Cy1-$CH_2$-, -Cy1-$C(CH_3)_2$-, and -Cy1-O-Cy2; further preferably, L is selected from

| | | | |
|---|---|---|---|
| 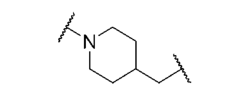 | 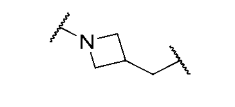 | 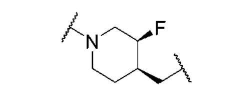 | 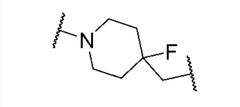 |
| 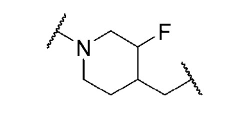 | 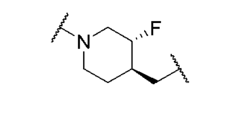 | 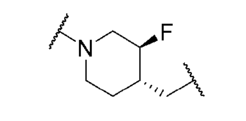 | 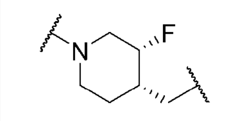 |
| 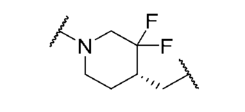 | 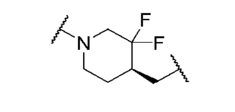 | 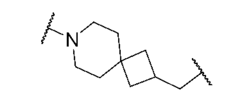 | 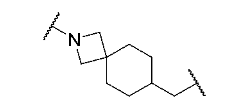 |
| 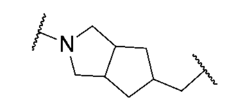 | 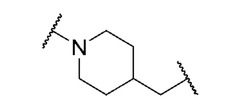 | 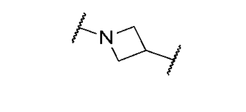 | 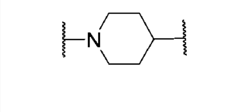 |
| 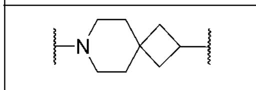 | 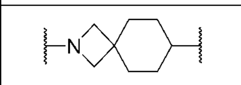 | 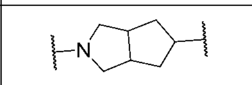 | 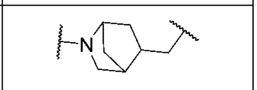 |
| 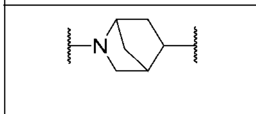 | 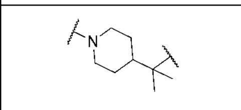 | 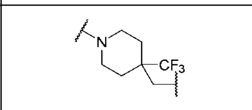 | 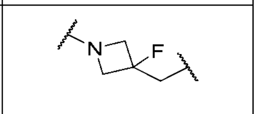 |
| 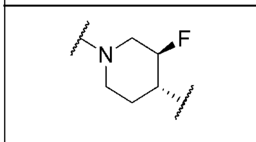 | 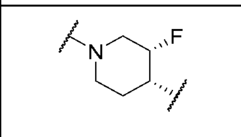 | 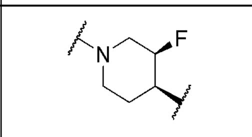 | 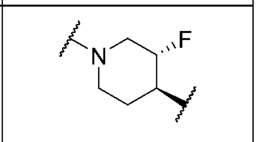 |
| 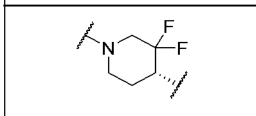 | 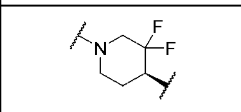 | 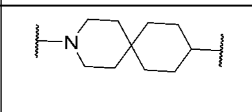 | 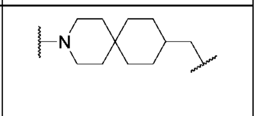 |
| 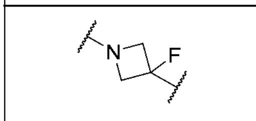 | 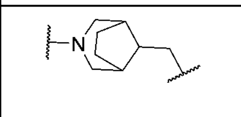 | 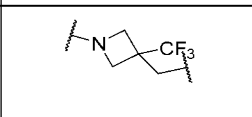 | 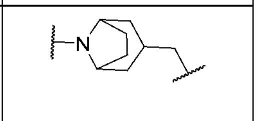 |
| 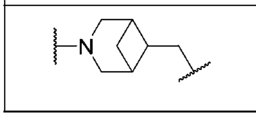 | 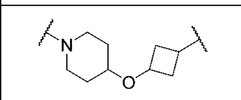 | | |

;

Cy1 and Cy2 are each independently selected from one of the following optionally substituted groups:

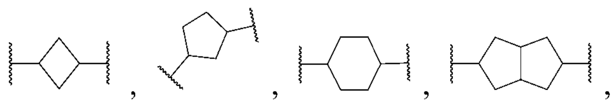

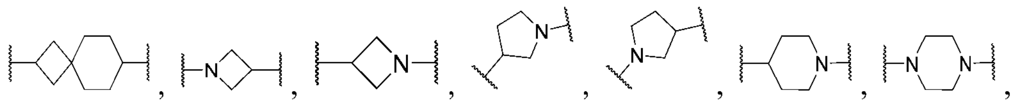

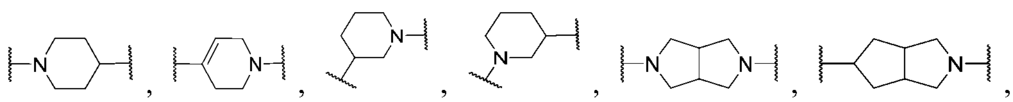

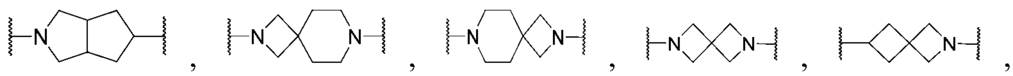

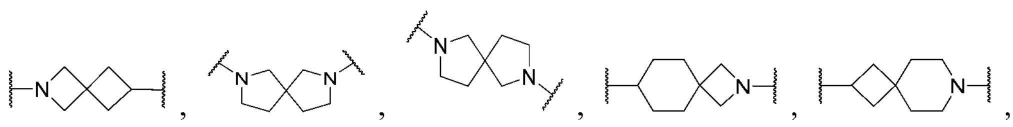

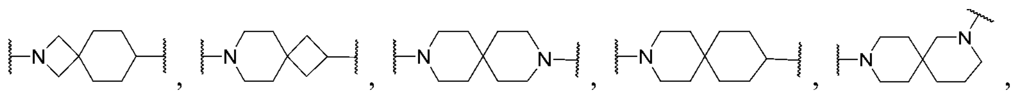

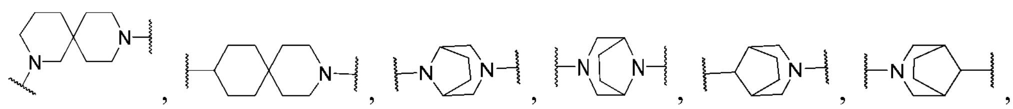

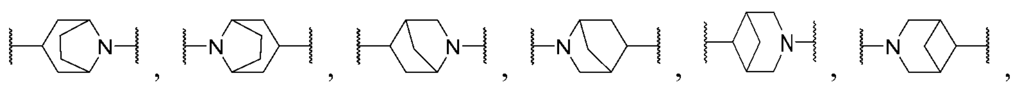

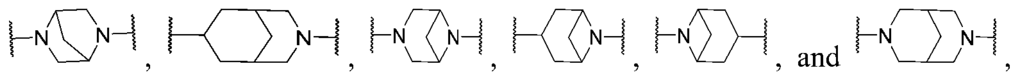

which, when substituted, are substituted with 1 to 4 substituents selected from deuterium, F, $CF_3$, OH, =O, COOH, CN, $NH_2$, hydroxymethyl, methyl, methoxy, and cyclopropyl; preferably, Cy1 and Cy2 are each independently selected from one of the following optionally substituted groups:

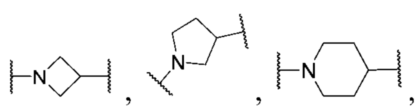

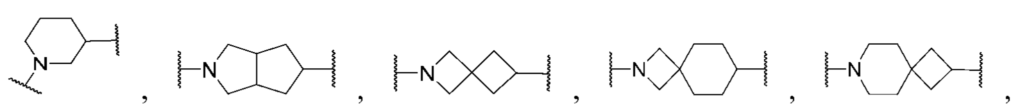

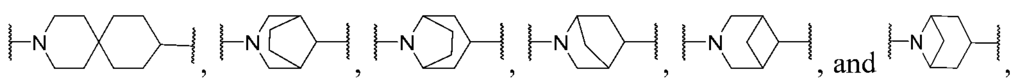

which, when substituted, are substituted with 1 to 4 substituents selected from deuterium, F, $CF_3$, OH, =O, COOH, CN, $NH_2$, hydroxymethyl, methyl, methoxy, and cyclopropyl;

Ak2 is selected from -$C(CH_3)_2$- or -$CH_2$-;
K is

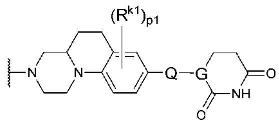
;

preferably, K is selected from

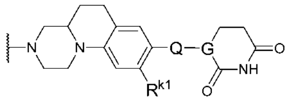
and
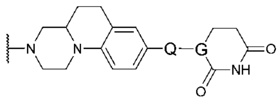
;

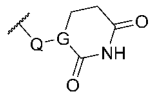

is selected from

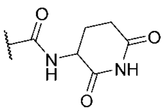
,
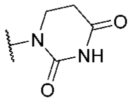
, and
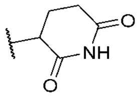
;

the definitions of the remaining groups are consistent with those described in any embodiment of the present invention.

The present invention relates to a compound as described below or a stereoisomer, tautomer, deuterated substance, solvate, prodrug, metabolite, pharmaceutically acceptable salt or co-crystal thereof, wherein the compound is selected from one of the structures shown in Table E below:

Table E

| | | |
|---|---|---|
| 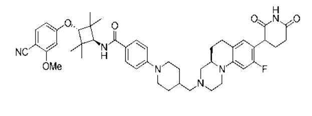 | 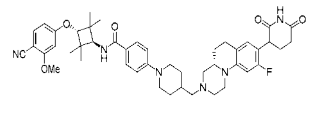 | 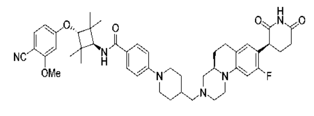 |
| 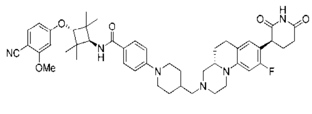 | 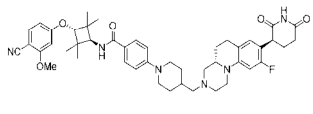 | 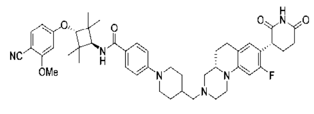 |
| 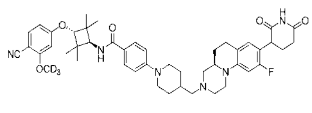 | 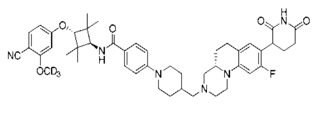 | 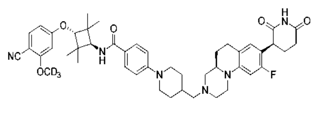 |
| 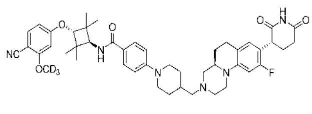 | 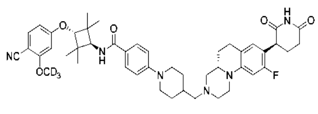 | 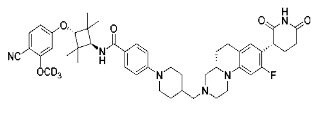 |
| 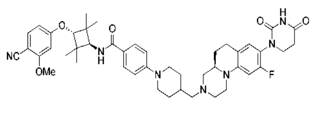 | 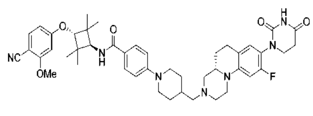 | 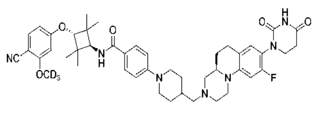 |
| 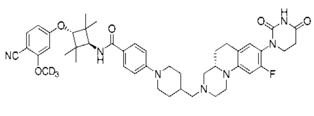 | 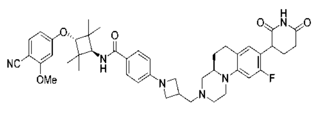 | 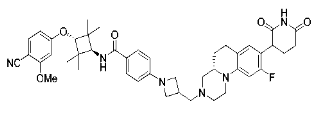 |
| 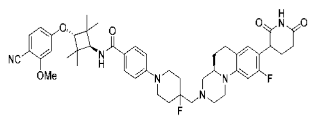 | 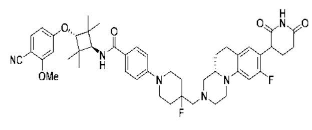 | 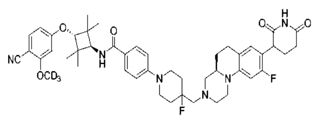 |

| | | |
|---|---|---|
| 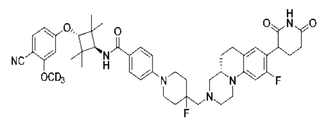 | 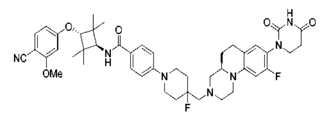 | 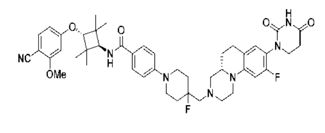 |
| 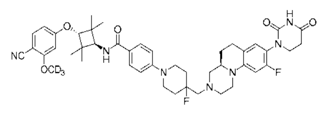 | 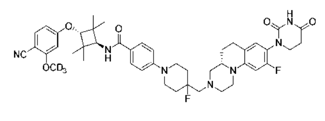 | 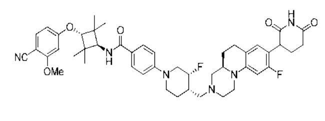 |
| 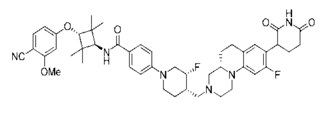 | 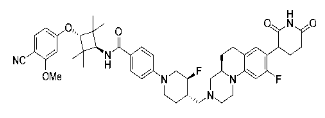 | 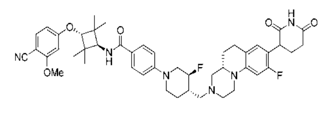 |
| 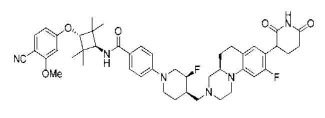 | 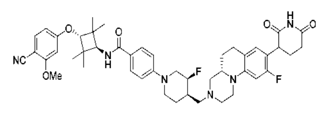 | 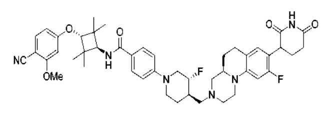 |
| 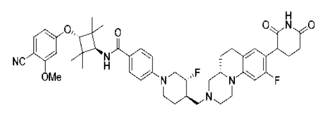 | 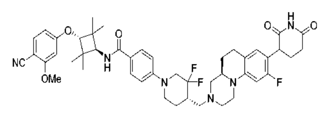 | 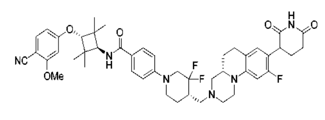 |
| 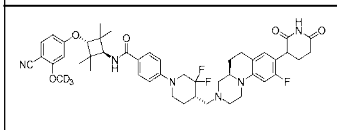 | 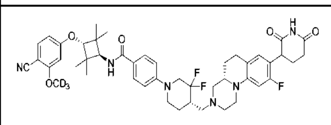 | 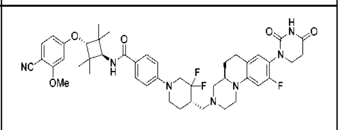 |
| 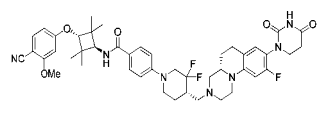 | 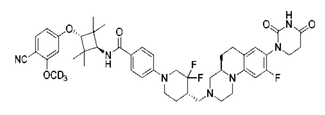 | 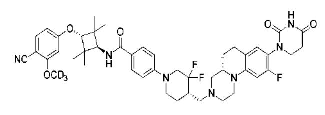 |
| 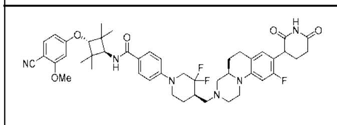 | 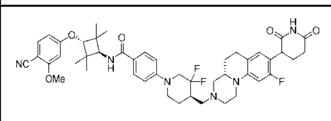 | 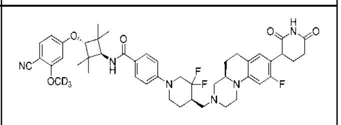 |
| 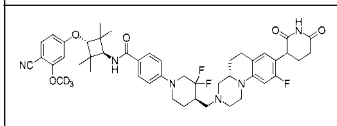 | 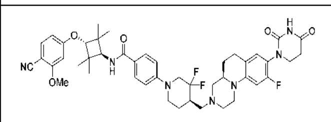 | 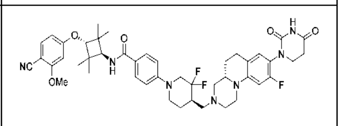 |
| 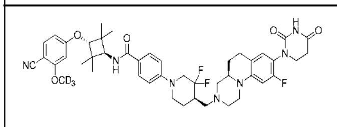 | 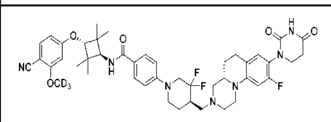 | 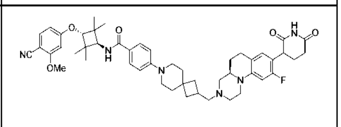 |
| 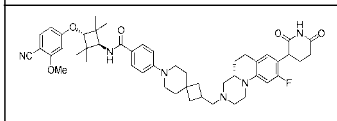 | 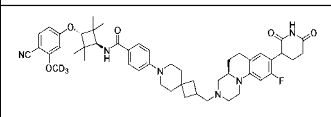 | 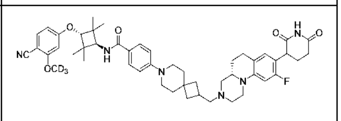 |
| 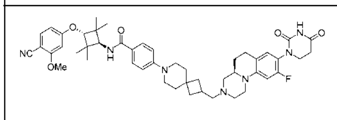 | 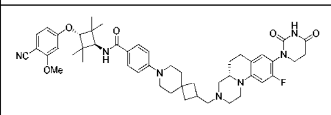 | 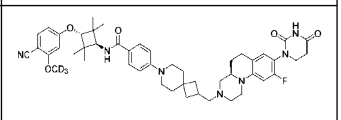 |
| 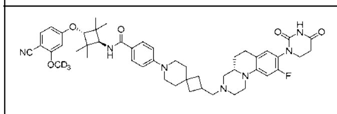 | 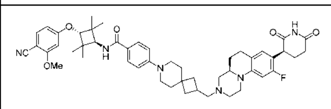 | 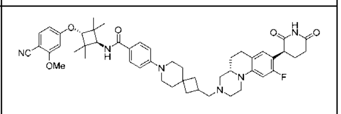 |
| 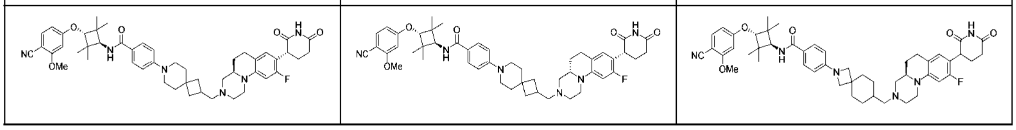 | | |

| | | |
|---|---|---|
| 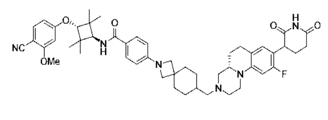 | 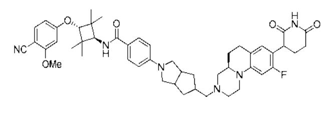 | 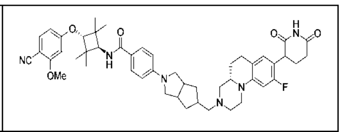 |
| 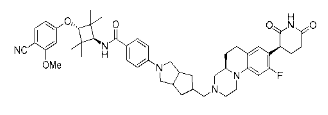 | 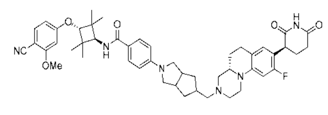 | 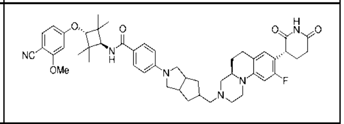 |
| 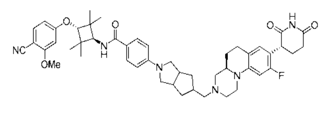 | 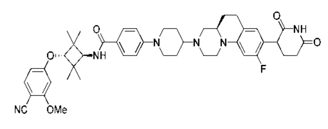 | 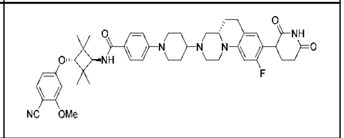 |
| 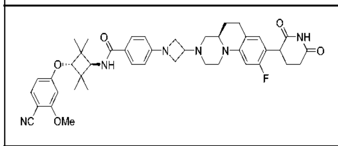 | 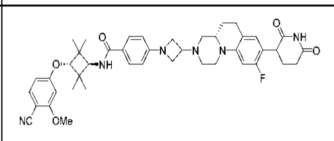 | 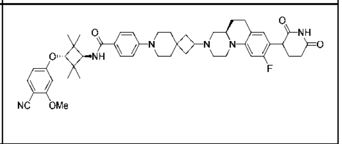 |
| 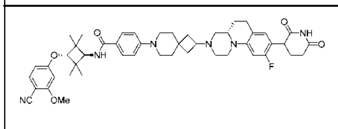 | 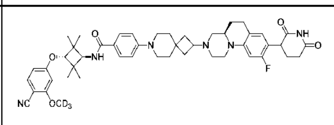 | 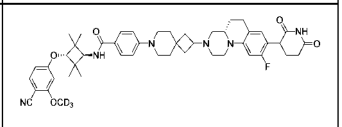 |
| 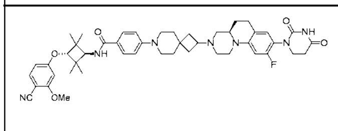 | 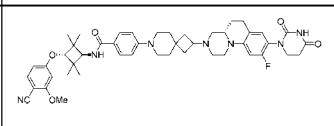 | 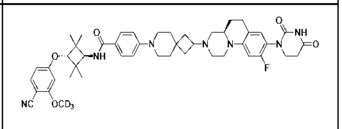 |
| 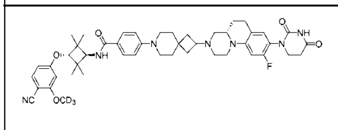 | 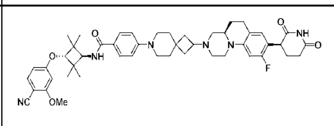 | 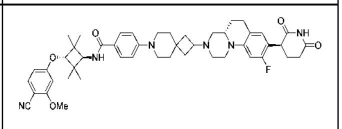 |
| 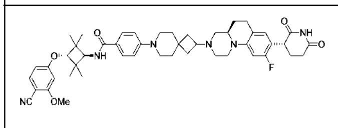 | 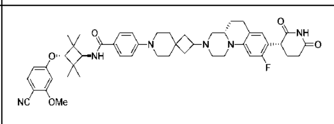 | 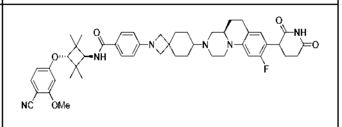 |
| 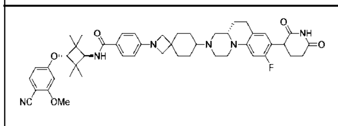 | 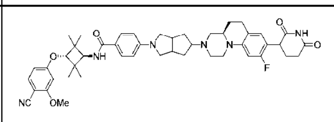 | 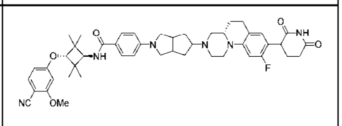 |
| 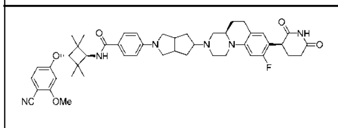 | 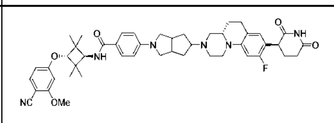 | 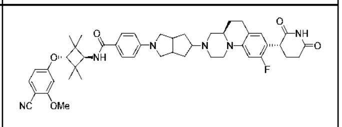 |
| 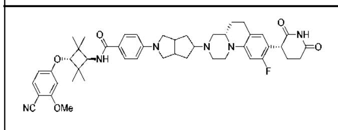 | 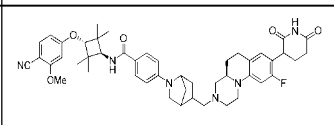 | 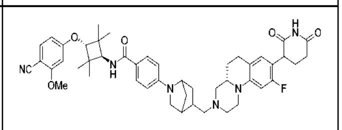 |
| 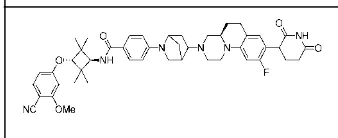 | 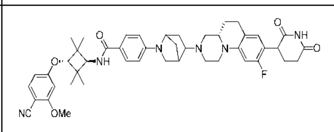 | 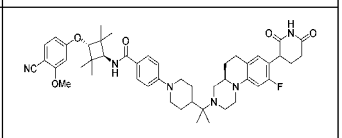 |
| 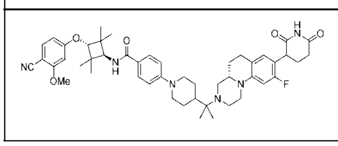 | 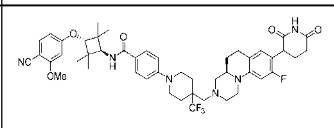 | 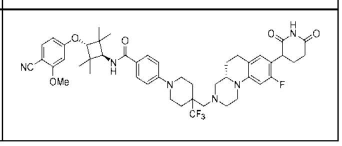 |

| | | |
|---|---|---|
| 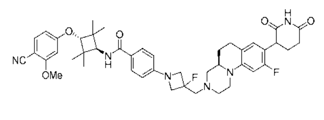 | 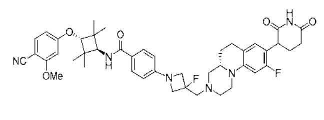 | 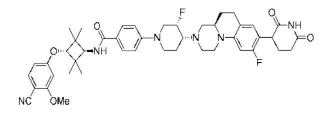 |
| 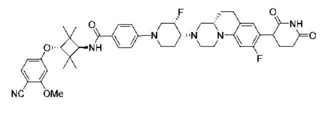 | 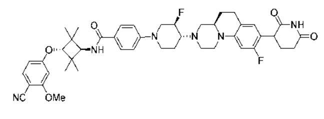 | 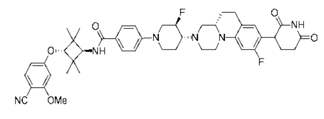 |
| 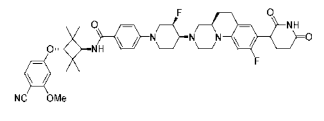 | 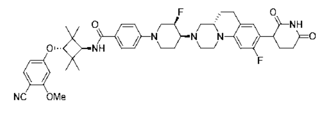 | 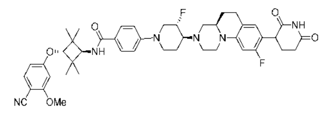 |
| 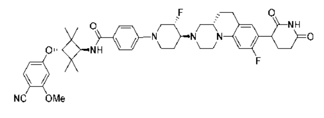 | 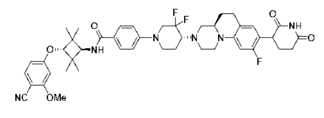 | 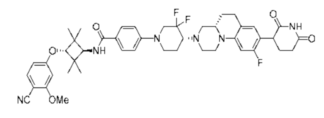 |
| 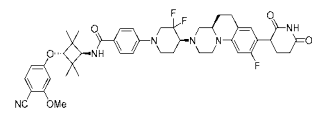 | 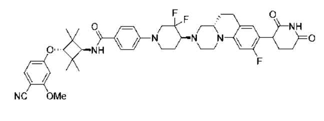 | 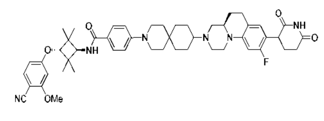 |
| 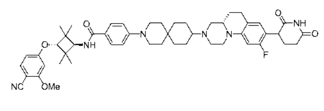 | 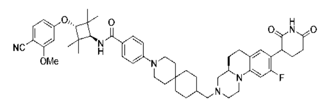 | 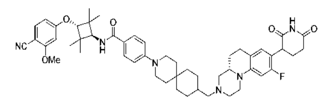 |
| 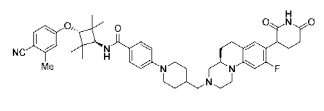 | 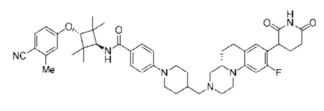 | 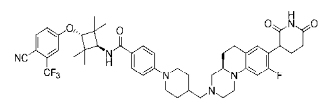 |
| 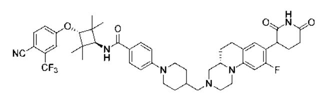 | 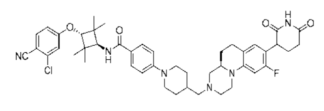 | 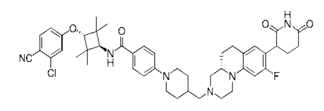 |
| 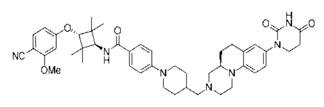 | 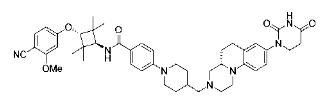 | 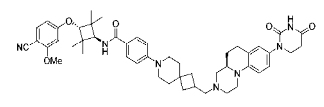 |
| 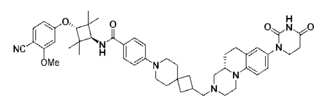 | 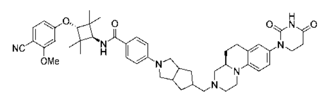 | 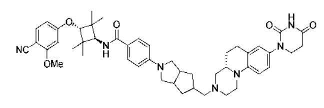 |
| 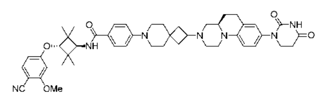 | 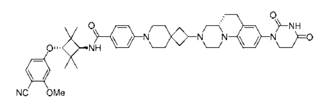 | 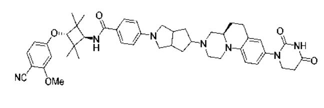 |
| 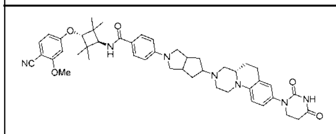 | 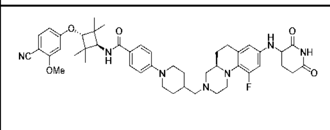 | 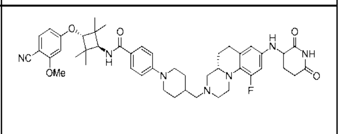 |
| 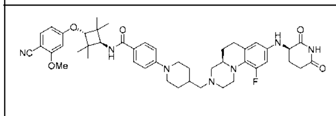 | 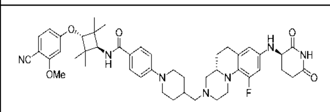 | 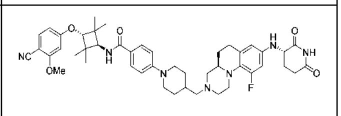 |
| 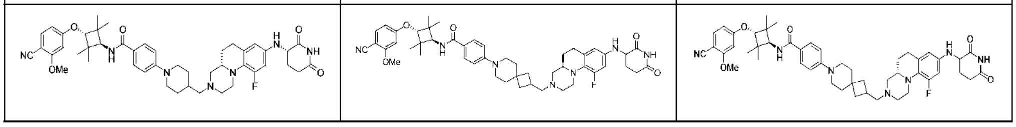 | | |

| | | |
|---|---|---|
| 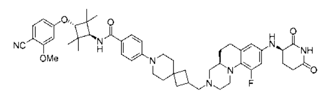 | 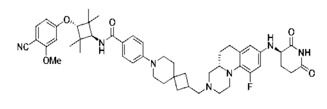 | 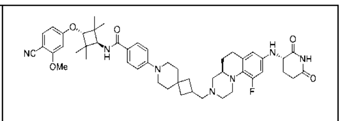 |
| 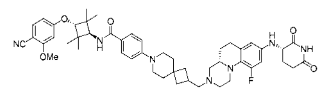 | 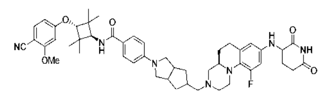 | 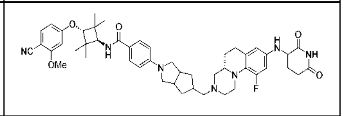 |
| 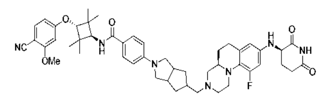 | 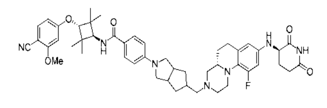 | 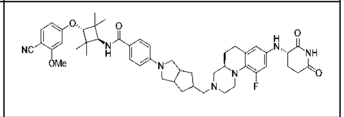 |
| 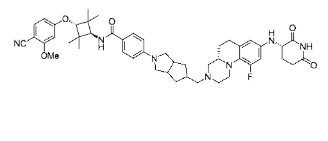 | 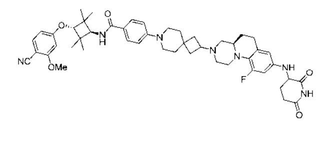 | 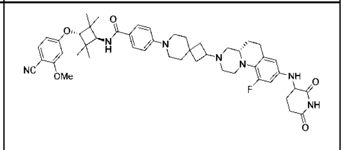 |
| 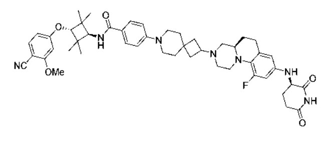 | 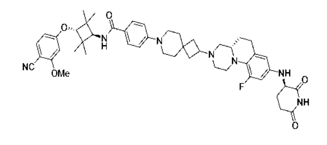 | 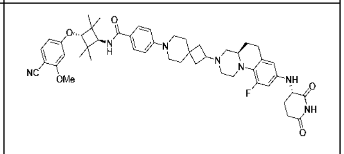 |
| 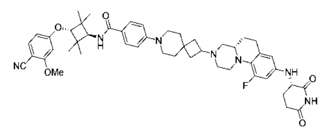 | 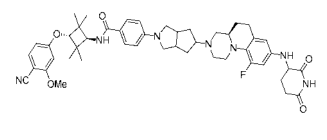 | 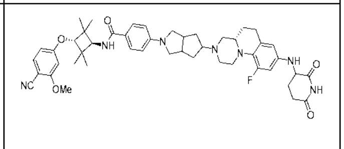 |
| 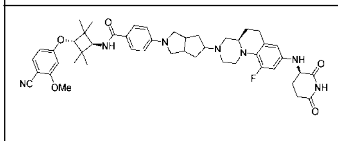 | 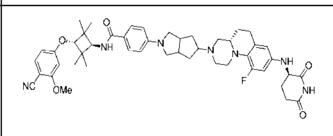 | 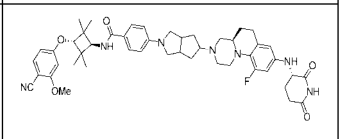 |
| 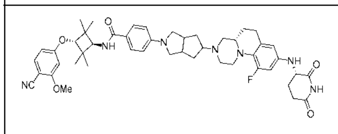 | 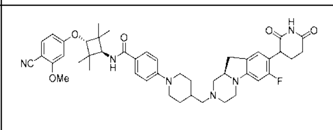 | 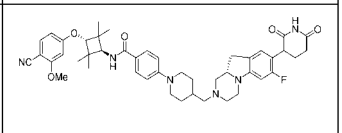 |
| 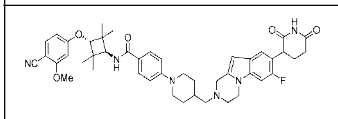 | 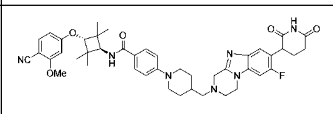 | 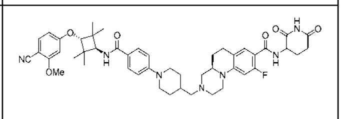 |
| 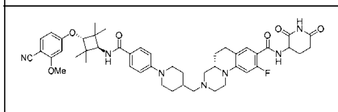 | 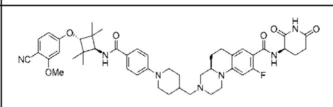 | 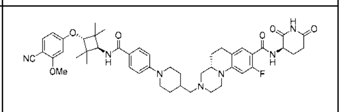 |
| 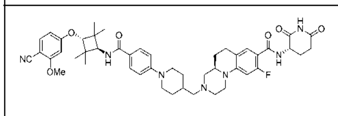 | 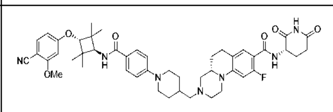 | 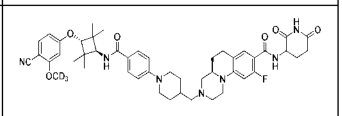 |
| 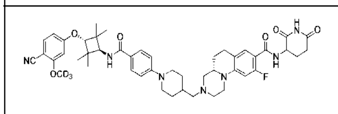 | 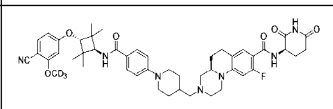 | 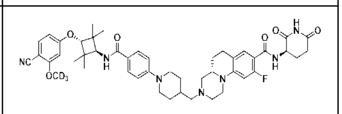 |
| 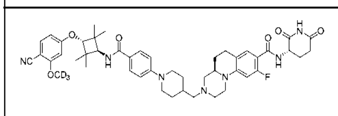 | 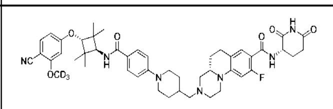 | 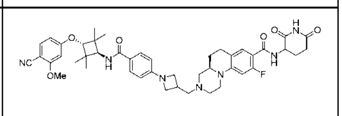 |

| | | |
|---|---|---|
| 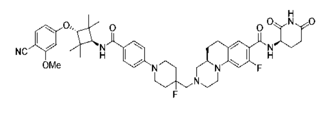 | 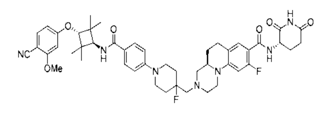 | 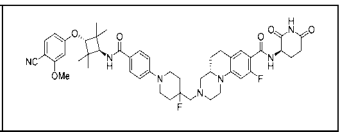 |
| 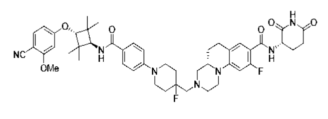 | 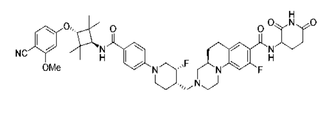 | 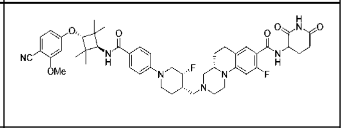 |
| 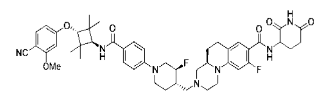 | 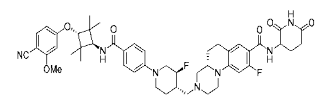 | 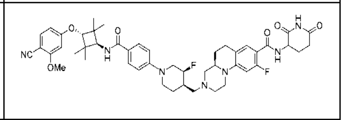 |
| 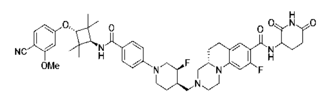 | 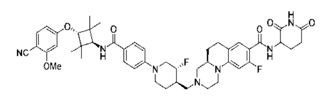 | 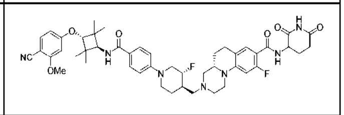 |
| 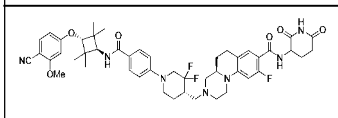 | 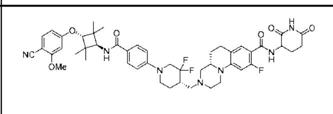 | 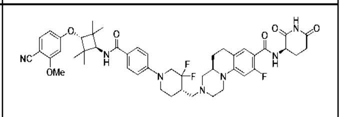 |
| 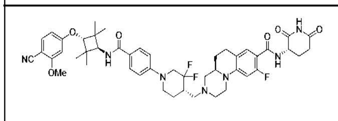 | 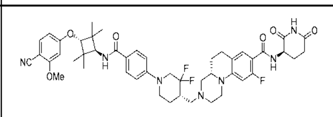 | 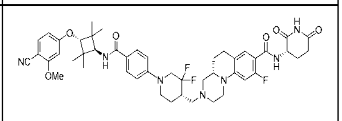 |
| 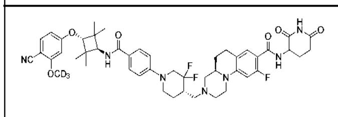 | 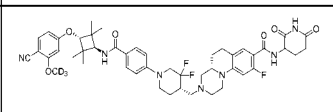 | 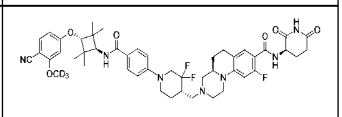 |
| 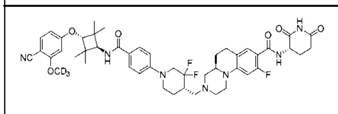 | 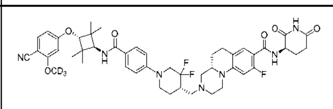 | 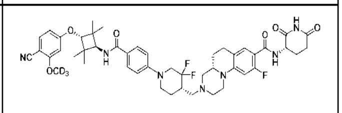 |
| 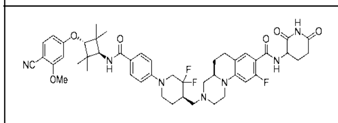 | 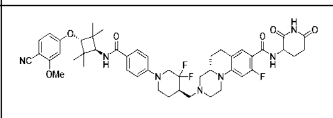 | 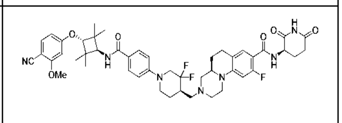 |
| 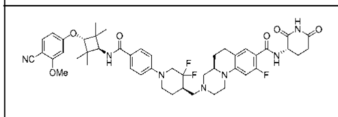 | 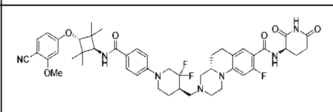 | 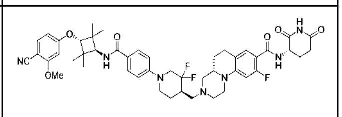 |
| 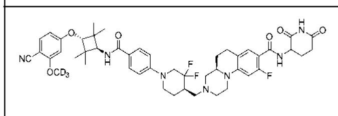 | 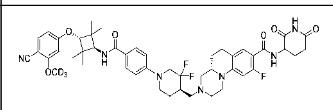 | 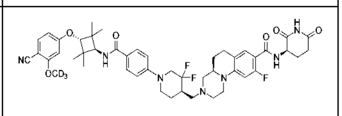 |
| 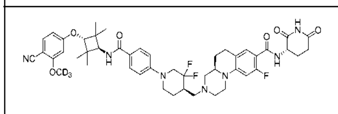 | 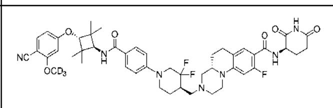 | 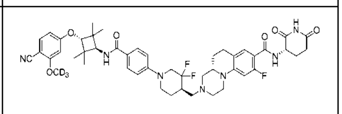 |
| 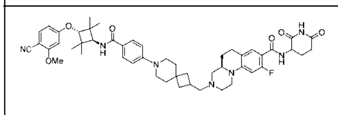 | 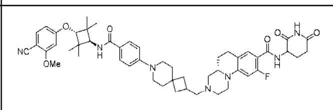 | 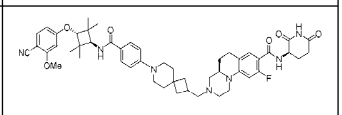 |
| 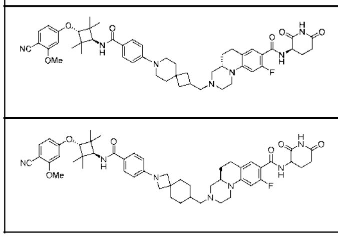 | | |

| | | |
|---|---|---|
| 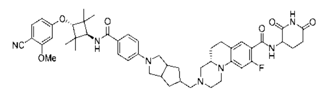 | 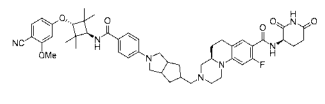 | 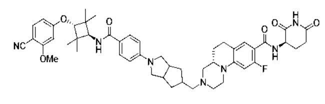 |
| 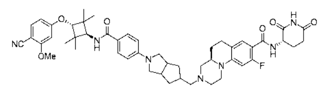 | 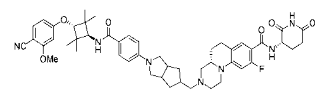 | 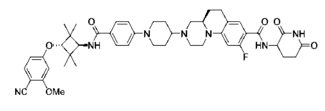 |
| 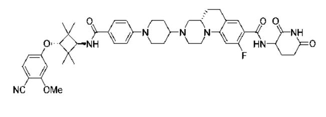 | 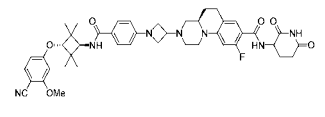 | 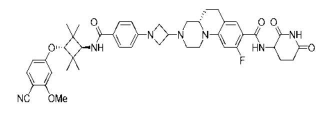 |
| 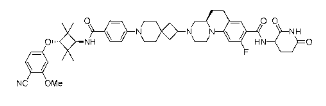 | 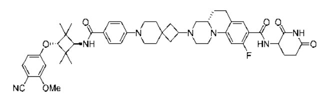 | 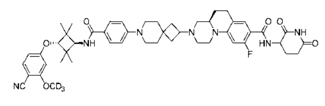 |
| 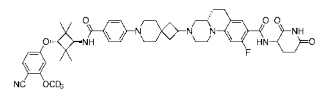 | 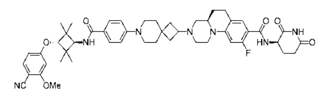 | 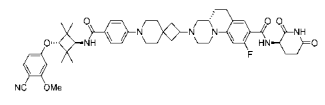 |
| 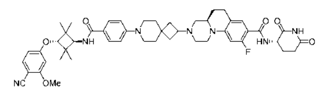 | 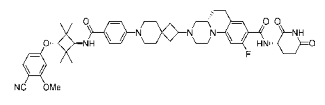 | 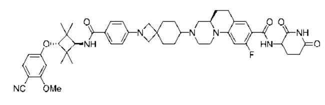 |
| 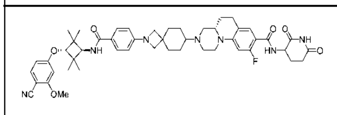 | 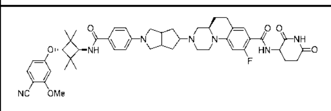 | 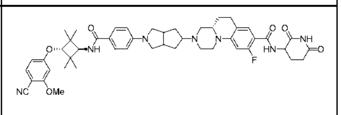 |
| 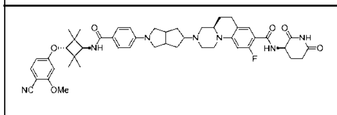 | 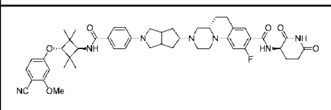 | 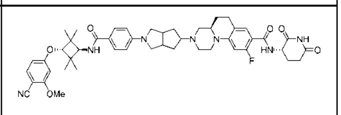 |
| 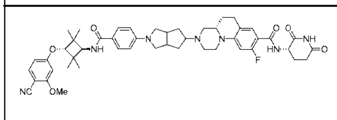 | 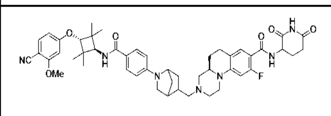 | 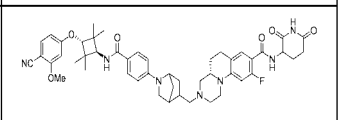 |
| 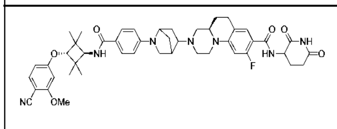 | 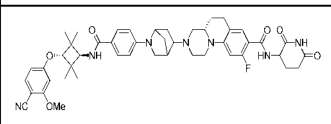 | 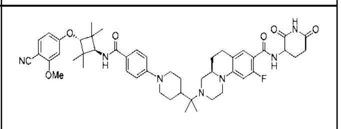 |
| 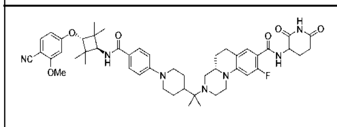 | 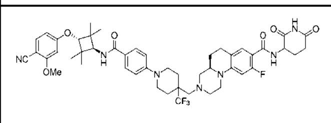 | 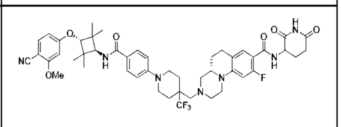 |
| 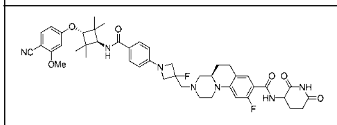 | 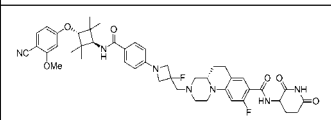 | 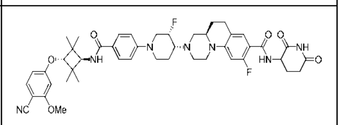 |
| 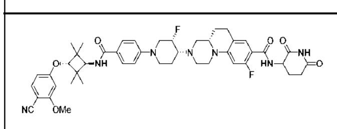 | 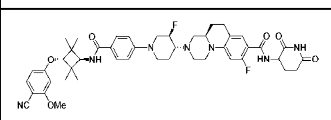 | 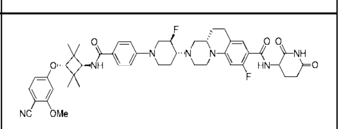 |
| 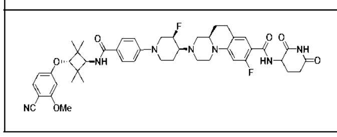 | | |

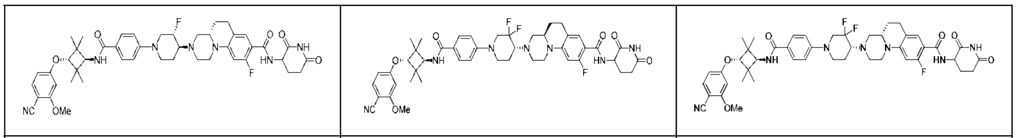

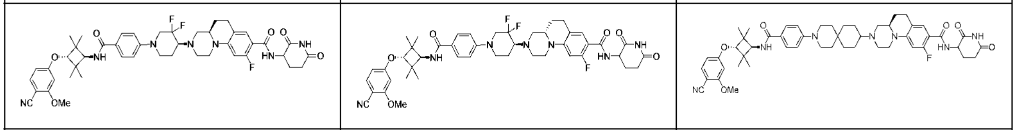

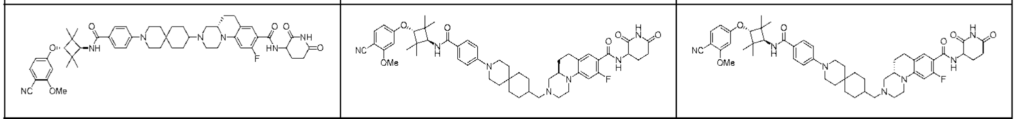

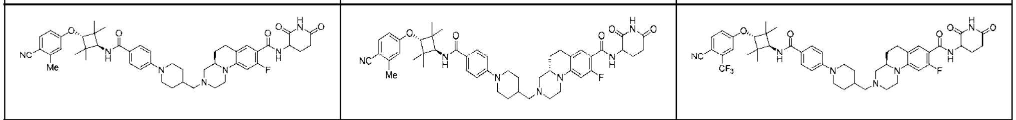

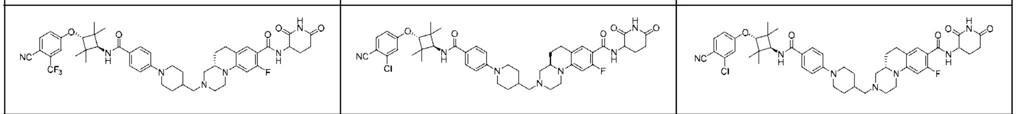

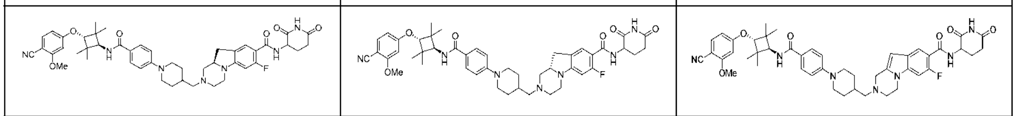

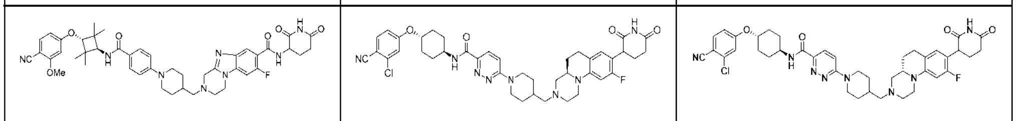

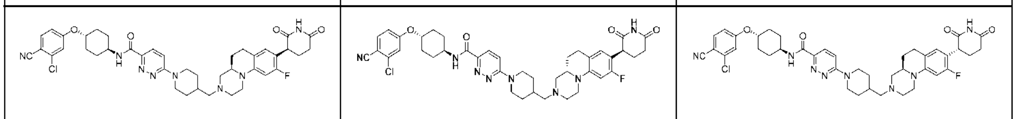

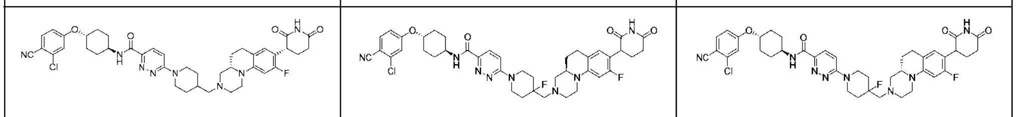

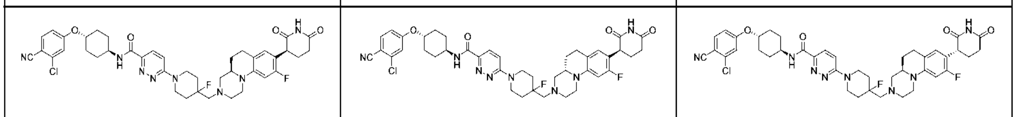

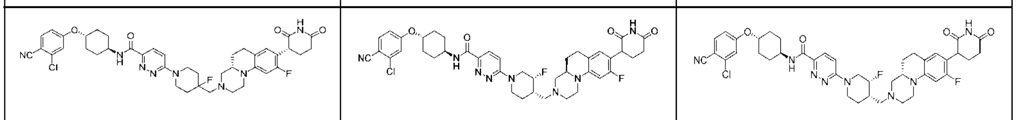

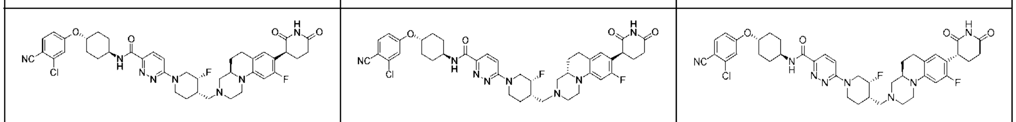

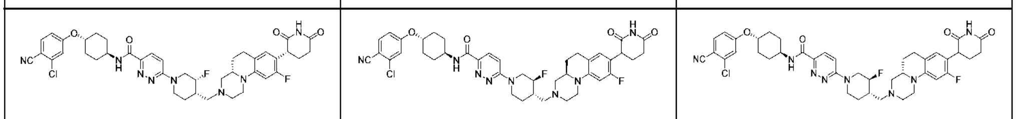

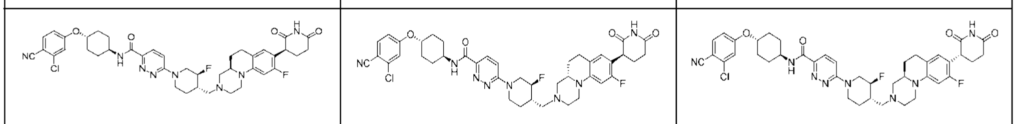

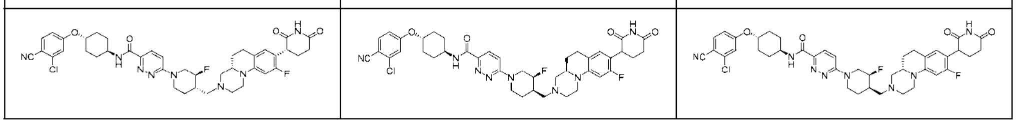

| | | |
|---|---|---|
| 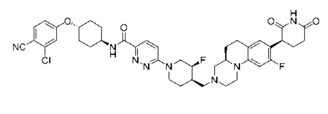 | 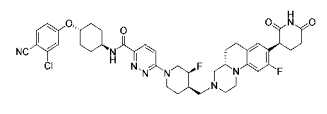 | 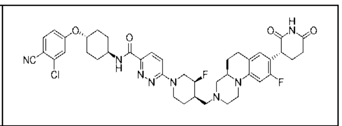 |
| 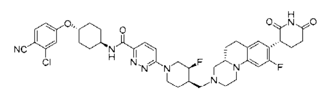 | 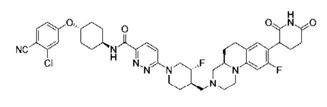 | 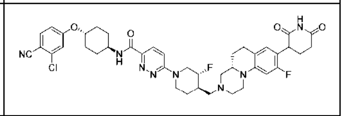 |
| 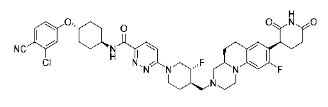 | 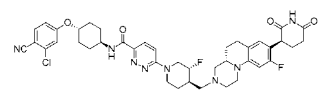 | 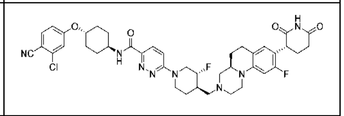 |
| 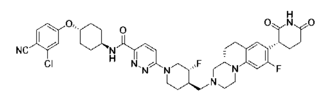 | 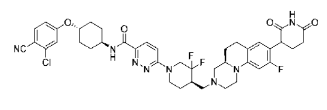 | 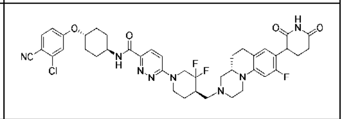 |
| 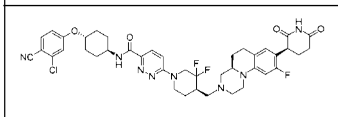 | 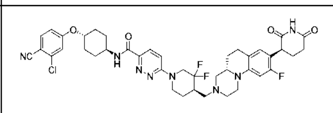 | 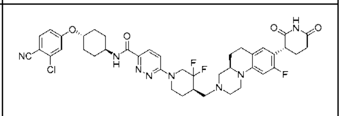 |
| 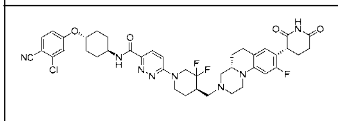 | 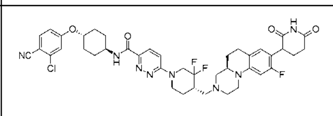 | 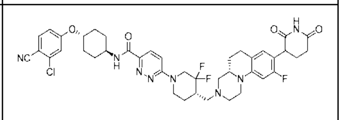 |
| 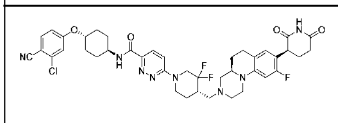 | 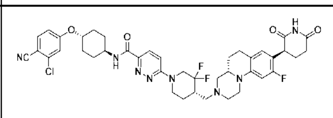 | 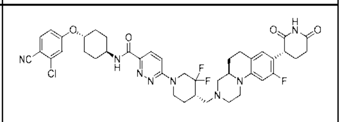 |
| 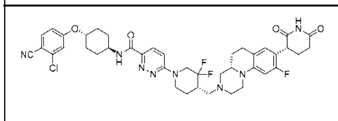 | 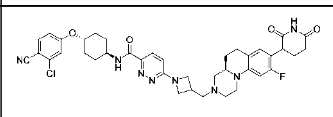 | 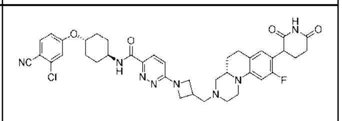 |
| 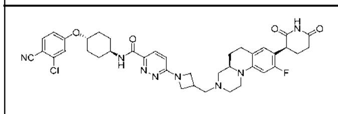 | 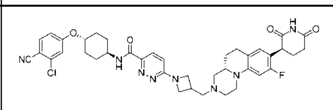 | 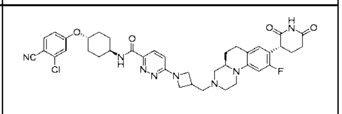 |
| 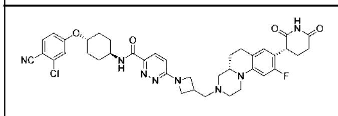 | 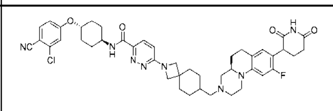 | 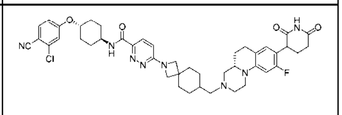 |
| 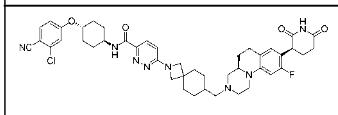 | 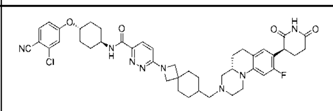 | 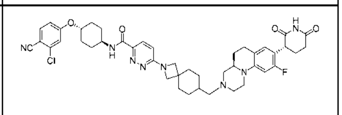 |
| 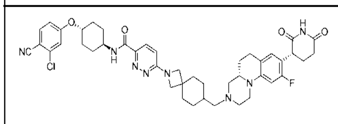 | 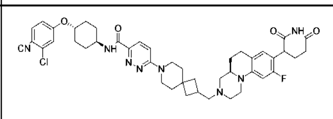 | 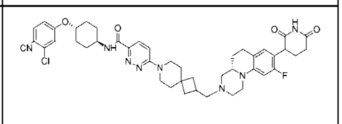 |
| 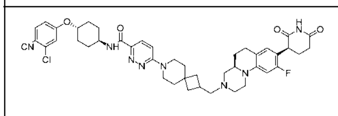 | 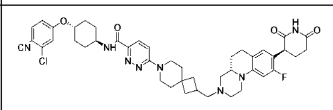 | 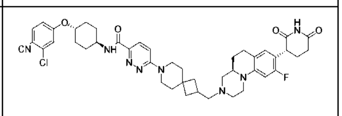 |
| 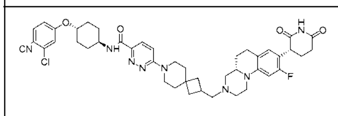 | | |
| | | |

| | | |
|---|---|---|
| 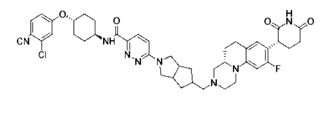 | 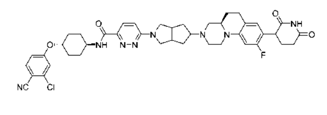 | 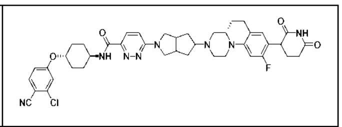 |
| 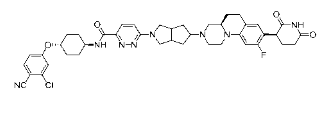 | 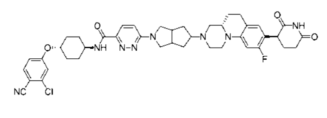 | 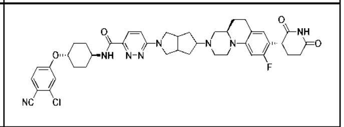 |
| 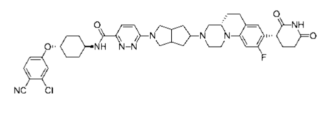 | 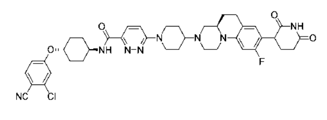 | 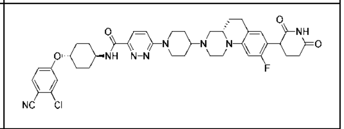 |
| 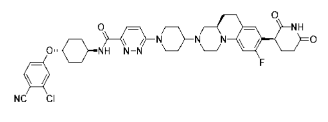 | 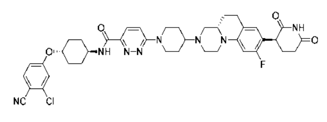 | 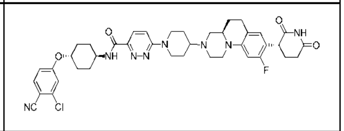 |
| 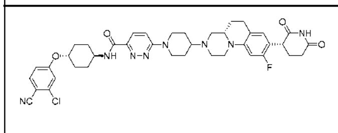 | 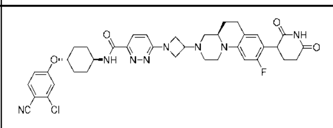 | 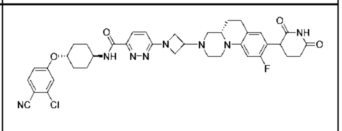 |
| 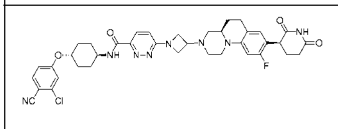 | 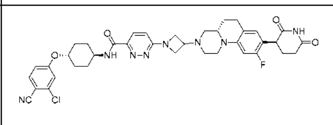 | 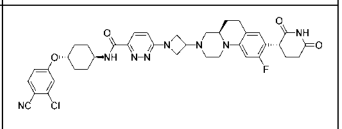 |
| 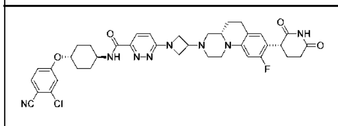 | 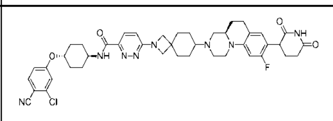 | 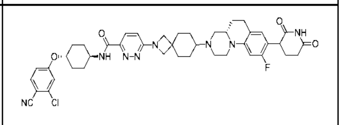 |
| 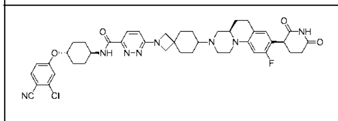 | 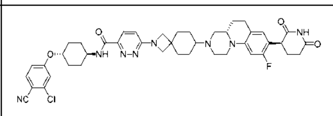 | 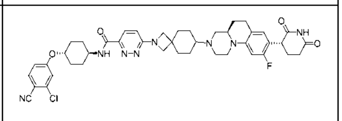 |
| 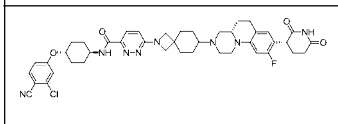 | 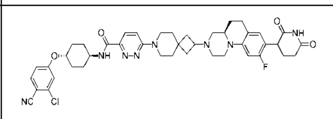 | 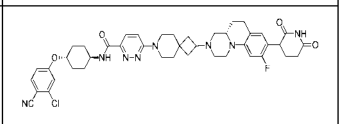 |
| 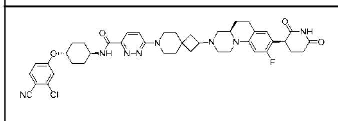 | 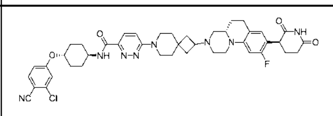 | 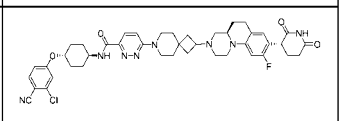 |
| 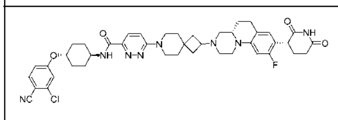 | 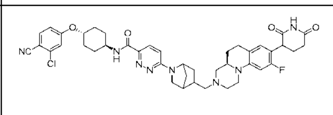 | 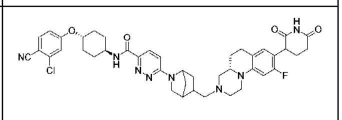 |
| 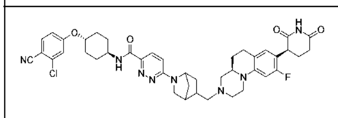 | 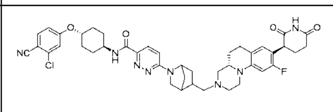 | 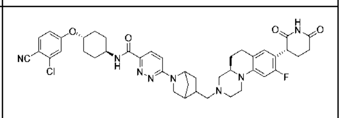 |
| 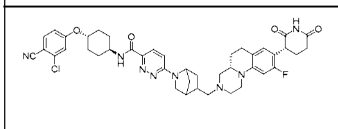 | 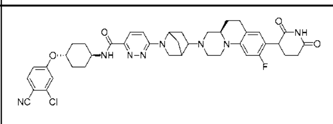 | 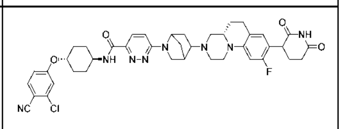 |
| 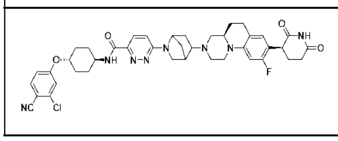 | | |

| | | |
|---|---|---|
| 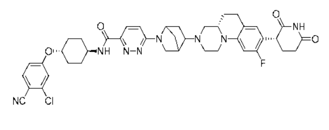 | 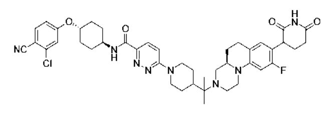 | 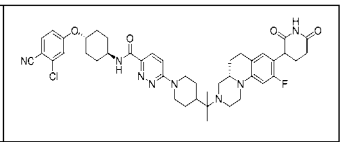 |
| 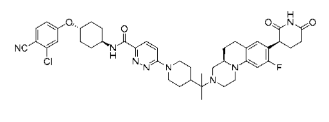 | 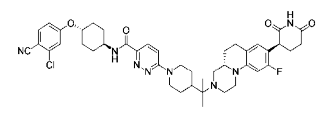 | 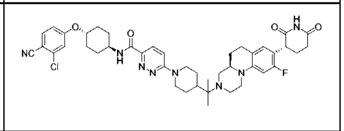 |
| 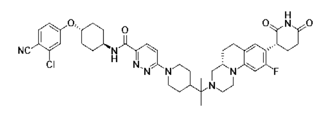 | 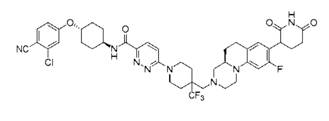 | 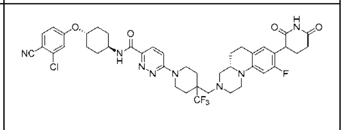 |
| 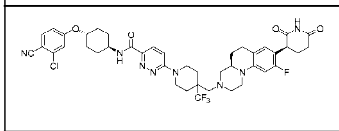 | 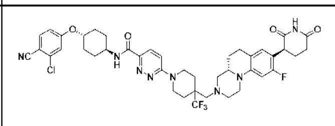 | 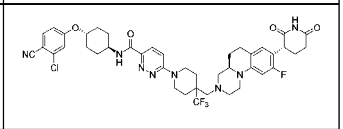 |
| 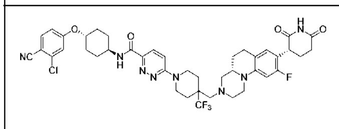 | 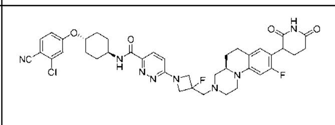 | 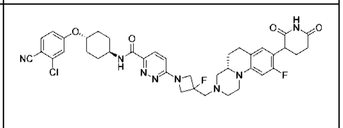 |
| 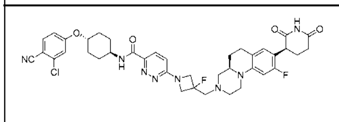 | 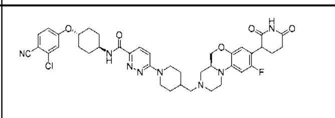 | 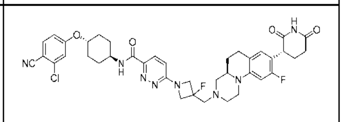 |
| 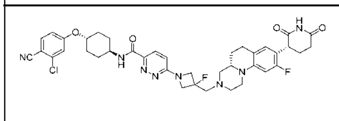 | 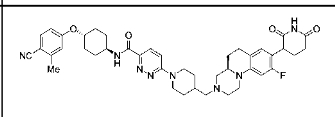 | 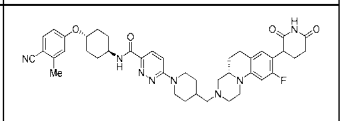 |
| 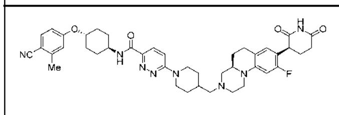 | 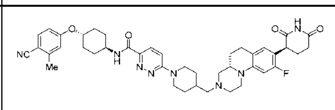 | 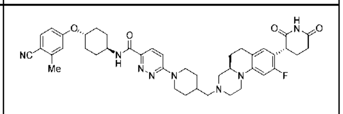 |
| 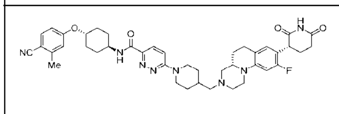 | 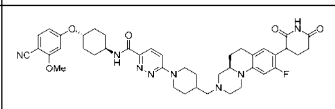 | 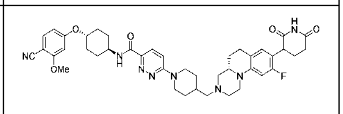 |
| 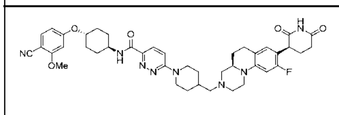 | 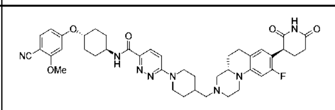 | 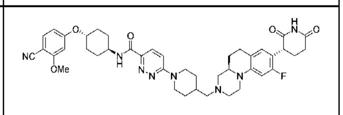 |
| 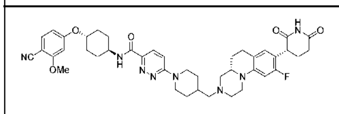 | 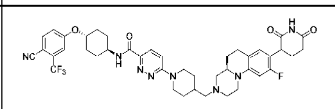 | 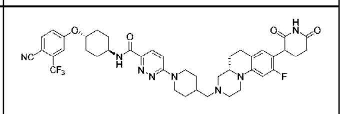 |
| 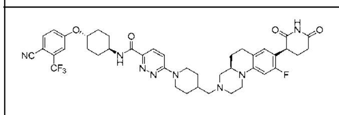 | 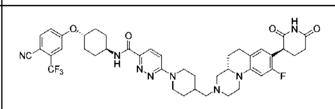 | 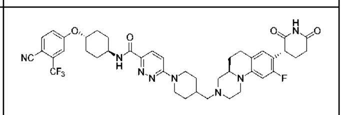 |
| 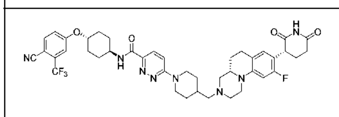 | 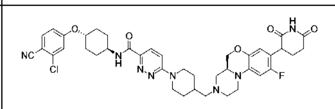 | 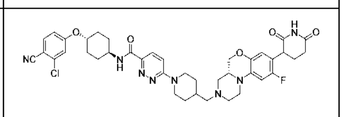 |
| 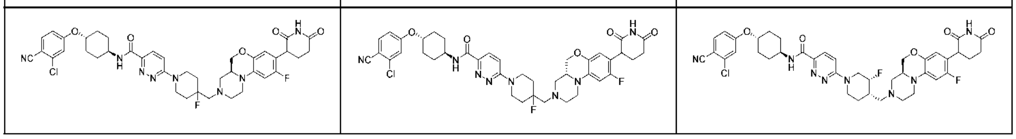 | | |

| | | |
|---|---|---|
| 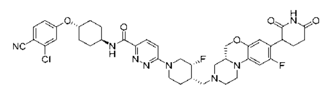 | 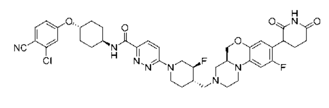 | 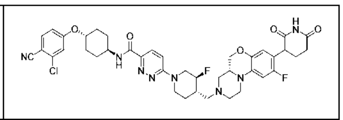 |
| 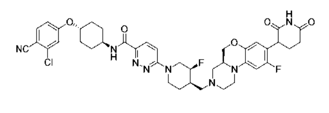 | 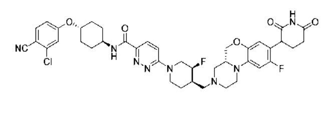 | 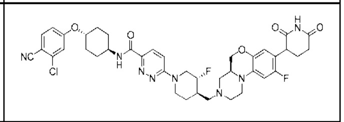 |
| 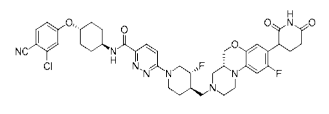 | 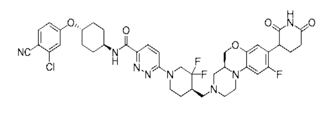 | 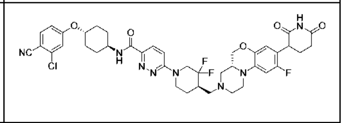 |
| 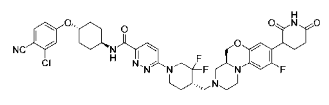 | 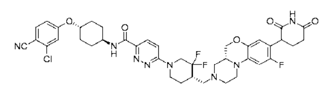 | 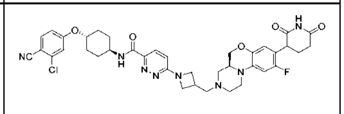 |
| 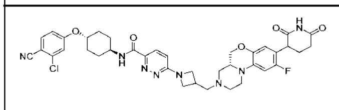 | 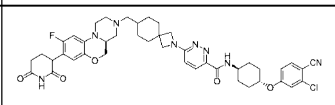 | 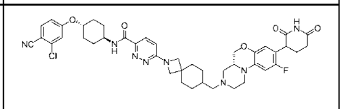 |
| 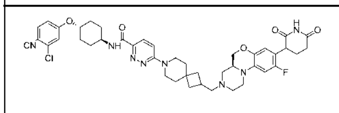 | 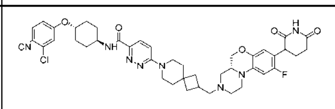 | 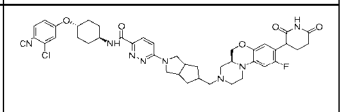 |
| 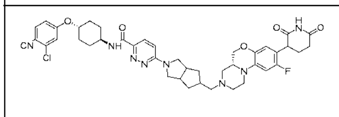 | 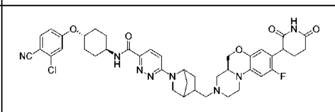 | 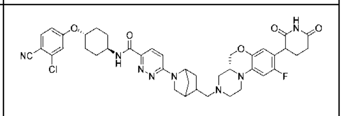 |
| 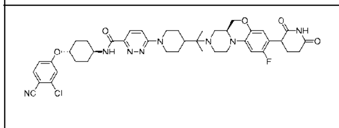 | 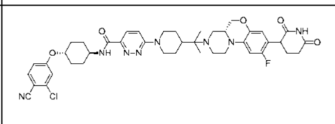 | 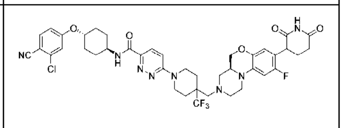 |
| 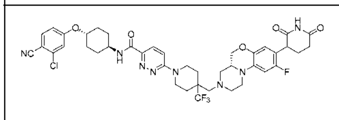 | 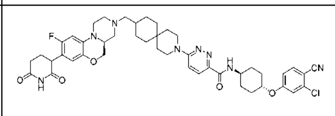 | 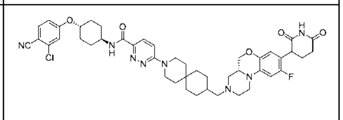 |
| 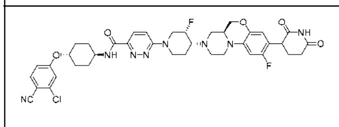 | 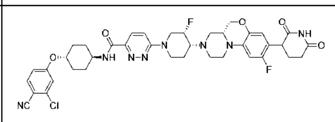 | 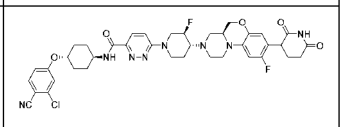 |
| 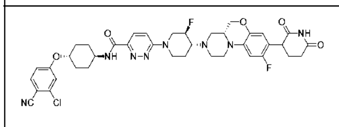 | 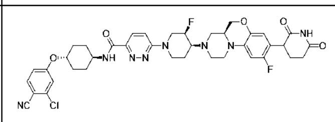 | 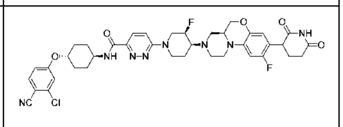 |
| 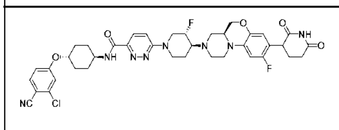 | 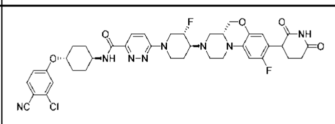 | 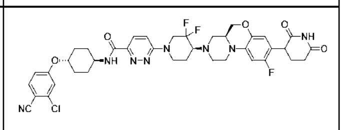 |
| 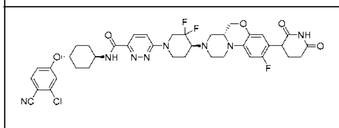 | 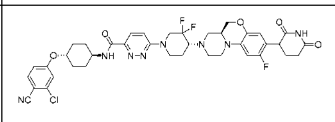 | 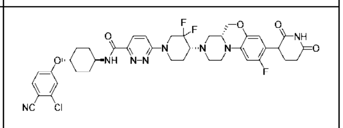 |
| 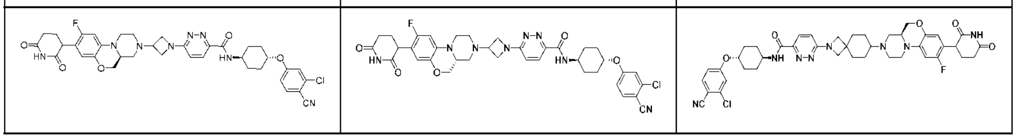 | | |

| | | |
|---|---|---|
| 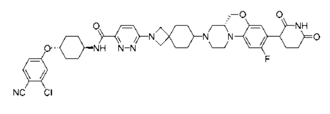 | 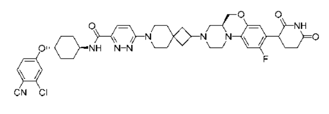 | 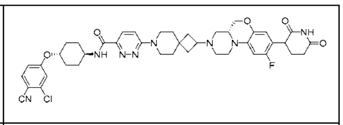 |
| 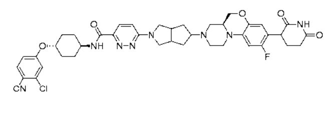 | 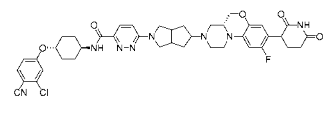 | 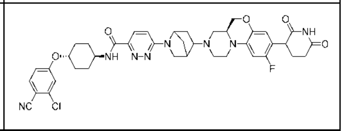 |
| 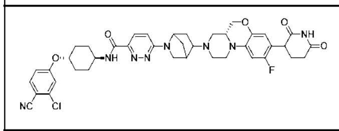 | 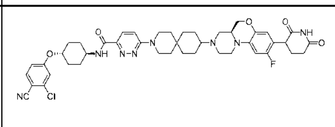 | 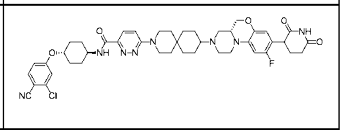 |
| 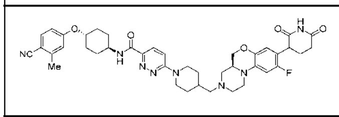 | 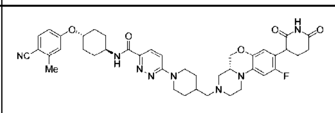 | 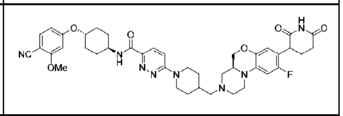 |
| 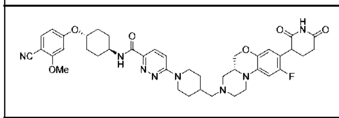 | 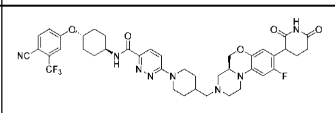 | 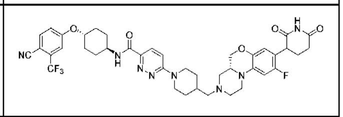 |
| 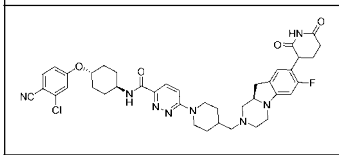 | 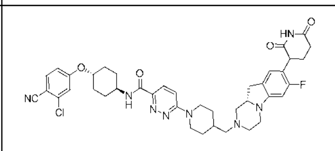 | 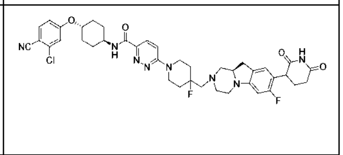 |
| 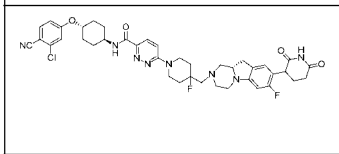 | 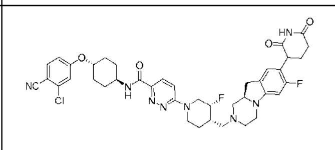 | 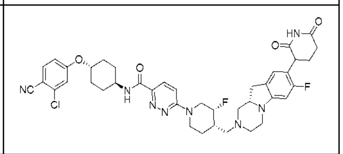 |
| 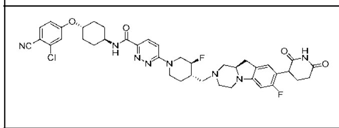 | 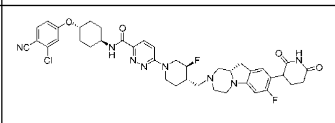 | 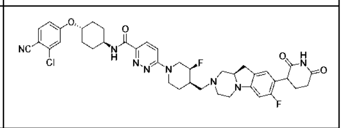 |
| 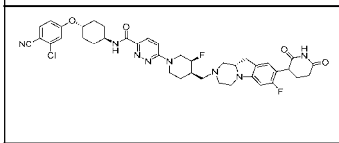 | 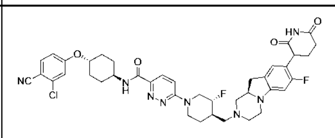 | 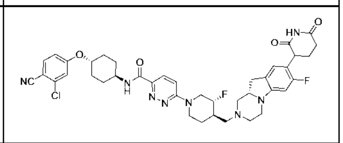 |
| 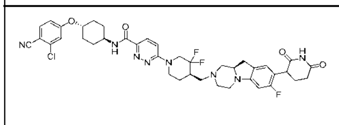 | 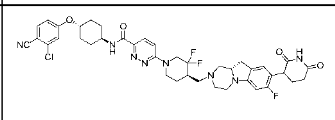 | 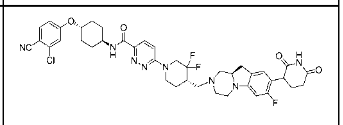 |
| 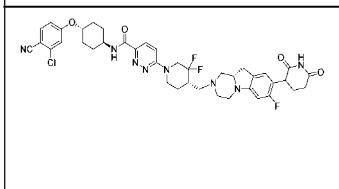 | 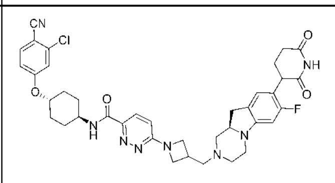 | 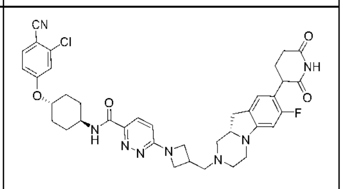 |
| 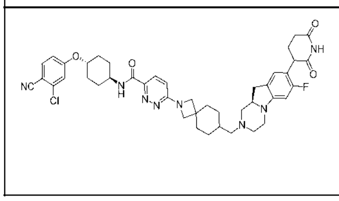 | 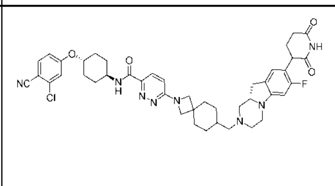 | 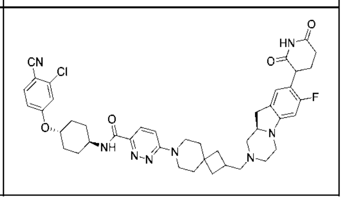 |

| | | |
|---|---|---|
| 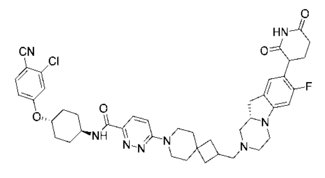 | 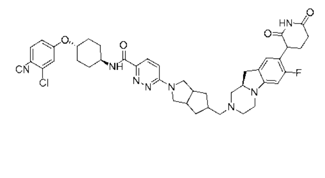 | 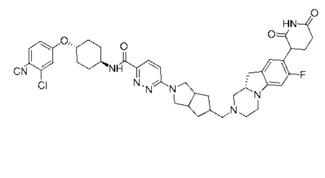 |
| 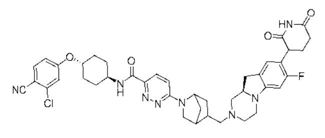 | 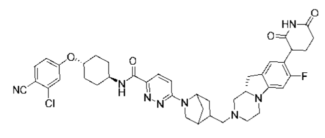 | 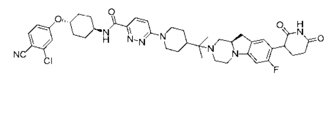 |
| 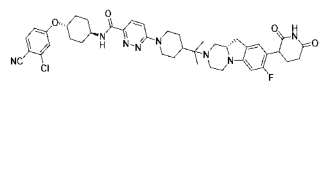 | 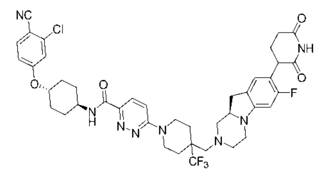 | 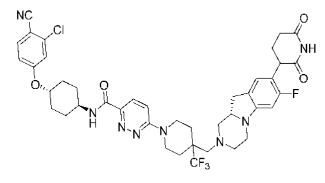 |
| 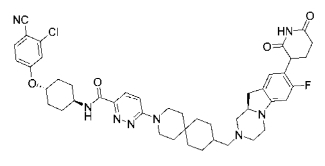 | 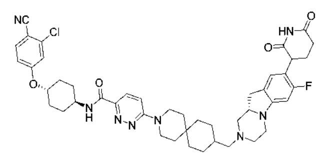 | 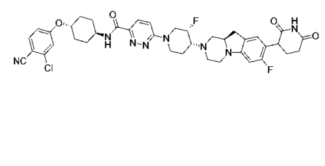 |
| 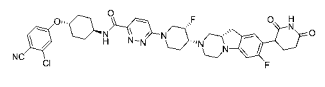 | 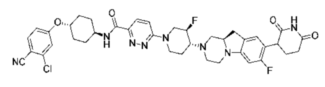 | 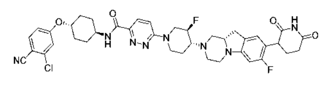 |
| 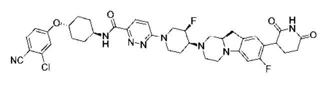 | 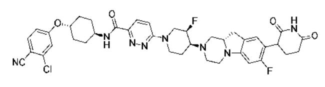 | 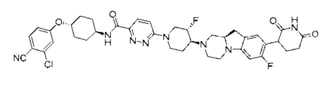 |
| 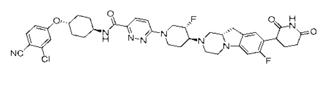 | 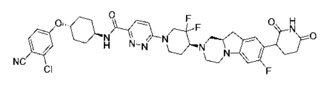 | 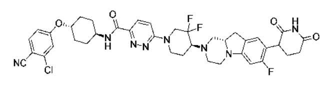 |
| 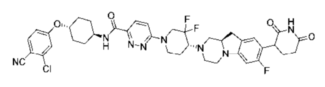 | 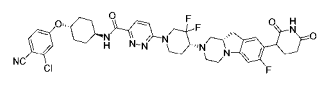 | 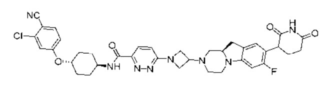 |
| 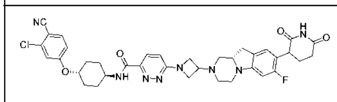 | 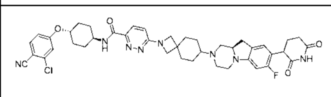 | 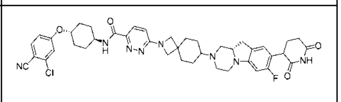 |
| 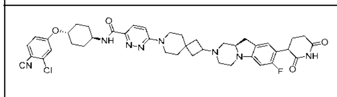 | 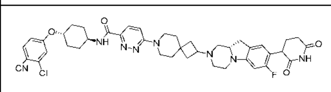 | 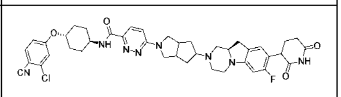 |
| 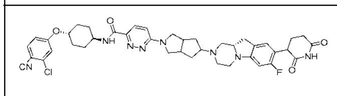 | 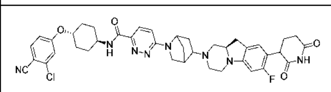 | 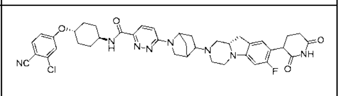 |
| 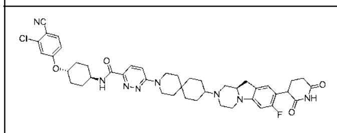 | 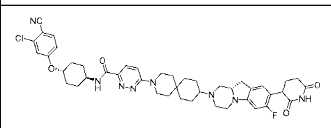 | 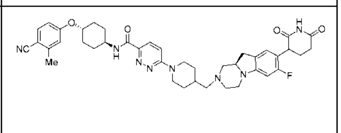 |
| 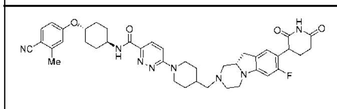 | 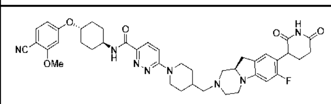 | 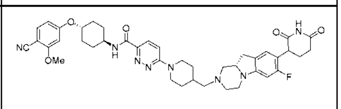 |
| 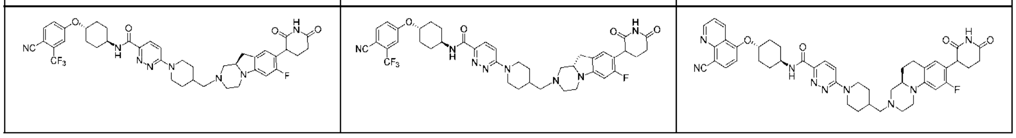 | | |

| | | |
|---|---|---|
| 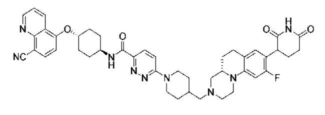 | 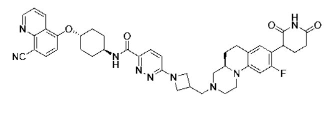 | 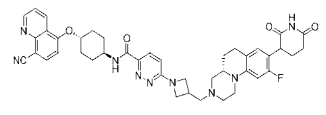 |
| 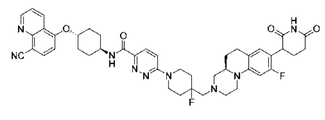 | 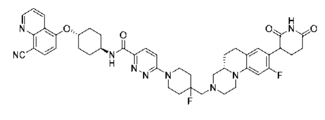 | 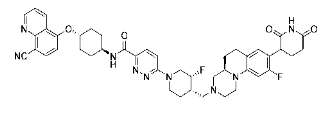 |
| 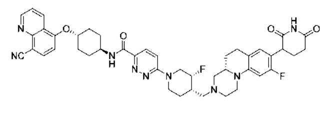 | 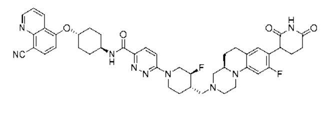 | 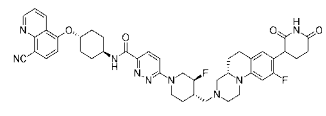 |
| 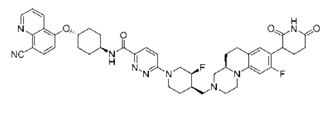 | 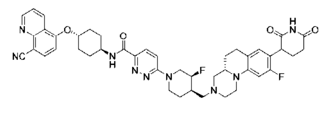 | 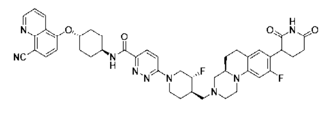 |
| 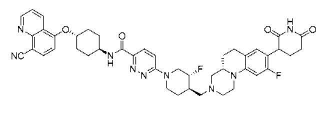 | 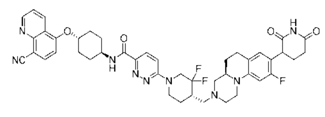 | 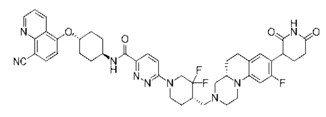 |
| 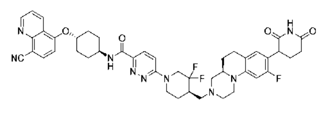 | 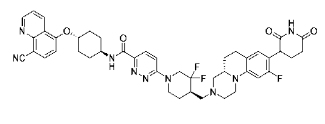 | 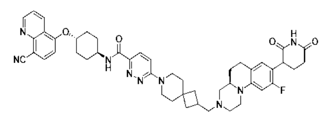 |
| 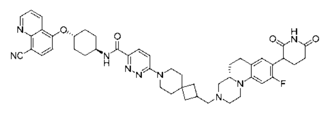 | 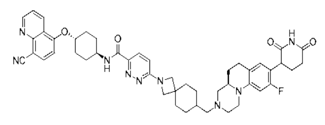 | 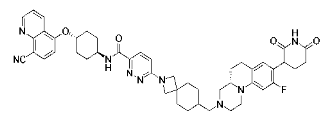 |
| 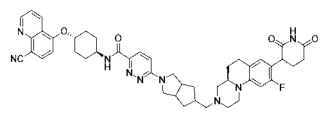 | 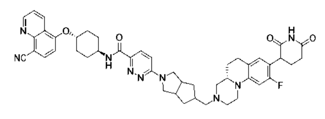 | 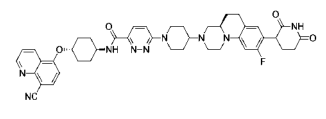 |
| 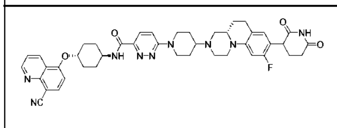 | 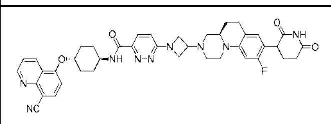 | 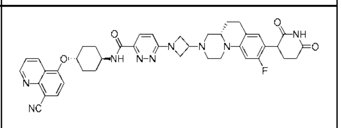 |
| 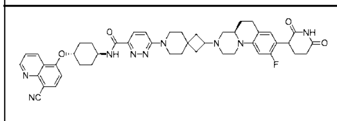 | 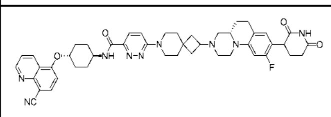 | 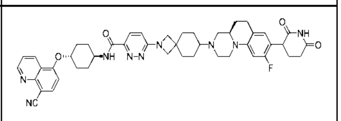 |
| 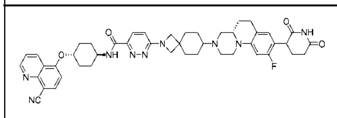 | 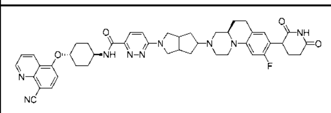 | 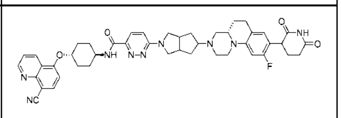 |
| 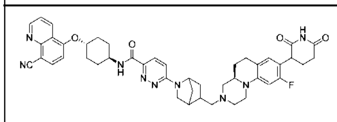 | 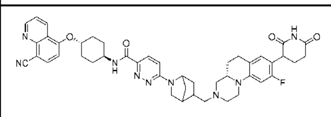 | 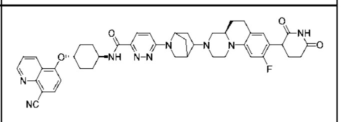 |
| 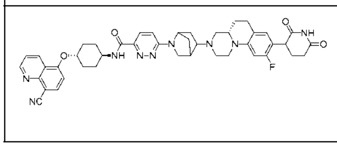 | 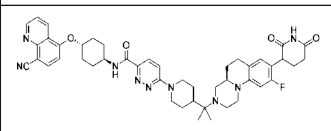 | 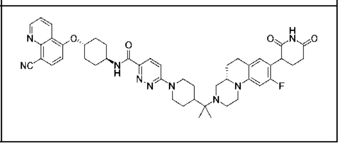 |

| | | |
|---|---|---|
| 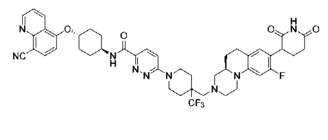 | 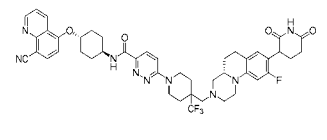 | 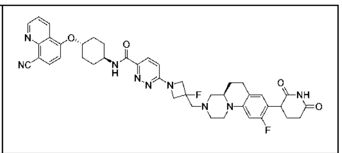 |
| 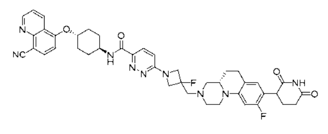 | 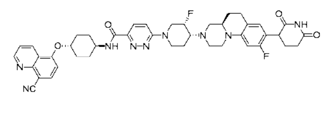 | 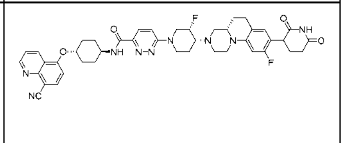 |
| 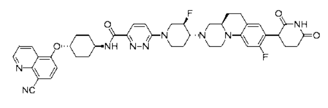 | 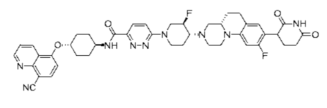 | 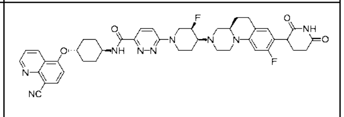 |
| 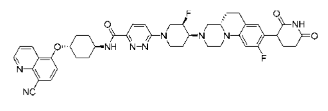 | 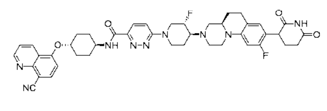 | 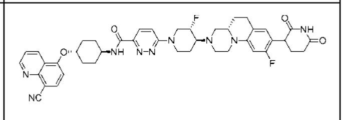 |
| 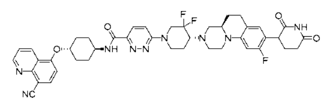 | 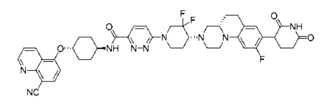 | 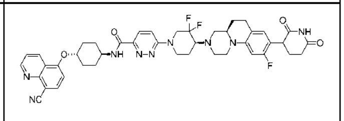 |
| 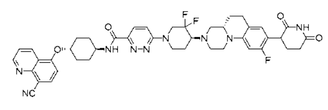 | 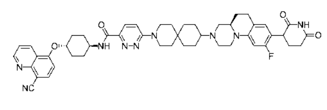 | 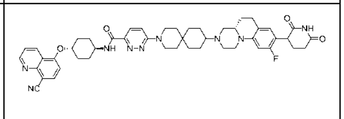 |
| 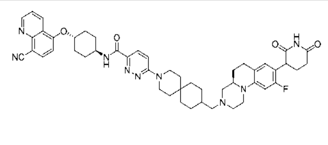 | 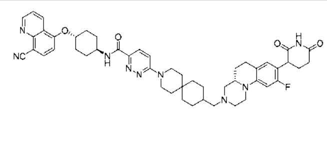 | 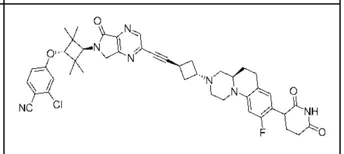 |
| 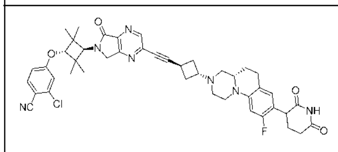 | 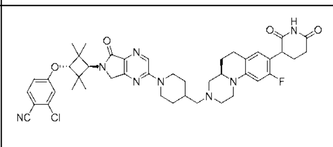 | 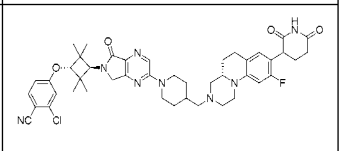 |
| 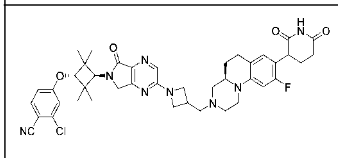 | 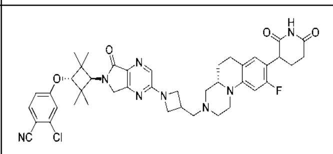 | 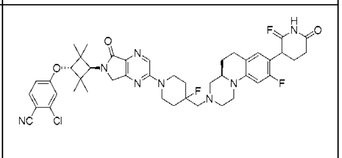 |
| 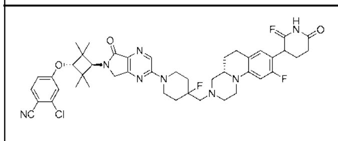 | 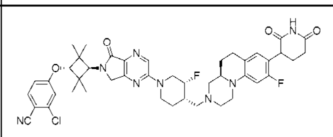 | 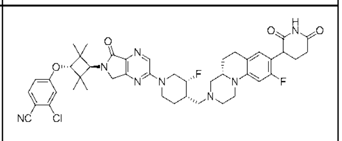 |
| 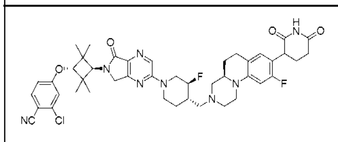 | 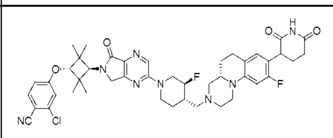 | 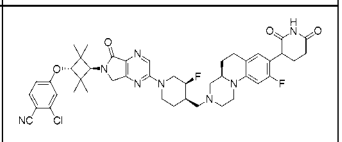 |
| 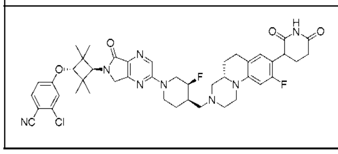 | 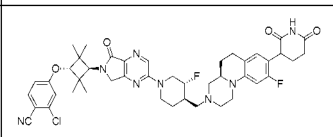 | 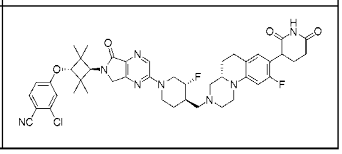 |

| | | |
|---|---|---|
| 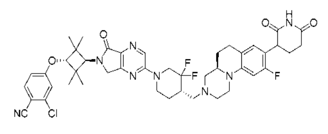 | 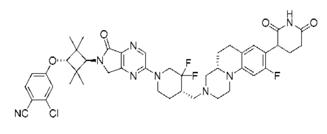 | 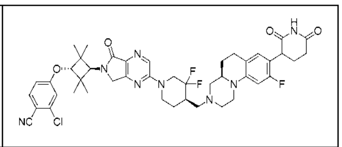 |
| 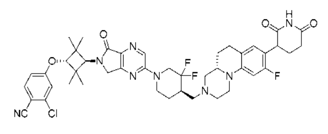 | 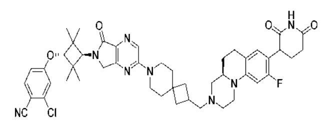 | 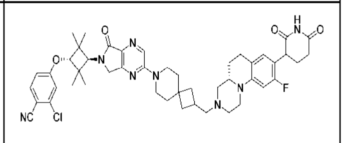 |
| 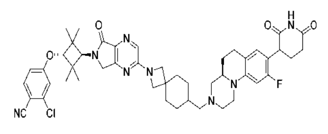 | 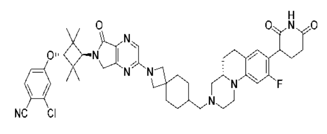 | 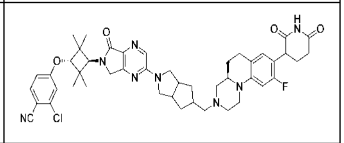 |
| 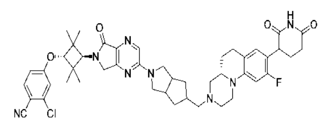 | 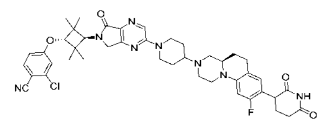 | 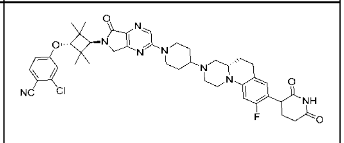 |
| 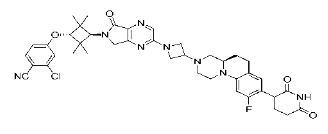 | 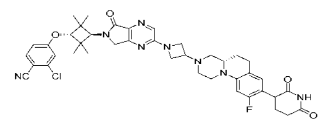 | 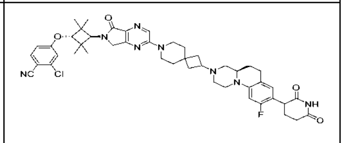 |
| 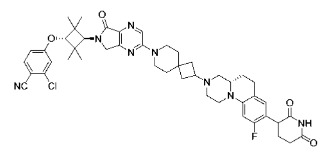 | 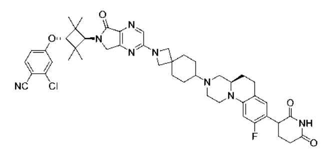 | 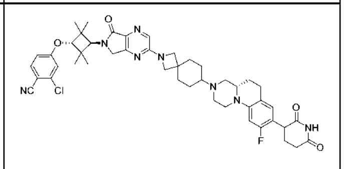 |
| 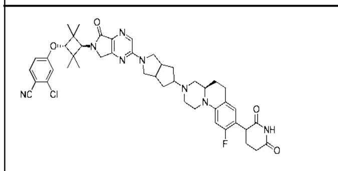 | 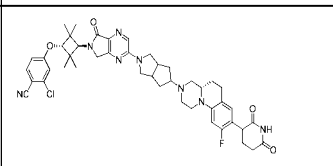 | 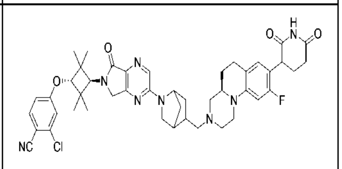 |
| 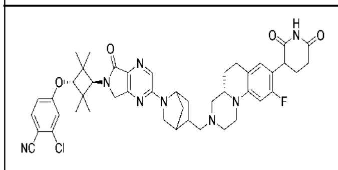 | 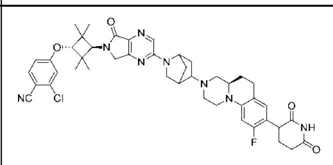 | 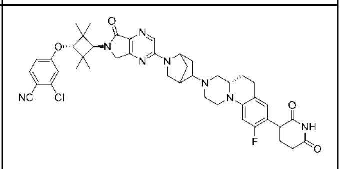 |
| 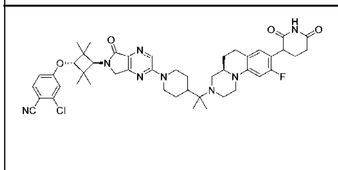 | 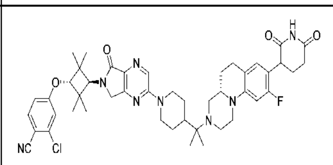 | 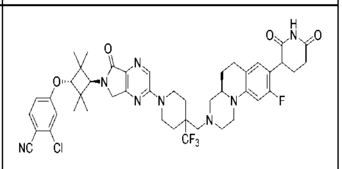 |
| 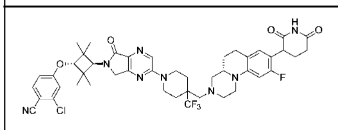 | 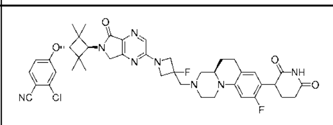 | 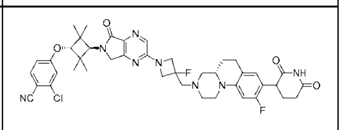 |
| 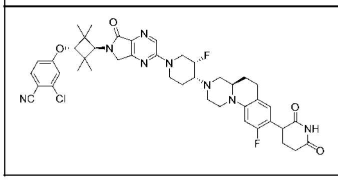 | 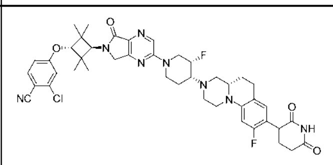 | 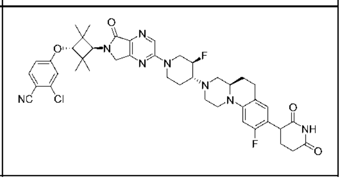 |

| | | |
|---|---|---|
| 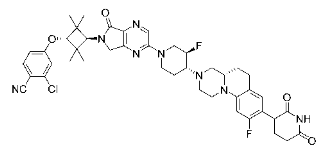 | 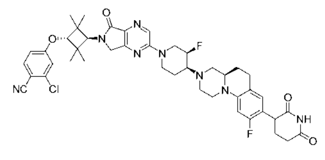 | 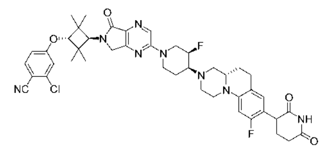 |
| 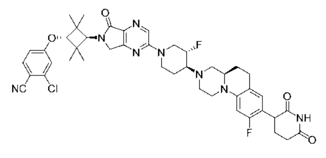 | 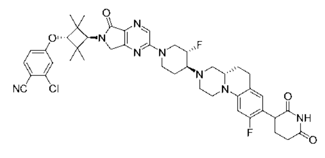 | 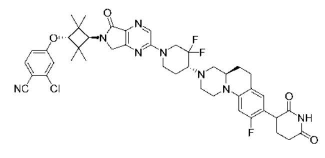 |
| 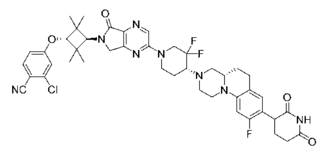 | 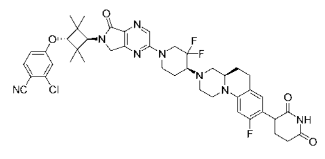 | 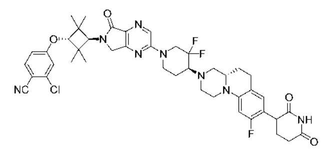 |
| 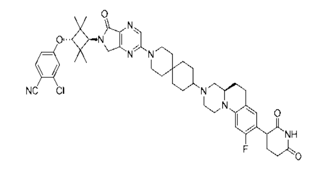 | 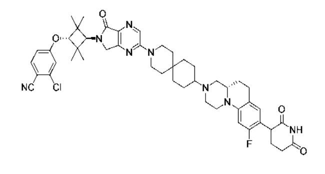 | 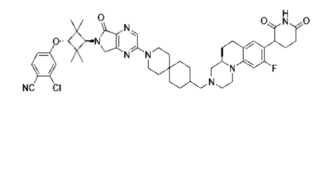 |
| 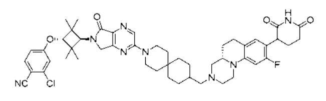 | 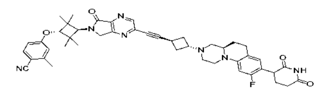 | 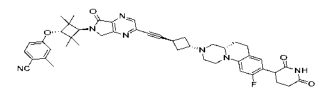 |
| 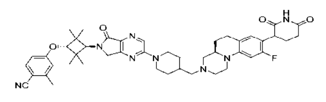 | 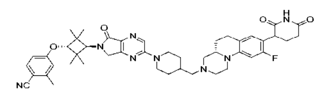 | 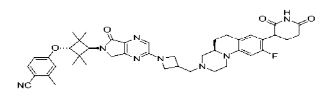 |
| 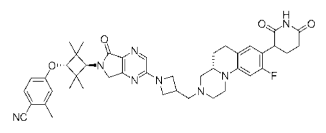 | 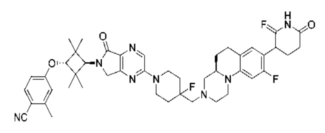 | 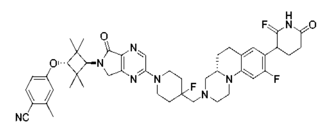 |
| 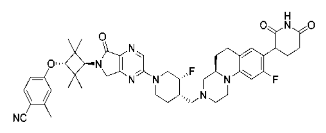 | 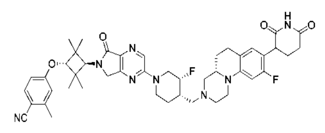 | 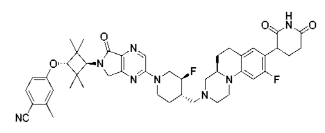 |
| 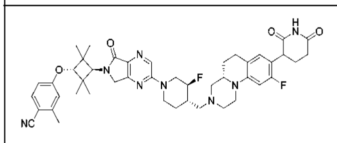 | 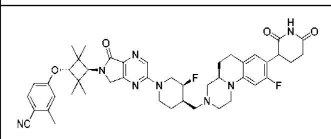 | 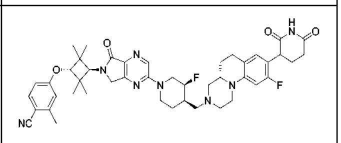 |
| 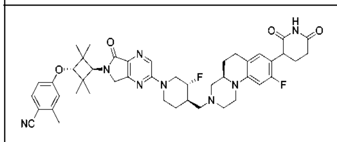 | 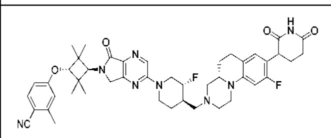 | 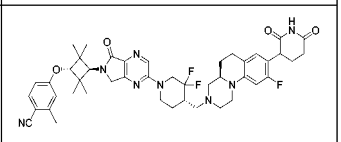 |
| 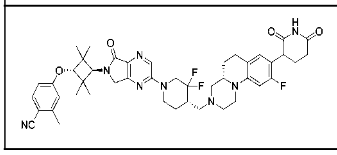 | 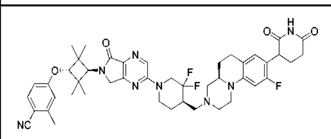 | 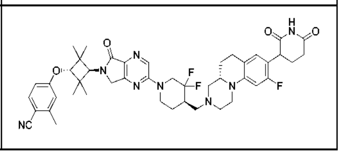 |

| | | |
|---|---|---|
| 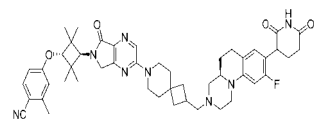 | 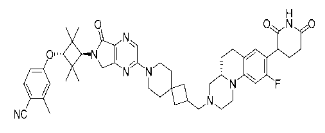 | 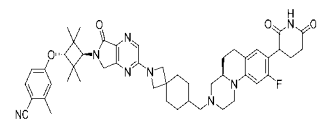 |
| 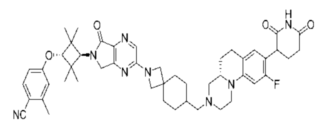 | 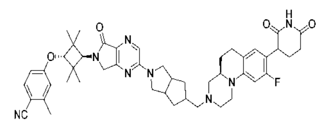 | 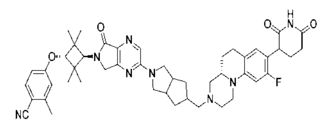 |
| 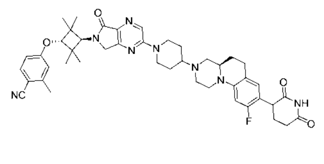 | 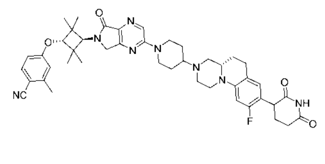 | 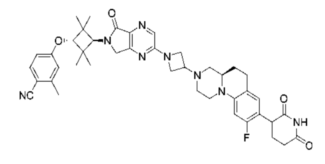 |
| 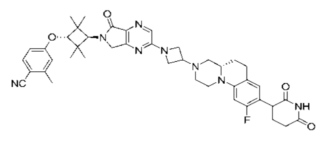 | 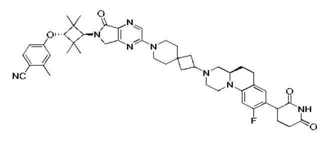 | 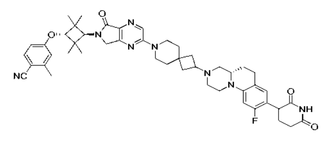 |
| 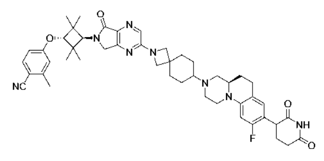 | 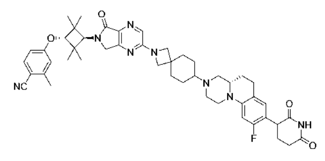 | 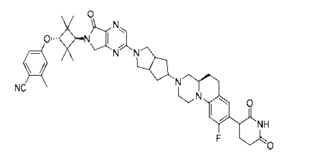 |
| 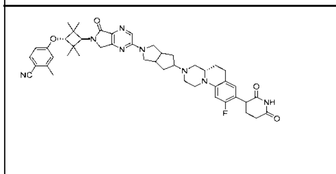 | 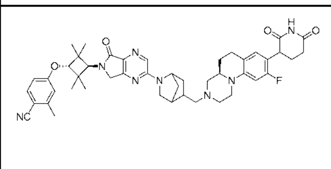 | 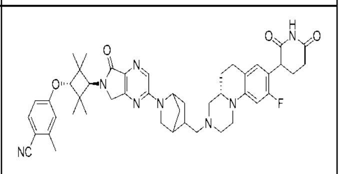 |
| 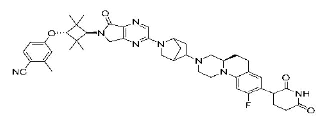 | 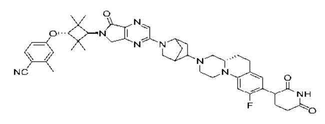 | 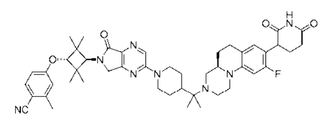 |
| 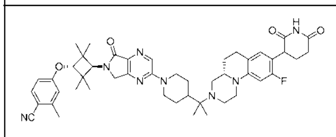 | 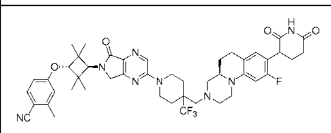 | 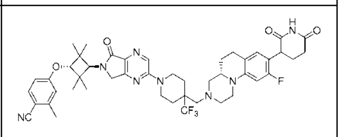 |
| 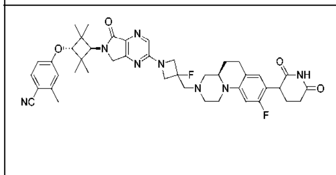 | 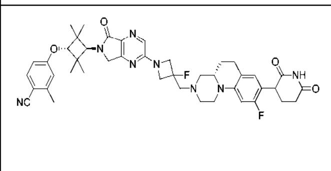 | 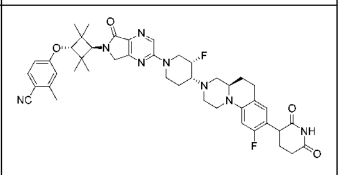 |
| 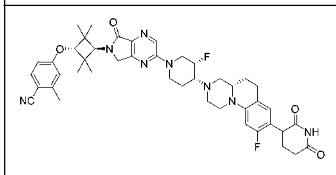 | 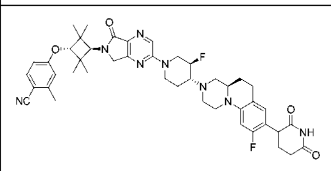 | 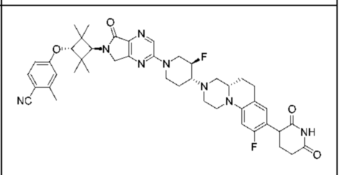 |
| 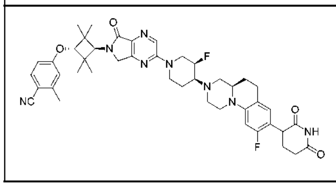 | 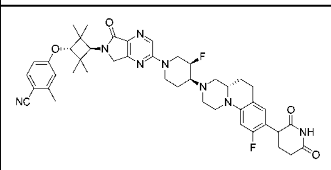 | 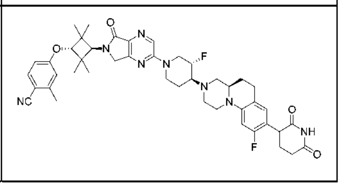 |

| | | |
|---|---|---|
| 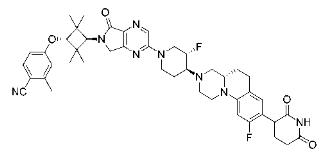 | 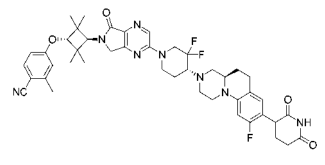 | 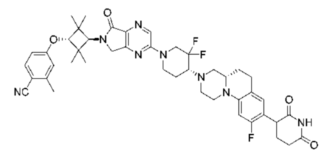 |
| 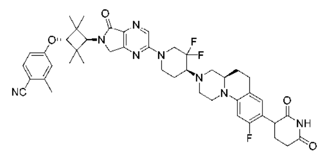 | 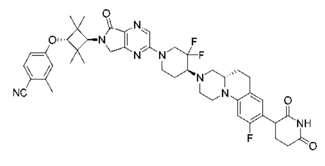 | 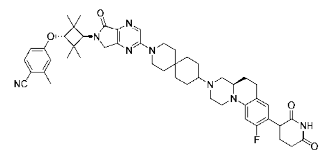 |
| 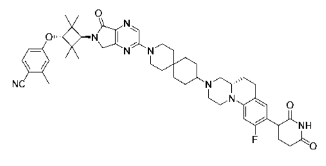 | 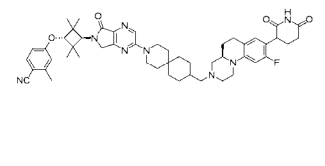 | 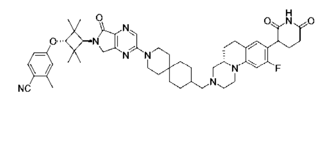 |
| 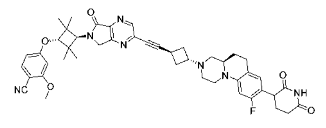 | 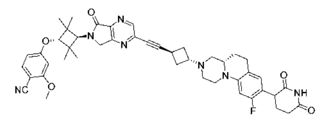 | 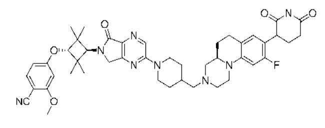 |
| 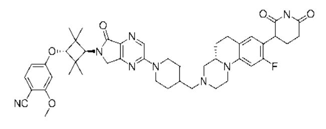 | 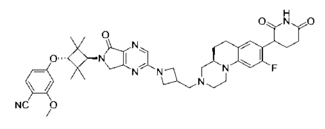 | 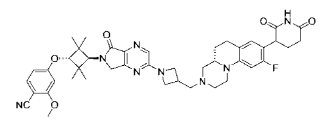 |
| 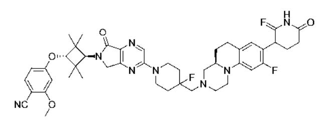 | 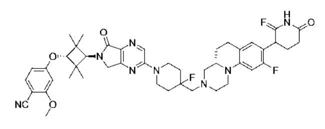 | 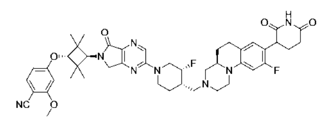 |
| 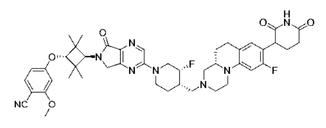 | 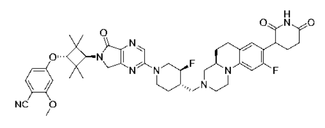 | 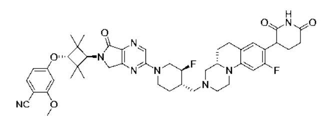 |
| 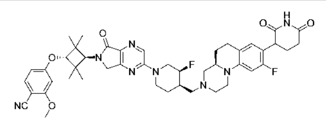 | 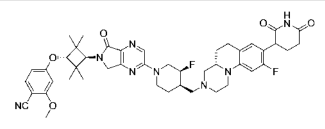 | 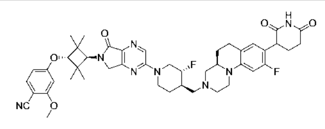 |
| 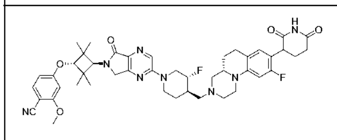 | 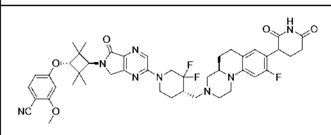 | 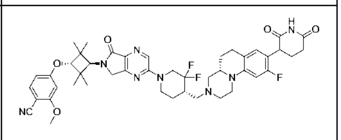 |
| 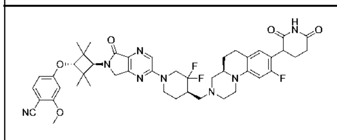 | 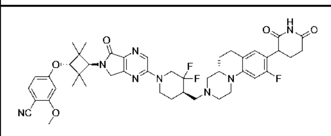 | 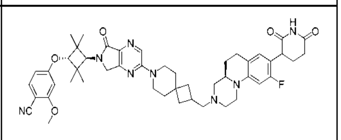 |
| 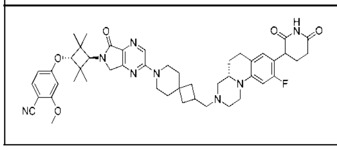 | 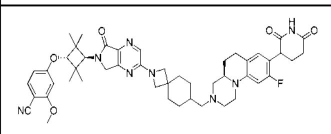 | 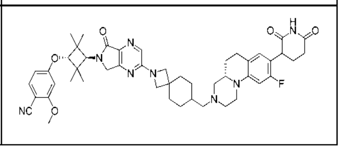 |

| | | |
|---|---|---|
| 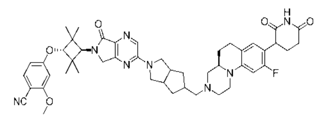 | 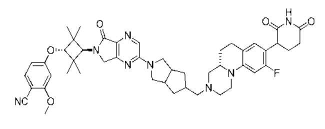 | 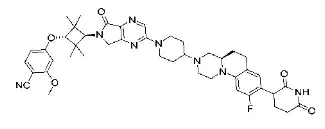 |
| 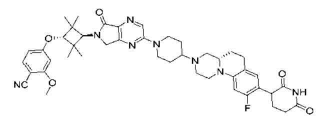 | 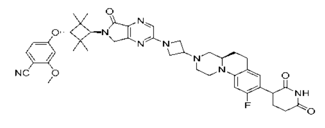 | 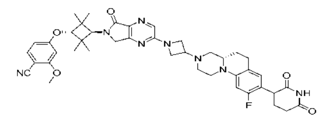 |
| 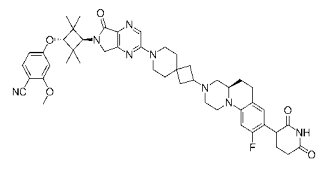 | 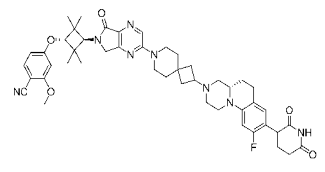 | 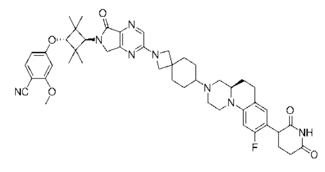 |
| 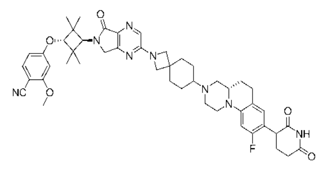 | 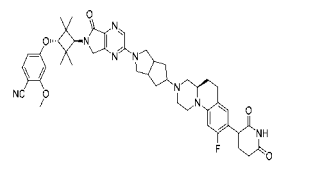 | 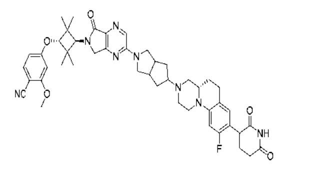 |
| 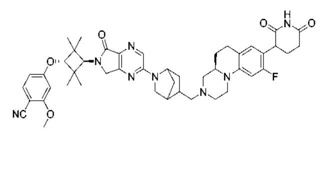 | 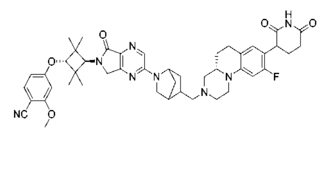 | 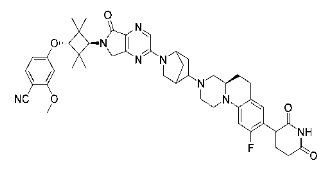 |
| 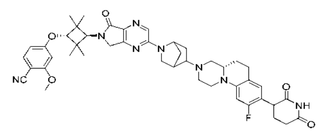 | 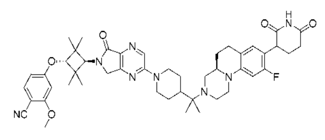 | 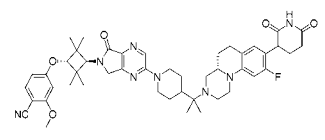 |
| 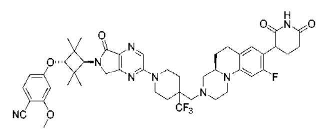 | 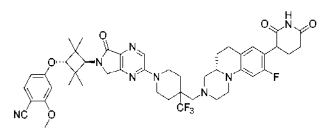 | 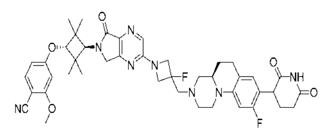 |
| 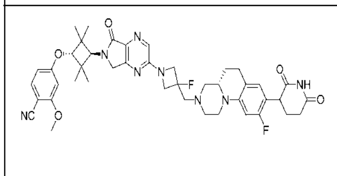 | 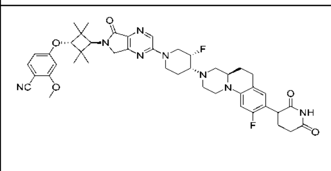 | 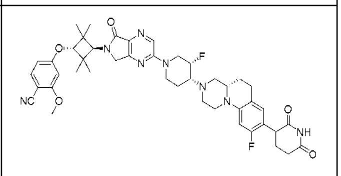 |
| 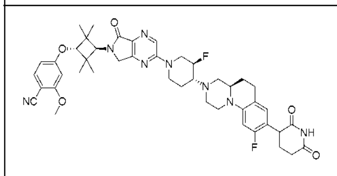 | 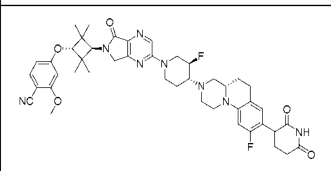 | 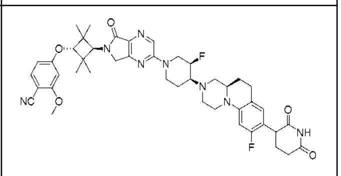 |
| 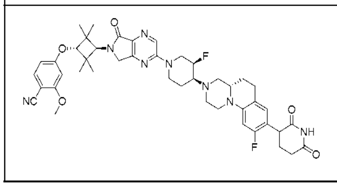 | 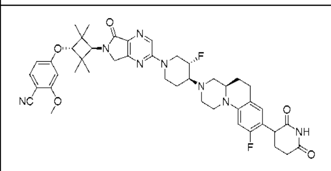 | 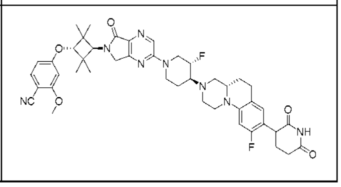 |

| | | |
|---|---|---|
| 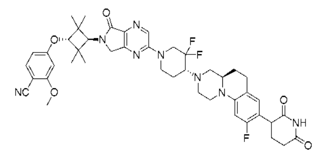 | 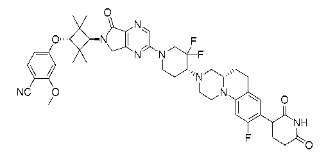 | 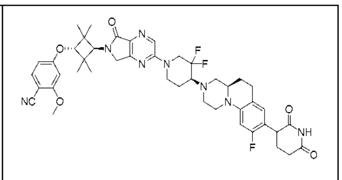 |
| 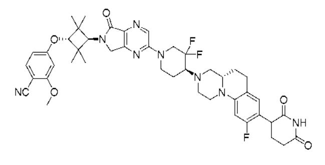 | 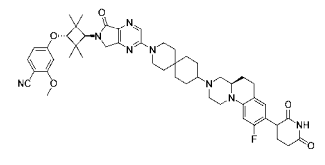 | 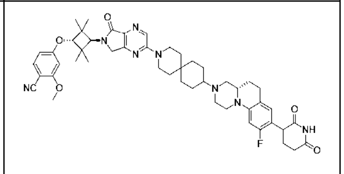 |
| 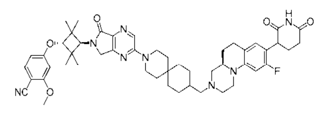 | 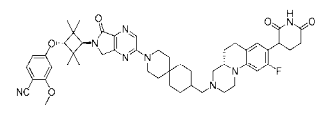 | 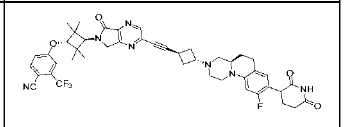 |
| 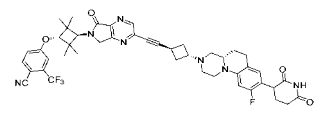 | 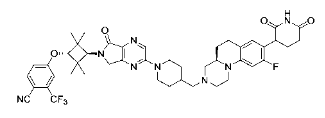 | 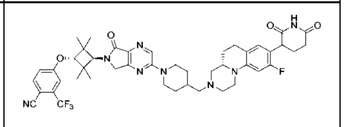 |
| 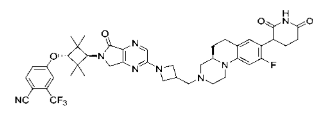 | 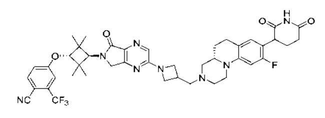 | 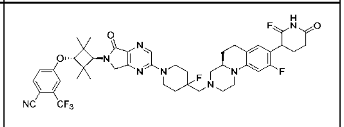 |
| 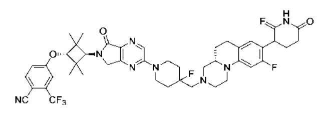 | 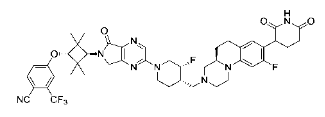 | 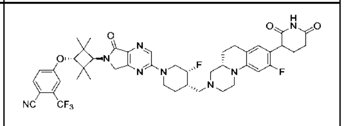 |
| 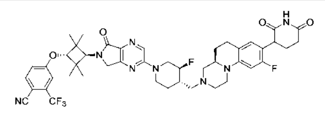 | 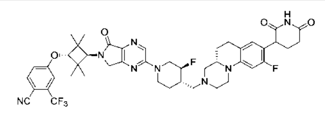 | 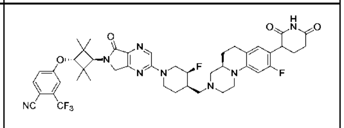 |
| 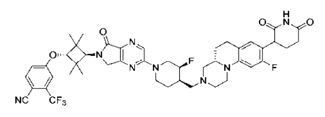 | 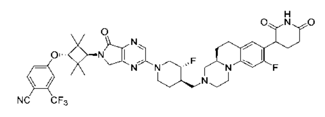 | 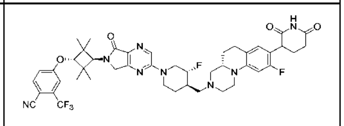 |
| 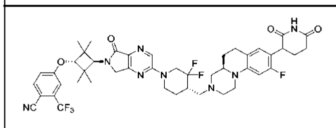 | 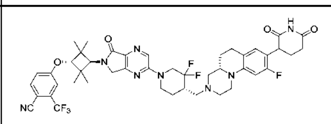 | 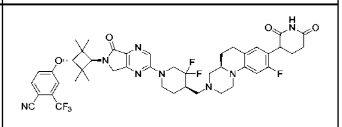 |
| 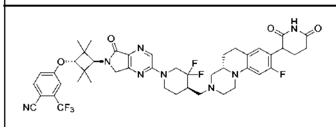 | 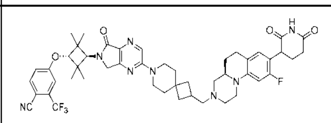 | 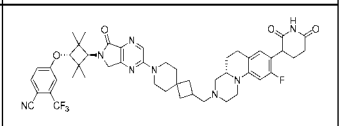 |
| 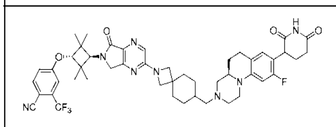 | 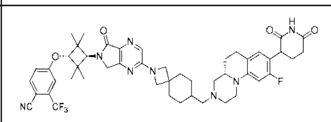 | 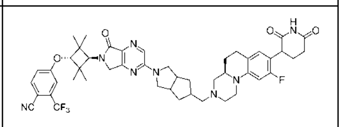 |
| 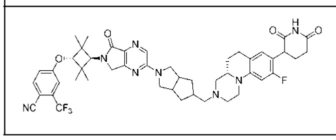 | 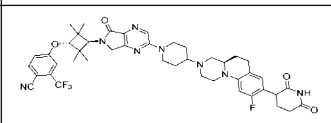 | 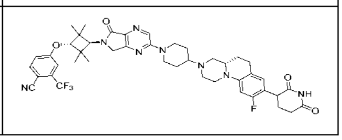 |

| | | |
|---|---|---|
| 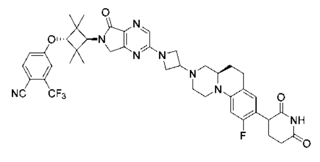 | 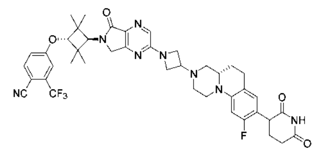 | 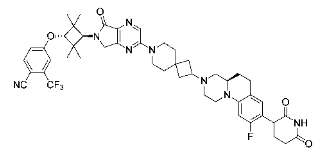 |
| 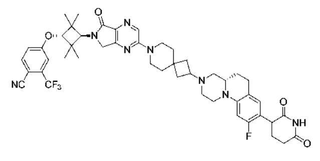 | 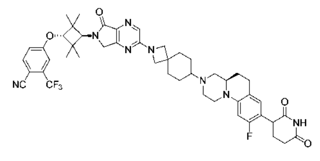 | 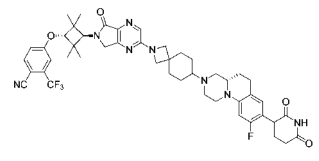 |
| 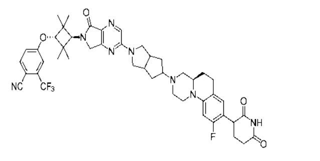 | 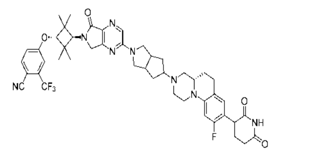 | 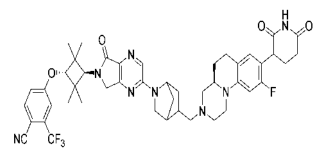 |
| 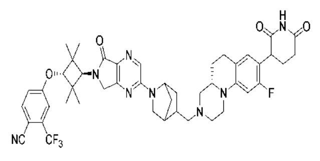 | 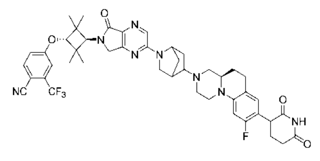 | 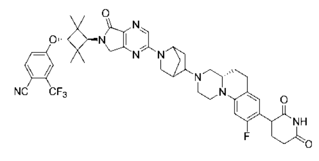 |
| 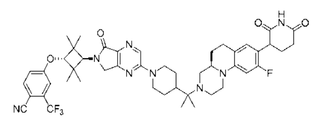 | 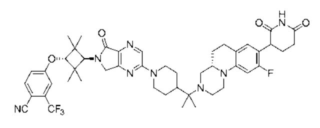 | 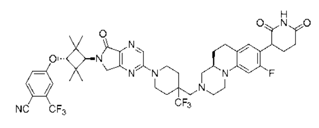 |
| 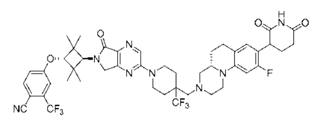 | 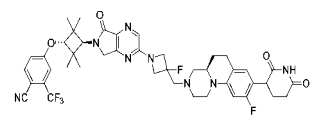 | 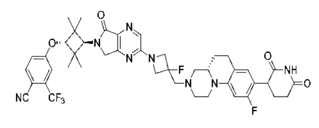 |
| 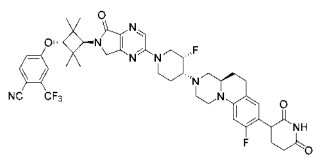 | 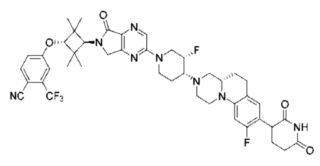 | 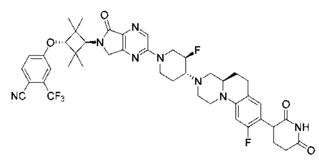 |
| 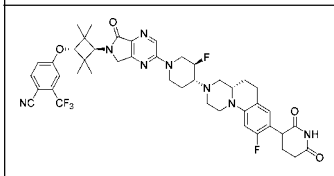 | 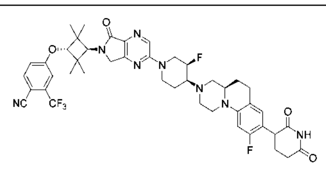 | 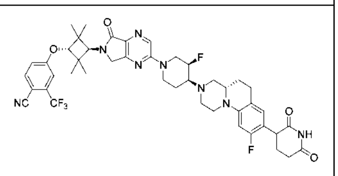 |
| 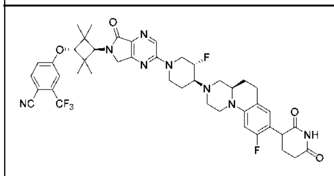 | 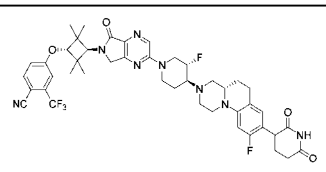 | 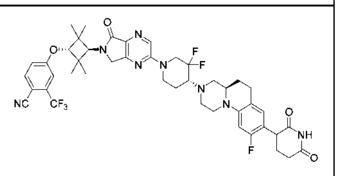 |
| 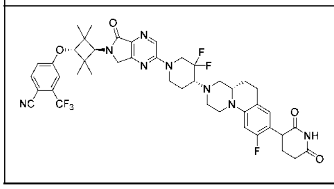 | 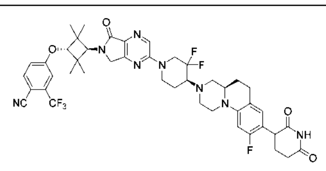 | 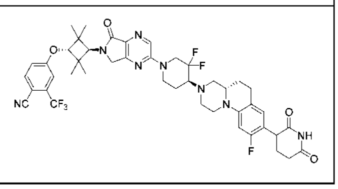 |

| | | |
|---|---|---|
| 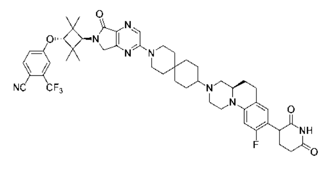 | 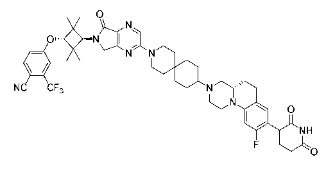 | 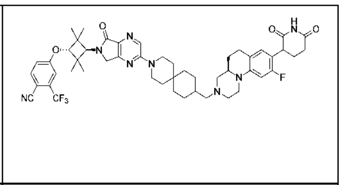 |
| 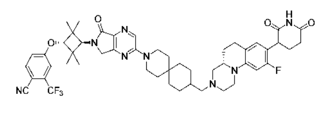 | 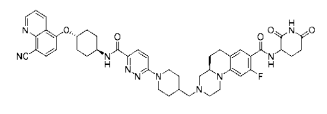 | 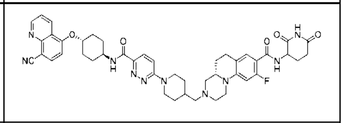 |
| 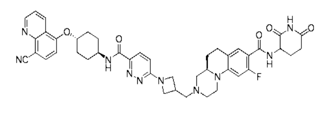 | 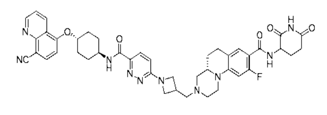 | 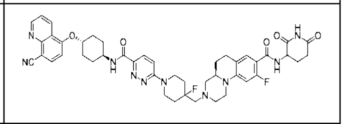 |
| 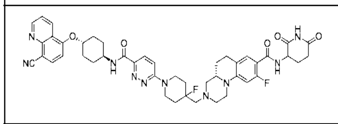 | 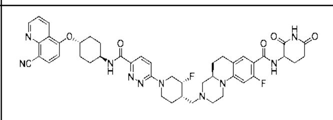 | 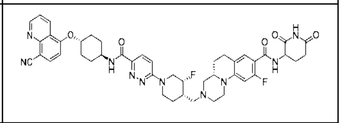 |
| 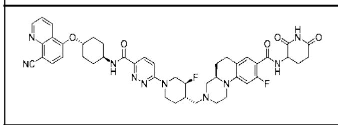 | 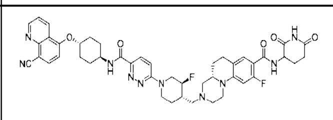 | 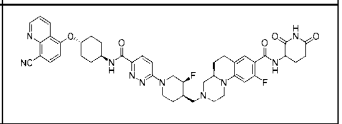 |
| 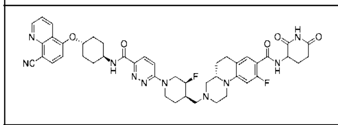 | 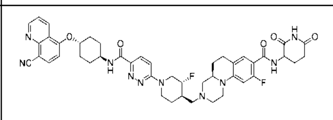 | 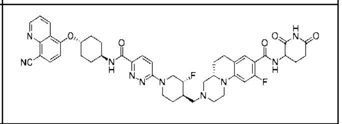 |
| 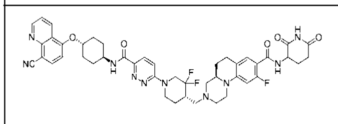 | 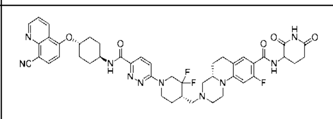 | 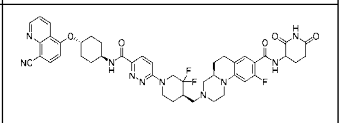 |
| 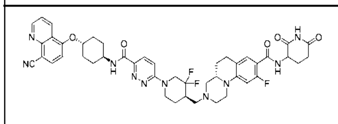 | 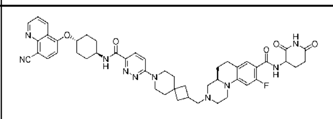 | 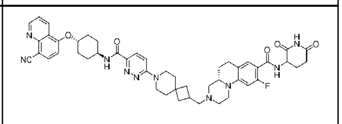 |
| 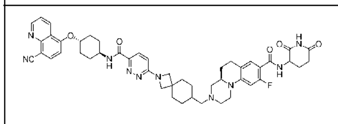 | 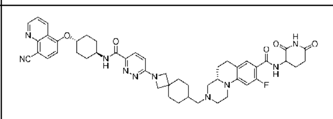 | 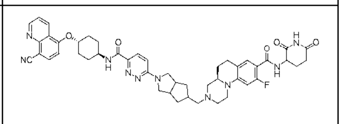 |
| 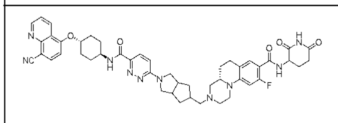 | 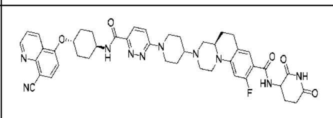 | 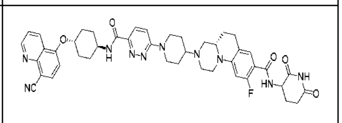 |
| 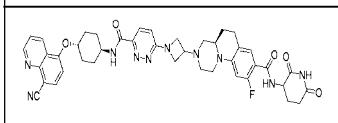 | 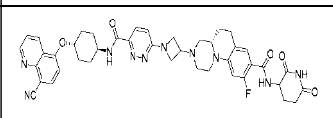 | 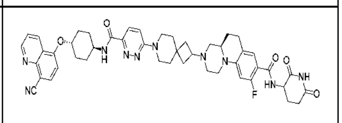 |
| 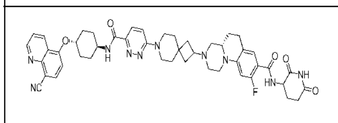 | 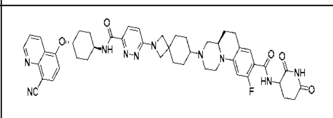 | 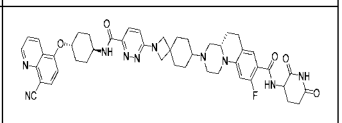 |
| 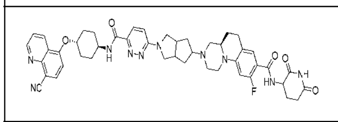 | 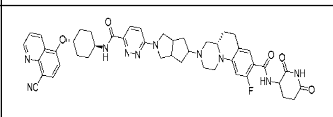 | 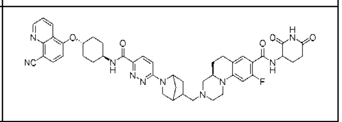 |
| 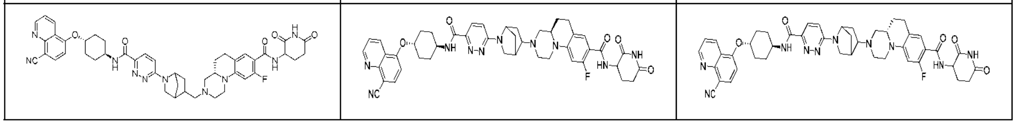 | | |

| | | |
|---|---|---|
| 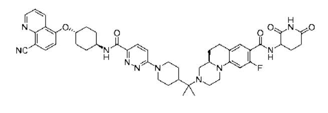 | 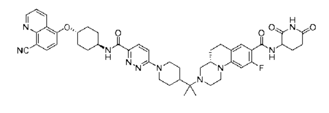 | 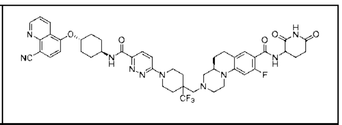 |
| 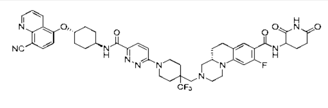 | 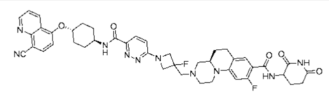 | 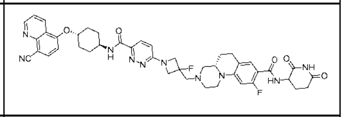 |
| 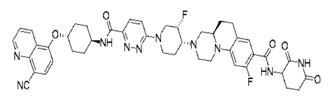 | 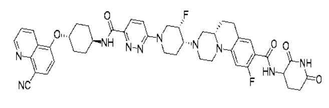 | 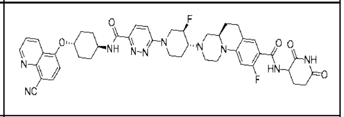 |
| 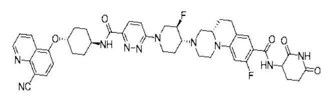 | 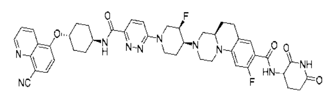 | 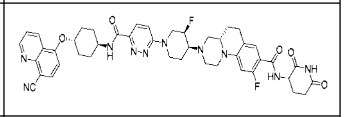 |
| 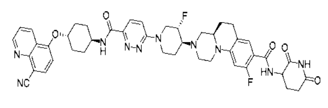 | 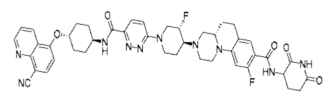 | 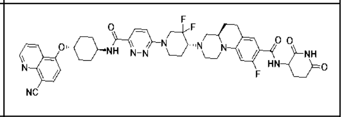 |
| 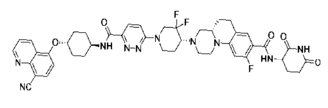 | 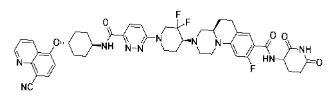 | 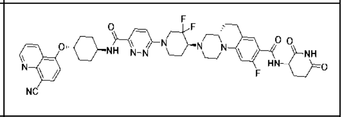 |
| 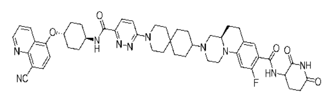 | 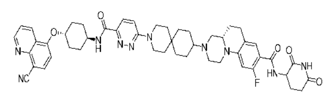 | 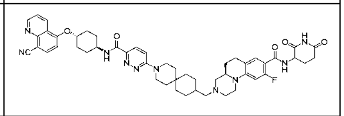 |
| 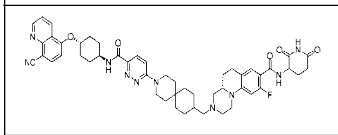 | 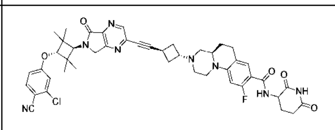 | 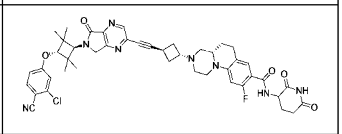 |
| 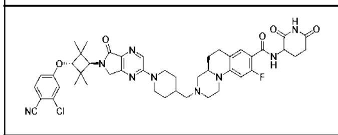 | 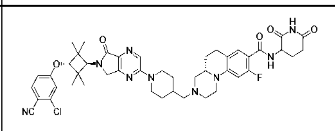 | 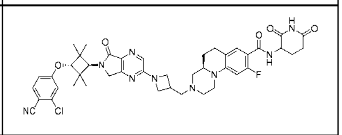 |
| 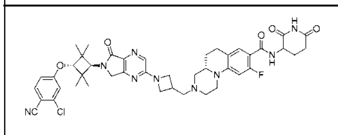 | 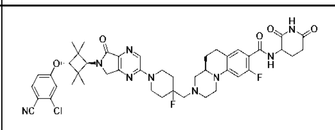 | 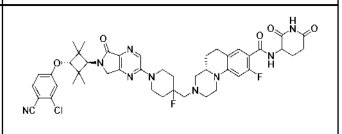 |
| 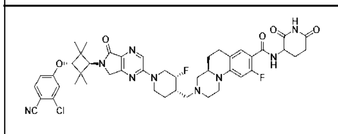 | 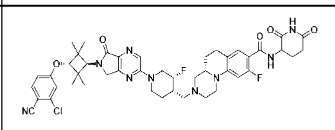 | 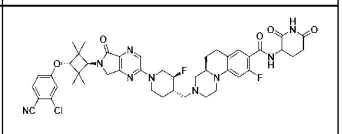 |
| 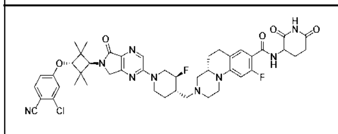 | 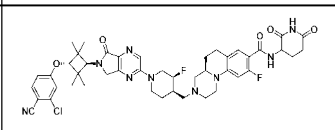 | 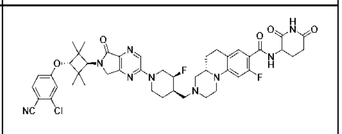 |
| 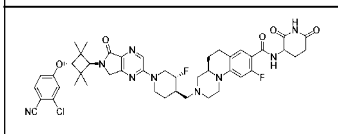 | 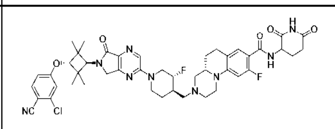 | 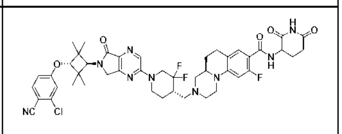 |
| 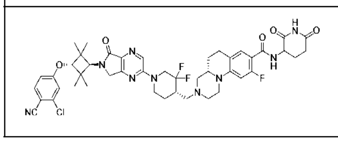 | | |

| | | |
|---|---|---|
| 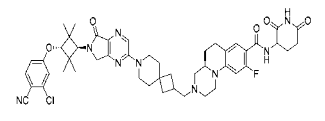 | 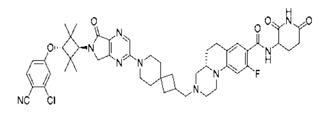 | 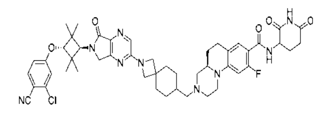 |
| 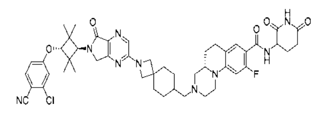 | 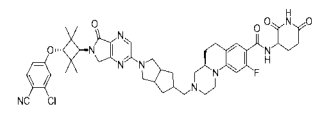 | 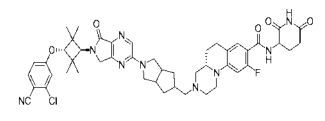 |
| 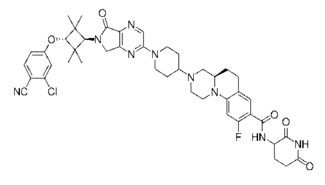 | 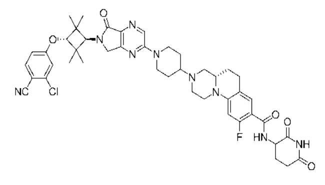 | 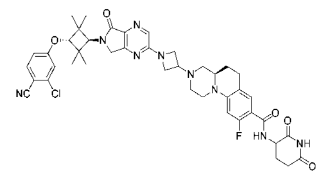 |
| 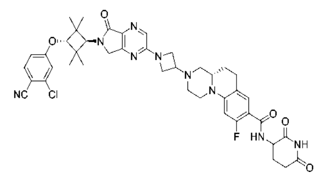 | 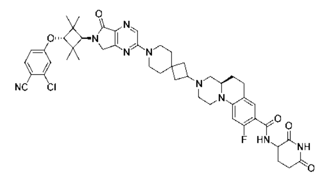 | 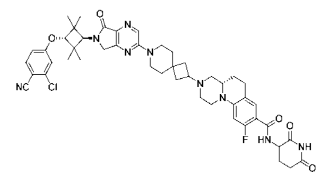 |
| 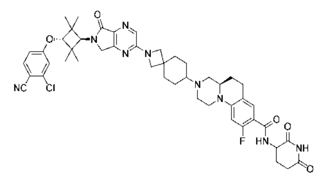 | 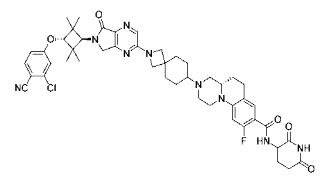 | 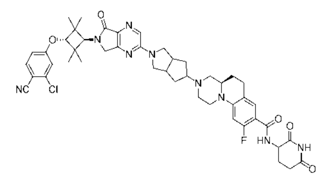 |
| 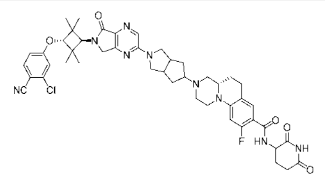 | 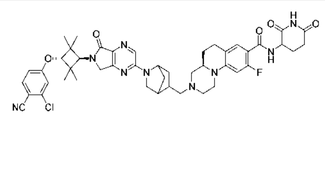 | 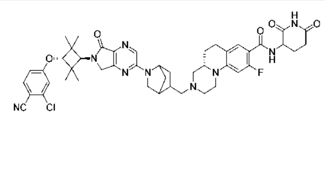 |
| 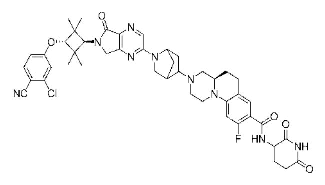 | 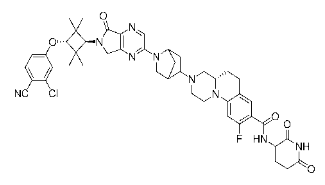 | 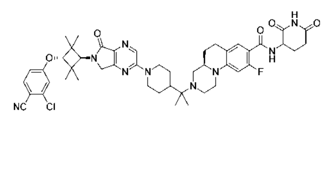 |
| 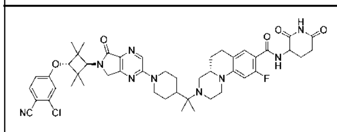 | 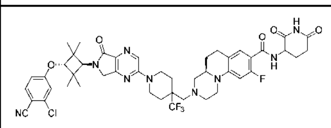 | 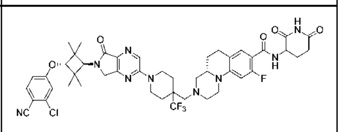 |
| 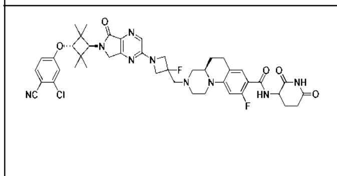 | 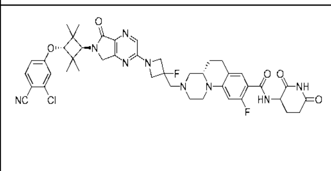 | 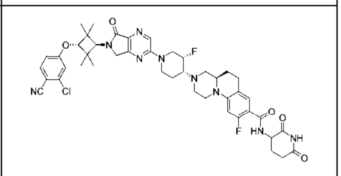 |
| 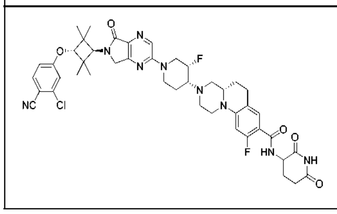 | 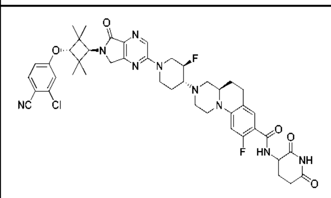 | 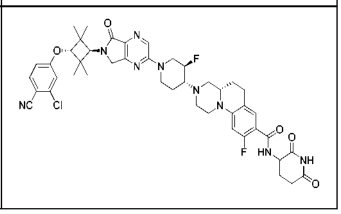 |

| | | |
|---|---|---|
| 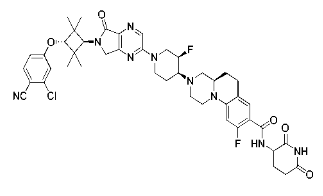 | 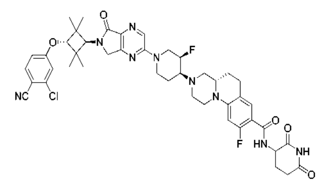 | 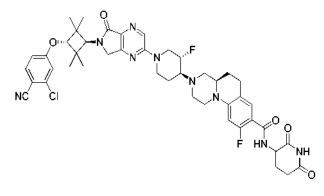 |
| 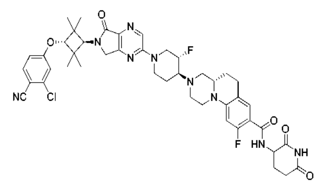 | 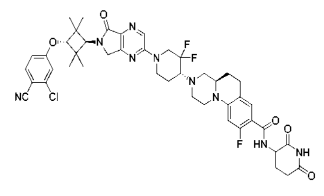 | 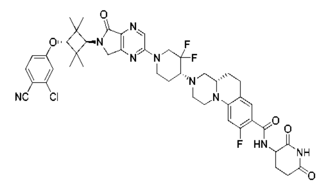 |
| 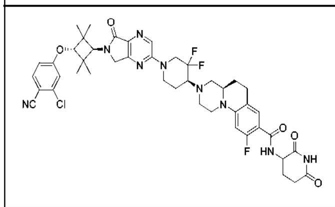 | 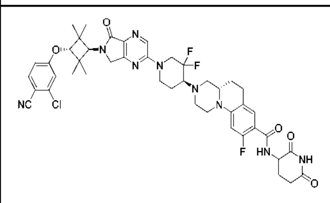 | 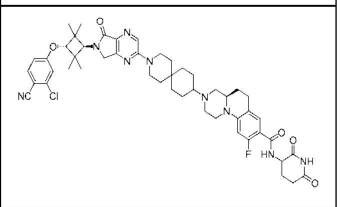 |
| 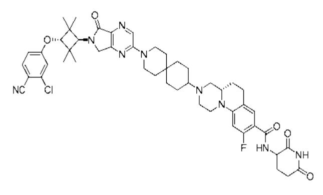 | 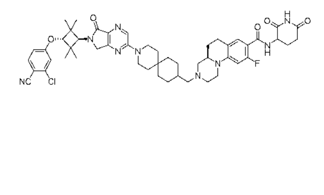 | 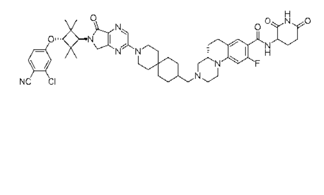 |
| 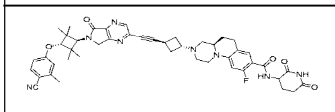 | 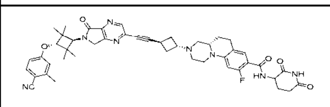 | 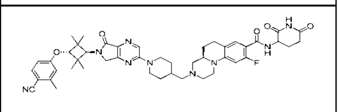 |
| 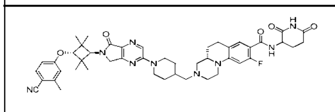 | 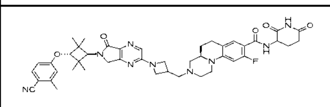 | 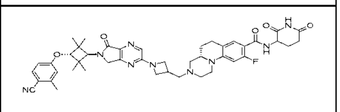 |
| 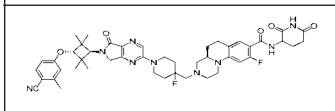 | 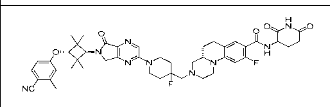 | 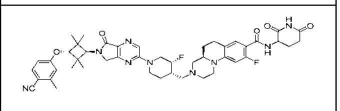 |
| 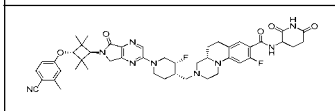 | 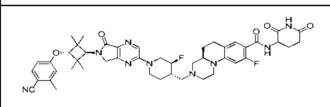 | 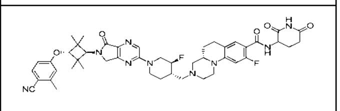 |
| 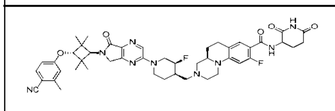 | 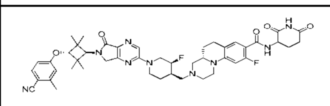 | 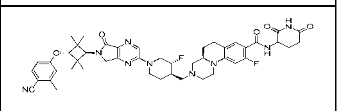 |
| 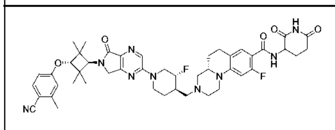 | 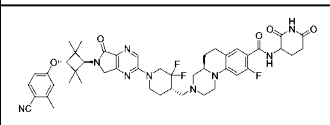 | 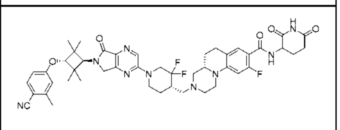 |
| 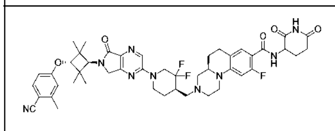 | 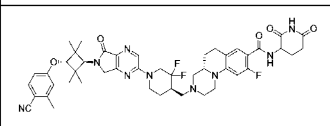 | 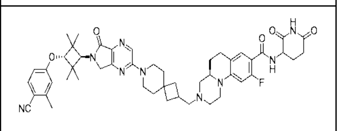 |
| 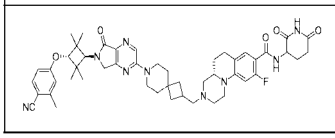 | 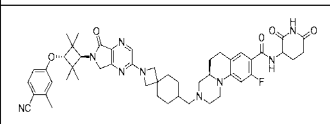 | 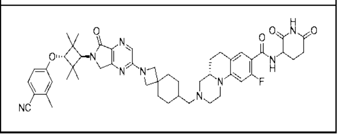 |

| | | |
|---|---|---|
| 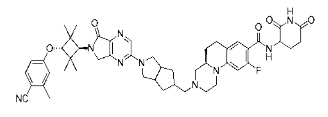 | 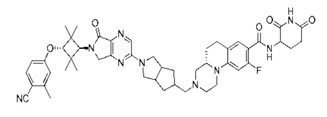 | 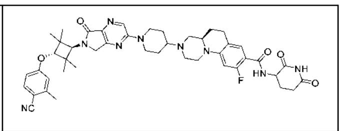 |
| 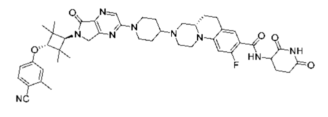 | 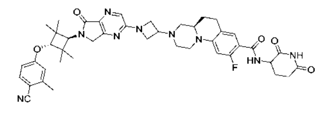 | 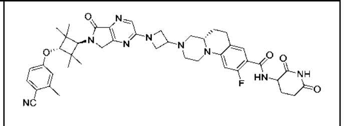 |
| 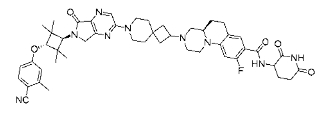 | 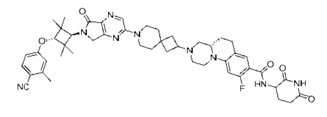 | 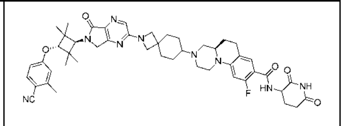 |
| 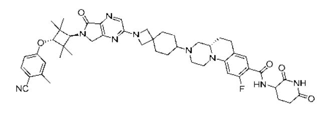 | 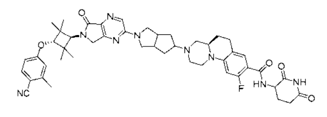 | 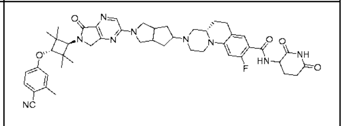 |
| 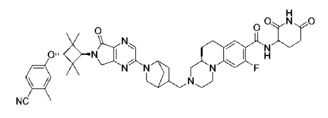 | 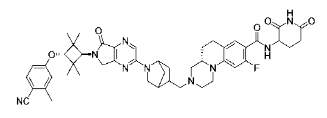 | 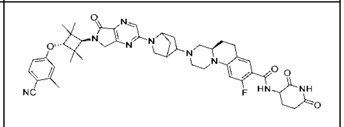 |
| 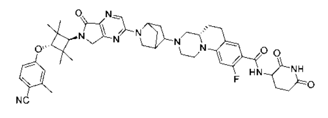 | 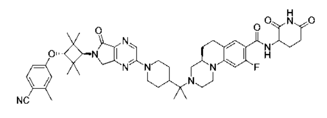 | 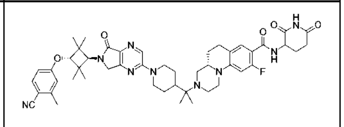 |
| 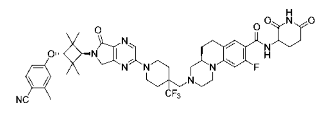 | 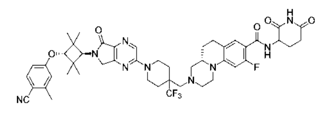 | 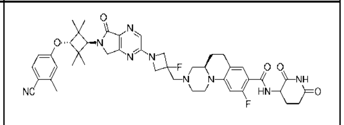 |
| 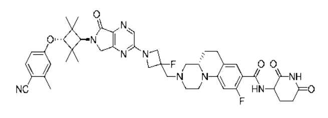 | 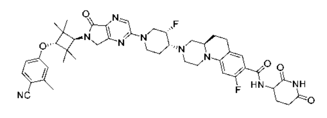 | 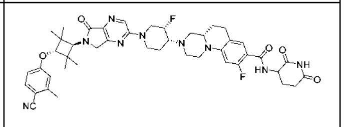 |
| 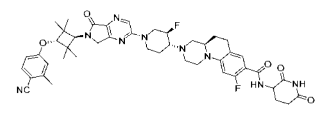 | 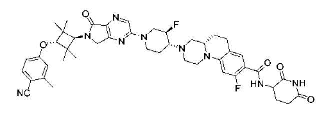 | 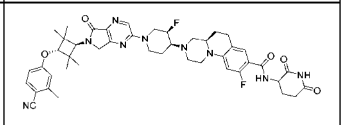 |
| 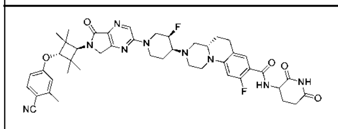 | 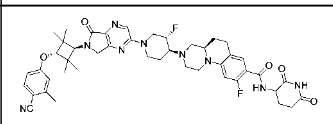 | 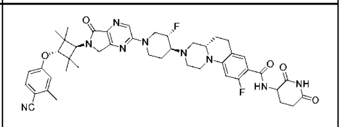 |
| 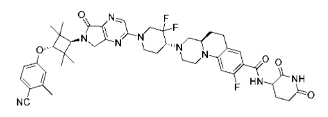 | 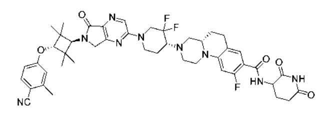 | 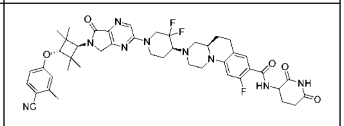 |
| 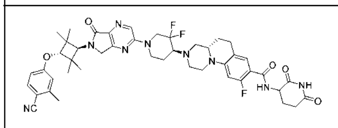 | 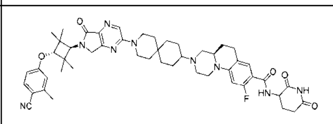 | 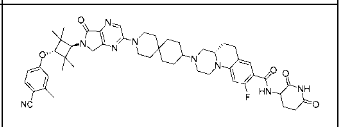 |
| 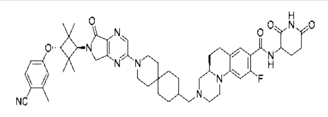 | 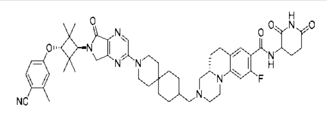 | 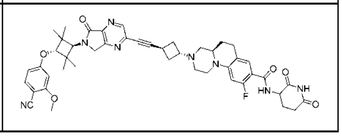 |

| | | |
|---|---|---|
| 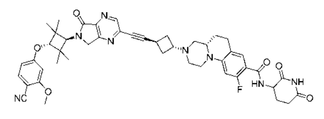 | 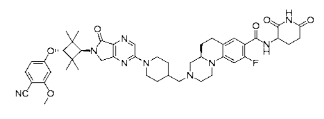 | 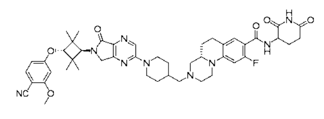 |
| 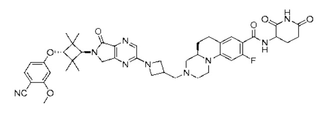 | 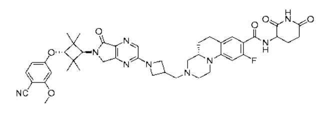 | 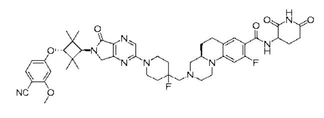 |
| 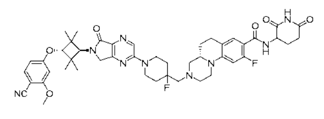 | 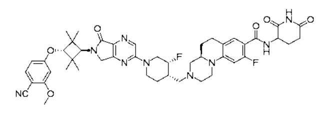 | 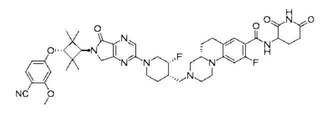 |
| 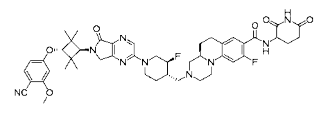 | 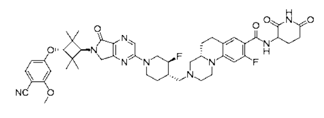 | 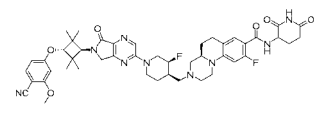 |
| 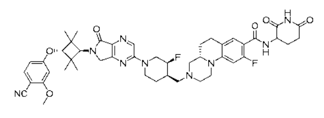 | 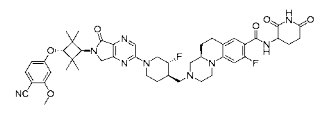 | 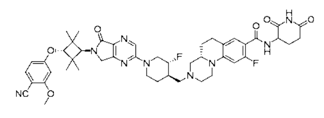 |
| 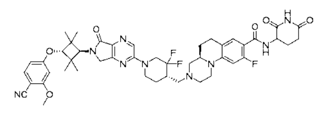 | 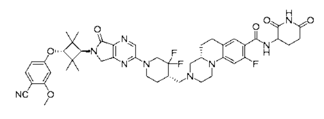 | 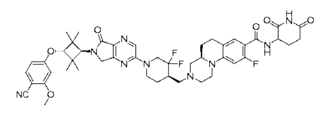 |
| 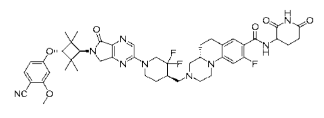 | 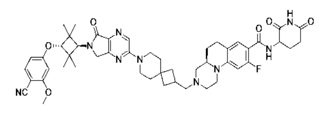 | 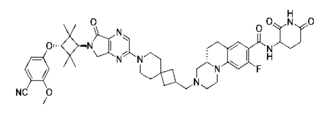 |
| 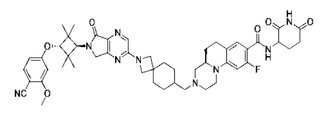 | 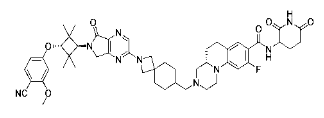 | 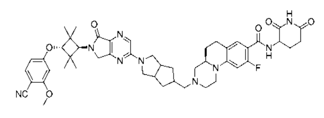 |
| 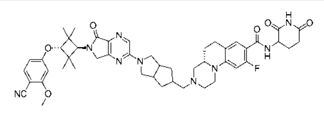 | 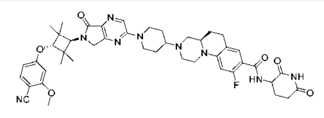 | 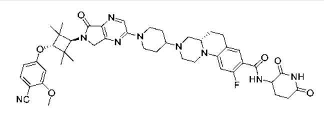 |
| 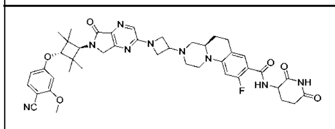 | 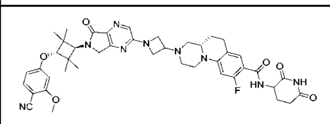 | 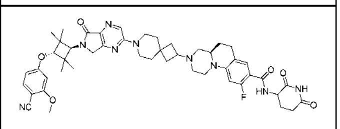 |
| 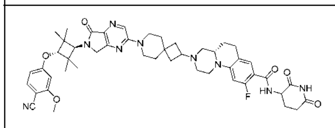 | 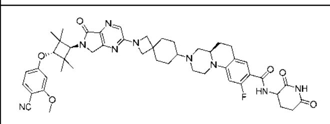 | 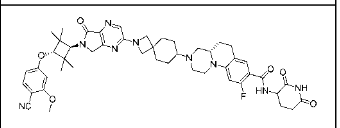 |
| 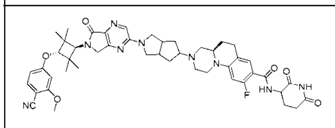 | 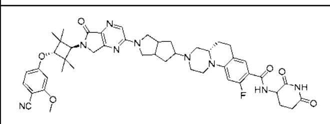 | 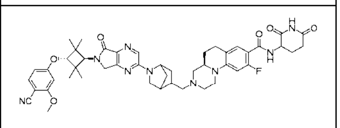 |
| 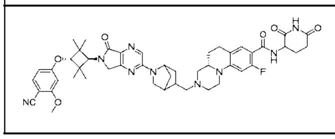 | 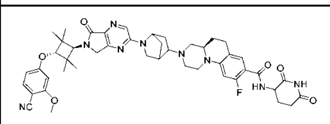 | 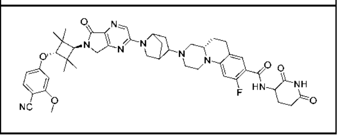 |

| | | |
|---|---|---|
| 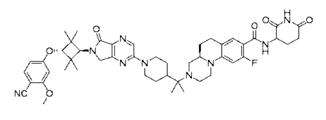 | 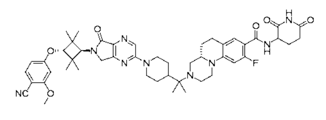 | 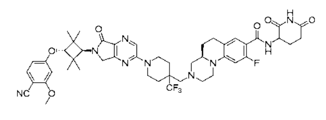 |
| 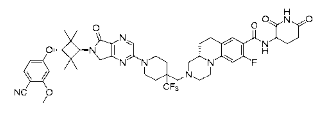 | 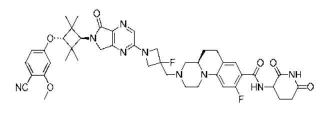 | 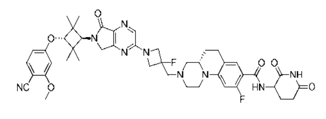 |
| 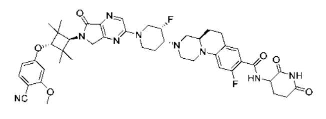 | 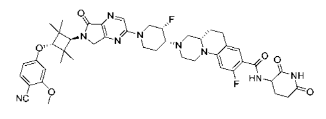 | 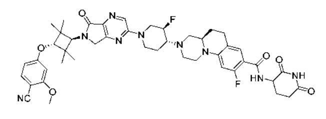 |
| 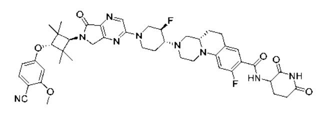 | 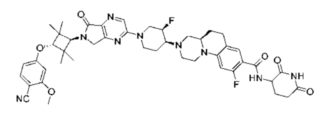 | 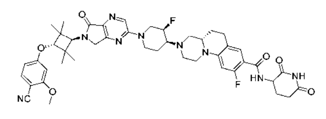 |
| 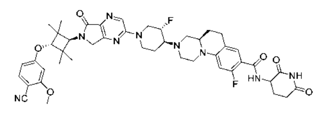 | 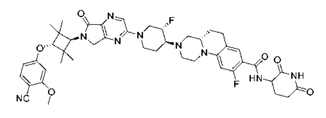 | 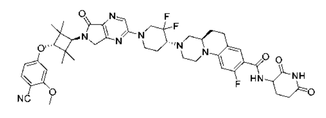 |
| 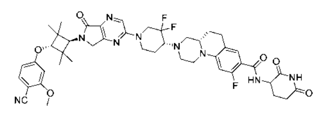 | 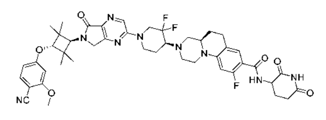 | 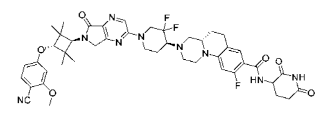 |
| 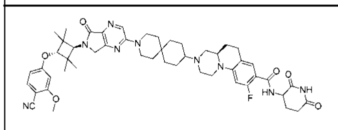 | 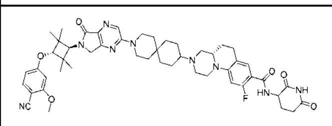 | 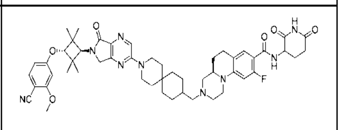 |
| 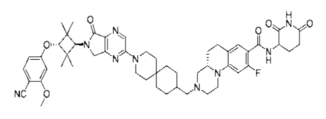 | 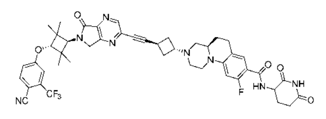 | 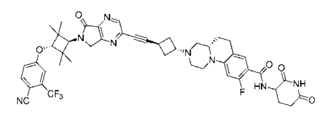 |
| 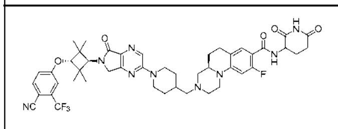 | 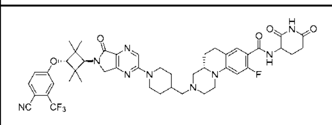 | 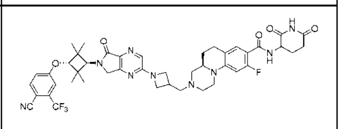 |
| 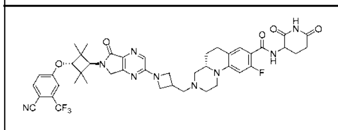 | 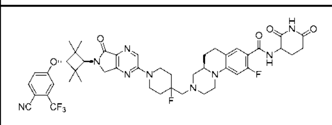 | 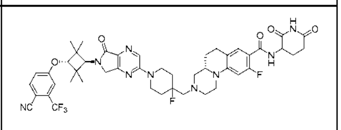 |
| 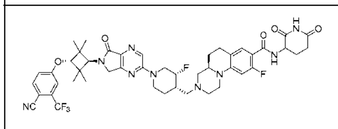 | 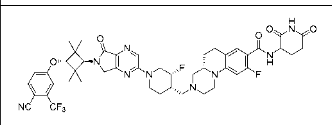 | 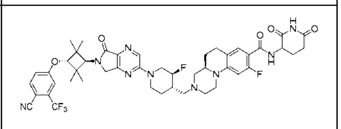 |
| 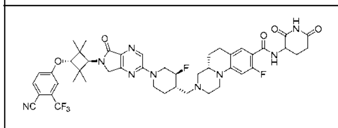 | 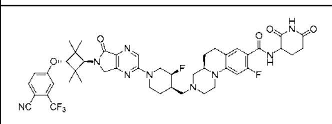 | 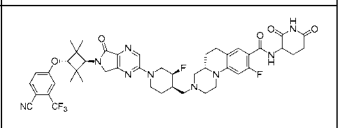 |
| 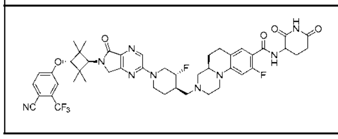 | 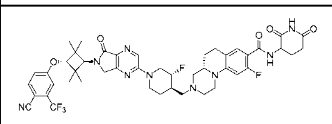 | 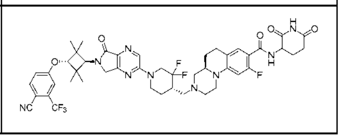 |

| | | |
|---|---|---|
| 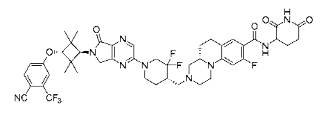 | 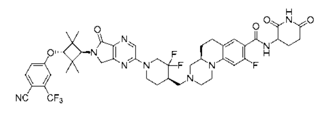 | 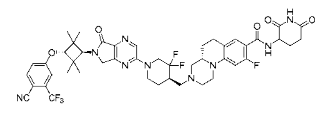 |
| 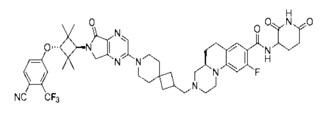 | 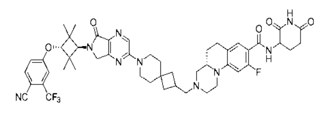 | 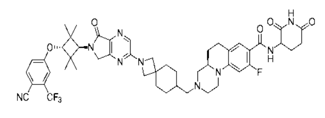 |
| 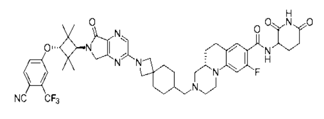 | 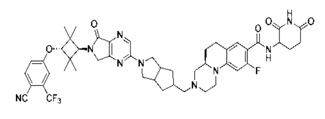 | 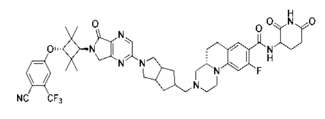 |
| 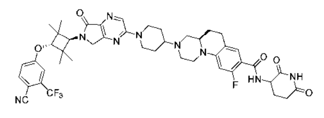 | 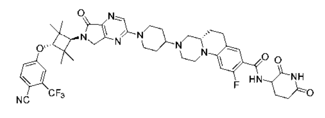 | 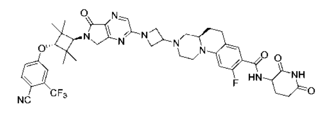 |
| 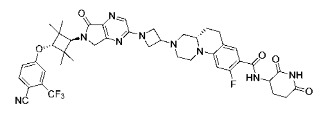 | 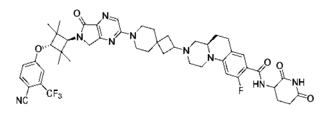 | 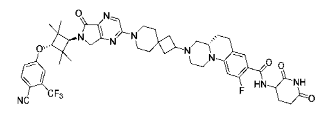 |
| 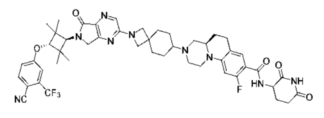 | 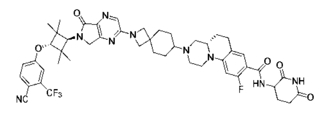 | 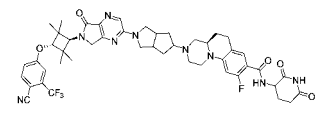 |
| 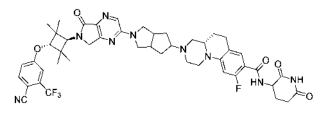 | 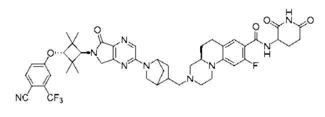 | 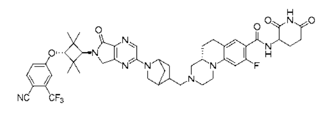 |
| 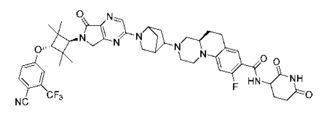 | 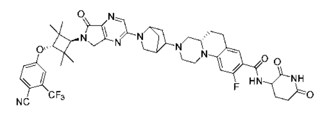 | 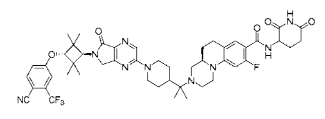 |
| 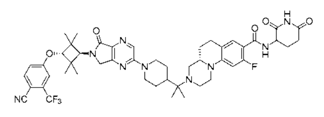 | 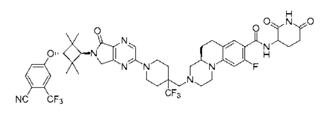 | 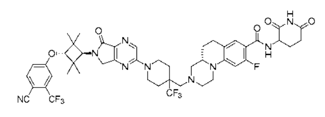 |
| 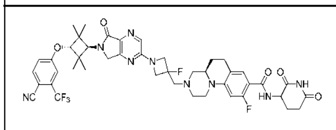 | 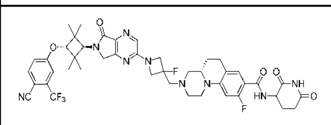 | 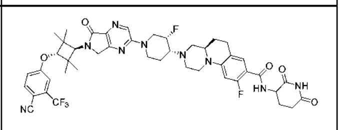 |
| 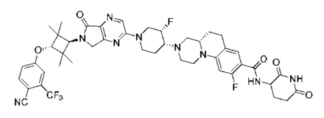 | 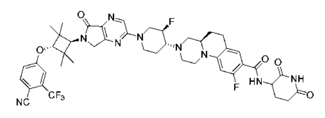 | 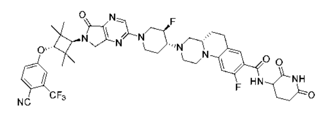 |
| 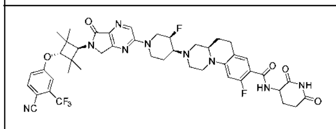 | 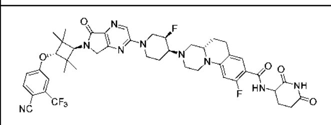 | 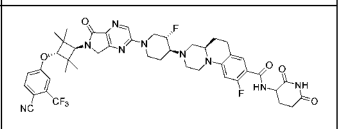 |
| 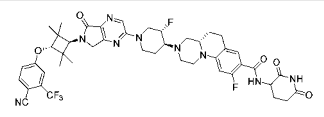 | 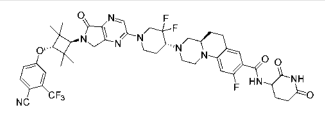 | 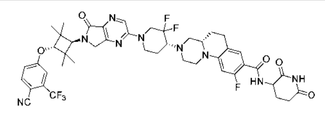 |

| | | |
|---|---|---|
| 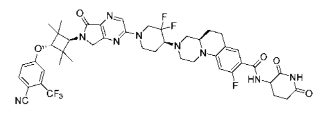 | 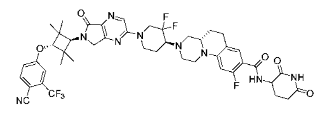 | 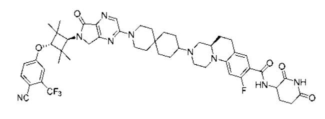 |
| 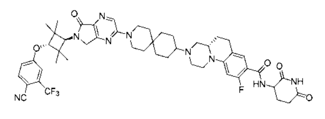 | 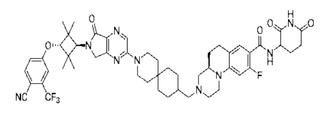 | 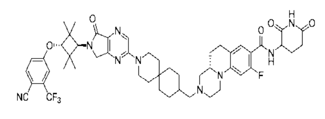 |
| 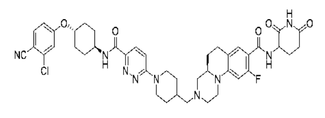 | 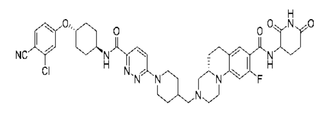 | 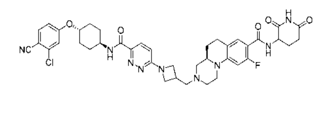 |
| 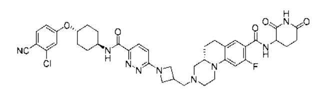 | 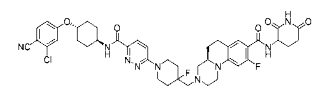 | 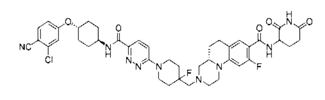 |
| 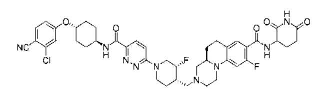 | 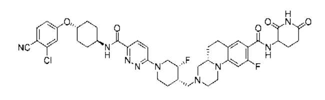 | 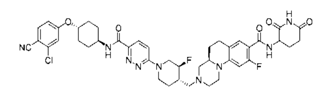 |
| 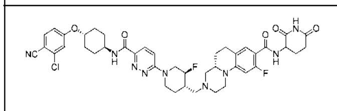 | 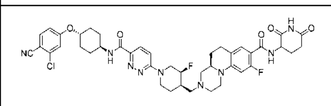 | 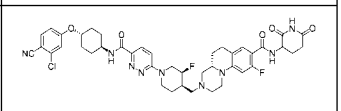 |
| 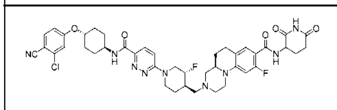 | 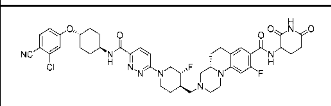 | 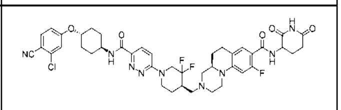 |
| 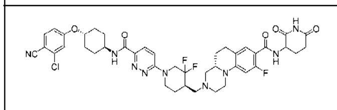 | 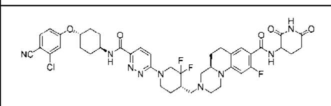 | 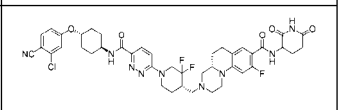 |
| 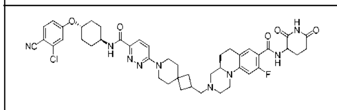 | 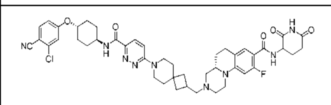 | 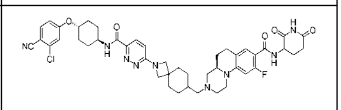 |
| 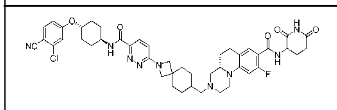 | 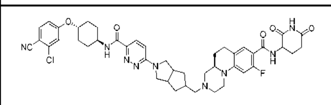 | 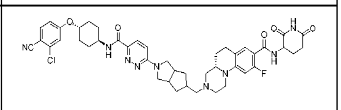 |
| 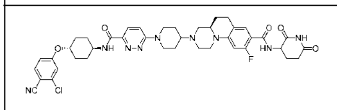 | 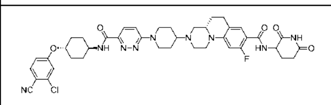 | 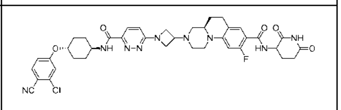 |
| 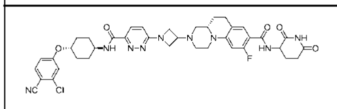 | 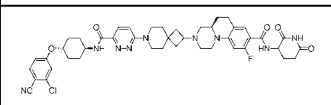 | 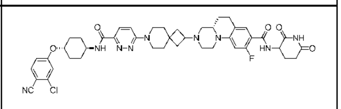 |
| 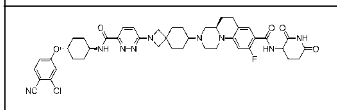 | 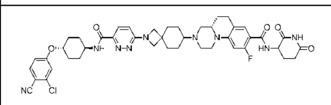 | 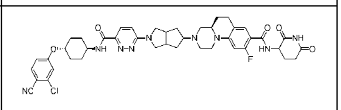 |
| 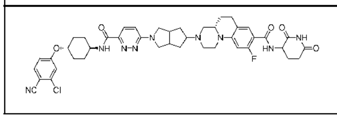 | | |

| | | |
|---|---|---|
| 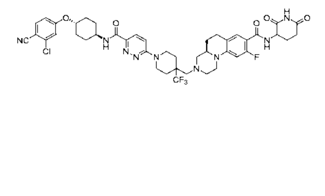 | 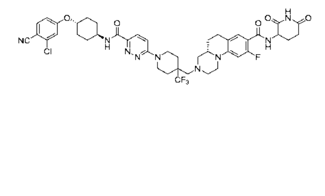 | 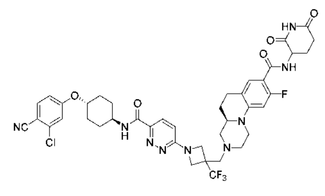 |
| 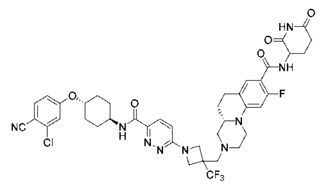 | 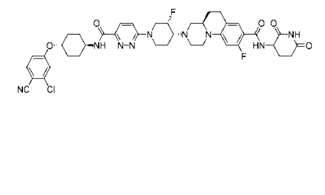 | 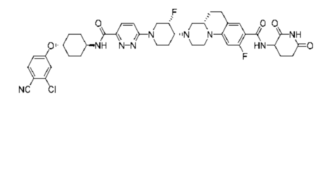 |
| 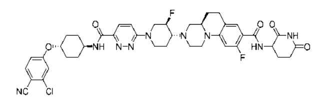 | 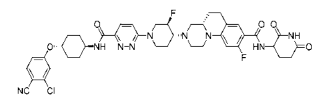 | 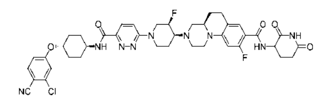 |
| 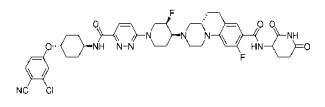 | 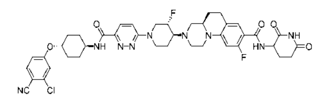 | 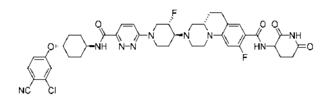 |
| 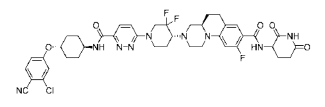 | 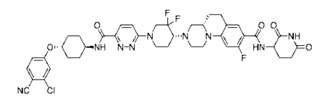 | 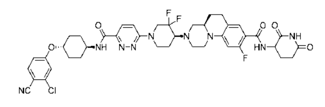 |
| 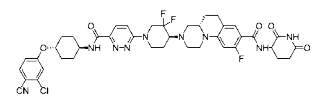 | 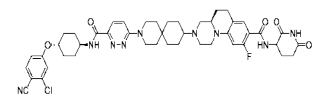 | 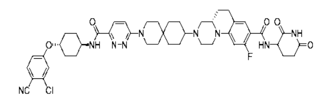 |
| 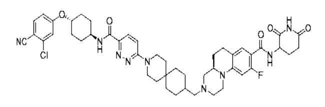 | 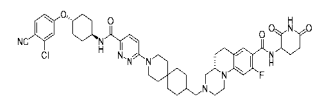 | 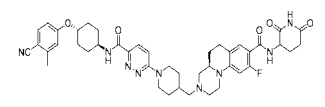 |
| 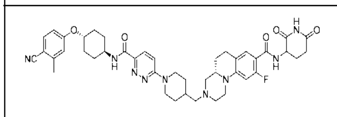 | 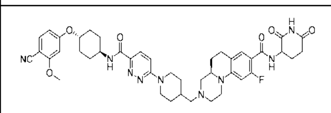 | 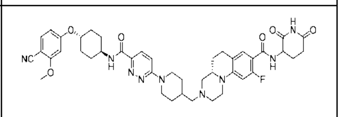 |
| 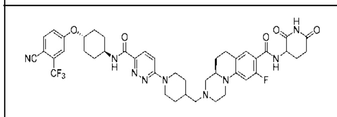 | 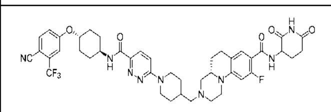 | 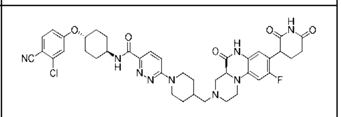 |
| 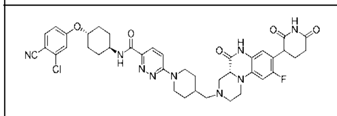 | 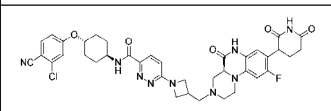 | 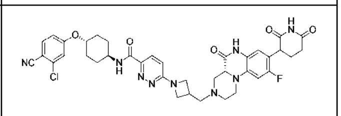 |
| 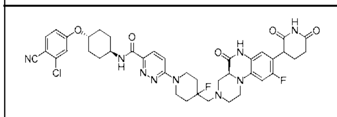 | 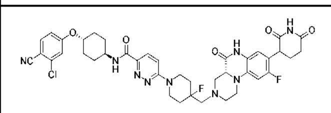 | 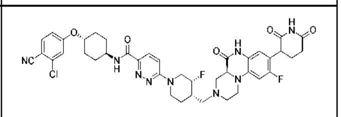 |
| 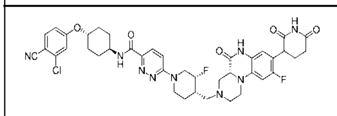 | 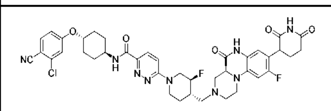 | 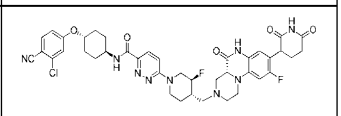 |
| 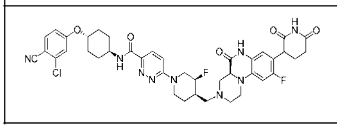 | 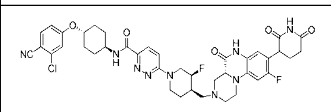 | 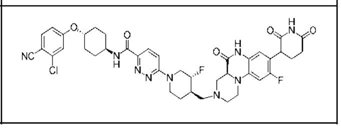 |

| | | |
|---|---|---|
| 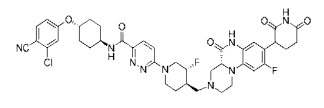 | 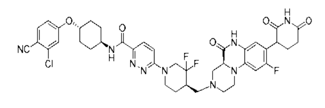 | 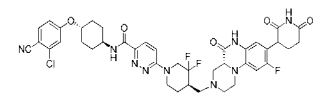 |
| 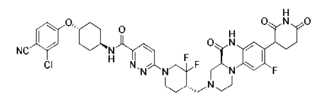 | 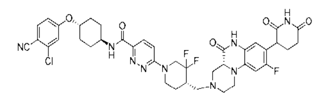 | 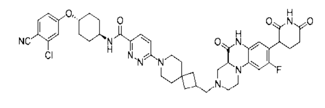 |
| 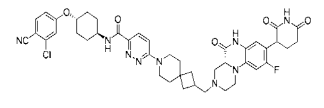 | 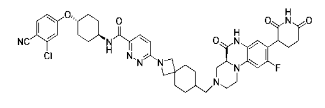 | 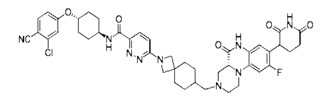 |
| 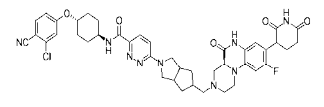 | 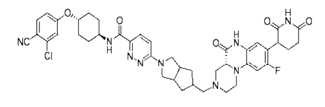 | 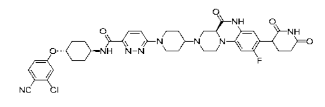 |
| 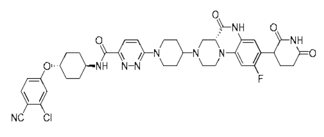 | 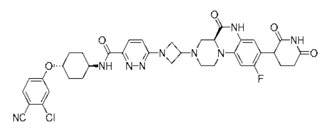 | 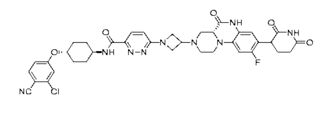 |
| 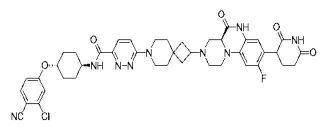 | 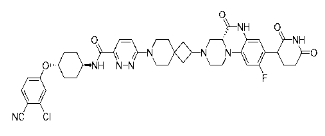 | 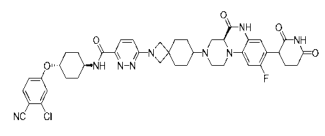 |
| 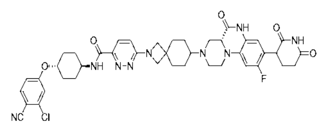 | 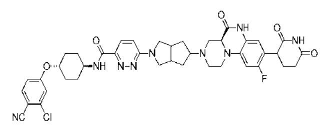 | 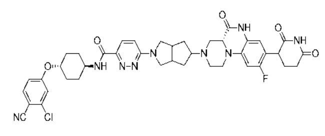 |
| 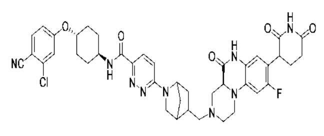 | 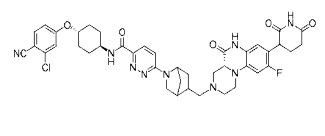 | 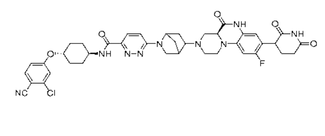 |
| 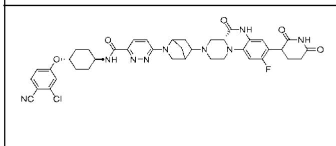 | 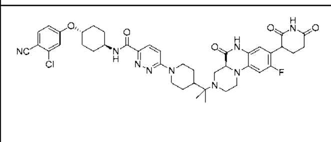 | 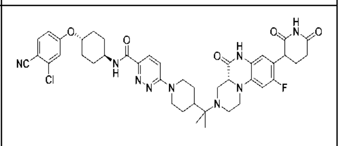 |
| 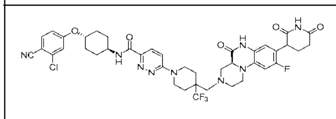 | 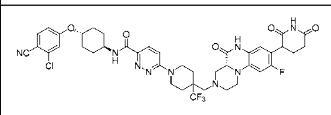 | 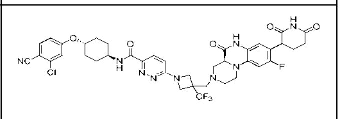 |
| 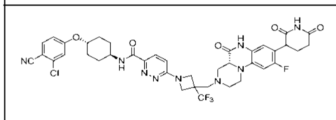 | 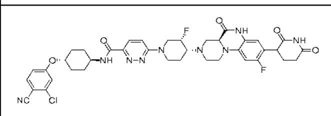 | 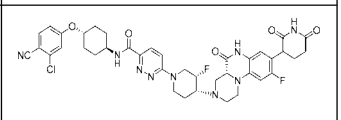 |
| 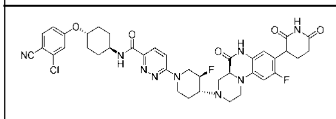 | 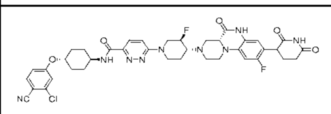 | 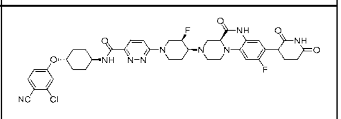 |
| 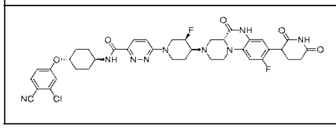 | 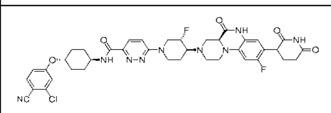 | 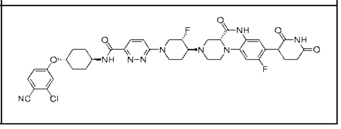 |

| | | |
|---|---|---|
| 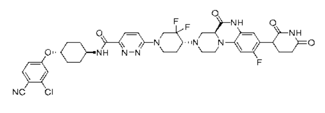 | 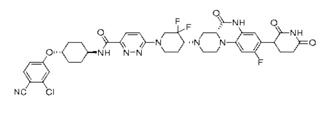 | 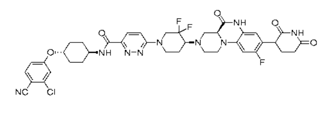 |
| 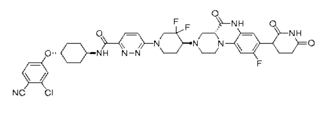 | 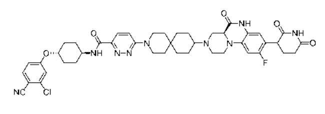 | 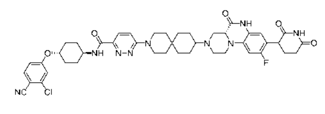 |
| 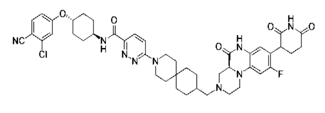 | 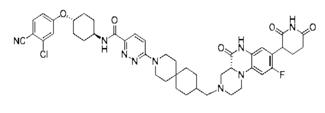 | 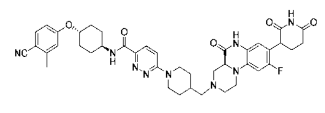 |
| 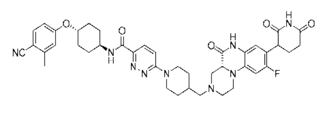 | 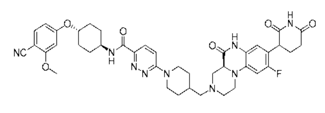 | 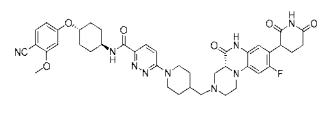 |
| 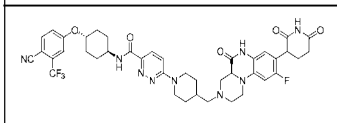 | 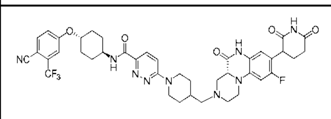 | 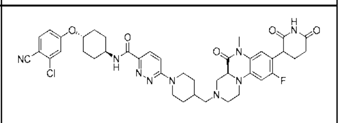 |
| 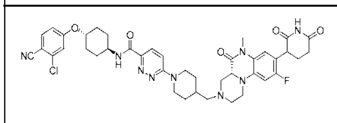 | 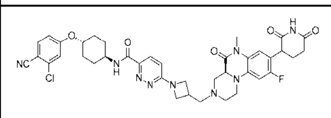 | 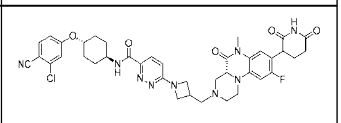 |
| 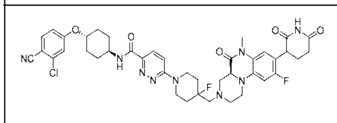 | 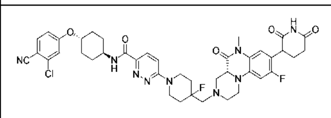 | 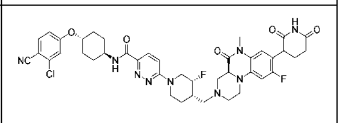 |
| 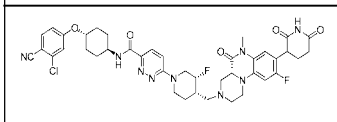 | 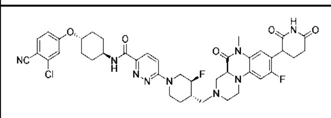 | 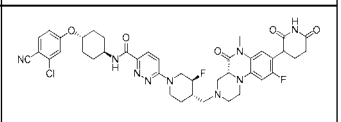 |
| 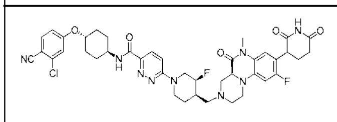 | 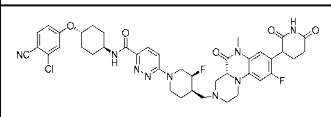 | 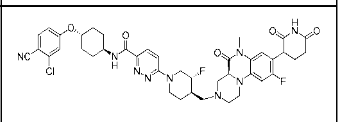 |
| 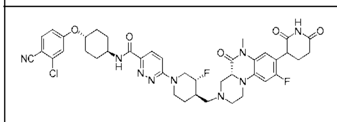 | 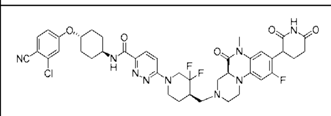 | 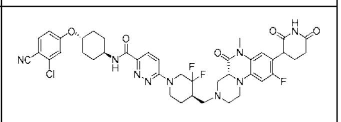 |
| 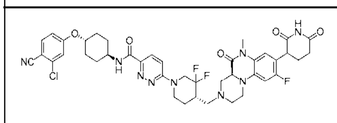 | 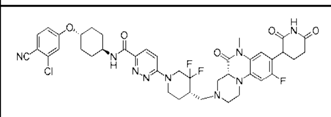 | 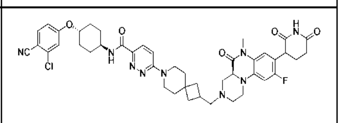 |
| 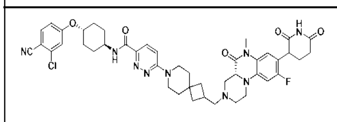 | 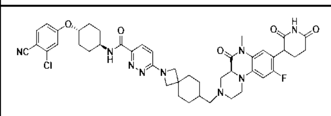 | 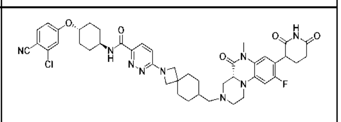 |
| 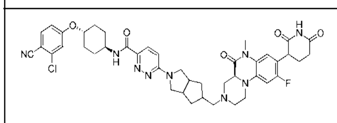 | 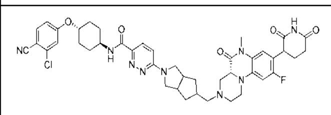 | 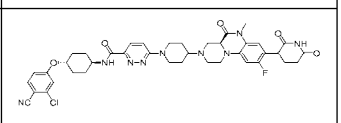 |
| 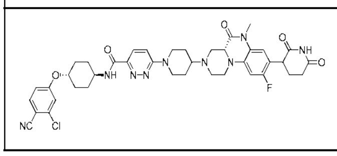 | | |

| | | |
|---|---|---|
| 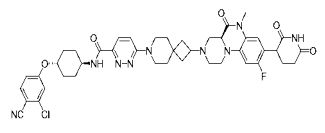 | 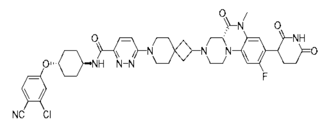 | 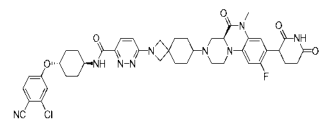 |
| 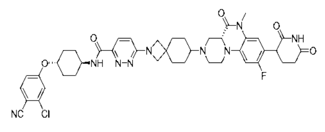 | 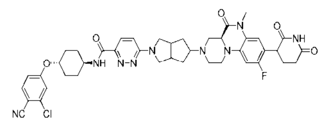 | 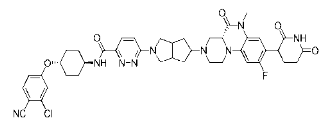 |
| 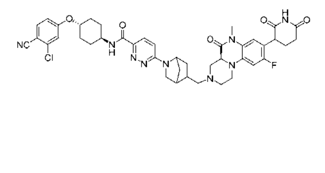 | 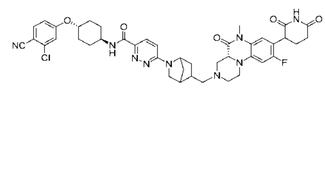 | 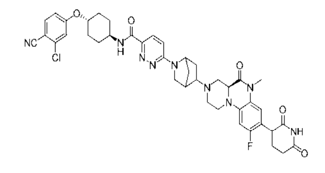 |
| 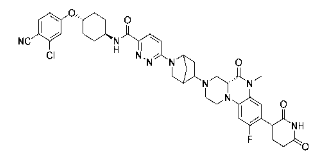 | 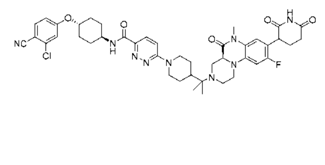 | 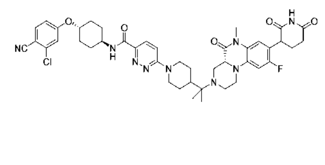 |
| 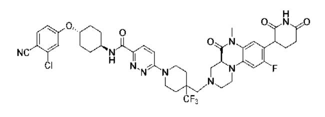 | 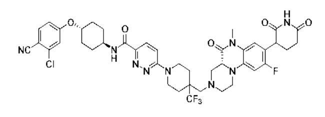 | 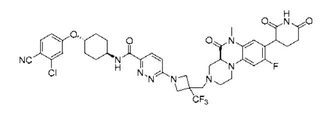 |
| 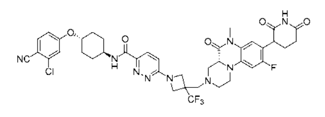 | 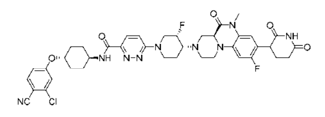 | 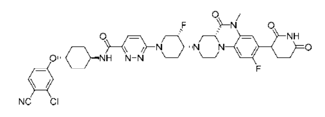 |
| 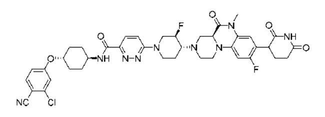 | 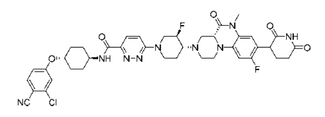 | 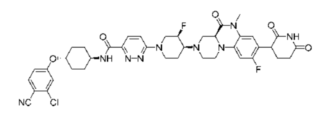 |
| 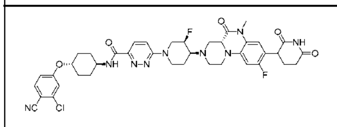 | 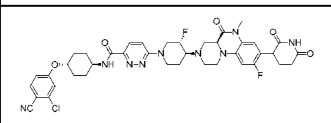 | 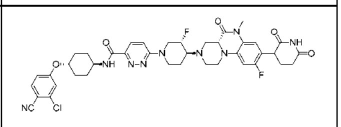 |
| 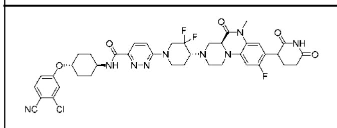 | 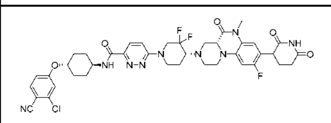 | 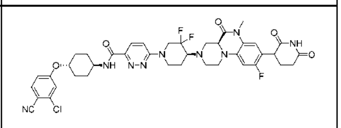 |
| 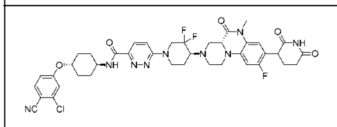 | 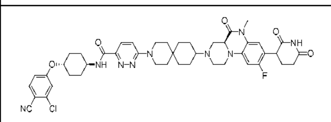 | 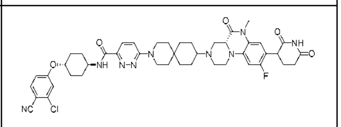 |
| 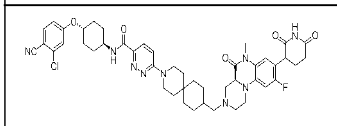 | 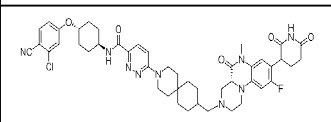 | 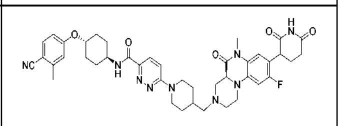 |
| 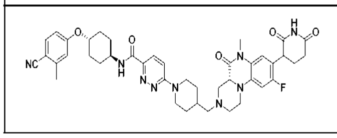 | 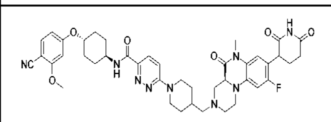 | 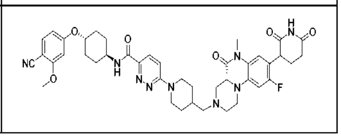 |

| | | |
|---|---|---|
| 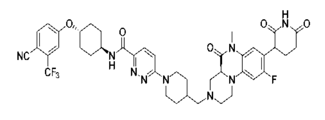 | 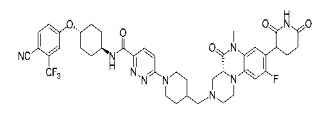 | 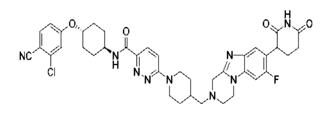 |
| 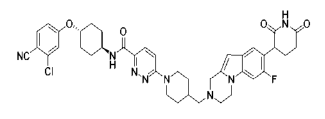 | 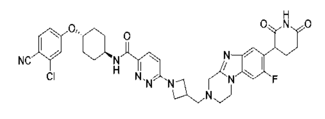 | 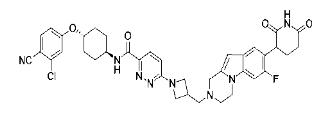 |
| 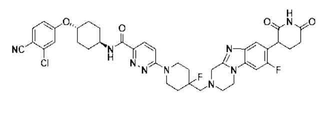 | 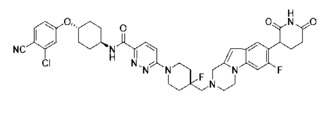 | 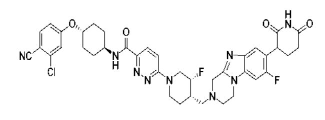 |
| 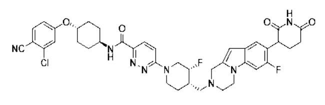 | 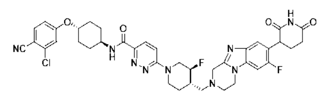 | 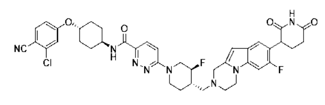 |
| 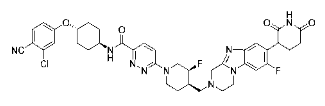 | 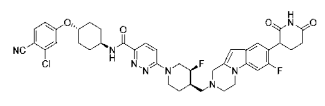 | 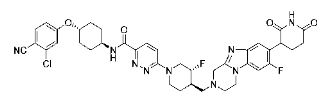 |
| 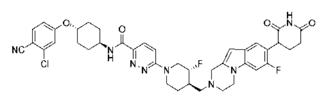 | 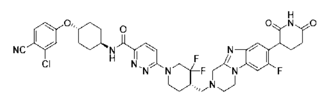 | 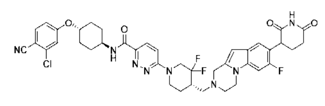 |
| 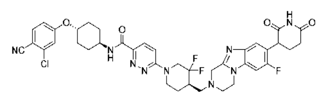 | 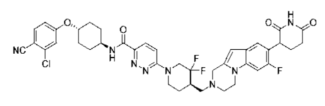 | 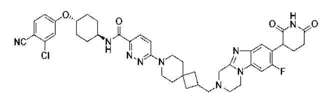 |
| 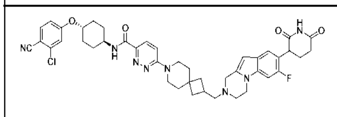 | 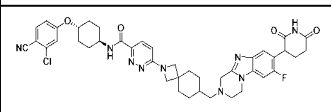 | 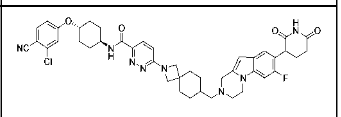 |
| 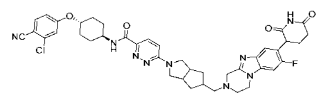 | 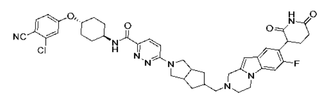 | 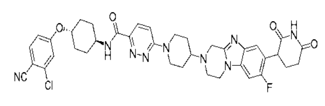 |
| 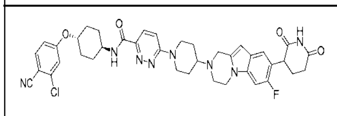 | 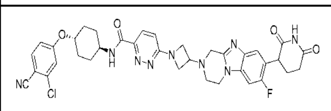 | 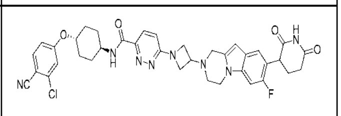 |
| 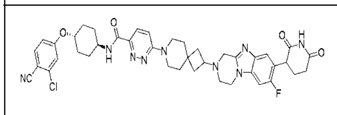 | 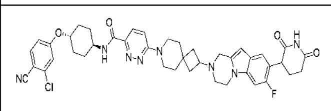 | 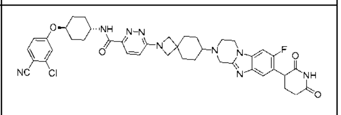 |
| 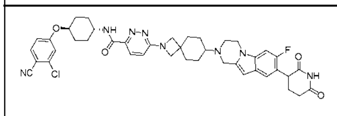 | 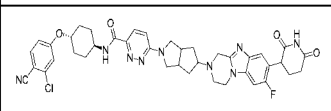 | 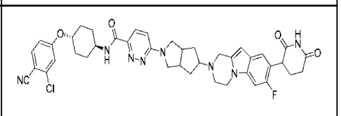 |
| 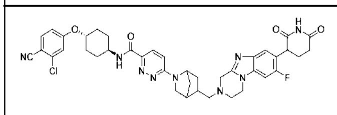 | 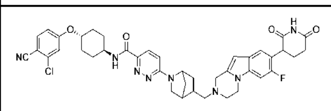 | 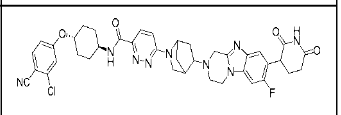 |
| 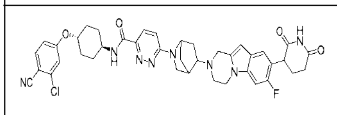 | | |
| | | |

| | | |
|---|---|---|
| 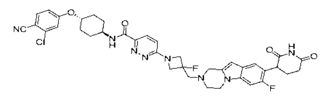 | 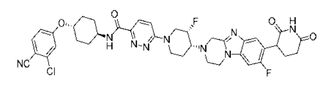 | 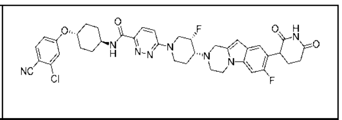 |
| 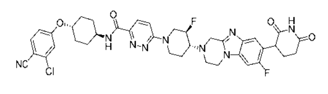 | 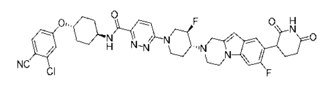 | 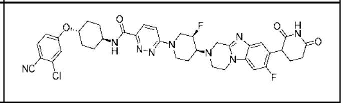 |
| 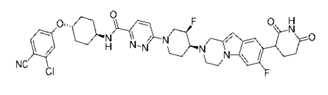 | 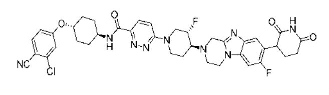 | 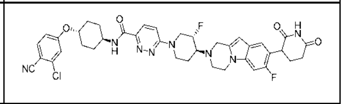 |
| 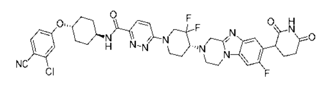 | 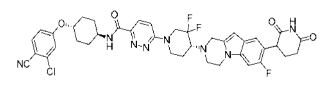 | 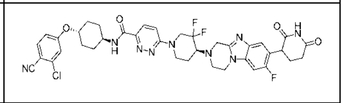 |
| 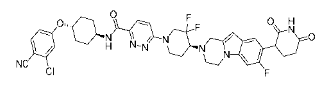 | 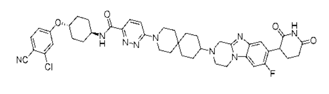 | 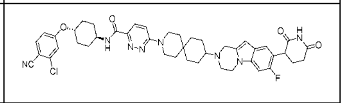 |
| 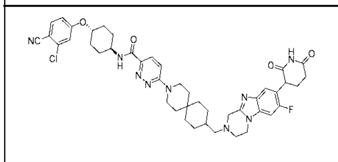 | 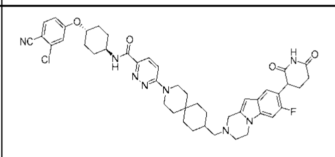 | 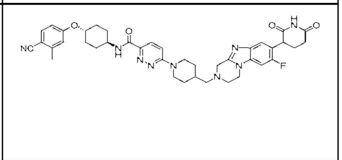 |
| 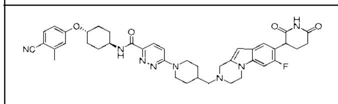 | 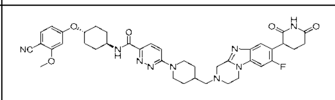 | 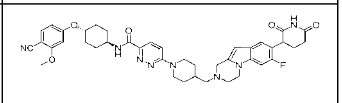 |
| 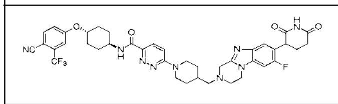 | 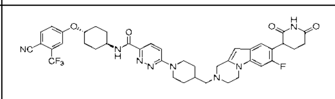 | 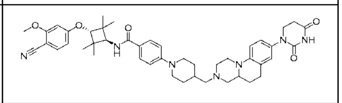 |
| | 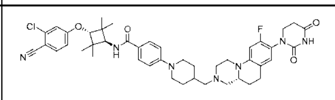 | 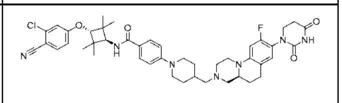 |
| 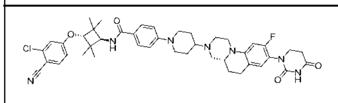 | 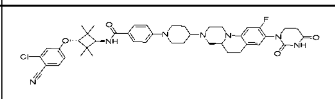 | 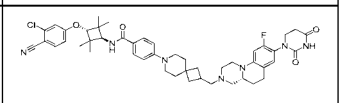 |
| 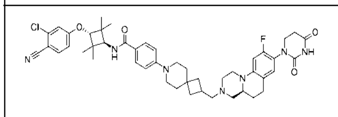 | 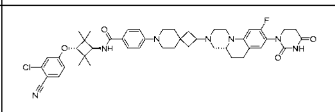 | 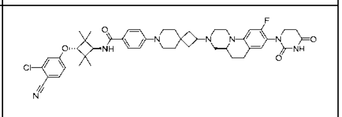 |
| 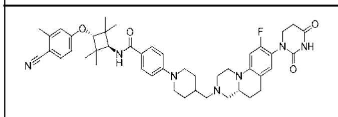 | 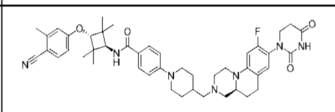 | 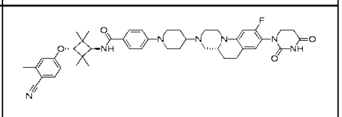 |
| 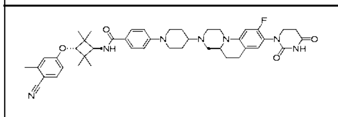 | 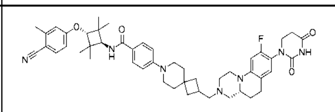 | 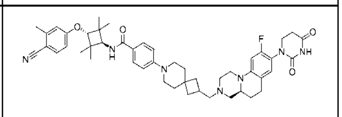 |
| 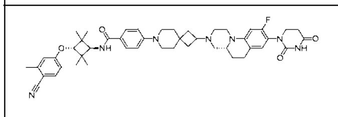 | 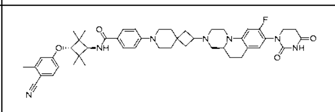 | |
| | | |
| | | |
| | | |

| | | |
|---|---|---|
| 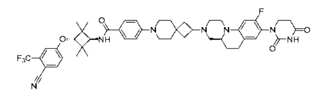 | 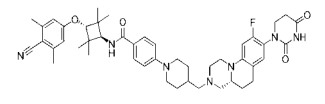 | 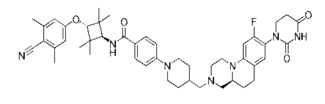 |
| 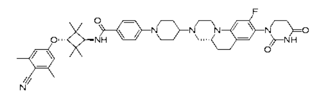 | 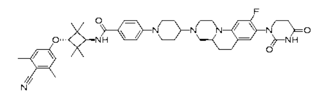 | 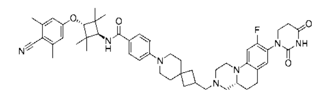 |
| 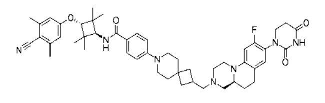 | 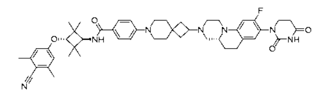 | 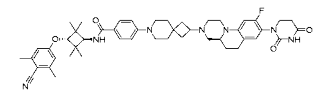 |
| 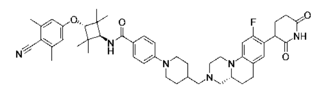 | 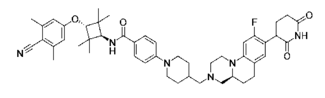 | 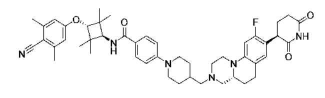 |
| 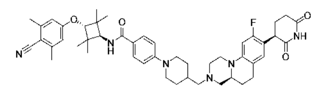 | 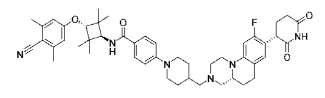 | 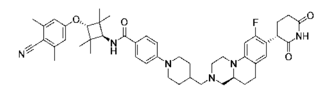 |
| 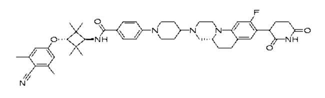 | 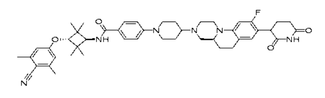 | 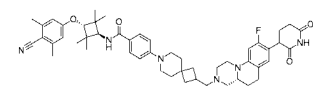 |
| 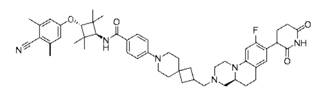 | 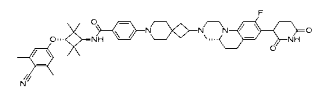 | 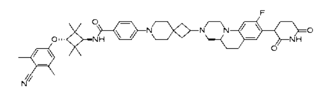 |
| 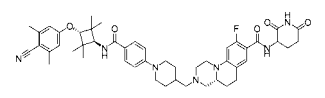 | 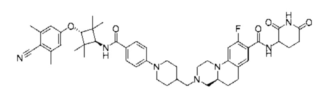 | 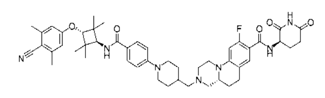 |
| 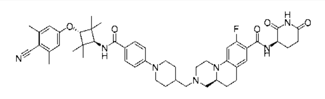 | 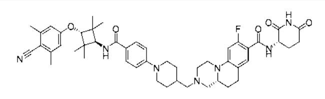 | 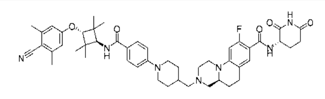 |
| 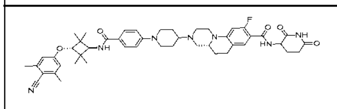 | 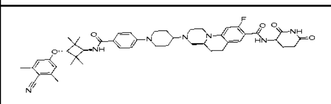 | 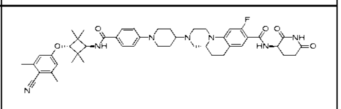 |
| 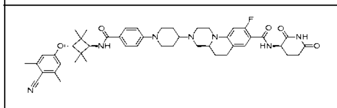 | 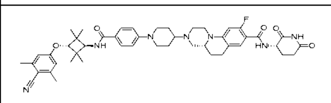 | 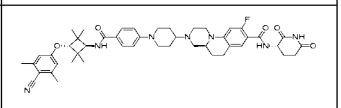 |
| 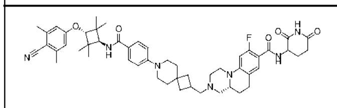 | 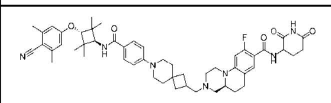 | 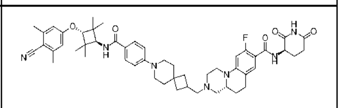 |
| 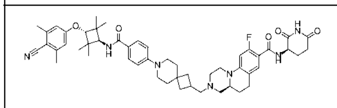 | 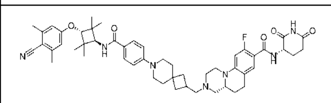 | 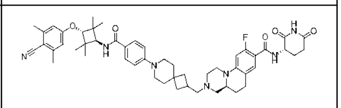 |
| 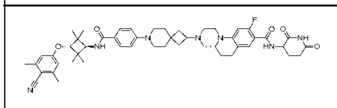 | | |
| | | |
| | | |

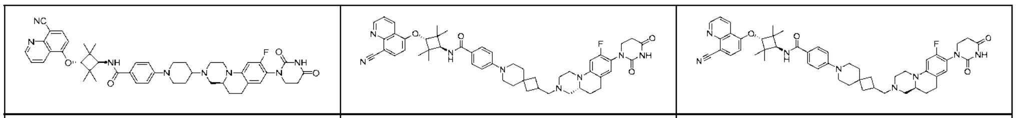

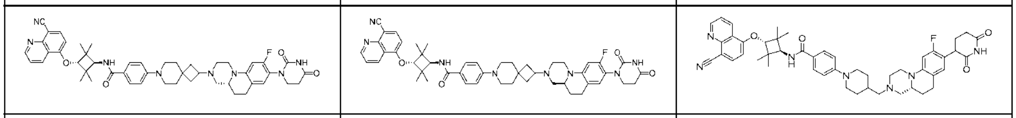

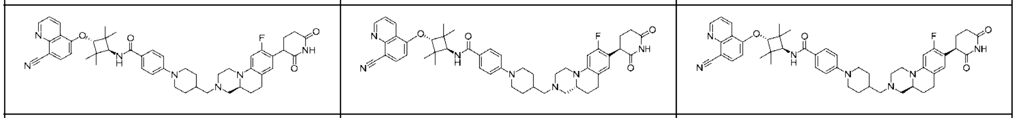

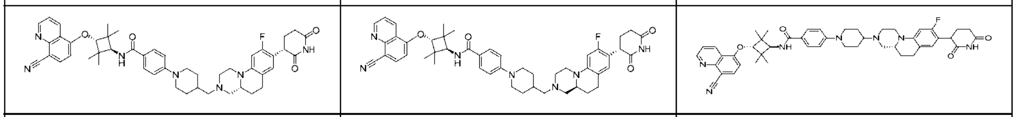

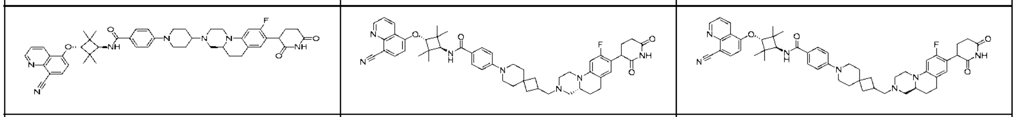

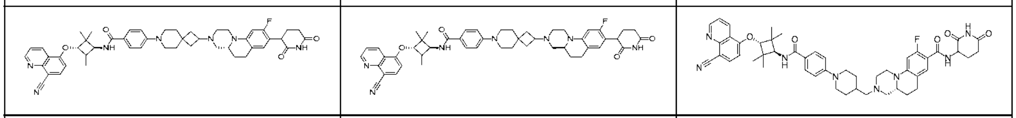

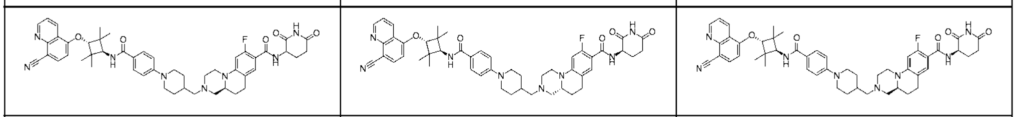

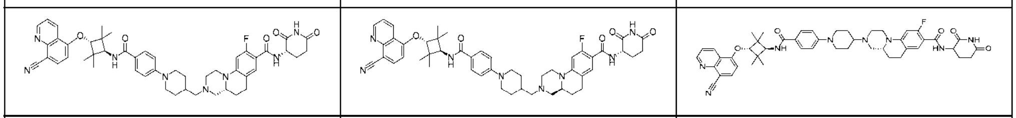

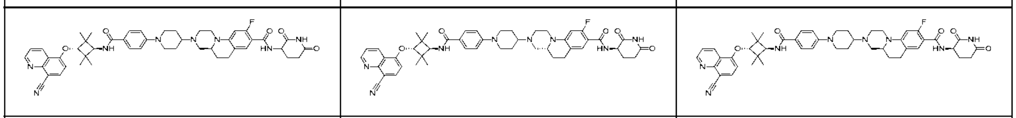

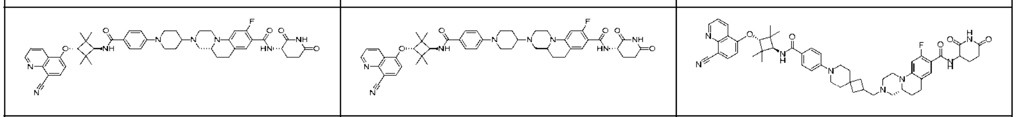

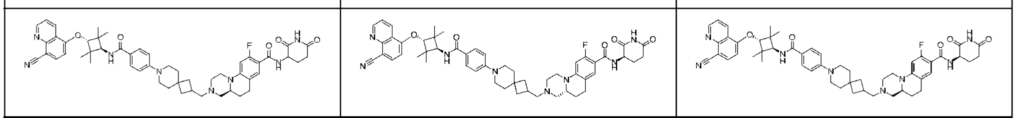

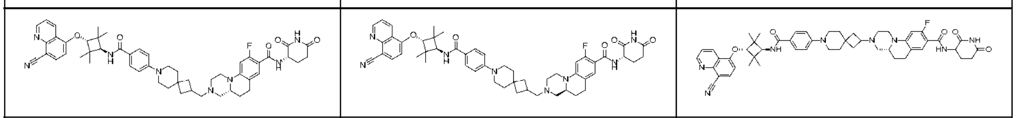

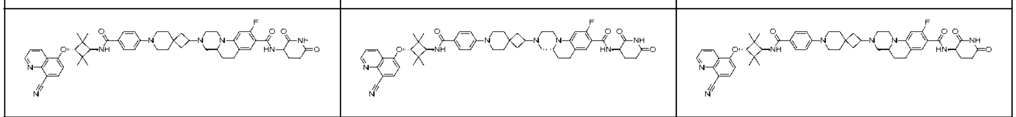

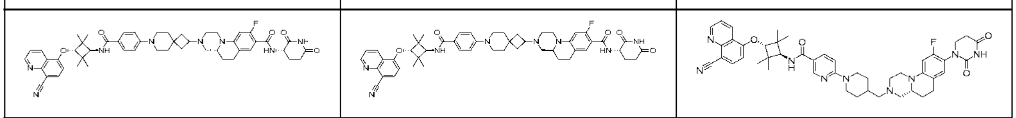

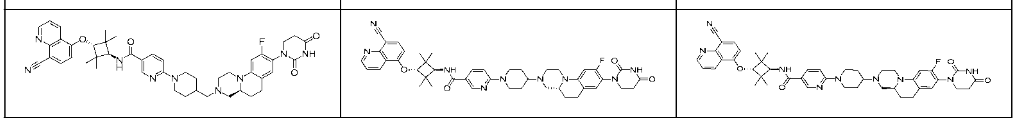

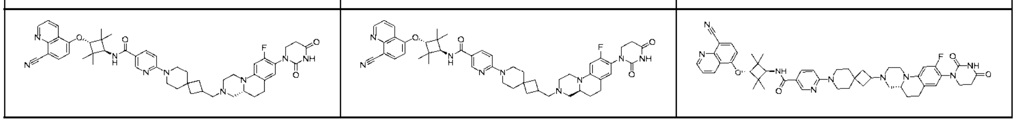

| | | |
|---|---|---|
| 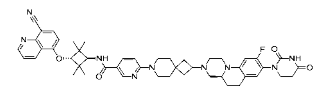 | 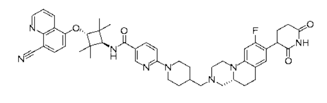 | 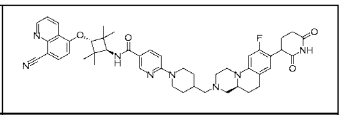 |
| 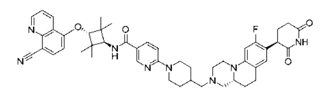 | 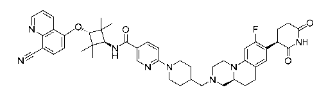 | 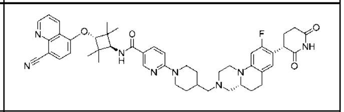 |
| 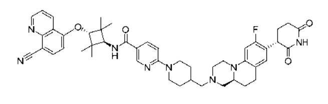 | 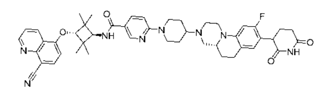 | 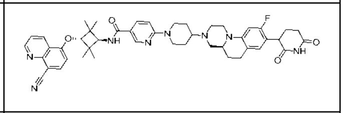 |
| 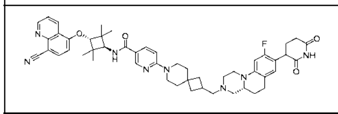 | 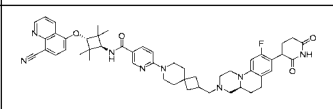 | 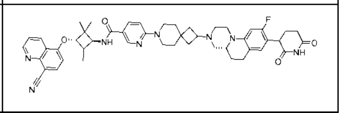 |
| 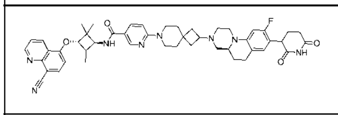 | 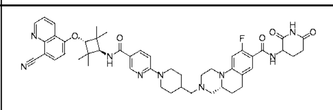 | 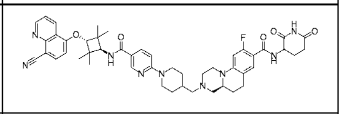 |
| 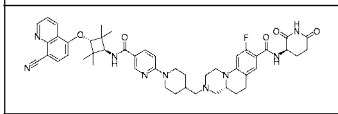 | 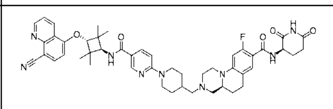 | 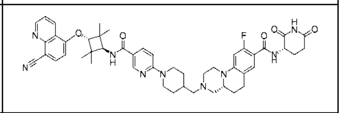 |
| 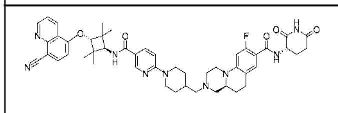 | 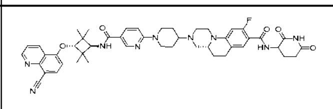 | 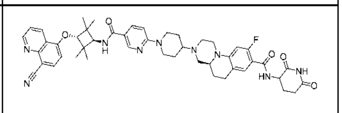 |
| 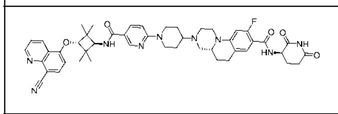 | 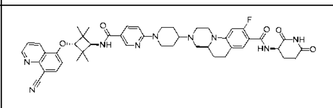 | 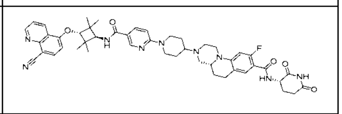 |
| 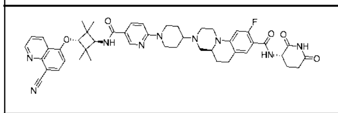 | 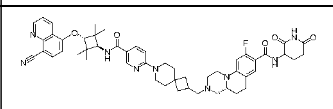 | 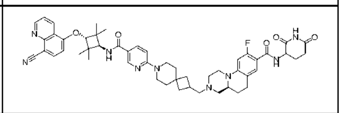 |
| 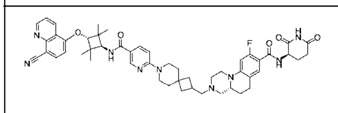 | 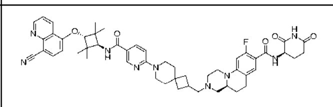 | 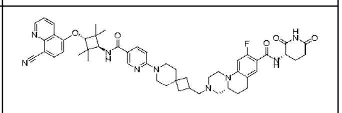 |
| 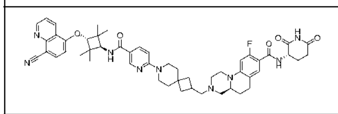 | 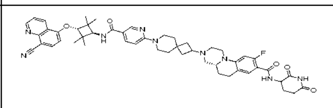 | 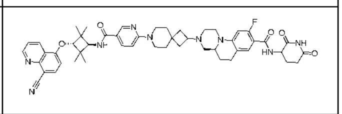 |
| 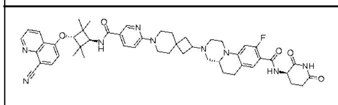 | 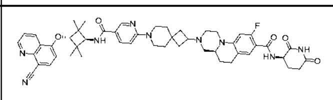 | 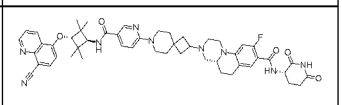 |
| 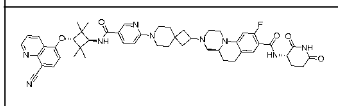 | 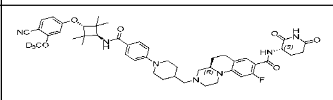 | 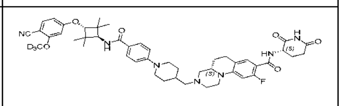 |
| 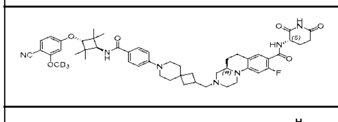 | | |
| | | |
| | | |

| | | |
|---|---|---|
| 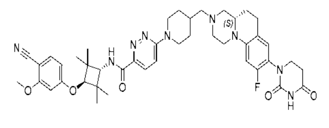 | 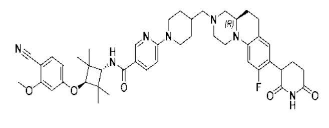 | 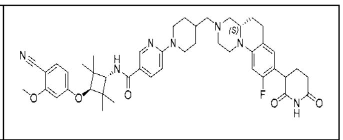 |
| 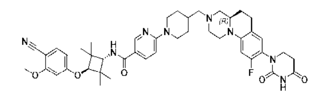 | 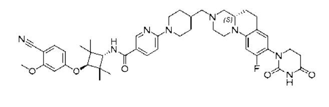 | 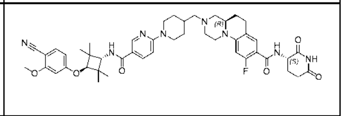 |
| 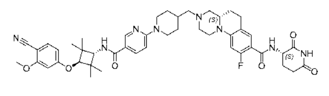 | 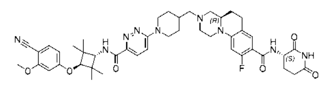 | 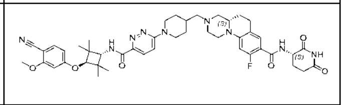 |
| 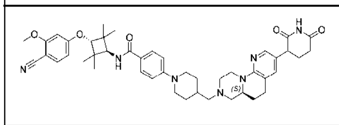 | 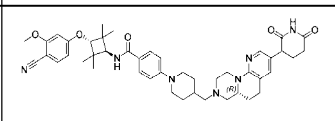 | 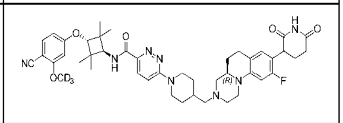 |
| 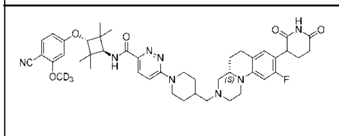 | 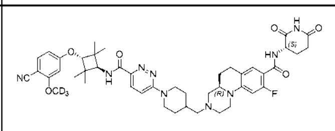 | 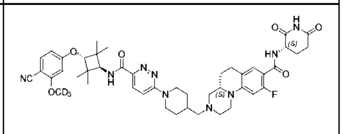 |
| 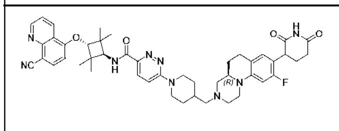 | 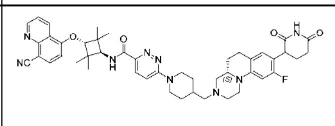 | 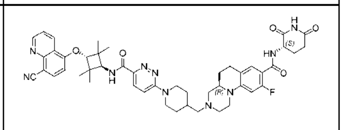 |
| 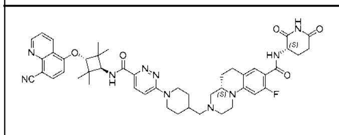 | 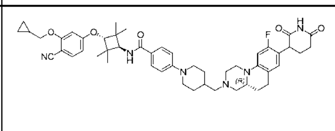 | 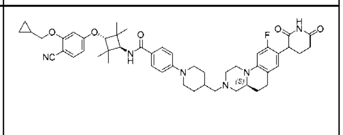 |
| 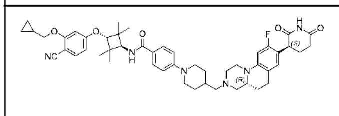 | 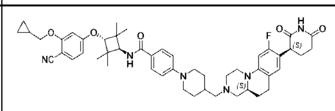 | 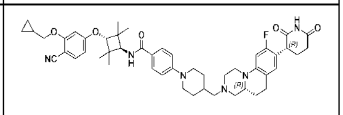 |
| 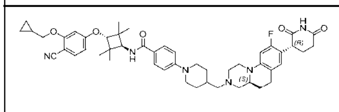 | 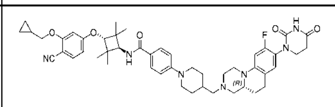 | 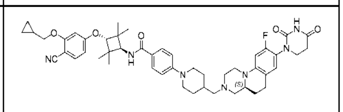 |
| 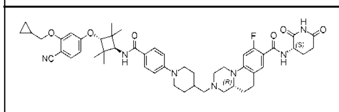 | 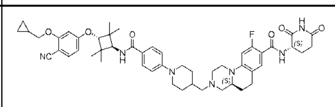 | 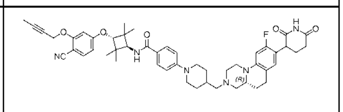 |
| 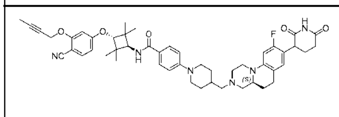 | 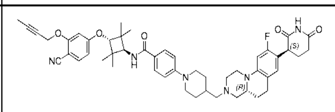 | 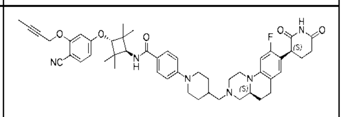 |
| 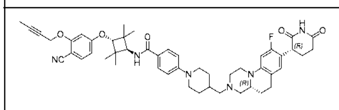 | 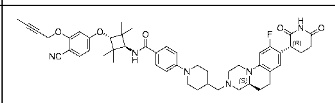 | 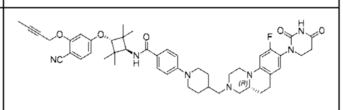 |
| 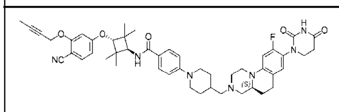 | 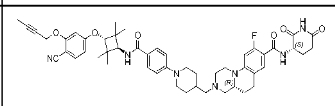 | 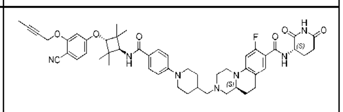 |
| 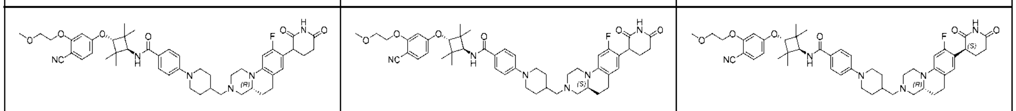 | | |

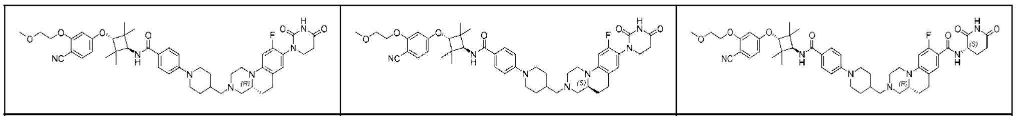

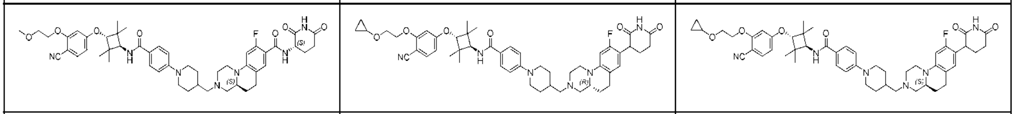

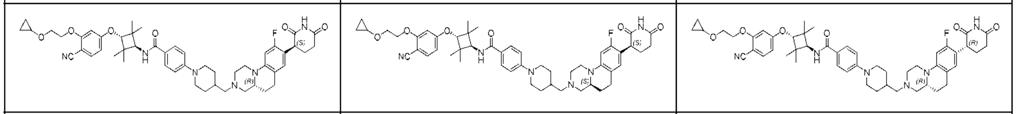

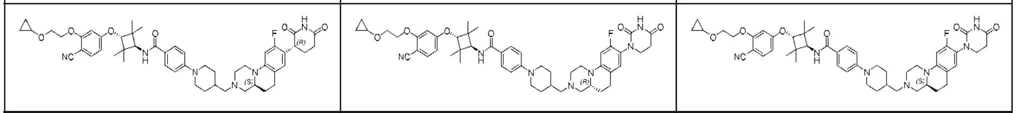

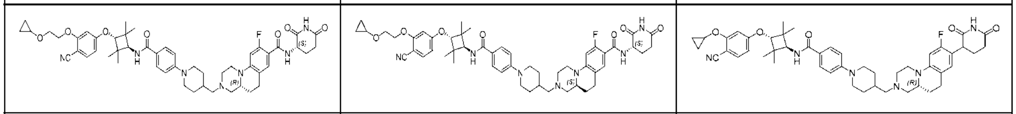

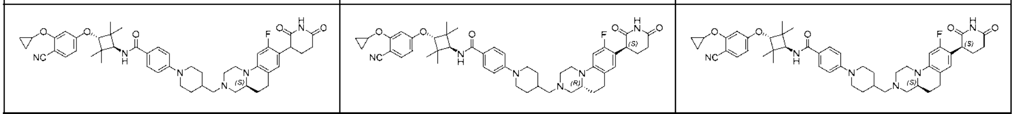

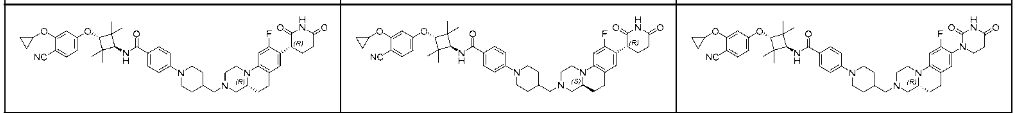

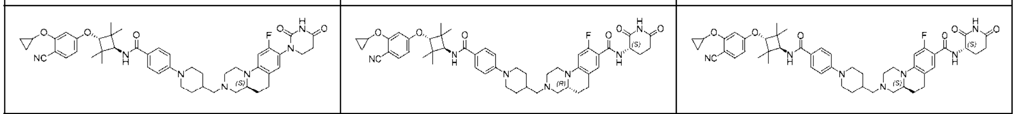

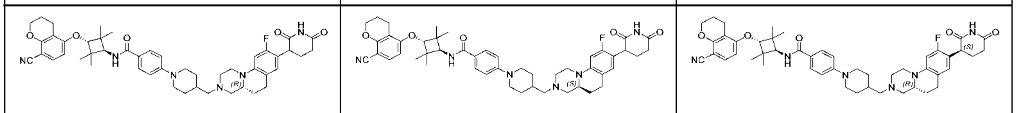

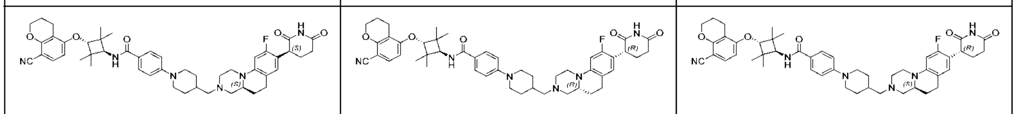

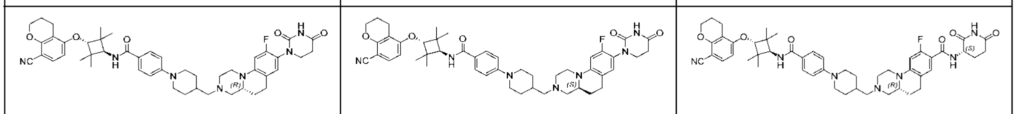

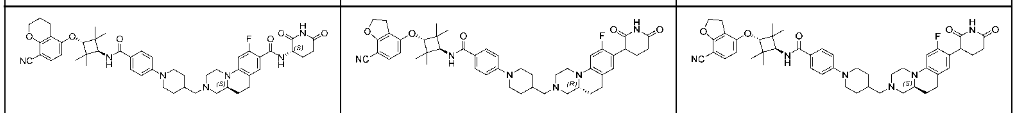

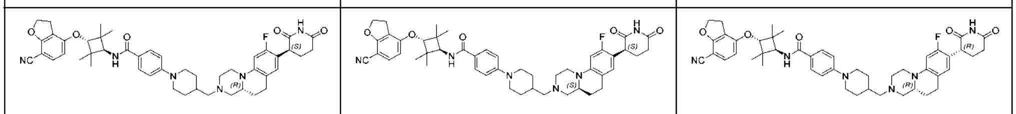

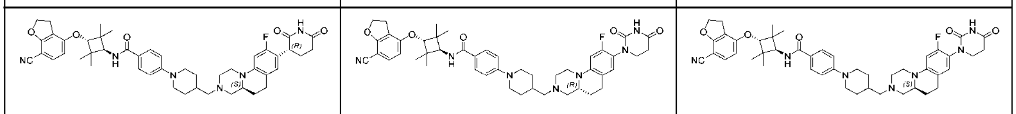

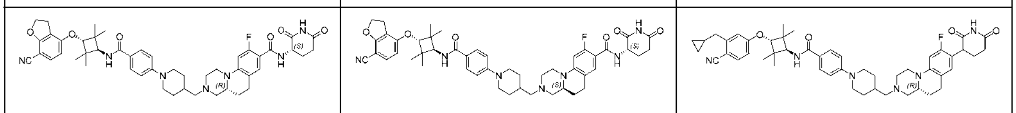

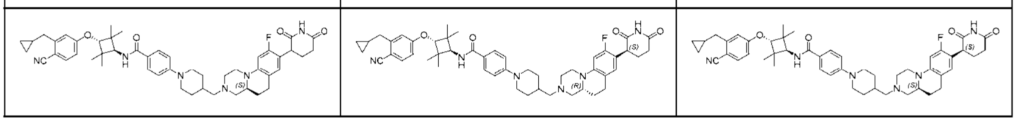

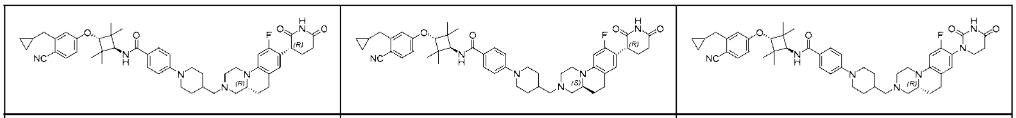

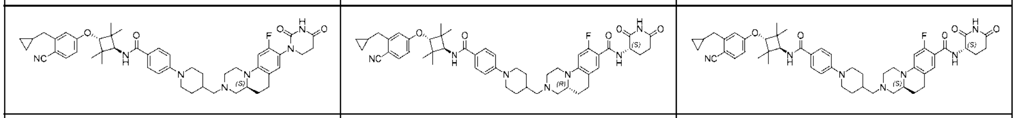

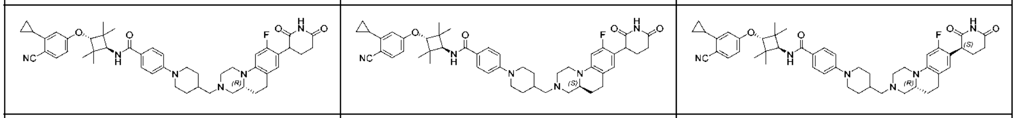

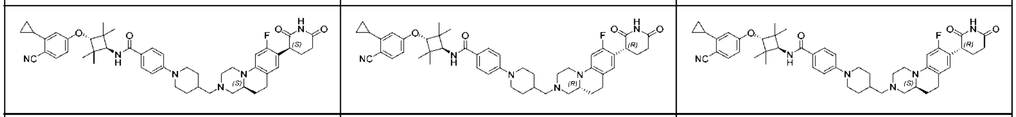

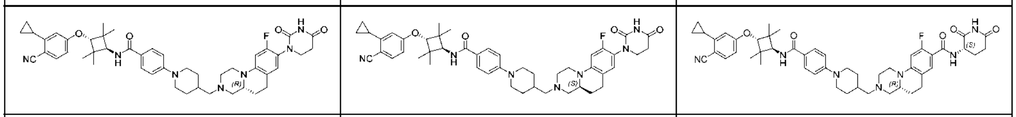

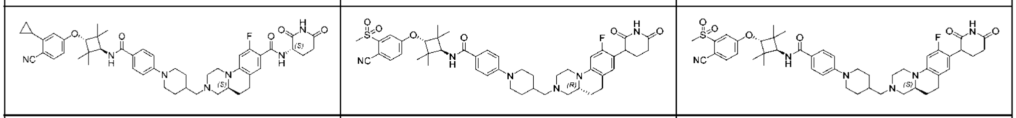

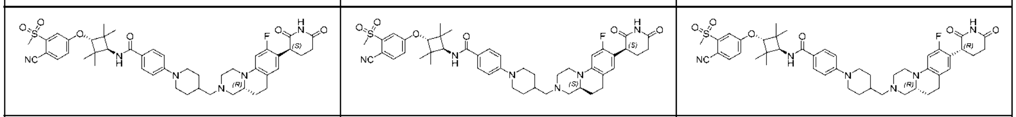

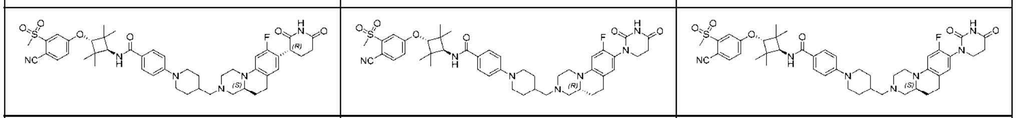

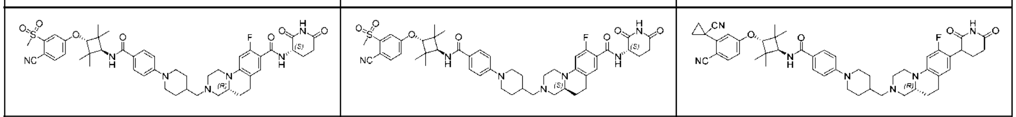

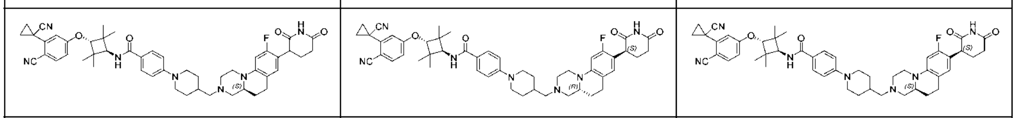

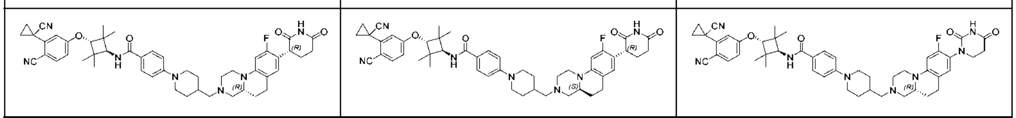

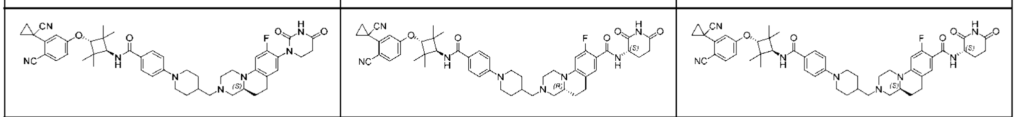

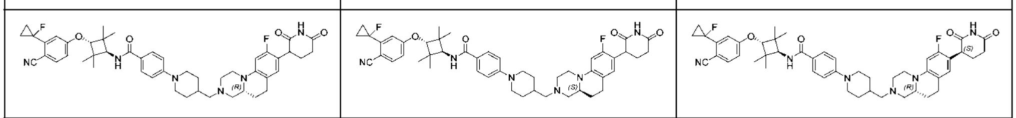

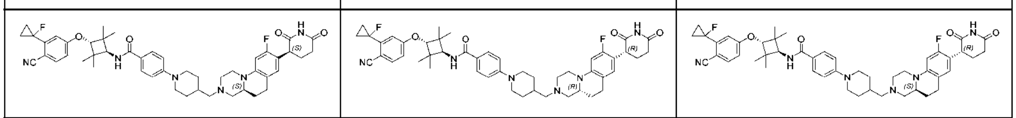

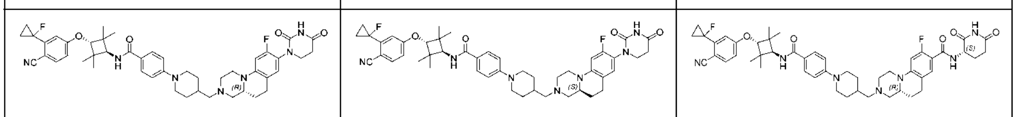

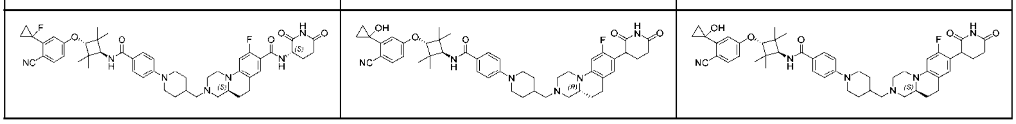

| | | |
|---|---|---|
| 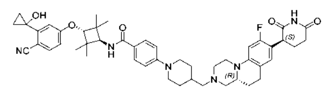 | 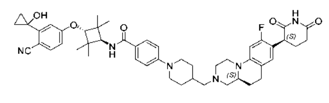 | 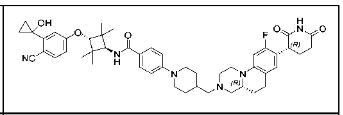 |
| 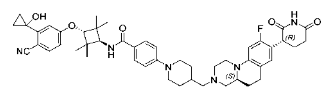 | 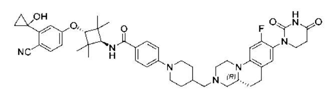 | 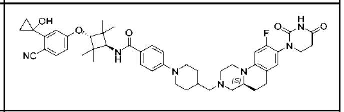 |
| 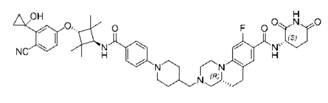 | 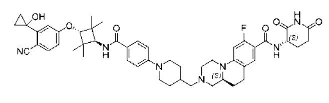 | 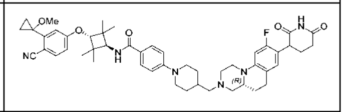 |
| 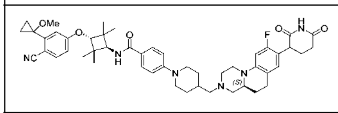 | 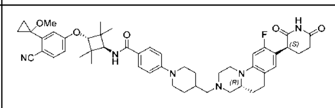 | 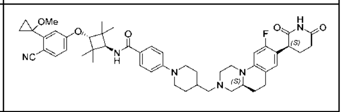 |
| 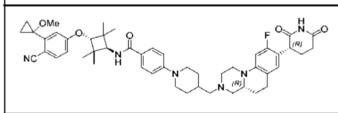 | 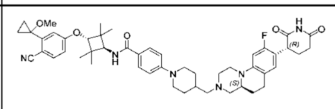 | 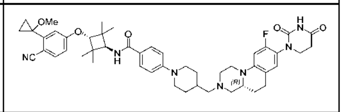 |
| 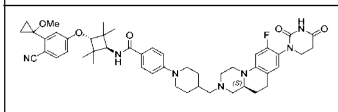 | 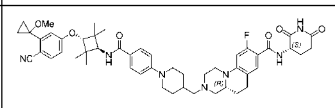 | 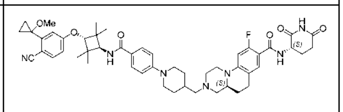 |
| 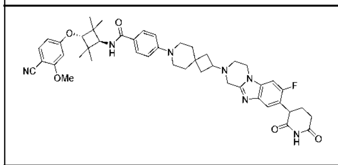 | 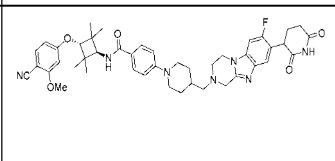 | 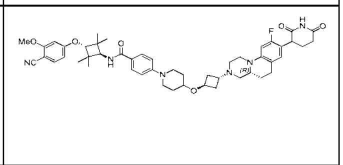 |
| 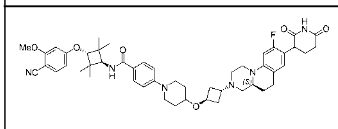 | 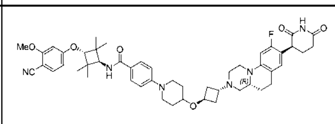 | 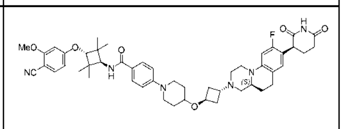 |
| 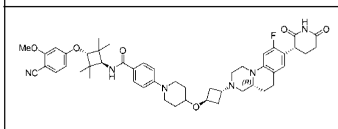 | 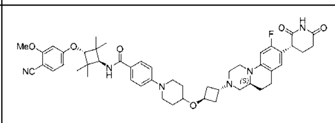 | 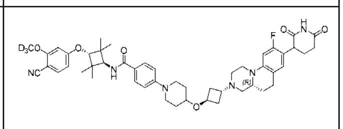 |
| 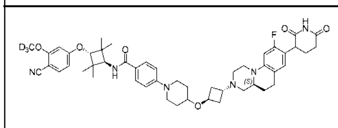 | 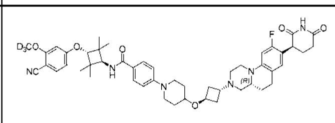 | 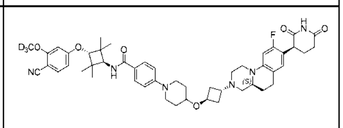 |
| 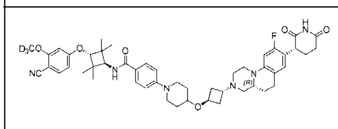 | 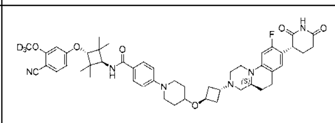 | 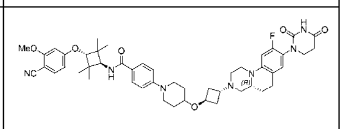 |
| 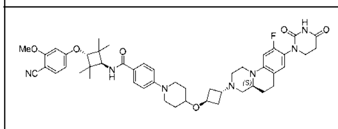 | 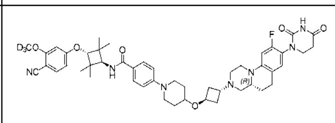 | 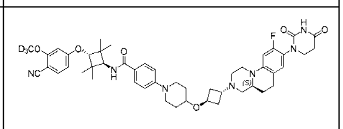 |
| 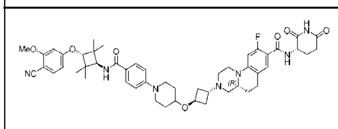 | 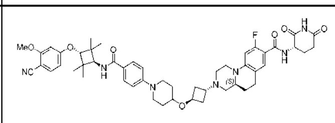 | 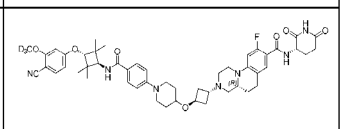 |
| 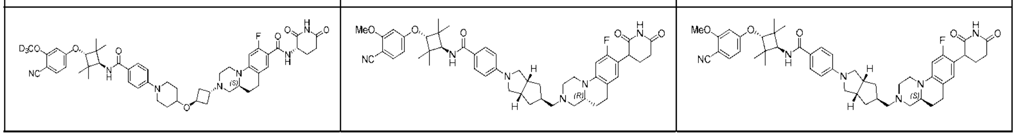 | | |

| | | |
|---|---|---|
| 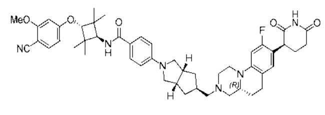 | 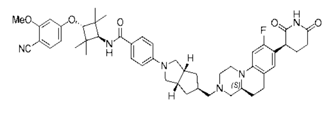 | 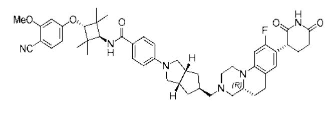 |
| 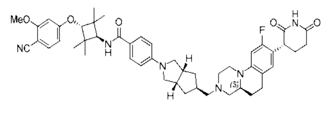 | 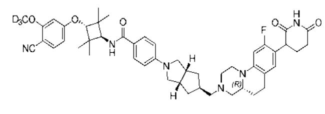 | 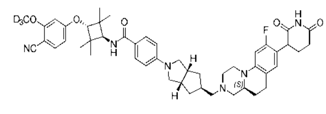 |
| 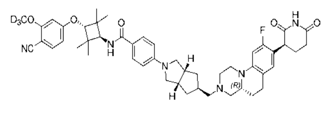 | 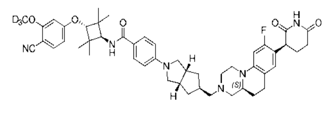 | 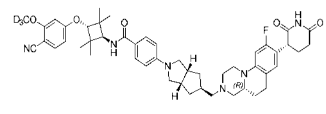 |
| 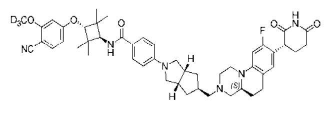 | 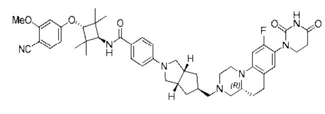 | 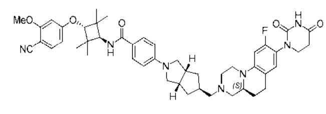 |
| 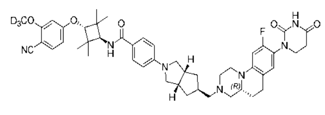 | 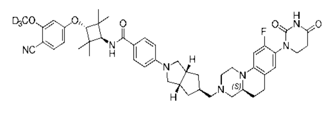 | 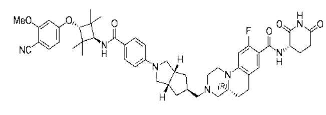 |
| 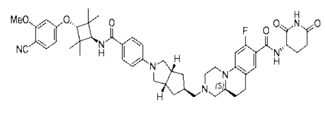 | 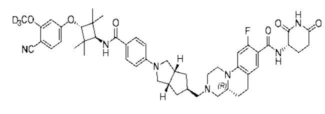 | 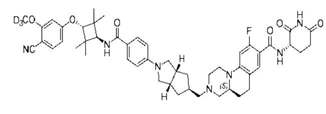 |
| 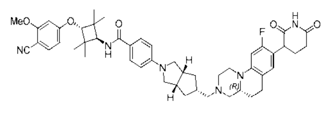 | 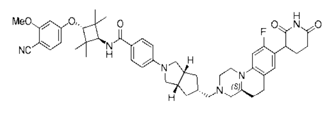 | 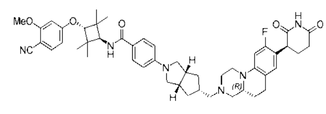 |
| 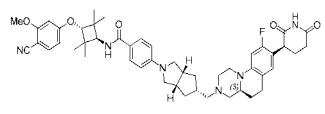 | 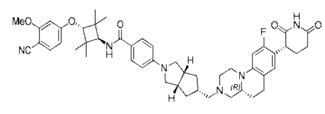 | 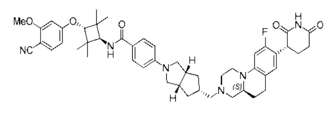 |
| 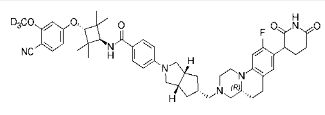 | 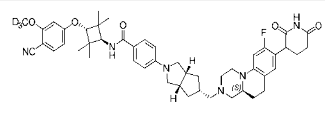 | 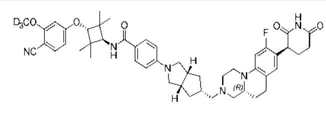 |
| 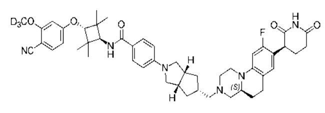 | 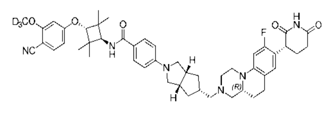 | 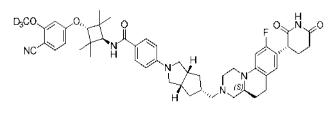 |
| 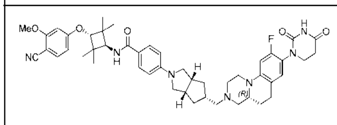 | 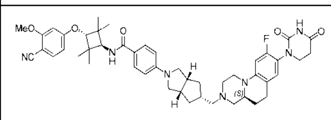 | 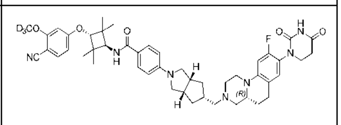 |
| 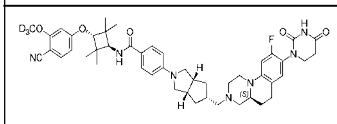 | 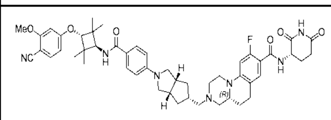 | 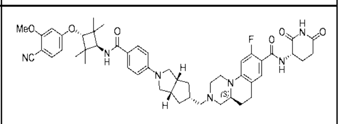 |
| 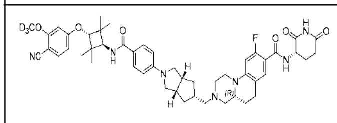 | 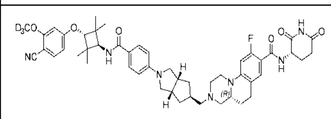 | 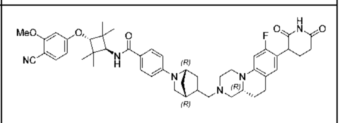 |
| 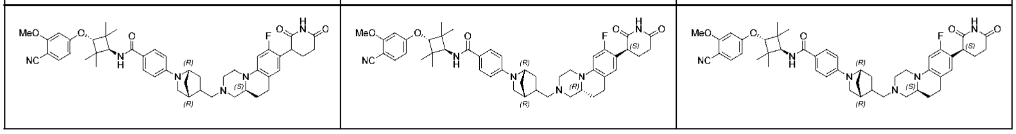 | | |

| | | |
|---|---|---|
| 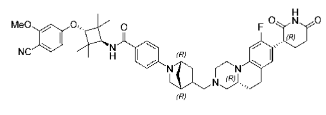 | 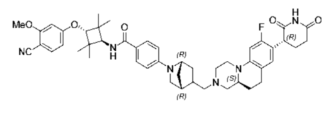 | 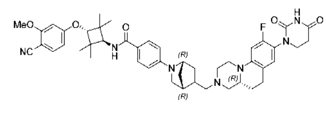 |
| 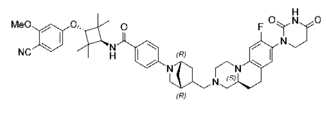 | 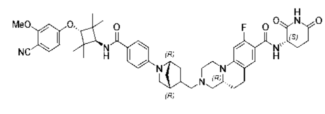 | 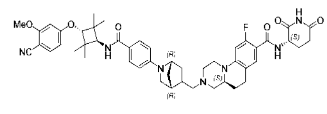 |
| 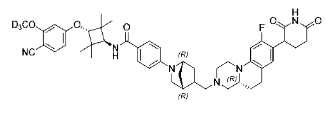 | 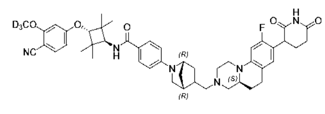 | 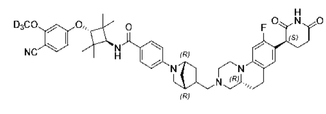 |
| 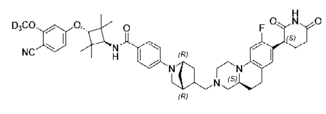 | 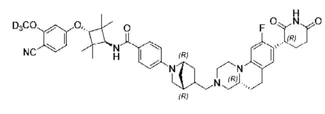 | 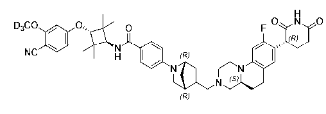 |
| 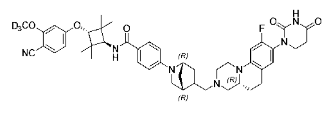 | 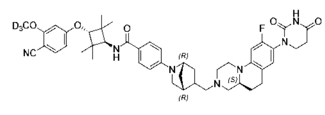 | 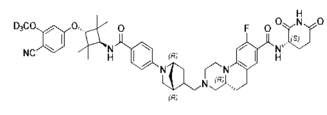 |
| 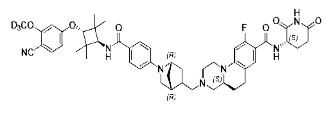 | 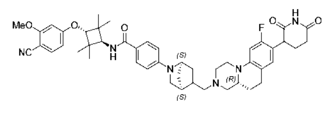 | 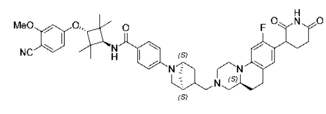 |
| 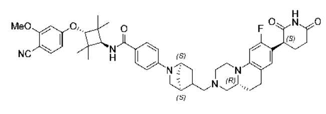 | 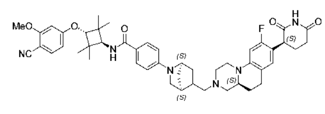 | 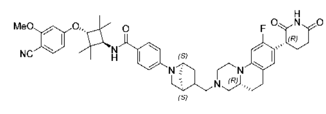 |
| 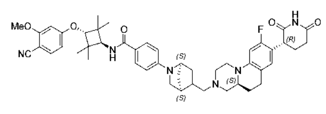 | 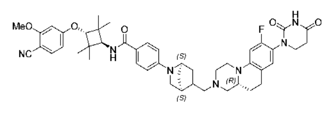 | 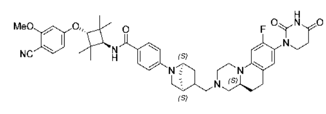 |
| 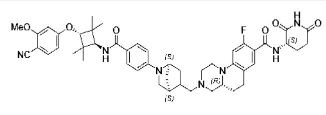 | 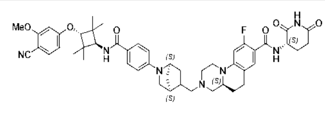 | 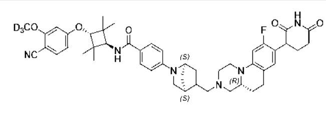 |
| 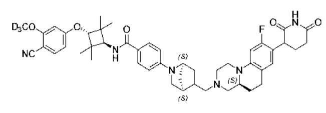 | 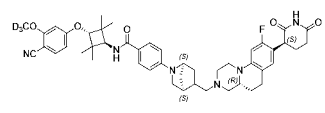 | 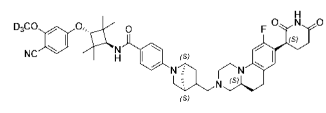 |
| 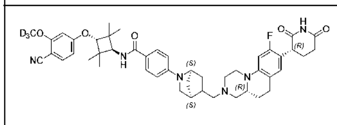 | 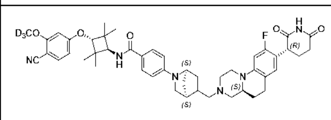 | 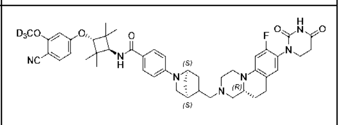 |
| 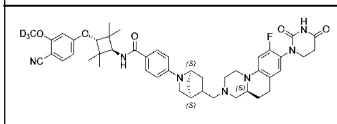 | 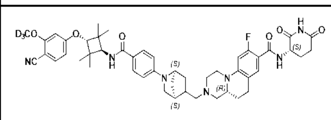 | 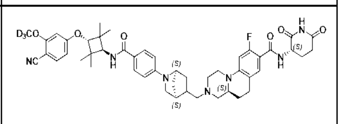 |
| 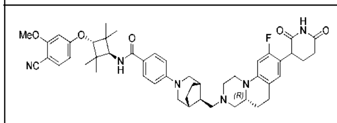 | 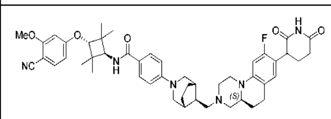 | 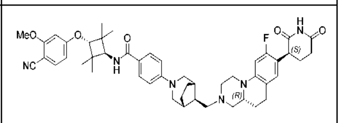 |
| 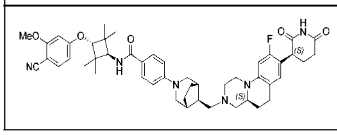 | | |

| | | |
|---|---|---|
| 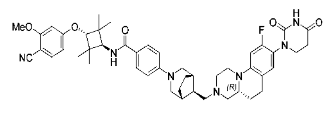 | 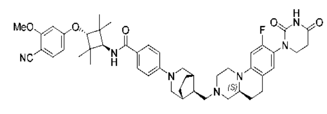 | 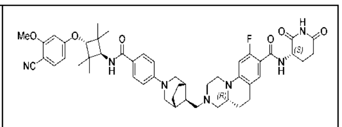 |
| 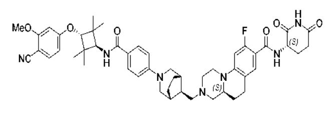 | 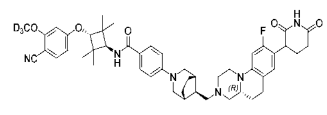 | 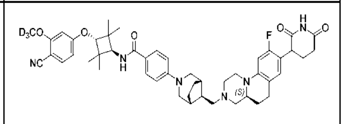 |
| 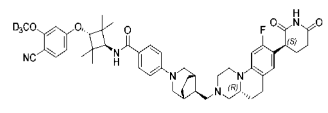 | 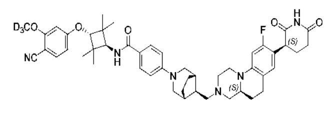 | 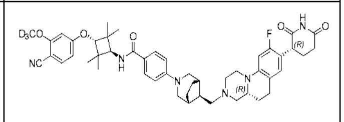 |
| 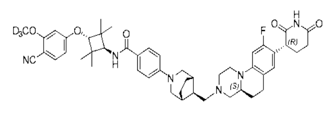 | 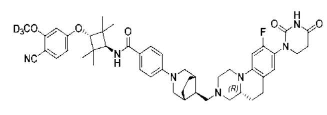 | 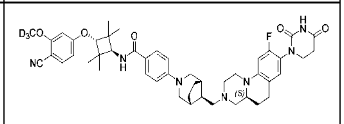 |
| 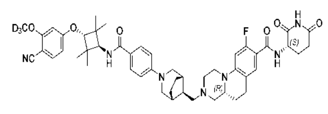 | 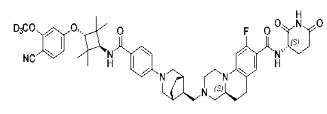 | 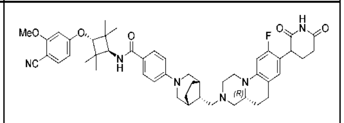 |
| 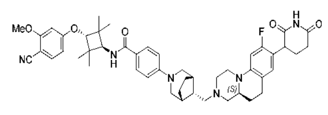 | 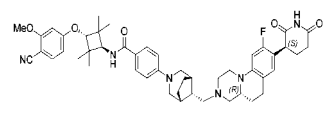 | 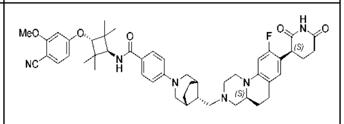 |
| 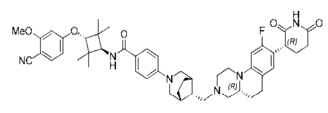 | 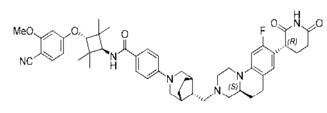 | 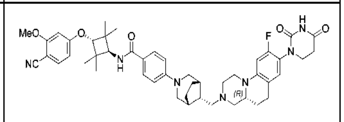 |
| 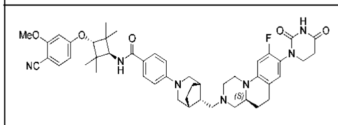 | 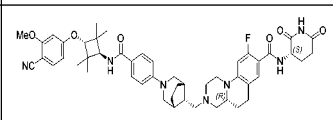 | 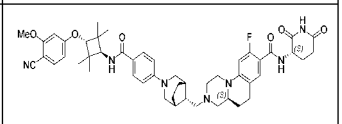 |
| 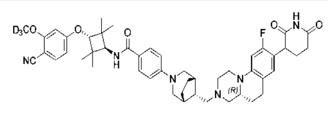 | 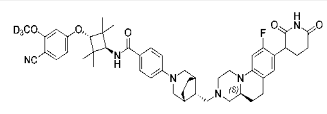 | 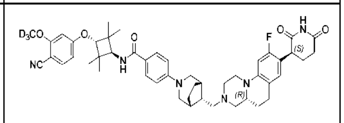 |
| 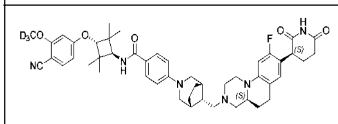 | 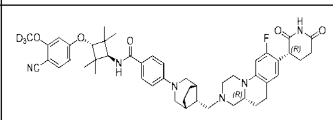 | 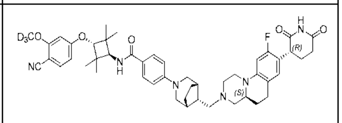 |
| 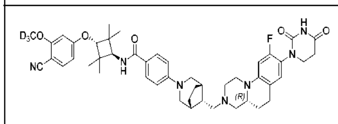 | 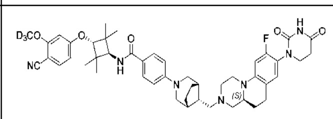 | 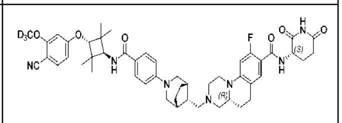 |
| 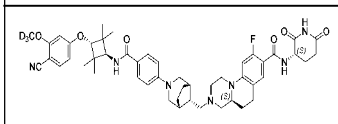 | 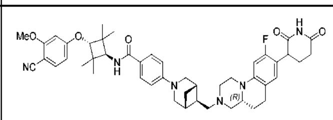 | 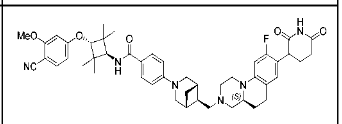 |
| 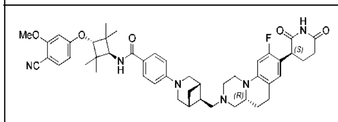 | 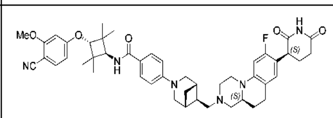 | 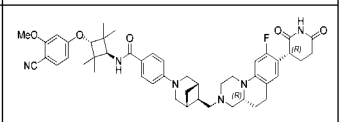 |
| 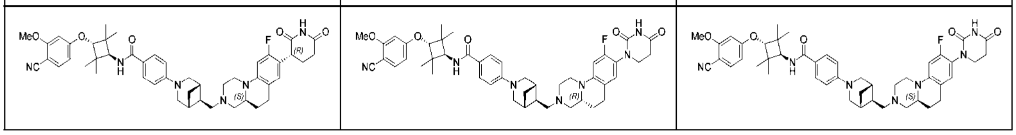 | | |

| | | |
|---|---|---|
| 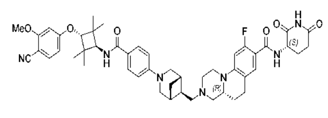 | 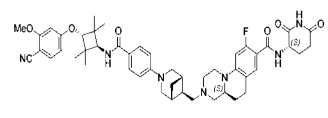 | 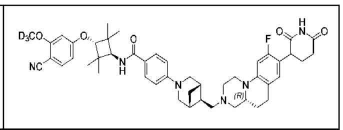 |
| 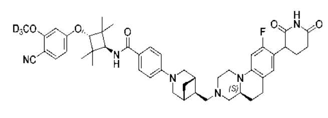 | 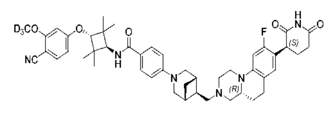 | 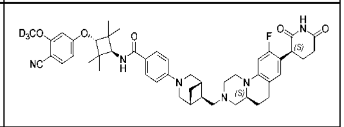 |
| 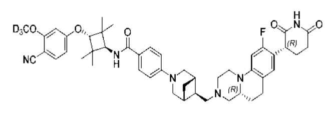 | 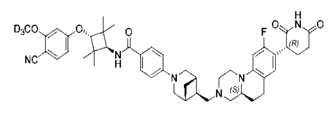 | 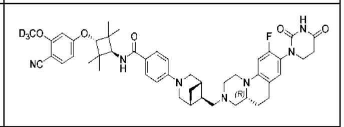 |
| 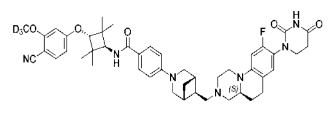 | 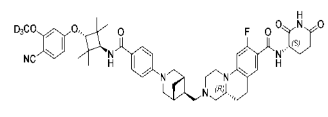 | 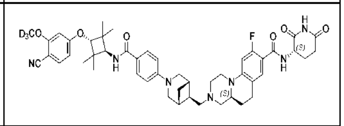 |
| 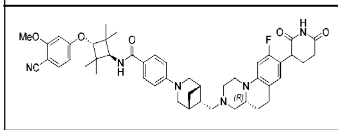 | 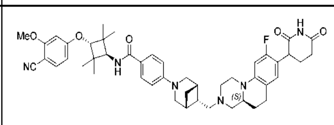 | 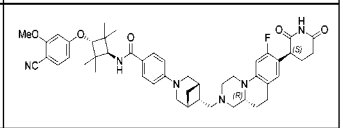 |
| 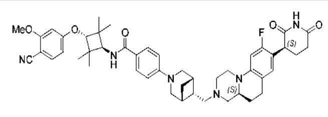 | 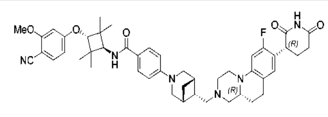 | 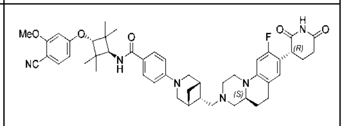 |
| 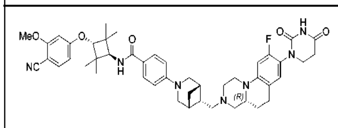 | 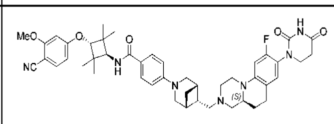 | 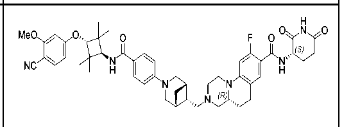 |
| 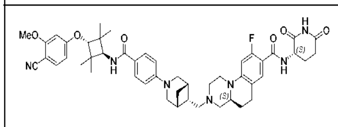 | 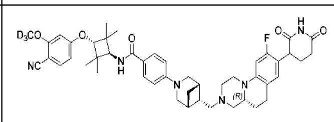 | 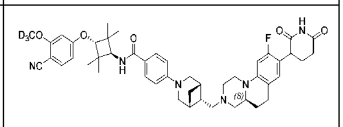 |
| 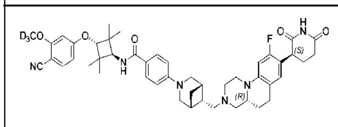 | 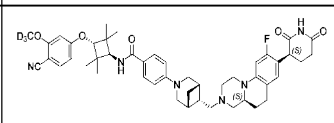 | 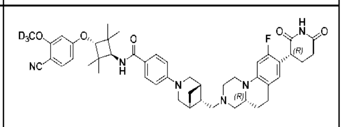 |
| 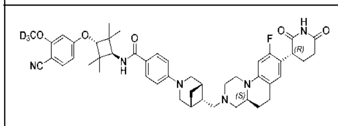 | 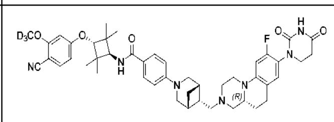 | 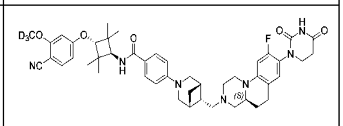 |
| 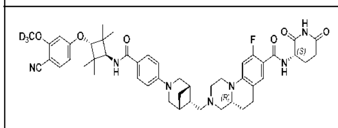 | 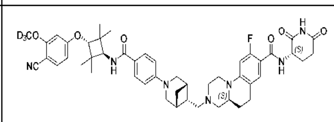 | 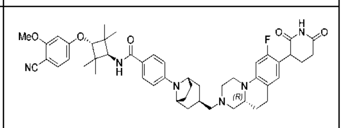 |
| 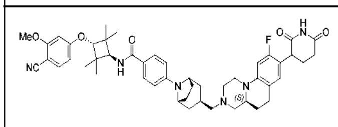 | 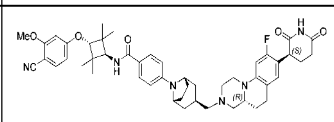 | 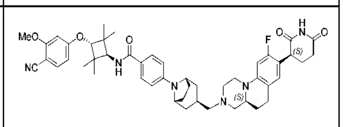 |
| 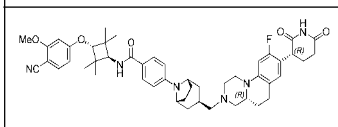 | 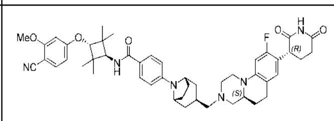 | 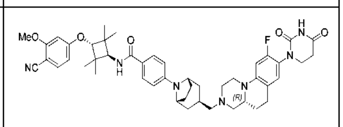 |
| 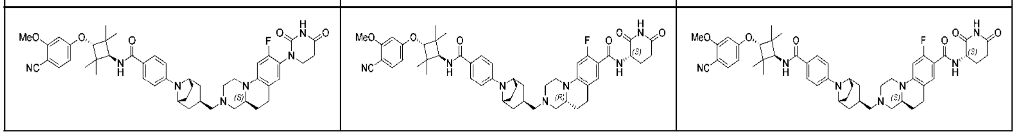 | | |

| | | |
|---|---|---|
| 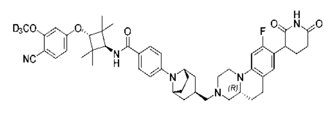 | 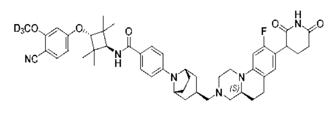 | 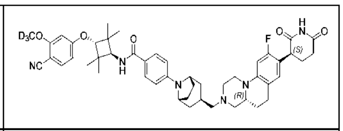 |
| 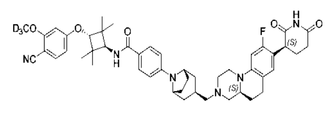 | 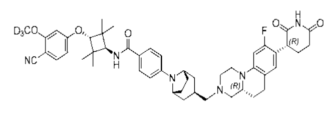 | 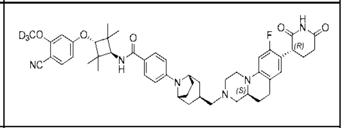 |
| 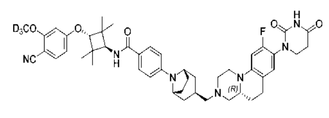 | 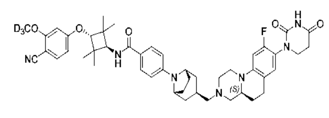 | 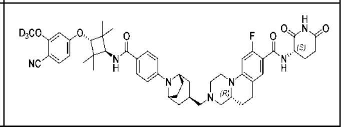 |
| 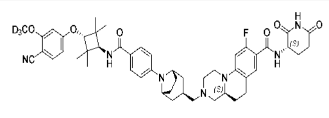 | 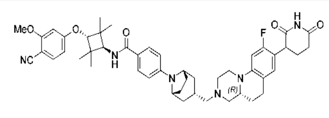 | 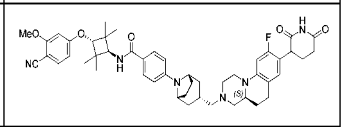 |
| 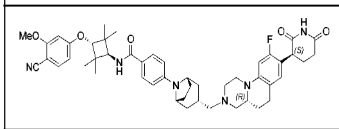 | 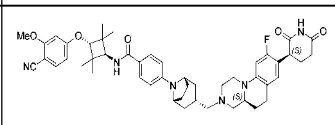 | 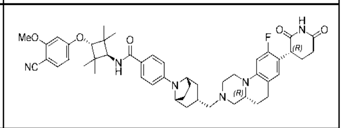 |
| 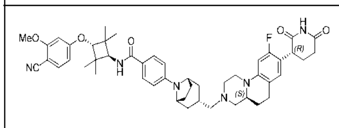 | 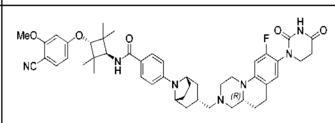 | 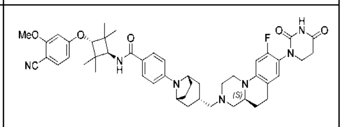 |
| 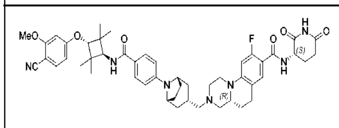 | 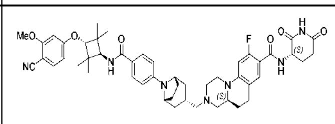 | 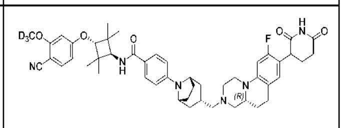 |
| 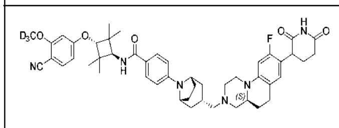 | 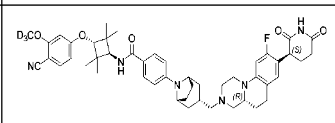 | 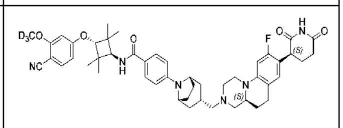 |
| 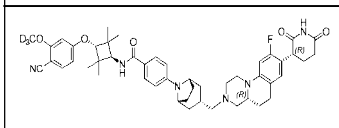 | 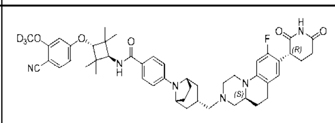 | 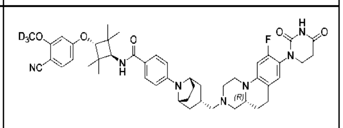 |
| 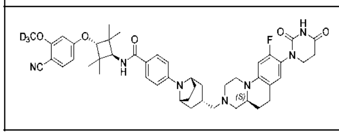 | 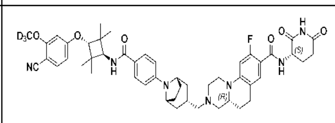 | 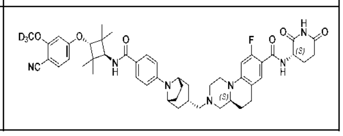 |

.

The present invention relates to a pharmaceutical composition, comprising the above-mentioned compound or the stereoisomer, tautomer, deuterated substance, solvate, prodrug, metabolite, pharmaceutically acceptable salt or co-crystal thereof according to the present invention, and a pharmaceutically acceptable carrier.

The present invention relates to the use of the above-mentioned compound or the stereoisomer, tautomer, deuterated substance, solvate, prodrug, metabolite, pharmaceutically acceptable salt or co-crystal thereof according to the present invention in the preparation of a medicament for the treatment of a disease related to AR activity or expression.

The present invention relates to the use of the above-mentioned compound or the stereoisomer, tautomer, deuterated substance, solvate, prodrug, metabolite, pharmaceutically acceptable salt or co-crystal thereof according to the present invention in the preparation of a medicament for the treatment of a disease related to the inhibition or degradation of AR. **In** some embodiments, the disease related to the inhibition or degradation of AR is cancer, preferably prostate cancer.

The present invention relates to a pharmaceutical composition or a pharmaceutical preparation, comprising a therapeutically effective amount of the compound or the stereoisomer, tautomer, deuterated substance, solvate, prodrug, metabolite, pharmaceutically acceptable salt or co-crystal thereof according to the present invention, and a pharmaceutically acceptable excipient. The pharmaceutical composition can be in a unit dosage form (the amount of the active pharmaceutical ingredient per unit dosage form is also referred to as the "dosage strength").

The present invention further provides a method for treating a disease in a mammal, comprising administering to the mammal a therapeutically effective amount of the compound or the stereoisomer, tautomer, deuterated substance, solvate, prodrug, metabolite, pharmaceutically acceptable salt or co-crystal thereof, or the pharmaceutical composition according to the present invention. **In** some embodiments, the mammal according to the present invention comprises human.

The term "effective amount" or "therapeutically effective amount" according to the present application refers to a sufficient amount of the compound disclosed in the present application that is administered to ameliorate, to some extent, one or more symptoms of a disease or condition (such as cancer) being treated. **In** some embodiments, the outcome is the reduction and/or remission of signs, symptoms or causes of the disease, or any other desired change in the biological system. For example, an "effective amount" in terms of the therapeutic use is an amount of the included compound disclosed in the present application that is required to provide clinically significant reduction of the symptoms of the disease. Examples of the therapeutically effective amount include, but are limited to 1-1500 mg, **1-1000** mg, 1-900 mg, 1-800 mg, 1-700 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 80-1500 mg, 80-1000 mg, and 80-800 mg.

In some embodiments, the pharmaceutical composition comprises the compound or the stereoisomer, tautomer, deuterated substance, solvate, prodrug, metabolite, pharmaceutically acceptable salt or co-crystal thereof according to the present invention in an amount including but not limited to 1-1500 mg, 1-1000 mg, 20-800 mg, 40-800 mg, 40-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 320 mg, 400 mg, 480 mg, 500 mg, 600 mg, 640 mg, 840 mg, and 1000 mg.

Provided is a method for treating a disease in a mammal, comprising administering to a subject a therapeutically effective amount of the compound or the stereoisomer, tautomer, deuterated substance, solvate, prodrug, metabolite, pharmaceutically acceptable salt or co-crystal thereof according to the present invention, wherein the therapeutically effective amount is preferably 1-1500 mg, and the disease is preferably cancer, further preferably prostate cancer.

Provided is a method for treating a disease in a mammal, comprising administering to a subject a drug, i.e., the compound or the stereoisomer, tautomer, deuterated substance, solvate, prodrug, metabolite, pharmaceutically acceptable salt or co-crystal thereof according to the present invention in a daily dose of 1-1500 mg/day, wherein the daily dose can be a single dose or divided doses; in some embodiments, the daily dose includes, but is not limited to 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, and 200-400 mg/day; in some embodiments, the daily dose includes, but is not limited to 10 mg/day, 20 mg/day, 25 mg/day, 50 mg/day, 80 mg/day, 100 mg/day, 125 mg/day, 150 mg/day, 160 mg/day, 200 mg/day, 300 mg/day, 320 mg/day, 400 mg/day, 480 mg/day, 600 mg/day, 640 mg/day, 800 mg/day, 1000 mg/day, and 1500 mg/day.

The present invention relates to a kit, wherein the kit may comprise a composition in the form of a single dose or multiple doses and comprises the compound or the stereoisomer, tautomer, deuterated substance, solvate, prodrug, metabolite, pharmaceutically acceptable salt or co-crystal thereof according to the present invention, and the amount of the compound or the stereoisomer, tautomer, deuterated substance, solvate, prodrug, metabolite, pharmaceutically acceptable salt or co-crystal thereof according to the present invention is identical to the amount of same in the above-mentioned pharmaceutical composition.

In the present invention, the amount of the compound or the stereoisomer, tautomer, deuterated substance, solvate, prodrug, metabolite, pharmaceutically acceptable salt or co-crystal thereof according to the present invention is calculated in the form of free base in each case.

Unless otherwise stated, the terms used in the description and claims have the following meanings.

The carbon, hydrogen, oxygen, sulphur, nitrogen, phosphorus, F, Cl, Br, I, etc. involved in the groups and compounds of the present invention all comprise their isotopes. That is, the carbon, hydrogen, oxygen, sulphur, nitrogen, phosphorus, F, Cl, Br, I, etc. involved in the groups and compounds of the present invention are optionally further substituted with one or more of their corresponding isotopes, wherein the isotopes of carbon comprise $^{11}C$, $^{12}C$, $^{13}C$ and $^{14}C$; the isotopes of hydrogen comprise protium (H), deuterium (D, also known as heavy hydrogen), and tritium (T, also known as superheavy hydrogen); the isotopes of oxygen comprise $^{15}O$, $^{16}O$, $^{17}O$ and $^{18}O$; the isotopes of sulphur

comprise $^{32}S$, $^{33}S$, $^{34}S$, $^{35}S$ and $^{36}S$; the isotopes of nitrogen comprise $^{13}N$, $^{14}N$ and $^{15}N$; the isotopes of fluorine comprise $^{17}F$, $^{18}F$ and $^{19}F$; the isotopes of chlorine comprise $^{35}Cl$, $^{36}Cl$ and $^{37}Cl$; the isotopes of bromine comprise $^{79}Br$ and $^{81}Br$; the isotopes of iodine comprise $^{123}I$ and $^{125}I$; and the isotopes of phosphorus comprise $^{31}P$ and $^{32}P$.

"CN" refers to cyano.

"Halogen" refers to F, Cl, Br or I.

"Alkyl" refers to a substituted or unsubstituted linear or branched saturated aliphatic hydrocarbon group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and various branched isomers thereof. The alkyl can be monovalent, divalent, trivalent or tetravalent.

"Alkylene" refers to a substituted or unsubstituted linear or branched divalent saturated hydrocarbon group, including $-(CH_2)_v-$ (v is an integer from 1 to 10). Examples of the alkylene include, but are not limited to, methylene, ethylene, propylene, and butylene.

"Cycloalkyl" refers to a substituted or unsubstituted saturated carbocyclic hydrocarbon group, typically having 3 to 12 carbon atoms, and the cycloalkyl can be monocyclic, fused, bridged and spirocyclic. Non-limiting examples include cyclopropyl, cyclobutyl, cyclopentyl, cyclohexyl, cycloheptyl, cyclobutyl fused cyclobutyl, cyclobutyl spiro cyclobutyl, adamantane, etc. The cycloalkyl can be monovalent, divalent, trivalent or tetravalent.

"Heterocycloalkyl" refers to a substituted or unsubstituted saturated heteroatom-containing cyclic hydrocarbon group, including but not limited to 3 to 12 atoms, or 3 to 8 atoms, wherein 1 to 3 heteroatoms are included and selected from N, O, S or Se. The C, N and S in the ring of the heterocycloalkyl can be oxidised to various oxidation states. The heterocycloalkyl can be monocyclic, fused, bridged and spirocyclic. The heterocycloalkyl can be connected to a heteroatom or a carbon atom, and non-limiting examples include oxiranyl, aziridinyl, oxetanyl, azetidinyl, tetrahydrofuryl, tetrahydro-2H-pyranyl, dioxolanyl, dioxanyl, pyrrolidyl, piperidyl, imidazolidinyl, oxazolidinyl, oxazinanyl, morpholinyl, hexahydropyrimidyl, piperazinyl,

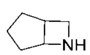, 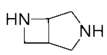, 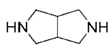, 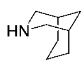, 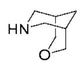, 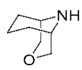, 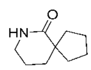,

and

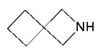.

The heterocycloalkyl can be monovalent, divalent, trivalent or tetravalent.

"Alkenyl" refers to a substituted or unsubstituted linear or branched unsaturated hydrocarbon group, having at least 1, typically 1, 2 or 3 carbon-carbon double bonds, with a main chain including but not limited to, 2 to 10, 2 to 6, or 2 to 4 carbon atoms. Examples of the alkenyl include, but are not limited to, vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, and 1,4-hexadiene. The alkenyl can be monovalent, divalent, trivalent or tetravalent.

"Alkynyl" refers to a substituted or unsubstituted linear or branched unsaturated hydrocarbon group, having at least 1, typically 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of the alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, and 4-decynyl. The alkynyl can be monovalent, divalent, trivalent or tetravalent.

"Alkoxy" refers to substituted or unsubstituted -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10- to 15-membered tricyclic ring, or 12- to 18-membered tetracyclic ring system. The carbocyclyl can be connected to an aromatic ring or non-aromatic ring, and the ring is optionally monocyclic, fused, bridged or spirocyclic. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring,

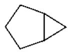, 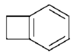, 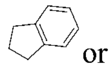 or 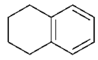.

The "carbocyclyl" or "carbocyclic ring" can be monovalent, divalent, trivalent or tetravalent.

"Heterocyclyl" or "heterocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10- to 15-membered tricyclic ring, or 12- to 18-membered tetracyclic ring system, and contains 1 or more (including but not limited to 2, 3, 4 or 5) heteroatoms selected from N, O, S or Se, and the selectively substituted C, N, S or Se in the ring of the heterocyclyl can be oxidised to various oxidation states. The heterocyclyl can be connected to a heteroatom or a carbon atom, can be connected to an aromatic ring or a non-aromatic ring, and is optionally monocyclic, bridged, fused or spirocyclic. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl,

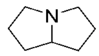, 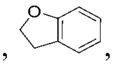,

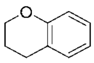, 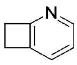, 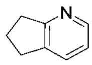, 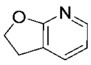, 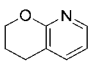, 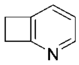, 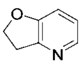, 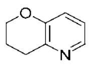, 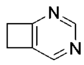, 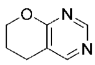,

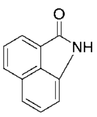, 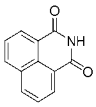, 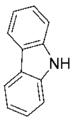, 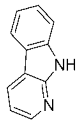, 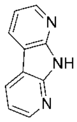, 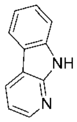, 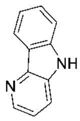, 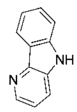, 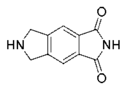,

and

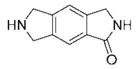.

The "heterocyclyl" or "heterocyclic ring" can be monovalent, divalent, trivalent or tetravalent.

"Spiro ring" or "spiro ring group" refers to a polycyclic group that shares one atom (called a spiro atom) between substituted or unsubstituted rings. The number of ring atoms in the spiro ring system includes, but is not limited to 5 to 20, 6 to 14, 6 to 12, or 6 to 10, wherein one or more rings may contain 0 or more (including but not limited to 1, 2, 3 or 4) double bonds, and may optionally contain 0 to 5 heteroatoms selected from N, O or $S(=O)_n$ (n is 0, 1, or 2). Non-limiting examples include:

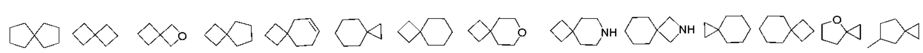

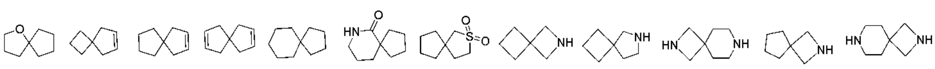.

The "spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, wherein n is 0, 1 or 2). The number of ring atoms in the fused ring system includes but is not limited to 5 to 20, 5 to 14, 5 to 12, or 5 to 10. Non-limiting examples include:

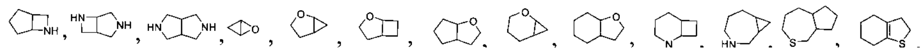

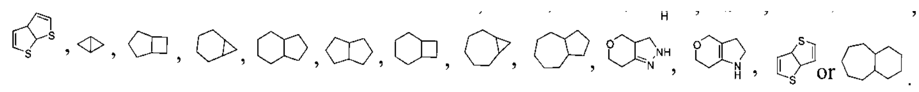

The "fused ring" or "fused ring group" can be monovalent, divalent, trivalent or tetravalent.

"Bridged ring" or "bridged ring group" refers to a substituted or unsubstituted polycyclic group containing any two atoms that are not directly connected, and may contain 0 or more double bonds. Any ring in the bridged ring system may contain 0 to 5 groups selected from heteroatoms or groups containing heteroatoms (including but not limited to N, S(=O)n or O, wherein n is 0, 1 or 2). The number of ring atoms includes, but is not limited to 5 to 20, 5 to 14, 5 to 12, or 5 to 10. Non-limiting examples include:

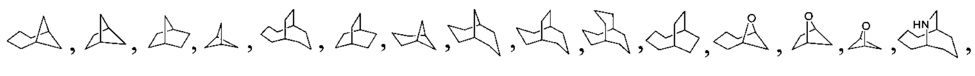

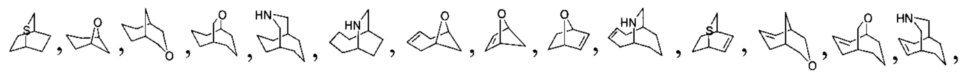

cubane, and adamantane. The "bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms.

"Carbo-fused ring", "fused ring carbocyclyl", "fused carbocyclyl" or "carbo-fused ring group" refers to a "fused ring" with a ring system consisting only of carbon atoms.

"Carbo-bridged ring", "bridged ring carbocyclyl", "bridged carbocyclyl" or "carbo-bridged ring group" refers to a "bridged ring" with a ring system consisting only of carbon atoms.

"Mono-heterocyclic ring", "monocyclic heterocyclyl" or "mono-heterocyclyl" refers to "heterocyclyl" or "heterocyclic ring" with a monocyclic system.

"Fused heterocyclic ring", "fused heterocyclyl", "fused ring heterocyclyl" or "fused heterocyclic ring group" refers to a "fused ring" containing a heteroatom.

"Spiro-heterocyclic ring", "spiroheterocyclyl", "spiro ring heterocyclyl" or "spiro-heterocyclic ring group" refers to a "spiro ring" containing a heteroatom.

"Bridged heterocyclic ring", "bridged heterocyclyl", "bridged ring heterocyclyl" or "bridged heterocyclic ring group" refers to a "bridged ring" containing a heteroatom.

"Aryl" or "aromatic ring" refers to a substituted or unsubstituted aromatic hydrocarbon group with a monocyclic ring or a fused ring, wherein the number of ring atoms in the aromatic ring includes, but is not limited to 6 to 18, 6 to 12, or 6 to 10 carbon atoms. The aryl ring can be fused to a saturated or unsaturated carbocyclic ring, wherein the ring connected to the parent structure is an aryl ring. Non-limiting examples include a benzene ring, a naphthalene ring, and

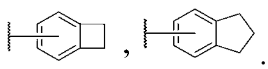

The "aryl" or "aromatic ring" can be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbon group containing 1 to 5 heteroatoms or groups containing heteroatoms (including but not limited to N, O, S(=O)n or Se(=O)n, wherein n is 0, 1 or 2), wherein the number of ring atoms in the heteroaromatic ring includes, but is not limited to, 5 to 15, 5 to 10, or 5 to 6. The atoms C, N and S in the ring are optionally oxidised (i.e., C(=O), NO, S(=O)n, and Se(=O)n, wherein n is 1 or 2). Non-limiting examples of the heteroaryl include, but are not limited to pyridyl, furyl, thienyl, selenophenyl, pyridyl, pyranyl, N-alkylpyrrolyl, pyrimidyl, pyrazinyl, pyridazinyl, imidazolyl, benzopyrazolyl, benzimidazolyl, benzopyridyl, pyrrolopyridyl, and pyridonyl. The heteroaryl ring can be fused to a saturated or unsaturated carbocyclic ring or heterocyclic ring, wherein the ring connected to the parent structure is an aryl ring. Non-limiting examples include

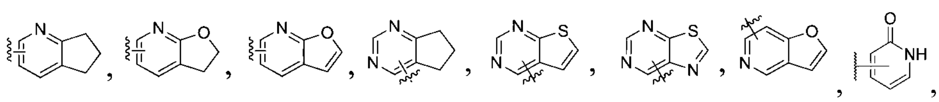

and

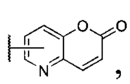, 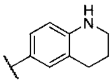, 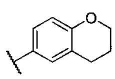.

The definition of the heteroaryl herein is consistent with this definition. The heteroaryl can be monovalent, divalent, trivalent or tetravalent. When divalent, trivalent or tetravalent, the point of connection is on the aromatic ring.

"Substitution" or "substituted" refers to substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, a bridged ring group, a spiro ring group, a fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_nR^a$, $-(CH_2)_m$-alkenyl-$R^a$, $OR^d$, $-(CH_2)_m$-alkynyl-$R^a$ (wherein m and n are 0, 1 or 2), arylthio, thiocarbonyl, silyl, $-NR^bR^c$, etc, wherein $R^b$ and $R^c$ are independently selected from H, hydroxyl, amino, carbonyl, alkyl, alkoxy, cycloalkyl, heterocyclyl, aryl, heteroaryl, sulphonyl, or trifluoromethylsulphonyl; alternatively, $R^b$ and $R^c$ may form five- or six-membered cycloalkyl or heterocyclyl; $R^a$ and $R^d$ are each independently selected from aryl, heteroaryl, alkyl, alkoxy, cycloalkyl, heterocyclyl, carbonyl, an ester group, a bridged ring group, a spiro ring group or a fused ring group.

"Substituted with 1 to X substituents selected from ..." refers to a substitution with 1, 2, 3 ... X substituents selected from ..., wherein X is selected from any integer between 1 and 10. For example, "substituted with 1 to 4 $R^k$" refers to a substitution with 1, 2, 3 or 4 $R^k$. For example, "substituted with 1 to 5 substituents selected from ..." refers to a substitution with 1, 2, 3, 4 or 5 substituents selected from ... For example, "bridged heterocyclic ring is optionally substituted with 1 to 4 substituents selected from H or F" means that the bridged heterocyclic ring is optionally substituted with 1, 2, 3 or 4 substituents selected from H or F.

An X- to Y-membered ring (X and Y are integers, $3 \leq X < Y$, and $X < Y \leq 20$ (i.e., any integer from 4 to 20)) includes X-, X+1-, X+2-, X+3-, X+4-,...or Y-membered rings. Rings include a heterocyclic ring, a carbocyclic ring, an aromatic ring, aryl, heteroaryl, cycloalkyl, a mono-heterocyclic ring, a fused heterocyclic ring, a spiro-heterocyclic ring, or a bridged heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

A $C_{x-y}$ carbocyclic ring (including aryl, cycloalkyl, a mono-carbocyclic ring, a spiro-carbocyclic ring, a fused-carbocyclic ring, or a bridged-carbocyclic ring) includes $C_x$-, $C_{x+1}$-, $C_{x+2}$-, $C_{x+3}$-, $C_{x+4}$-,...or $C_y$-membered rings (x is an integer, $3 \leq x < y$, and y is any integer from 4 to 20). For example, "$C_{3-6}$ cycloalkyl" refers to $C_3$, $C_4$, $C_5$ or $C_6$ cycloalkyl.

When a group has one or more connectable sites, any one or more sites of the group can be connected to other groups via chemical bonds. When the connection mode of the chemical bond is indeterminate and there are hydrogen atoms at the connectable sites, the number of H atoms at the site will decrease correspondingly with the number of connected chemical bonds when the chemical bond is connected, forming a group with the corresponding valence. For example,

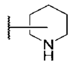

indicates that any connectable site on the piperidyl group can be connected to other groups via one chemical bond, including at least

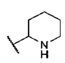, 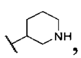, 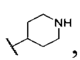, 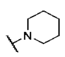

these 4 connection modes. Even if an H atom is shown on the N atom,

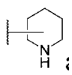

also includes

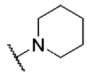.

For example,

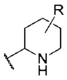

indicates that the R group on the piperidyl group can be located on C or N, including at least

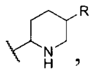, 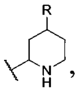, 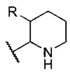, 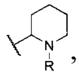, 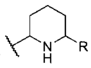.

For example, the general formula fragment is

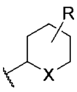.

When X is selected from $CH_2$ or NH, it indicates that the R group on this general formula fragment can be located on C or X. When X is $CH_2$, the general formula fragment may be

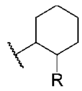

and

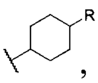, 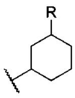, 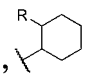, 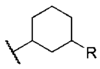;

when X is NH, the general formula fragment may be

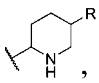, 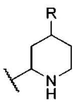, 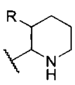, 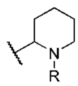, 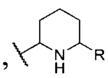.

When the connecting groups listed do not specify their connection direction, their connection directions include both the left-to-right and right-to-left reading orders. For example, for A-L-B, wherein L is -M-W-, it includes both A-M-W-B and A-W-M-B.

The term "optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound according to the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated substances, solvates, prodrugs, metabolites, pharmaceutically acceptable salts or co-crystals thereof and other chemical components, wherein the "other chemical components" refer to pharmaceutically acceptable carriers, excipients and/or one or more other therapeutic agents.

The term "dosage strength" refers to the amount of the active pharmaceutical ingredient contained in each vial,

tablet or any other single unit dosage form.

"Carrier" refers to a material that does not cause significant irritation to an organism and does not eliminate the biological activity and characteristics of a compound administered.

"Prodrug" refers to a compound that can be converted into the compound of the present invention with the biological activity by metabolism *in vivo.* The prodrug of the present invention is prepared by modifying an amino or carboxyl group in the compound of the present invention, and the modification can be removed by conventional operations or *in vivo* to obtain a parent compound. When the prodrug of the present invention is administered to a mammalian individual, the prodrug is split to form a free amino or carboxyl group.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

"Animal" is meant to include mammals, such as humans, companion animals, zoo animals, and domestic animals, preferably humans, horses, or dogs.

"Stereoisomer" refers to an isomer produced as a result of different spatial arrangement of atoms in molecules, including cis-trans isomers, enantiomers, diastereomers and conformational isomers.

"Tautomer" refers to a functional group isomer produced by the rapid movement of an atom in two positions in a molecule, such as keto-enol isomerisation and amide-imino alcohol isomerisation.

Synthesis method I:

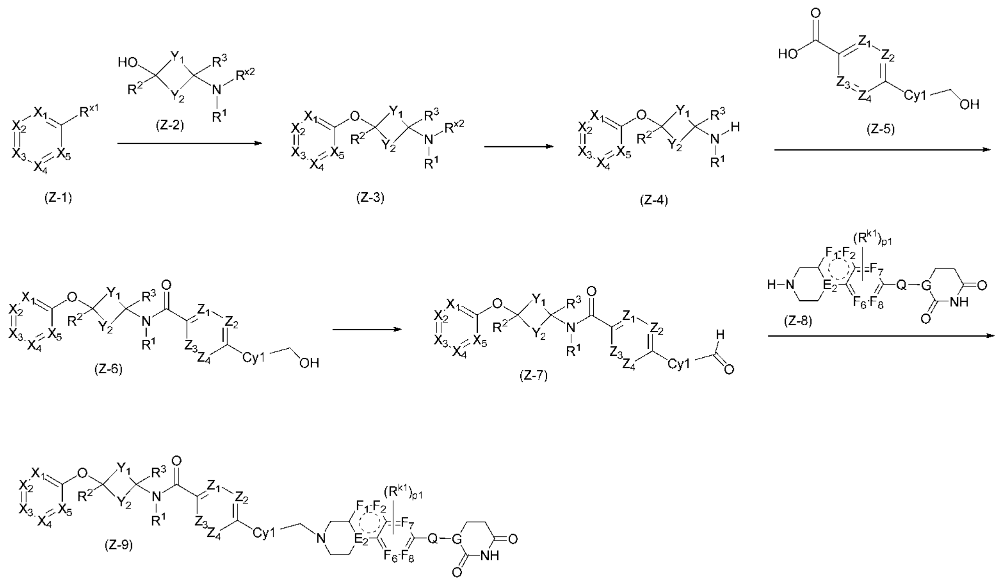

a compound of general formula (Z-1) and a compound of general formula (Z-2) are subjected to a nucleophilic substitution or coupling reaction to generate a compound of general formula (Z-3);

the compound of general formula (Z-3) is subjected to a deprotection reaction to obtain a compound of general formula (Z-4);

the compound of general formula (Z-4) and a compound of general formula (Z-5) are subjected to a condensation reaction to obtain a compound of general formula (Z-6);

the compound of general formula (Z-6) is subjected to an oxidation reaction to obtain a compound of general formula (Z-7);

the compound of general formula (Z-7) and a compound of general formula (Z-8) are subjected to a reductive amination reaction to obtain a compound of general formula (Z-9).

$R^{x1}$ is selected from F, Cl, Br, I, OTf or a boronic acid ester;

$R^{x2}$ is selected from Boc, Cbz, Fmoc, SEM, and other protecting groups.

Detailed Description of Embodiments

The technical solutions of the present invention will be described in detail by the following examples, but the scope of protection of the present invention includes but is not limited thereto.

The compounds used in the reactions described herein are prepared according to organic synthesis techniques known to those skilled in the art, and starting from commercially available chemicals and(or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

The structures of the compounds are determined by nuclear magnetic resonance (NMR) or (and) mass spectrometry (MS). The NMR shift (δ) is given in the unit of $10^{-6}$ (ppm). NMR is determined with (Bruker Avance III 400 and Bruker Avance 300) nuclear magnetic resonance instrument; the solvents for determination are deuterated dimethyl sulphoxide (DMSO-$d_6$), deuterated chloroform ($CDCl_3$) and deuterated methanol ($CD_3OD$); and the internal standard is tetramethylsilane (TMS).

MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 μM).

For thin layer chromatography (TLC), silica gel plates from Yantai Huanghai HSGF254 or Qingdao GF254 are used. Silica gel plates with a thickness of 0.15 mm-0.20 mm are employed for TLC, while plates with a thickness of 0.4 mm-0.5 mm are used for TLC separation and purification.

For column chromatography, Yantai Huanghai silica gel of 200-300 mesh is typically used as a carrier.

Abbreviations of synthetic reagents:

DMAP: CAS No.: 1122-58-3; NBS: CAS No.: 128-08-5; HATU: CAS No.: 148893-10-1;

**Synthesis of intermediate 1**:

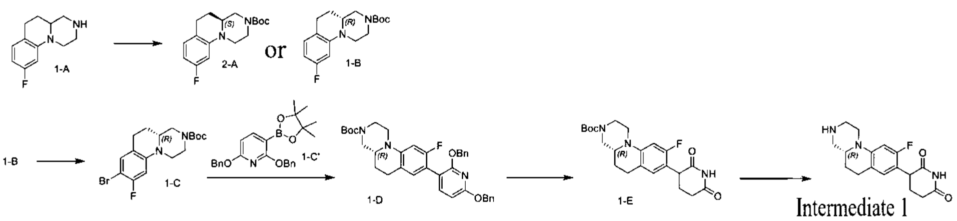
1-A
2-A or 1-B
1-B
1-C
1-C'
1-D
1-E
Intermediate 1

Step 1: Preparation of 1-B

1-A (2.8 g, 13.58 mmol) (see Bioorganic & Medicinal Chemistry Letters, 2016, 26, 5877-5882 for the synthetic method) was dissolved in dichloromethane (50 mL), $Boc_2O$ (5.93 g, 27.17 mmol) and DMAP (3.32 g, 27.18 mmol) were added, and the mixture was reacted at room temperature for 16 h. The reaction system was washed with 0.5 mol/L hydrochloric acid (50 mL), dried over anhydrous sodium sulphate, and concentrated under reduced pressure. The crude product was separated and purified by silica gel column chromatography (ethyl acetate : petroleum ether (v/v) = 0 : 1-1 : 9) to obtain the racemate of 1-B (3.4 g, yield: 82%).

The racemate of 1-B was subjected to chiral resolution, and the chiral resolution method was as follows:

1. Instrument: SFC Prep 150 AP; chromatography column: Daicel IC-H (19 mm × 250 mm).
2. The sample was dissolved in methanol and filtered through a 0.45 μm filter head to prepare a sample solution.
3. Preparative chromatography conditions: a. the mobile phase consists of systems A and B: mobile phase A: $CO_2$; mobile phase B: methanol/isopropanol (v/v) = 1 : 1; b. isocratic elution, mobile phase B: 20%; c. flow rate: 40 mL/min.

Retention time: chiral isomer 1 (compound 1-B): 5.7 min, chiral isomer 2 (compound 2-A): 6.47 min.

According to the MicroED structure determination of compound 2-B, compound 1-B is in the R configuration, and compound 2-A is in the S configuration.

LCMS m/z = 307.3 [M+1]+.

Step 2: Preparation of 1-C

1-B (1.8 g, 5.88 mmol) was dissolved in acetonitrile (50 mL), NBS (1.05 g, 5.90 mmol) was added, and the mixture was reacted at room temperature for 1 h. The reaction system was concentrated under reduced pressure, and the crude product was separated and purified by silica gel column chromatography (ethyl acetate : petroleum ether (v/v) = 0 : 1-1 : 9) to obtain 1-C (1.6 g, yield: 71%).

LCMS m/z = 385.3 [M+1]+.

Step 3: Preparation of 1-D

1-C (0.77 g, 2.0 mmol), 1-C' (1.67 g, 4.0 mmol) (see WO 2022235945 for the synthetic method), Pd(dppf)$Cl_2$·DCM (0.16 g, 0.20 mmol) and caesium carbonate (1.30 g, 4.0 mmol) were added to a reaction flask, followed by 1,4-dioxane (30 mL) and water (3 mL), and the mixture was reacted at 100°C under nitrogen atmosphere for 20 h. The reaction system was cooled to room temperature, and water (50 mL) and ethyl acetate (50 mL) were added. The aqueous phase was extracted with ethyl acetate (50 mL), and the organic phase was washed with a saturated aqueous sodium chloride solution (30 mL), dried over anhydrous sodium sulphate, and concentrated under reduced pressure. The crude product was separated and purified by silica gel column chromatography (petroleum ether/ethyl acetate (v/v) = 1 : 0-9 : 1) to obtain 1-D (0.70 g, yield: 59%).

Step 4: Preparation of 1-E

1-D (0.70 g, 1.18 mmol) was dissolved in THF (20 mL), and 10% palladium on carbon (0.63 g) was added. The mixture was reacted at 45°C under hydrogen balloon atmosphere for 20 h. The reaction system was cooled to room temperature, and filtered by suction. The filtrate was concentrated under reduced pressure, and the crude product was separated and purified by silica gel column chromatography (MeOH : DCM (v/v) = 0 : 1-5 : 95) to obtain 1-E (0.25 g, yield: 51%).

LCMS m/z = 418.1 [M+1]+.

Step 5: Preparation of intermediate 1

1-E (250 mg, 0.6 mmol) was dissolved in dichloromethane (2 mL), trifluoroacetic acid (2 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude intermediate 1 (190 mg).

LCMS m/z = 318.3 [M+1]+.

Synthesis of intermediate 2:

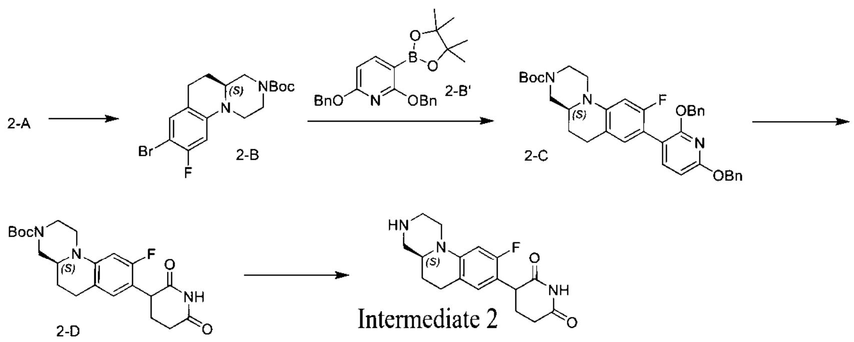

Step 1: Preparation of 2-B

2-A (1.4 g, 4.57 mmol) was dissolved in acetonitrile (50 mL), NBS (0.81 g, 4.55 mmol) was added, and the mixture was reacted at room temperature for 1 h. The reaction system was concentrated under reduced pressure, and the crude

product was separated and purified by silica gel column chromatography (ethyl acetate : petroleum ether (v/v) = 0 : 1-1 : 9) to obtain 2-B (1.6 g, yield: 92%).

LCMS m/z = 385.3 $[M+1]^+$.

According to the MicroED structure determination, compound 2-B is in the S configuration.

Step 2: Preparation of 2-C

2-B (1.6 g, 4.16 mmol), 2-B' (3.46 g, 8.29 mmol), Pd(dppf)$Cl_2$·DCM (0.34 g, 0.42 mmol) and caesium carbonate (2.70 g, 8.3 mmol) were added to a reaction flask, followed by 1,4-dioxane (50 mL) and water (5 mL), and the mixture was reacted at 100°C under nitrogen atmosphere for 20 h. The reaction system was cooled to room temperature, and water (50 mL) and ethyl acetate (50 mL) were added. The aqueous phase was extracted with ethyl acetate (50 mL), and the organic phase was washed with a saturated aqueous sodium chloride solution (30 mL), dried over anhydrous sodium sulphate, and concentrated under reduced pressure. The crude product was separated and purified by silica gel column chromatography (petroleum ether/ethyl acetate (v/v) = 1 : 0-9 : 1) to obtain 2-C (1.4 g, yield: 56%).

Step 3: Preparation of 2-D

2-C (1.4 g, 2.35 mmol) was dissolved in THF (50 mL), and 10% palladium on carbon (1.25 g) was added. The mixture was reacted at 45°C under hydrogen balloon atmosphere for 20 h. The reaction system was cooled to room temperature, and filtered by suction. The filtrate was concentrated under reduced pressure, and the crude product was separated and purified by silica gel column chromatography (MeOH : DCM (v/v) = 0 : 1-5 : 95) to obtain 2-D (0.85 g, yield: 87%).

LCMS m/z = 418.1 $[M+1]^+$.

Step 4: Preparation of intermediate 2

2-D (620 mg, 1.49 mmol) was dissolved in dichloromethane (5 mL), trifluoroacetic acid (3 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude intermediate 2 (460 mg).

LCMS m/z = 318.3 $[M+1]^+$.

**Preparation of intermediate 3:**

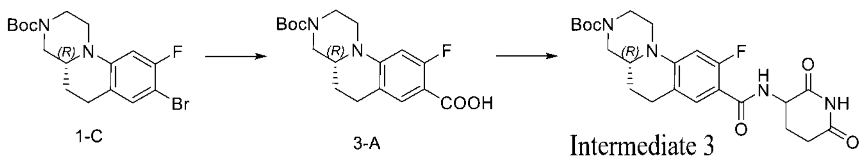

Step 1: Preparation of 3-A

Under nitrogen atmosphere, 1-C (7.00 g, 18.17 mmol) and tetrahydrofuran (70 mL) were respectively added to a reaction flask, followed by slowly dropwise addition of 2.5 mol/L solution of n-butyllithium in n-hexane (14.50 mL, 36.25 mmol) at -78°C, and the mixture was stirred for another 1.5 h at -78°C. Subsequently, the system was purged three times with carbon dioxide, and reacted for 0.5 h under carbon dioxide balloon atmosphere with the system temperature maintained below - 40°C. The reaction system was allowed to warm to room temperature, and ethyl acetate (20 mL) was added. The mixture was adjusted to pH 2 with 1 mol/L hydrochloric acid, and extracted with ethyl acetate (30 mL×3). The organic phase was dried over anhydrous sodium sulphate, and concentrated under reduced pressure, and the crude product was separated and purified by silica gel column chromatography (petroleum ether/ethyl acetate (v/v) = 10 : 1-2 : 1) to obtain 3-A (2.4 g, yield: 38%).

$^1$H NMR (400 MHz, DMSO-$d_6$) δ 7.42 (d, 1H), 6.64 (d, 1H), 4.00 - 3.82 (m, 3H), 3.23 - 3.09 (m, 1H), 3.08 - 2.90 (m, 1H), 2.90 - 2.77 (m, 1H), 2.76 - 2.60 (m, 3H), 2.05 - 1.90 (m, 1H), 1.65 - 1.50 (m, 1H), 1.49 - 1.35 (m, 9H).

Step 2: Preparation of intermediate 3

To a reaction flask were respectively added 3-A (2.4 g, 6.85 mmol), HATU (3.13 g, 8.23 mmol), diisopropylethylamine (2.66 g, 20.58 mmol) and DMF (10 mL), and the mixture was stirred at room temperature for 10 min. Subsequently, 3-amino-2,6-piperidinedione hydrochloride (1.35 g, 8.20 mmol) was added, and the resulting mixture was reacted at room temperature for 30 min. The reaction solution was poured into water (100 mL), and filtered. The filter cake was washed with water (50 mL), and dried under reduced pressure to obtain intermediate 3 (2.0 g, yield: 63%). LCMS m/z = 461.2 $[M+1]^+$

**Preparation of intermediate 4:**

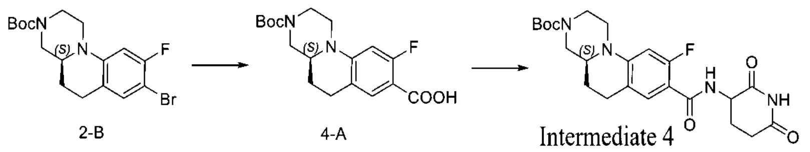

2-B 4-A Intermediate 4

Starting from compound 2-B and following the synthetic method of intermediate 3, intermediate 4 was obtained. LCMS m/z = 461.2 $[M+1]^+$

Preparation of intermediate 5:

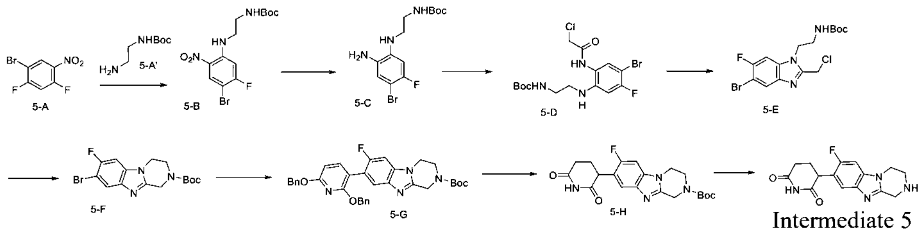

5-A 5-B 5-C 5-D 5-E 5-F 5-G 5-H Intermediate 5

Step 1: Preparation of 5-B

5-A (10 g, 42.02 mmol) was dissolved in DMSO (80 mL), 5-A' (7.41 g, 46.25 mmol) and DIPEA (16.29 g, 126.03 mmol) were added, and the mixture was reacted at 50°C for 3 h. The reaction system was cooled to room temperature, and water (300 mL) and ethyl acetate (400 mL) were added. The organic phase was washed with a saturated aqueous sodium chloride solution (300 mL), dried over anhydrous sodium sulphate, and concentrated under reduced pressure to afford crude 5-B (6.2 g).

Step 2: Preparation of 5-C

The above-mentioned crude 5-B (6.2 g) was added to methanol (150 mL) and water (30 mL), followed by iron powder (9.15 g, 163.4 mmol) and ammonium chloride (8.77 g, 163.96 mmol), and the mixture was reacted at 70°C for 20 h. The reaction system was cooled to room temperature, and filtered through a pad of diatomaceous earth. The filtrate was concentrated under reduced pressure, and water (300 mL) and ethyl acetate (400 mL) were added to the residue. The organic phase was washed with a saturated aqueous sodium chloride solution (300 mL), dried over anhydrous sodium sulphate, and concentrated under reduced pressure. The crude product was separated and purified by silica gel column chromatography (methanol : dichloromethane (v/v) = 0 : 1-1 : 9) to afford 5-C (1.9 g, two-step yield from compound 5-A: 13%).

Step 3: Preparation of 5-D

5-C (1.9 g, 5.46 mmol) and TEA (1.66 g, 16.40 mmol) were dissolved in dichloromethane (20 mL), chloroacetyl chloride (0.99 g, 8.77 mmol) was added dropwise, and the mixture was reacted at room temperature for 20 h. To the

reaction solution was added water (50 mL). The organic phase was dried over anhydrous sodium sulphate, and concentrated under reduced pressure to obtain crude 5-D (2.1 g).

Step 4: Preparation of 5-E

The above-mentioned crude 5-D (0.95 g) was added to acetic acid (15 mL), and the mixture was reacted at 50°C for 20 h. The reaction system was cooled to 0°C, adjusted to pH 8 with 25% aqueous ammonia, and extracted with ethyl acetate (150 mL). The organic phase was dried over anhydrous sodium sulphate, and concentrated under reduced pressure. The crude product was separated and purified by silica gel column chromatography (methanol : dichloromethane (v/v) = 0 : 1-1 : 9) to obtain 5-E (0.85 g, two-step yield from compound 5-C: 85%).

Step 5: Preparation of 5-F

5-E (7.3 g, 17.95 mmol) was dissolved in THF (80 mL), 1 mol/L solution of potassium tert-butoxide in tetrahydrofuran (20 mL) was added, and the mixture was reacted at room temperature for 19 h. To the reaction system were added water (30 mL) and ethyl acetate (50 mL). The organic phase was washed with a saturated aqueous sodium chloride solution (300 mL), dried over anhydrous sodium sulphate, and concentrated under reduced pressure. The crude product was separated and purified by silica gel column chromatography (methanol : dichloromethane (v/v) = 0 : 1-1 : 9) to obtain 5-F (3.6 g, yield: 54%).

Step 6: Preparation of 5-G

5-F (2.0 g, 5.4 mmol), 2,6-bisbenzyloxypyridine-3-boronic acid pinacol ester (3.4 g, 8.15 mmol), Pd(dppf)$Cl_2$·DCM (0.44 g, 0.54 mmol) and caesium carbonate (3.5 g, 10.74 mmol) were successively added to a reaction flask, followed by 1,4-dioxane (100 mL) and water (10 mL), and the mixture was reacted at 100°C under nitrogen atmosphere for 20 h. The reaction system was cooled to room temperature, and water (300 mL) and ethyl acetate (300 mL) were added. The organic phase was washed with a saturated aqueous sodium chloride solution (100 mL), dried over anhydrous sodium sulphate, and concentrated under reduced pressure. The crude product was separated and purified by silica gel column chromatography (petroleum ether/ethyl acetate (v/v) = 1 : 0-9 : 1) to obtain 5-G (2.6 g, yield: 83%).

LCMS m/z = 581.4 $[M+1]^+$.

Step 7: Preparation of 5-H

5-G (2.6 g, 4.48 mmol) was dissolved in THF (10 mL) and methanol (30 mL), 10% palladium on carbon (2.5 g) was added, and the mixture was reacted at 40°C under hydrogen balloon atmosphere for 20 h. The reaction system was cooled to room temperature, and filtered. The filtrate was concentrated under reduced pressure to obtain crude 5-H (0.98 g).

LCMS m/z = 403.3 $[M+1]^+$.

Step 8: Preparation of intermediate 5

The above-mentioned crude 5-H (100 mg) was dissolved in dichloromethane (2 mL), trifluoroacetic acid (1 mL) was added, and the mixture was reacted at room temperature for 3 h. The reaction system was concentrated under reduced pressure. Methanol (5 mL) and sodium bicarbonate (solid, 150 mg) were added, and the mixture was stirred for 10 min. Subsequently, the system was filtered by suction, and the filtrate was concentrated under reduced pressure to obtain crude intermediate 5 (80 mg).

Example 1: Preparation of trifluoroacetate of **compound 1**

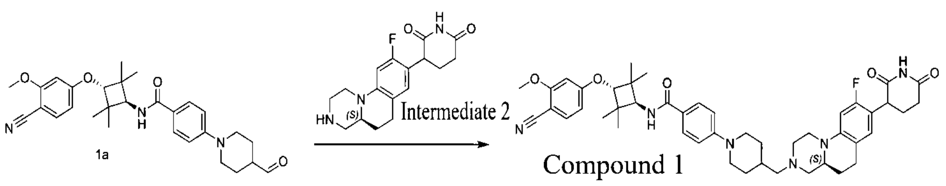


1a (0.23 g, 0.47 mmol) (see WO 2021127443 for the synthetic method) was dissolved in 1,2-dichloroethane (10

mL), the above-mentioned crude intermediate 2 (0.15 g) was added, followed by acetic acid (1 mL), and the mixture was stirred at room temperature for 1 h. Sodium triacetoxyborohydride (0.30 g, 1.42 mmol) was then added, and the resulting mixture was reacted at room temperature for 16 h. To the reaction solution was slowly added a saturated aqueous sodium bicarbonate solution (20 mL), and the mixture was extracted with dichloromethane (50 mL). The organic phase was washed with a saturated aqueous sodium chloride solution (50 mL), dried over anhydrous sodium sulphate, and concentrated under reduced pressure. The crude product was separated and purified by silica gel column chromatography (dichloromethane : methanol (v/v) = 10 : 1). The resulting crude product was subjected to preparative HPLC (preparative method: a solution of the crude product in DMSO was filtered through a 0.45 μm filter membrane to prepare a sample solution. mobile phase system: water (containing 0.1% TFA)/acetonitrile. Gradient elution method: gradient elution with acetonitrile from 10% to 50% (elution time: 15 min)). Lyophilisation was performed to obtain the trifluoroacetate of compound 1 (10 mg).

$^1$H NMR (400 MHz, $CD_3OD$) δ 7.80 - 7.72 (m, 2H), 7.52 (d, 1H), 7.08 - 7.00 (m, 2H), 6.91 (d, 1H), 6.71 (d, 1H), 6.64 - 6.60 (m, 1H), 6.55 (dd, 1H), 4.26 (s, 1H), 4.18 - 4.03 (m, 2H), 4.02 - 3.83 (m, 6H), 3.80 - 3.60 (m, 2H), 3.43 - 3.30 (m, 1H), 3.28 - 3.06 (m, 4H), 3.04 - 2.57 (m, 7H), 2.33 - 2.02 (m, 4H), 2.01 - 1.70 (m, 3H), 1.56 - 1.40 (m, 2H), 1.36 - 1.19 (m, 12H). LCMS m/z = 791.4 $[M+1]^+$

The resulting crude reaction solution from this step can also be subjected to neutral preparative HPLC (preparative method: a solution of the crude product in DMSO was filtered through a 0.45 μm filter membrane to prepare a sample solution. mobile phase system: 10 mmol/L ammonium bicarbonate in water/acetonitrile. Gradient elution method: gradient elution with acetonitrile from 47% to 87% (elution time: 28 min)). Lyophilisation was performed to obtain compound 1.

$^1$H NMR (400 MHz, $CDCl_3$) δ 7.91 (s, 1H), 7.73 - 7.64 (m, 2H), 7.45 (d, 1H), 6.95 - 6.87 (m, 2H), 6.72 (d, 1H), 6.53 - 6.44 (m, 2H), 6.40 (dd, 1H), 6.10 (d, 1H), 4.18 - 4.11 (m, 1H), 4.04 (s, 1H), 3.91 (s, 3H), 3.89 - 3.72 (m, 3H), 3.65 - 3.54 (m, 1H), 3.14 - 3.00 (m, 1H), 2.97 - 2.72 (m, 7H), 2.70 - 2.57 (m, 2H), 2.32 - 2.11 (m, 5H), 1.99 - 1.82 (m, 4H), 1.81 - 1.65 (m, 2H), 1.40 - 1.19 (m, 14H).

Compound 1 was subjected to chiral resolution to obtain chiral isomer 1 and chiral isomer 2, respectively.

Purification conditions for the first chiral resolution:

1. Instrument: SHIMADZU LC-20AP; chromatography column: Chiral Whelk column.
2. The sample was dissolved in acetonitrile and filtered through a 0.45 μm filter head to prepare a sample solution.
3. Preparative chromatography conditions: a. the mobile phase consists of systems A and B: mobile phase A: n-hexane; mobile phase B: a mixed solvent of ethanol/acetonitrile containing 0.1% aqueous ammonia; b. isocratic elution, mobile phase B: 70%; c. flow rate: 100 mL/min.

Purification conditions for the second chiral resolution:

1. Instrument: Waters 150 Prep-SFC; chromatography column: Chiral AD column.
2. The sample was dissolved in acetonitrile and filtered through a 0.45 μm filter head to prepare a sample solution.
3. Preparative chromatography conditions: a. the mobile phase consists of systems A and B: mobile phase A: $CO_2$; mobile phase B: a mixed solvent of isopropanol/acetonitrile containing 0.1% aqueous ammonia; b. isocratic elution, mobile phase B: 70%; c. flow rate: 100 mL/min.

Chiral analytical method:

1. Instrument: SHIMAZU LC-20AD; chromatography column: Chiral Whelk column.
2. Analytical chromatography conditions: a. the mobile phase consists of systems A and B: mobile phase A: n-hexane; mobile phase B: a solution of ethanol and acetonitrile containing 0.1% isopropylamine; b. isocratic elution, mobile phase B: 60%; c. flow rate: 1 mL/min.

Retention time: chiral isomer 1: 2.594 min, chiral isomer 2: 5.557 min.

**Example 3: Preparation of trifluoroacetate of compound 3**

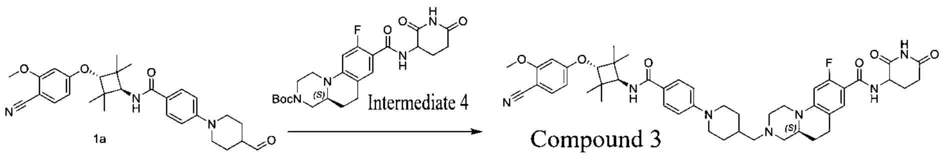

1a Compound 3

Dichloromethane (2 mL) and trifluoroacetic acid (1 mL) were added to intermediate 4 (160 mg, 0.35 mmol), and the mixture was reacted at room temperature for 2 h. The reaction system was concentrated under reduced pressure. Dichloromethane (5 mL) and triethylamine (1 mL) were added, and the mixture was concentrated under reduced pressure to obtain crude 1 (120 mg). 1a (0.160 g, 0.33 mmol) was dissolved in 1,2-dichloroethane (10 mL), the above-mentioned crude 1 (0.120 g) was added, followed by acetic acid (1 mL), and the mixture was stirred at room temperature for 1 h. Sodium triacetoxyborohydride (0.21 g, 0.99 mmol) was then added, and the resulting mixture was reacted at room temperature for 16 h. To the reaction solution was slowly added a saturated aqueous sodium bicarbonate solution (20 mL), and the mixture was extracted with dichloromethane (50 mL). The organic phase was washed with a saturated aqueous sodium chloride solution (50 mL), dried over anhydrous sodium sulphate, and concentrated under reduced pressure. The crude product was separated and purified by silica gel column chromatography (dichloromethane : methanol (v/v) = 10 : 1). The resulting crude product was subjected to preparative HPLC (preparative method: a solution of the crude product in DMSO was filtered through a 0.45 μm filter membrane to prepare a sample solution. mobile phase system: water (containing 0.1% TFA)/acetonitrile. Gradient elution method: gradient elution with acetonitrile from 10% to 50% (elution time: 15 min)). Lyophilisation was performed to obtain the trifluoroacetate of compound 3 (45 mg).

$^1$H NMR (400 MHz, $CD_3OD$) δ 7.80 - 7.72 (m, 2H), 7.60 - 7.48 (m, 2H), 7.10 - 7.00 (m, 2H), 6.76 (d, 1H), 6.65 - 6.60 (m, 1H), 6.55 (dd, 1H), 4.85 - 4.72 (m, 1H), 4.30 - 4.10 (m, 3H), 4.02 - 3.88 (m, 5H), 3.82 - 3.63 (m, 2H), 3.60 - 3.43 (m, 1H), 3.33 - 3.10 (m, 4H), 3.07 - 2.62 (m, 7H), 2.37 - 2.06 (m, 4H), 2.02 - 1.88 (m, 2H), 1.85 - 1.72 (m, 1H), 1.57 - 1.42 (m, 2H), 1.35 - 1.17 (m, 12H).

LCMS m/z = 834.6 $[M+1]^+$

**Example 5: Preparation of trifluoroacetate of compound 5**

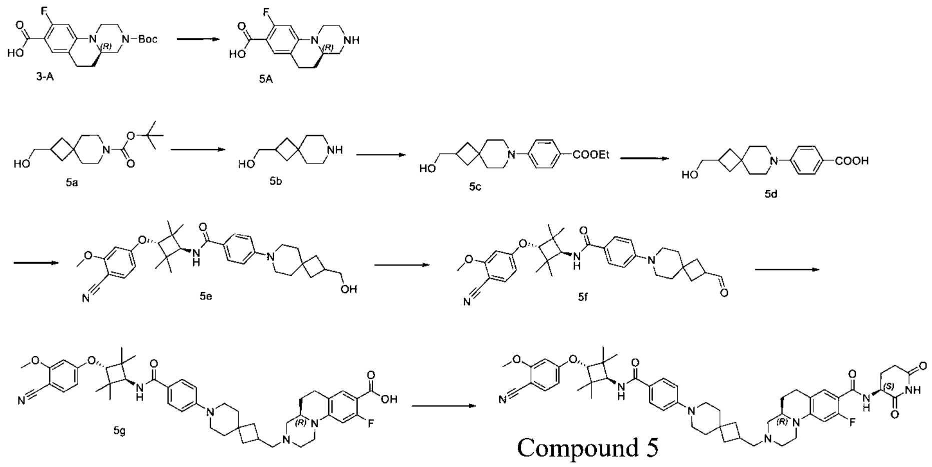
3-A 5A 5a 5b 5c 5d 5e 5f 5g Compound 5

Step 1: Preparation of trifluoroacetate of 5A

3-A (650 mg, 1.86 mmol) was dissolved in dichloromethane (2 mL), trifluoroacetic acid (2 mL) was added, and the mixture was reacted at room temperature for 3 h. The reaction system was concentrated under reduced pressure to obtain the trifluoroacetate of crude 5A (700 mg).

Step 2: Preparation of trifluoroacetate of 5b

5a (3.00 g, 11.75 mmol) was dissolved in dichloromethane (5 mL), trifluoroacetic acid (5 mL) was added, and the mixture was reacted at room temperature for 3 h. The reaction system was concentrated under reduced pressure to obtain the trifluoroacetate of crude 5b (3.2 g).

Step 3: Preparation of 5c

To a reaction flask were respectively added the above-mentioned trifluoroacetate of crude 5b (3.2 g), ethyl 4-fluorobenzoate (1.85 g, 11.00 mmol), potassium carbonate (3.04 g, 22.0 mmol) and dimethyl sulphoxide (15 mL), and the mixture was reacted at 100°C for 16 h. The reaction system was cooled to room temperature, and water (100 mL) and ethyl acetate (100 mL) were added to the reaction solution. After phase separation, the aqueous phase was extracted with ethyl acetate (50 mL×3), and the organic phases were combined, washed with a saturated aqueous sodium chloride solution (50 mL), dried over anhydrous sodium sulphate, and filtered. The filtrate was concentrated under reduced pressure, and the crude product was separated and purified by silica gel column chromatography (petroleum ether/ethyl acetate (v/v) = 1 : 0-1 : 1) to obtain 5c (2.40 g, yield: 72%).

Step 4: Preparation of 5d

To a reaction flask were respectively added 5c (2.40 g, 7.91 mmol), potassium hydroxide (1.78 g, 31.79 mmol), ethanol (10 mL), tetrahydrofuran (10 mL) and water (10 mL), and the mixture was stirred at room temperature for 16 h. The reaction system was concentrated under reduced pressure, adjusted to pH 2 with 2 mol/L hydrochloric acid, and filtered. The filter cake was collected, and dried under reduced pressure to obtain crude 5d (1.50 g).
LCMS m/z = 276.3 $[M+1]^+$

Step 5: Preparation of 5e

To a reaction flask were respectively added the above-mentioned crude 5d (0.6 g), HATU (0.99 g, 2.60 mmol), diisopropylethylamine (1.41 g, 10.91 mmol) and DMF (10 mL), and the mixture was stirred at room temperature for 0.5 h. trans-4-(3-Amino-2,2,4,4-tetramethylcyclobutoxy)-2-methoxybenzonitrile hydrochloride (0.66 g, 2.12 mmol) (see CN115175901 for the synthetic method) was then added, and the resulting mixture was stirred at room temperature for 2 h. Water (100 mL) and ethyl acetate (100 mL) were added to the reaction solution. After phase separation, the aqueous phase was extracted with ethyl acetate (50 mL×3), and the organic phases were combined, washed with a saturated aqueous sodium chloride solution (50 mL), dried over anhydrous sodium sulphate, and filtered. The filtrate was concentrated under reduced pressure, and the crude product was separated and purified by silica gel column chromatography (petroleum ether/ethyl acetate (v/v) = 1 : 0-0 : 1) to obtain 5e (1.10 g, yield: 98%).
LCMS m/z = 532.6 $[M+1]^+$

Step 6: Preparation of 5f

To a reaction flask were respectively added 5e (1.10 g, 2.07 mmol), Dess-Martin periodinane (1.05 g, 2.48 mmol) and dichloromethane (10 mL), and the mixture was reacted at room temperature for 4 h. The reaction system was filtered through a pad of diatomaceous earth, the filtrate was concentrated under reduced pressure, and the crude product was separated and purified by silica gel column chromatography (methanol/dichloromethane (v/v) = 0 : 1-1 : 9) to obtain 5f (0.52 g, yield: 47%).
LCMS m/z = 530.2 $[M+1]^+$

Step 7: Preparation of 5g

To a reaction flask were respectively added 5f (0.25 g, 0.47 mmol), the above-mentioned trifluoroacetate of crude 5A (0.18 g), glacial acetic acid (0.2 mL) and DMAc (3 mL), and the mixture was reacted at room temperature for 1 h. Sodium triacetoxyborohydride (0.30 g, 1.42 mmol) was then added, and the resulting mixture was reacted at room temperature for 16 h. Water (20 mL) and ethyl acetate (20 mL) were added to the reaction solution. After phase separation, the aqueous phase was extracted with ethyl acetate (20 mL×3), and the organic phases were combined, washed with a saturated aqueous sodium chloride solution (50 mL), dried over anhydrous sodium sulphate, and filtered. The filtrate was concentrated under reduced pressure, and the crude product was separated and purified by silica gel column chromatography (methanol/dichloromethane (v/v) = 0 : 1-1 : 9) to obtain 5g (0.20 g, yield: 56%).

Step 8: Preparation of trifluoroacetate of compound 5

To a reaction flask were respectively added 5g (0.10 g, 0.13 mmol), (S)-3-aminopiperidine-2,6-dione hydrochloride (0.043 g, 0.26 mmol), EDCI (0.037 g, 0.19 mmol), HOBt (0.026 g, 0.19 mmol), N-methylmorpholine (0.065 g, 0.64 mmol) and DMF (4 mL), and the mixture was reacted at room temperature for 16 h. The reaction solution was subjected to Pre-HPLC (instrument and preparative column: using Waters 2767 preparative liquid phase chromatographic instrument;

preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 0.1% TFA)/acetonitrile. Gradient elution method: gradient elution with acetonitrile from 10% to 50% (elution time: 15 min). Lyophilisation was performed to obtain the trifluoroacetate of compound 5 (20 mg).

$^1$H NMR (400 MHz, $CD_3OD$) δ 7.74 (d, 2H), 7.57 - 7.48 (m, 2H), 7.02 (d, 2H), 6.75 (d, 1H), 6.62 (d, 1H), 6.55 (dd, 1H), 4.82 - 4.74 (m, 1H), 4.30 - 4.23 (m, 1H), 4.23 - 4.17 (m, 1H), 4.13 (s, 1H), 3.93 (s, 3H), 3.68 - 3.49 (m, 2H), 3.48 - 3.30 (m, 5H), 3.28 - 3.08 (m, 4H), 3.02 - 2.90 (m, 1H), 2.88 - 2.65 (m, 5H), 2.35 - 2.05 (m, 5H), 1.89 - 1.82 (m, 2H), 1.82 - 1.65 (m, 5H), 1.26 (d, 12H).
LCMS m/z = 874.4 [M+1]$^+$

**Example 7: Preparation** of trifluoroacetate **of compound 7**

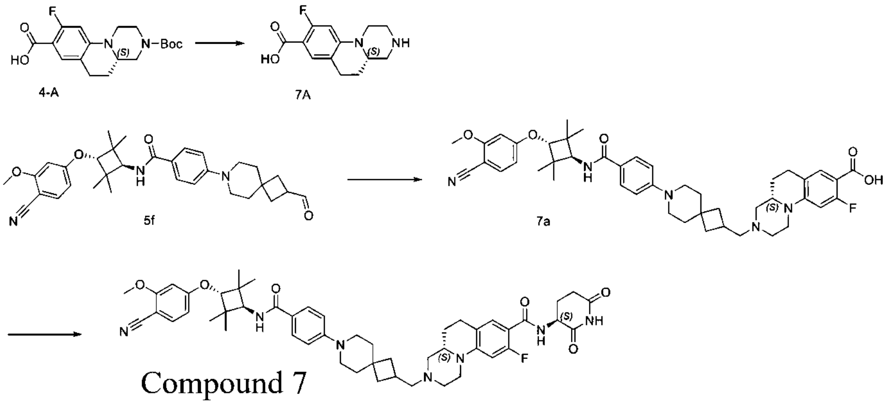
4-A
7A
5f
7a
Compound 7

Starting from compound 4-A and following the synthetic method of example 5, the trifluoroacetate of compound 7 was obtained.

$^1$H NMR (400 MHz, CD3OD) δ 7.81 - 7.71 (m, 2H), 7.57 - 7.48 (m, 2H), 7.13 - 7.01 (m, 2H), 6.75 (d, 1H), 6.62 (d, 1H), 6.55 (dd, 1H), 4.80 - 4.74 (m, 1H), 4.26 (s, 1H), 4.24 - 4.16 (m, 1H), 4.13 (s, 1H), 3.93 (s, 3H), 3.67 - 3.50 (m, 2H), 3.50 - 3.15 (m, 9H), 3.01 - 2.91 (m, 1H), 2.91 - 2.63 (m, 5H), 2.35 - 2.07 (m, 5H), 1.94 - 1.84 (m, 2H), 1.84 - 1.67 (m, 5H), 1.26 (d, 12H).
LCMS m/z = 437.8 [M/2 +1]$^+$

Following the synthetic method of other examples, the following compounds were obtained:

| Example No. | Structure | Starting material | LCMS / $^{1}$H NMR |
|---|---|---|---|
| Example 2 (tri-fluoroac etate of compound 2) | 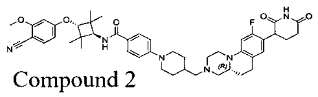 Compound 2 | Intermediate 1+1a ( following the synthetic method of example 1) | $^{1}$H NMR (400 MHz, $CD_3OD$) δ 7.79 - 7.73 (m, 2H), 7.52 (d, 1H), 7.08 - 6.99 (m, 2H), 6.91 (d, 1H), 6.71 (d, 1H), 6.62 (d, 1H), 6.55 (dd, 1H), 4.29 - 4.23 (m, 1H), 4.18 - 4.03 (m, 2H), 4.00 - 3.91 (m, 5H), 3.91 - 3.83 (m, 1H), 3.79 - 3.60 (m, 2H), 3.41 - 3.33 (m, 1H), 3.26 - 3.08 (m, 4H), 3.01 - 2.88 (m, 3H), 2.87 - 2.60 (m, 4H), 2.30 - 2.15 (m, 2H), 2.15 - 2.03 (m, 2H), 2.00 - 1.88 (m, 2H), 1.85 - 1.71 (m, 1H), 1.56 - 1.42 (m, 2H), 1.26 (d, 12H). LCMS m/z = 791.4 [M+1]$^{+}$ |
| Example 2 (compound 2) | | | $^{1}$H NMR (400 MHz, DMSO-$d_6$) δ 10.75 (s, 1H), 7.73 (d, 2H), 7.64 (d, 1H), 7.46 (d, 1H), 6.95 (d, 2H), 6.79 (d, 1H), 6.69 - 6.56 (m, 2H), 6.54 (dd, 1H), 4.27 (s, 1H), 4.05 (d, 1H), 3.91 (s, 3H), 3.90 - 3.76 (m, 3H), 3.76 - 3.66 (m, 1H), 3.05 - 2.84 (m, 3H), 2.84 - 2.64 (m, 5H), 2.64 - 2.55 (m, 1H), 2.54 - 2.45 (m, 1H), 2.26 - 2.13 (m, 2H), 2.13 - 2.00 (m, 2H), 1.99 - 1.70 (m, 6H), 1.68 - 1.50 (m, 1H), 1.29 - 1.08 (m, 14H). |
| Example 4 (tri-fluoroacetate of compound 4) | 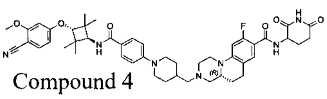 Compound 4 | Intermediate 3+1a ( following the synthetic method of example 3) | $^{1}$H NMR (400 MHz, $CD_3OD$) δ 7.81-7.72 (m, 2H), 7.60 - 7.47 (m, 2H), 7.07 - 6.96 (m, 2H), 6.76 (d, 1H), 6.65 - 6.60 (m, 1H), 6.55 (dd, 1H), 4.87 - 4.72 (m, 1H), 4.31 - 4.10 (m, 3H), 4.02 - 3.88 (m, 5H), 3.83 - 3.63 (m, 2H), 3.60 - 3.42 (m, 1H), 3.34 - 3.10 (m, 4H), 3.07 - 2.62 (m, 7H), 2.35 - 2.06 (m, 4H), 2.02 - 1.88 (m, 2H), 1.85 - 1.72 (m, 1H), 1.57 - 1.40 (m, 2H), 1.35 - 1.15 (m, 12H). LCMS m/z = 834.4 [M+1]$^{+}$ |
| Example 6 (tri-fluoroacetate of compound 6) | 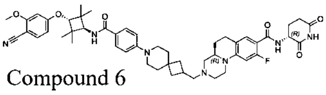 Compound 6 | 5g+ (R)-3-aminopiperi-dine-2,6-dione hydro-chloride (following the synthetic method of example 5) | $^{1}$H NMR (400 MHz, $CD_3OD$) δ 7.74 (d, 2H), 7.57 - 7.45 (m, 2H), 7.02 (d, 2H), 6.75 (d, 1H), 6.62 (d, 1H), 6.55 (dd, 1H), 4.80 - 4.73 (m, 1H), 4.32 - 4.23 (m, 1H), 4.23 - 4.16 (m, 1H), 4.13 (s, 1H), 3.93 (s, 3H), 3.69 - 3.49 (m, 2H), 3.48 - 3.30 (m, 5H), 3.28 - 3.05 (m, 4H), 3.02 - 2.89 (m, 1H), 2.88 - 2.65 (m, 5H), 2.35 - 2.05 (m, 5H), 1.89 - 1.82 (m, 2H), 1.82 - 1.65 (m, 5H), 1.26 (d, 12H). LCMS m/z = 437.8 [M/2 +1]$^{+}$ |

(continued)

| Example No. | Structure | Starting material | LCMS / $^1$H NMR |
|---|---|---|---|
| Example 8 (trifluoroacetate of compound 8) | 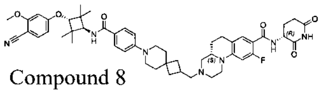 Compound 8 | 7a+ (R)-3-aminopiperidine-2,6-dione hydrochloride (following the synthetic method of example 5) | $^1$H NMR (400 MHz, $CD_3OD$) δ 7.79 - 7.71 (m, 2H), 7.57 - 7.48 (m, 2H), 7.10 - 6.99 (m, 2H), 6.75 (d, 1H), 6.62 (d, 1H), 6.55 (dd, 1H), 4.79 - 4.74 (m, 1H), 4.30 - 4.23 (m, 1H), 4.23 - 4.15 (m, 1H), 4.13 (s, 1H), 3.93 (s, 3H), 3.68 - 3.48 (m, 2H), 3.48 - 3.30 (m, 5H), 3.28 - 3.07 (m, 4H), 3.02 - 2.90 (m, 1H), 2.88 - 2.65 (m, 5H), 2.35 - 2.05 (m, 5H), 1.89 - 1.82 (m, 2H), 1.82 - 1.65 (m, 5H), 1.26 (d, 12H).<br>LCMS m/z = 874.4 $[M+1]^+$ |
| Example 9 (trifluoroacetate of compound 9) | 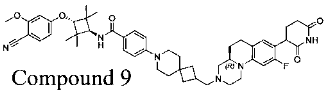 Compound 9 | 5f+ intermediate 1 (following the synthetic method of example 1) | $^1$H NMR (400 MHz, $CD_3OD$) δ 7.80 - 7.69 (m, 2H), 7.52 (d, 1H), 7.10 - 6.99 (m, 2H), 6.90 (d, 1H), 6.70 (d, 1H), 6.63 (d, 1H), 6.56 (dd, 1H), 4.26 (s, 1H), 4.16 - 4.05 (m, 2H), 3.93 (s, 3H), 3.91 - 3.83 (m, 1H), 3.65 - 3.49 (m, 2H), 3.41 - 3.33 (m, 2H), 3.32 - 3.23 (m, 5H), 3.23 - 3.12 (m, 1H), 3.12 - 3.00 (m, 1H), 2.99 - 2.89 (m, 1H), 2.89 - 2.63 (m, 5H), 2.31 - 2.16 (m, 3H), 2.15 - 1.99 (m, 2H), 1.91 - 1.83 (m, 2H), 1.83 - 1.65 (m, 5H), 1.28 (s, 6H), 1.24 (s, 6H).<br>LCMS m/z = 831.8 $[M+1]^+$ |
| Example 10 (trifluoroacetate of compound 10) | 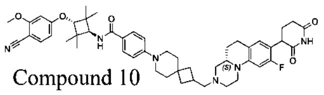 Compound 10 | 5f+ intermediate 2 (following the synthetic method of example 1) | $^1$H NMR (400 MHz, $CD_3OD$) δ 7.82 - 7.69 (m, 2H), 7.52 (d, 1H), 7.10 - 6.96 (m, 2H), 6.90 (d, 1H), 6.70 (d, 1H), 6.63 (d, 1H), 6.56 (dd, 1H), 4.26 (s, 1H), 4.16 - 4.03 (m, 2H), 3.93 (s, 3H), 3.91 - 3.82 (m, 1H), 3.65 - 3.49 (m, 2H), 3.41 - 3.33 (m, 2H), 3.33 - 3.23 (m, 5H), 3.23 - 3.12 (m, 1H), 3.12 - 3.00 (m, 1H), 2.99 - 2.89 (m, 1H), 2.89 - 2.63 (m, 5H), 2.34 - 2.16 (m, 3H), 2.15 - 1.97 (m, 2H), 1.92 - 1.83 (m, 2H), 1.83 - 1.65 (m, 5H), 1.28 (s, 6H), 1.24 (s, 6H).<br>LCMS m/z = 831.6 $[M+1]^+$ |
| Example 12 (compound 12) | 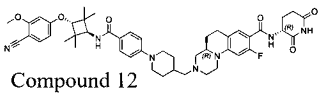 Compound 12 | 11a+ (R)-3-aminopiperidine-2,6-dione hydrochloride (following the synthetic method of example 5) | $^1$H NMR (400 MHz, DMSO-$d_6$) δ 10.82 (s, 1H), 7.94 (t, 1H), 7.74 (d, 2H), 7.64 (d, 1H), 7.47 (d, 1H), 7.36 (d, 1H), 6.95 (d, 2H), 6.74 - 6.61 (m, 2H), 6.54 (dd, 1H), 4.82 - 4.60 (m, 1H), 4.27 (s, 1H), 4.05 (d, 1H), 3.95 - 3.79 (m, 6H), 3.17 - 3.04 (m, 1H), 2.97 - 2.87 (m, 2H), 2.87 - 2.62 (m, 6H), 2.57 - 2.52 (m, 1H), 2.25 - 1.88 (m, 6H), 1.88 - 1.70 (m, 4H), 1.67 - 1.51 (m, 1H), 1.29 - 1.08 (m, 14H).<br>LCMS m/z = 418.0 $[M/2 +1]^+$ |

(continued)

| Example No. | Structure | Starting material | LCMS / $^1$H NMR |
|---|---|---|---|
| Example 14 (compound 14) | 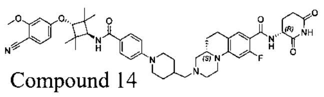 Compound 14 | 13a+(R)-3-aminopiperidine-2,6-dione hydrochloride (following the synthetic method of example 5) | $^1$H NMR (400 MHz, DMSO-$d_6$) δ 10.82 (s, 1H), 7.94 (t, 1H), 7.74 (d, 2H), 7.64 (d, 1H), 7.47 (d, 1H), 7.36 (d, 1H), 6.95 (d, 2H), 6.74 - 6.60 (m, 2H), 6.54 (dd, 1H), 4.83 - 4.59 (m, 1H), 4.27 (s, 1H), 4.05 (d, 1H), 3.97 - 3.79 (m, 6H), 3.16 - 3.04 (m, 1H), 2.98 - 2.87 (m, 2H), 2.87 - 2.62 (m, 6H), 2.58 - 2.52 (m, 1H), 2.25 - 1.88 (m, 6H), 1.88 - 1.70 (m, 4H), 1.69 - 1.51 (m, 1H), 1.29 - 1.07 (m, 14H). LCMS m/z = 418.0 [M/2 +1]$^+$ |

Example 11: Preparation of compound 11

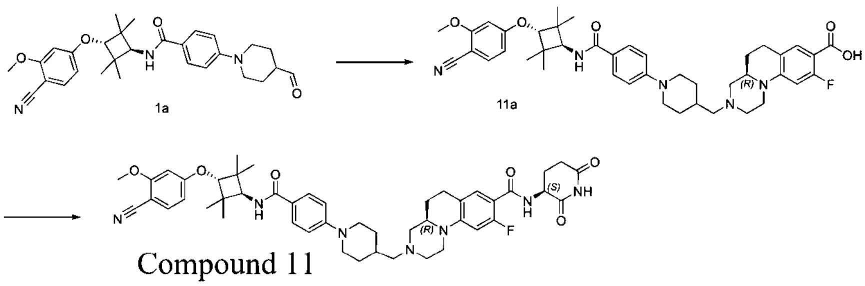
1a 11a Compound 11

Starting from compound 1a and the trifluoroacetate of 5A and following the synthetic method of example 5, compound 11 was obtained by neutral preparative chromatography (ammonium acetate in water (5 mmol/L)/acetonitrile) and lyophilisation.

$^1$H NMR (400 MHz, DMSO-$d_6$) δ 10.82 (s, 1H), 7.94 (t, 1H), 7.78 - 7.69 (m, 2H), 7.64 (d, 1H), 7.47 (d, 1H), 7.36 (d, 1H), 7.00 - 6.90 (m, 2H), 6.73 - 6.61 (m, 2H), 6.54 (dd, 1H), 4.78 - 4.65 (m, 1H), 4.27 (s, 1H), 4.05 (d, 1H), 3.95 - 3.79 (m, 6H), 3.16 - 3.06 (m, 1H), 2.97 - 2.61 (m, 8H), 2.57 - 2.50 (m, 1H), 2.26 - 2.14 (m, 2H), 2.13 - 1.88 (m, 4H), 1.87 - 1.72 (m, 4H), 1.66 - 1.52 (m, 1H), 1.28 - 1.09 (m, 14H).
LCMS m/z = 418.0 [M/2 +1]$^+$

Example 13: Preparation of compound 13

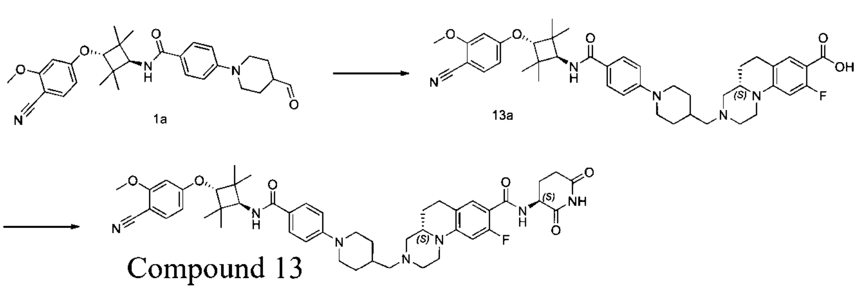
1a 13a Compound 13

Starting from compounds 1a and 7A and following the synthetic method of example 11, compound 13 was obtained by neutral preparative chromatography (ammonium acetate in water (5 mmol/L)/acetonitrile) and lyophilisation.

$^1$H NMR (400 MHz, DMSO-$d_6$) δ 10.82 (s, 1H), 7.94 (t, 1H), 7.74 (d, 2H), 7.64 (d, 1H), 7.47 (d, 1H), 7.36 (d, 1H), 6.95 (d, 2H), 6.74 - 6.61 (m, 2H), 6.54 (dd, 1H), 4.82 - 4.60 (m, 1H), 4.27 (s, 1H), 4.05 (d, 1H), 3.95 - 3.79 (m, 6H), 3.17 - 3.04

(m, 1H), 2.97 - 2.87 (m, 2H), 2.87 - 2.62 (m, 6H), 2.57 - 2.52 (m, 1H), 2.25 - 1.88 (m, 6H), 1.88 - 1.70 (m, 4H), 1.67 - 1.51 (m, 1H), 1.29 - 1.08 (m, 14H).
LCMS m/z = 418.0 [M/2 +1]$^+$

Example 15: Preparation of compound 15

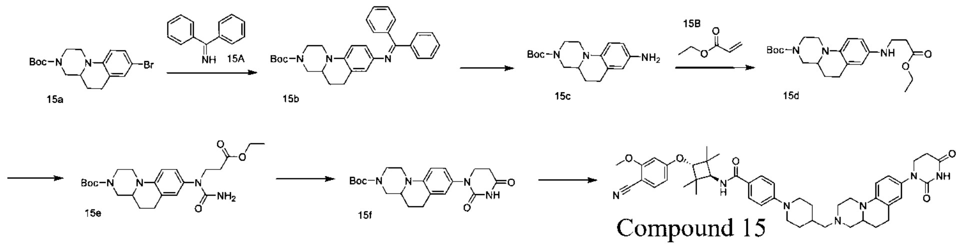

Compound 15

Step 1: Preparation of 15b

15a (1.28 g, 3.48 mmol) (see Bioorg. Med. Chem. Lett. 2016, 26, 5877-5882 for the synthetic method), caesium carbonate (2.27 g, 6.97 mmol), palladium acetate (0.16 g, 0.71 mmol), XantPhos (0.20 g, 0.35 mmol) and 15A (0.89 g, 4.91 mmol) were added to a 1,4-dioxane solution (40 mL), and the mixture was reacted at 105°C under nitrogen atmosphere for 3 h. The reaction system was cooled to room temperature. Water (100 mL) was added, and the mixture was extracted with ethyl acetate (80 mL×3). The organic phase was concentrated under reduced pressure, and the crude product was separated and purified by silica gel column chromatography (petroleum ether/ethyl acetate (v/v) = 1 : 0-7 : 3) to obtain 15b (1.30 g, yield: 80%).
LCMS m/z = 468.4 [M+1]$^+$

Step 2: Preparation of 15c

15b (1.30 g, 2.78 mmol) was added to methanol (10 mL), followed by 10% palladium on carbon (1.33 g) and ammonium acetate (1.32 g, 17.13 mmol), and the mixture was reacted at room temperature under hydrogen balloon atmosphere for 12 h. The reaction system was filtered through a pad of diatomaceous earth, the filtrate was concentrated under reduced pressure, and the crude product was separated and purified by silica gel column chromatography (petroleum ether/ethyl acetate (v/v) = 1 : 0-1 : 1) to obtain 15c (0.76 g, yield: 90%).
LCMS m/z = 304.4 [M+1]$^+$

Step 3: Preparation of 15d

15c (0.76 g, 2.55 mmol), 15B (0.75 g, 7.49 mmol) and DIPEA (0.97 g, 7.50 mmol) were successively added to ethanol (50 mL), and the mixture was reacted at 100°C for 48 h. The reaction system was cooled to room temperature, and concentrated under reduced pressure, and the crude product was separated and purified by silica gel column chromatography (methanol/dichloromethane (v/v) = 0 : 1-6 : 94) to obtain 15d (0.70 g, yield: 68%).
LCMS m/z = 404.2 [M+1]$^+$

Step 4: Preparation of 15e

15d (700 mg, 1.74 mmol) and DIPEA (671 mg, 5.19 mmol) were added to THF (20 mL), and then triphosgene (565 mg, 1.90 mmol) was slowly added, and the mixture was reacted at room temperature for 1 h. To the reaction system was added aqueous ammonia (5 mL), and the mixture was reacted at 50°C for 2 h. The reaction system was cooled to room temperature. Water (50 mL) was added, and the mixture was extracted with ethyl acetate (50 mL×2). The organic phase was concentrated under reduced pressure, and the crude product was separated and purified by silica gel column chromatography (methanol/dichloromethane (v/v) = 0 : 1-1 : 9) to obtain 15e (700 mg, yield: 90%).

Step 5: Preparation of 15f

15e (0.70 g, 1.57 mmol) was added to acetonitrile (30 mL), a 40% solution of benzyltrimethylammonium hydroxide in methanol (2 mL) was added, and the mixture was reacted at 60°C for 2 h. The reaction system was cooled

to room temperature, and concentrated under reduced pressure, and the crude product was separated and purified by silica gel column chromatography (methanol/dichloromethane (v/v) = 0 : 1-8 : 92) to obtain 15f (530 mg, yield: 84%). LCMS m/z = 401.4 $[M+1]^+$

Step 6: Preparation of compound 15

To a reaction flask were added 15f (0.15 g, 0.37 mmol), trifluoroacetic acid (2 mL) and dichloromethane (3 mL), and the mixture was reacted at room temperature for 2 h. The reaction solution was concentrated under reduced pressure, and adjusted to pH 7 with triethylamine. The mixture was concentrated under reduced pressure, and the residue was dissolved with DMA (5 mL). 1a (0.23 g, 0.47 mmol), acetic acid (0.5 mL) and sodium triacetoxyborohydride (0.24 g, 1.13 mmol) were successively added, and the resulting mixture was reacted at room temperature for 16 h. Methanol (10 mL) was added to the reaction solution, and the mixture was concentrated under reduced pressure. The reaction solution was subjected to Pre-HPLC (instrument and preparative column: using Waters 2767 preparative liquid phase chromatographic instrument; preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: ammonium acetate in water (5 mmol/L)/acetonitrile. Gradient elution method: gradient elution with acetonitrile from 10% to 70% (elution time: 12 min). Lyophilisation was performed to obtain compound 15 (0.20 g, yield: 70%).

$^1H$ NMR (400 MHz, DMSO-$d_6$) δ 10.21 (s, 1H), 7.74 (d, 2H), 7.64 (d, 1H), 7.47 (d, 1H), 6.99 - 6.91 (m, 3H), 6.91 - 6.85 (m, 1H), 6.85 - 6.77 (m, 1H), 6.64 (d, 1H), 6.54 (dd, 1H), 4.27 (s, 1H), 4.05 (d, 1H), 3.97 - 3.81 (m, 5H), 3.80 - 3.72 (m, 1H), 3.70 - 3.61 (m, 2H), 2.99 - 2.73 (m, 6H), 2.73 - 2.57 (m, 4H), 2.24 - 2.13 (m, 2H), 2.13 - 2.02 (m, 1H), 1.93 - 1.72 (m, 5H), 1.69 - 1.54 (m, 1H), 1.29 - 1.10 (m, 14H).

LCMS m/z = 774.6 $[M+1]^+$.

**Example 16: Preparation of trifluoroacetate of compound 16**

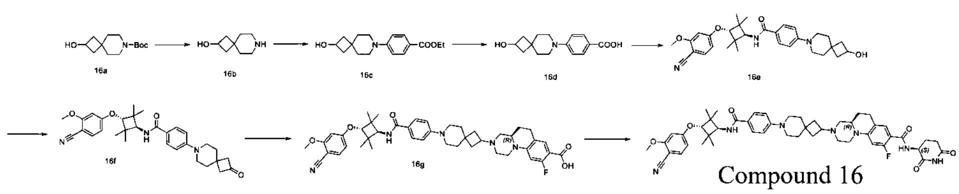

Compound 16

Step 1: Preparation of trifluoroacetate of 16b

16a (2.77 g, 11.48 mmol) was dissolved in dichloromethane (5 mL), trifluoroacetic acid (5 mL) was added, and the mixture was reacted at room temperature for 3 h. The reaction system was concentrated under reduced pressure to obtain the trifluoroacetate of crude 16b (3.0 g).

Step 2: Preparation of 16c

To a reaction flask were respectively added the above-mentioned trifluoroacetate of crude 16b (3.0 g), ethyl 4-fluorobenzoate (1.85 g, 11.00 mmol), potassium carbonate (3.04 g, 22.00 mmol) and dimethyl sulphoxide (15 mL), and the mixture was reacted at 100°C for 16 h. The reaction system was cooled to room temperature, and water (100 mL) and ethyl acetate (100 mL) were added to the reaction solution. After phase separation, the aqueous phase was extracted with ethyl acetate (50 mL×3), and the organic phases were combined, washed with a saturated aqueous sodium chloride solution (50 mL), dried over anhydrous sodium sulphate, and filtered. The filtrate was concentrated under reduced pressure, and the crude product was separated and purified by silica gel column chromatography (petroleum ether/ethyl acetate (v/v) = 1 : 0-1 : 1) to obtain 16c (2.5 g, yield: 79%).

Step 3: Preparation of 16d

To a reaction flask were respectively added 16c (2.4 g, 8.29 mmol), potassium hydroxide (1.86 g, 33.21 mmol), ethanol (10 mL), tetrahydrofuran (10 mL) and water (10 mL), and the mixture was reacted at room temperature for 16 h. The reaction system was concentrated under reduced pressure, adjusted to pH 2 with 2 mol/L hydrochloric acid, and filtered. The filter cake was collected, and dried under reduced pressure to obtain crude 16d (1.80 g).

LCMS m/z = 262.1 $[M+1]^+$

Step 4: Preparation of 16e

To a reaction flask were respectively added the above-mentioned crude 16d (0.68 g), HATU (0.99 g, 2.60 mmol), diisopropylethylamine (1.41 g, 10.91 mmol) and DMF (10 mL), and the mixture was stirred at room temperature for 0.5 h. trans-4-(3-Amino-2,2,4,4-tetramethylcyclobutoxy)-2-methoxybenzonitrile hydrochloride (0.57 g, 1.83 mmol) (see CN115175901 for the synthetic method) was then added, and the resulting mixture was reacted at room temperature for 2 h. Water (100 mL) and ethyl acetate (100 mL) were added to the reaction solution. After phase separation, the aqueous phase was extracted with ethyl acetate (50 mL×3), and the organic phases were combined, washed with a saturated aqueous sodium chloride solution (50 mL), dried over anhydrous sodium sulphate, and filtered. The filtrate was concentrated under reduced pressure, and the crude product was separated and purified by silica gel column chromatography (petroleum ether/ethyl acetate (v/v) = 1 : 0-0 : 1) to obtain 16e (1.10 g, two-step yield from compound 16c: 68%).
LCMS m/z = 518.5 $[M+1]^+$

Step 5: Preparation of 16f

To a reaction flask were respectively added 16e (1.10 g, 2.13 mmol), Dess-Martin periodinane (1.08 g, 2.55 mmol) and dichloromethane (10 mL), and the mixture was reacted at room temperature for 4 h. The reaction system was filtered through a pad of diatomaceous earth, the filtrate was concentrated under reduced pressure, and the crude product was separated and purified by silica gel column chromatography (methanol/dichloromethane (v/v) = 0 : 1-1 : 9) to obtain 16f (0.9 g, yield: 82%).
LCMS m/z = 516.2 $[M+1]^+$

Step 6: Preparation of 16g

To a reaction flask were respectively added 16f (0.25 g, 0.485 mmol), the above-mentioned trifluoroacetate of crude 5A (0.18 g), glacial acetic acid (0.2 mL) and DMAc (3 mL), and the mixture was reacted at room temperature for 1 h. Sodium triacetoxyborohydride (0.30 g, 1.42 mmol) was then added, and the resulting mixture was reacted at room temperature for 16 h. Water (20 mL) and ethyl acetate (20 mL) were added to the reaction solution. After phase separation, the aqueous phase was extracted with ethyl acetate (20 mL×3), and the organic phases were combined, washed with a saturated aqueous sodium chloride solution (50 mL), dried over anhydrous sodium sulphate, and filtered. The filtrate was concentrated under reduced pressure, and the crude product was separated and purified by silica gel column chromatography (methanol/dichloromethane (v/v) = 0 : 1-1 : 9) to obtain 16g (0.25 g, yield: 69%).
LCMS m/z = 750.4 $[M+1]^+$

Step 7: Preparation of trifluoroacetate of compound 16

To a reaction flask were respectively added 16g (0.2 g, 0.27 mmol), (S)-3-aminopiperidine-2,6-dione hydrochloride (0.069 g, 0.42 mmol), EDCI (0.13 g, 0.68 mmol), HOBt (0.073 g, 0.54 mmol), N-methylmorpholine (0.17 g, 1.68 mmol) and DMF (4 mL), and the mixture was reacted at room temperature for 16 h. The reaction solution was subjected to Pre-HPLC (instrument and preparative column: using Waters 2767 preparative liquid phase chromatographic instrument; preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 0.1% TFA)/acetonitrile. Gradient elution method: gradient elution with acetonitrile from 10% to 50% (elution time: 15 min). Lyophilisation was performed to obtain the trifluoroacetate of compound 16 (50 mg).

$^1H$ NMR (400 MHz, $CD_3OD$) δ 7.79 - 7.69 (m, 2H), 7.59 - 7.47 (m, 2H), 7.07 - 6.98 (m, 2H), 6.76 (d, 1H), 6.62 (d, 1H), 6.55 (dd, 1H), 4.79 - 4.74 (m, 1H), 4.29 - 4.18 (m, 2H), 4.13 (s, 1H), 3.93 (s, 3H), 3.86 - 3.72 (m, 1H), 3.67 - 3.54 (m, 2H), 3.51 - 3.40 (m, 1H), 3.40 - 3.30 (m, 4H), 3.26 - 3.15 (m, 1H), 3.08 - 2.94 (m, 1H), 2.89 - 2.74 (m, 4H), 2.74 - 2.65 (m, 1H), 2.47 - 2.37 (m, 2H), 2.35 - 2.25 (m, 1H), 2.21 - 2.05 (m, 4H), 1.87 - 1.70 (m, 5H), 1.26 (d, 12H).
LCMS m/z = 431.0 $[M/2 +1]^+$

Following the synthetic method of other examples, the following compounds were obtained:

| Example No. | Structure | Starting material | LCMS / $^{1}$H NMR |
|---|---|---|---|
| Example 17 (trifluoroacetate of compound 17) | 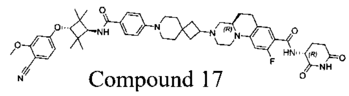 Compound 17 | 16g+(R)-3-aminopiperidine-2,6-dione hydrochloride (following the synthetic method of example 16) | $^{1}$H NMR (400 MHz, $CD_3OD$) δ 7.78 - 7.69 (m, 2H), 7.59 - 7.47 (m, 2H), 7.06 - 6.98 (m, 2H), 6.76 (d, 1H), 6.64 - 6.60 (m, 1H), 6.55 (dd, 1H), 4.79 - 4.73 (m, 1H), 4.29 - 4.16 (m, 2H), 4.13 (s, 1H), 3.93 (s, 3H), 3.87 - 3.72 (m, 1H), 3.68 - 3.54 (m, 2H), 3.51 - 3.40 (m, 1H), 3.40 - 3.30 (m, 4H), 3.25 - 3.15 (m, 1H), 3.08 - 2.94 (m, 1H), 2.89 - 2.74 (m, 4H), 2.74 - 2.65 (m, 1H), 2.46 - 2.37 (m, 2H), 2.35 - 2.25 (m, 1H), 2.22 - 2.05 (m, 4H), 1.87 - 1.70 (m, 5H), 1.26 (d, 12H). LCMS m/z = 431.0 [M/2 +1]$^{+}$ |
| Example 19 (trifluoroaceta te of compound 19) | 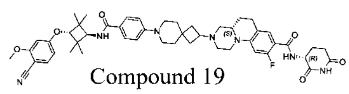 Compound 19 | 18a+ (R)-3-aminopiperidine-2,6-dione hydrochloride (following the synthetic method of example 16) | $^{1}$H NMR (400 MHz, $CD_3OD$) δ 7.78 - 7.69 (m, 2H), 7.59 - 7.47 (m, 2H), 7.07 - 6.98 (m, 2H), 6.76 (d, 1H), 6.62 (d, 1H), 6.55 (dd, 1H), 4.79 - 4.73 (m, 1H), 4.31 - 4.18 (m, 2H), 4.13 (s, 1H), 3.93 (s, 3H), 3.86 - 3.71 (m, 1H), 3.67 - 3.54 (m, 2H), 3.52 - 3.40 (m, 1H), 3.40 - 3.30 (m, 4H), 3.26 - 3.15 (m, 1H), 3.08 - 2.94 (m, 1H), 2.89 - 2.74 (m, 4H), 2.74 - 2.65 (m, 1H), 2.48 - 2.36 (m, 2H), 2.35 - 2.24 (m, 1H), 2.21 - 2.05 (m, 4H), 1.88 - 1.70 (m, 5H), 1.26 (d, 12H). LCMS m/z = 430.9 [M/2 +1]$^{+}$ |

Example 18: Preparation of trifluoroacetate of compound 18

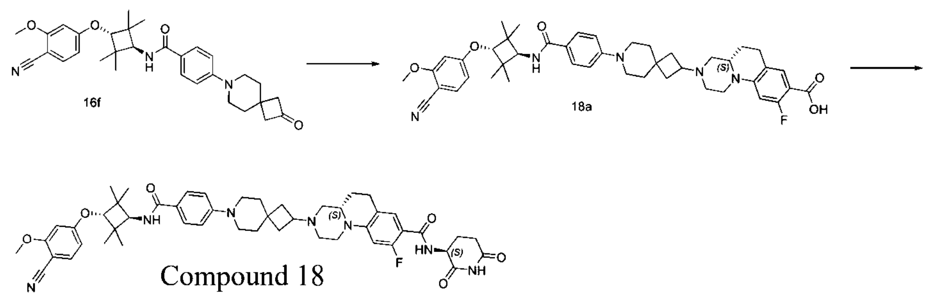
16f
18a
Compound 18

Starting from compounds 16f and 7A and following the synthetic method of example 16, the trifluoroacetate of compound 18 was obtained.

$^{1}$H NMR (400 MHz, $CD_3OD$) δ 7.77 - 7.69 (m, 2H), 7.59 - 7.47 (m, 2H), 7.05 - 6.98 (m, 2H), 6.76 (d, 1H), 6.66 - 6.60 (m, 1H), 6.55 (dd, 1H), 4.79 - 4.74 (m, 1H), 4.28 - 4.18 (m, 2H), 4.13 (s, 1H), 3.93 (s, 3H), 3.86 - 3.72 (m, 1H), 3.67 - 3.54 (m, 2H), 3.51 - 3.40 (m, 1H), 3.40 - 3.31 (m, 4H), 3.27 - 3.15 (m, 1H), 3.10 - 2.94 (m, 1H), 2.89 - 2.74 (m, 4H), 2.74 - 2.63 (m, 1H), 2.48 - 2.37 (m, 2H), 2.36 - 2.25 (m, 1H), 2.21 - 2.05 (m, 4H), 1.88 - 1.70 (m, 5H), 1.26 (d, 12H).
LCMS m/z = 860.4 [M+1]$^{+}$

**Example 20: Preparation of trifluoroacetate of compound 20**

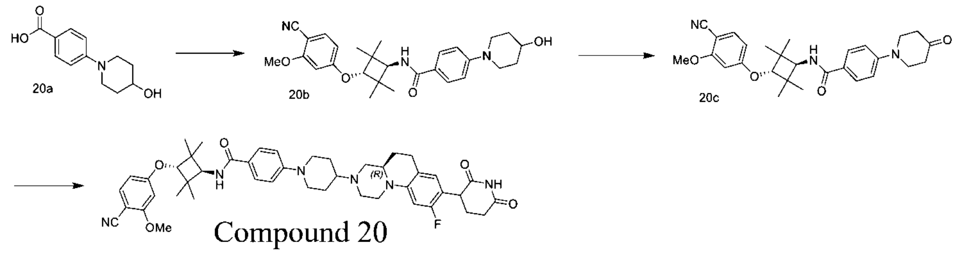


Step 1: Preparation of 20b

trans-4-(3-Amino-2,2,4,4-tetramethylcyclobutoxy)-2-methoxybenzonitrile hydrochloride (3.93 g, 12.64 mmol) (see CN115175901 for the synthetic method) was dissolved in DMF (30 mL), DIPEA (5.84 g, 45.18 mmol), HATU (5.16 g, 13.57 mmol) and 20a (2.0 g, 9.04 mmol) were added, and the mixture was reacted at room temperature under nitrogen atmosphere for 16 h. Ethyl acetate (50 mL) and purified water (50 mL) were added to the reaction solution. The aqueous phase was extracted with ethyl acetate (25 mL×2), and the organic phases were combined, washed with a saturated aqueous sodium chloride solution (25 mL), dried over anhydrous sodium sulphate, and filtered. The filtrate was concentrated under reduced pressure, and the crude product was separated and purified by silica gel column chromatography (petroleum ether : ethyl acetate (v/v) = 1 : 2) to obtain 20b (4.0 g, yield: 93%).
LCMS m/z = 478.3 $[M+1]^+$

Step 2: Preparation of 20c

20b (4.0 g, 8.38 mmol) was dissolved in dichloromethane (80 mL), Dess-Martin periodinane (4.27 g, 10.07 mmol) was added, and the mixture was reacted under reflux for 3 h. The reaction system was cooled to room temperature, the reaction solution was concentrated under reduced pressure, and the crude product was separated and purified by silica gel column chromatography (petroleum ether : ethyl acetate (v/v) = 1 : 2) to obtain 20c (2.5 g, yield: 63%).
LCMS m/z = 476.2 $[M+1]^+$

Step 3: Preparation of trifluoroacetate of compound 20

To a reaction flask were added 1-E (0.3 g, 0.72 mmol), trifluoroacetic acid (1 mL) and dichloromethane (3 mL), and the mixture was reacted at room temperature for 3 h. The reaction solution was concentrated under reduced pressure, and adjusted to pH 7 with triethylamine. The mixture was concentrated under reduced pressure, and N,N-dimethylacetamide (5 mL) was added to the residue. 20c (0.34 g, 0.72 mmol), acetic acid (0.3 mL) and sodium triacetoxyborohydride (0.31 g, 1.46 mmol) were successively added, and the resulting mixture was reacted at room temperature for 16 h. Dichloromethane (15 mL) and a saturated aqueous sodium bicarbonate solution (15 mL) were added to the reaction solution. The aqueous phase was extracted with dichloromethane (15 mL×3), and the organic phases were combined, washed with a saturated aqueous sodium chloride solution (15 mL), dried over anhydrous sodium sulphate, and concentrated under reduced pressure. The crude product was separated and purified by silica gel column chromatography (dichloromethane : methanol (v/v) = 10 : 1). The resulting crude product was subjected to Pre-HPLC (instrument and preparative column: using Waters 2767 preparative liquid phase chromatographic instrument; preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 0.1% TFA)/acetonitrile. Gradient elution method: gradient elution with acetonitrile from 10% to 50% (elution time: 15 min). Lyophilisation was performed to obtain the trifluoroacetate of compound 20 (50 mg).

$^1H$ NMR (400 MHz, DMSO-$d_6$) δ 10.75 (s, 1H), 7.74 (d, 2H), 7.64 (d, 1H), 7.48 (d, 1H), 6.97 (d, 2H), 6.78 (d, 1H), 6.66 - 6.57 (m, 2H), 6.54 (dd, 1H), 4.27 (s, 1H), 4.05 (d, 1H), 3.97 - 3.85 (m, 5H), 3.81 (dd, 1H), 3.77 - 3.68 (m, 1H), 3.02 - 2.86 (m, 3H), 2.86 - 2.75 (m, 2H), 2.75 - 2.55 (m, 4H), 2.50 - 2.39 (m, 2H), 2.35 - 2.22 (m, 1H), 2.18 - 2.04 (m, 1H), 2.03 - 1.80 (m, 5H), 1.67 - 1.43 (m, 3H), 1.19 (d, 12H).
LCMS m/z = 389.4 $[M/2+1]^+$

**Example 21: Preparation of trifluoroacetate of compound 21**

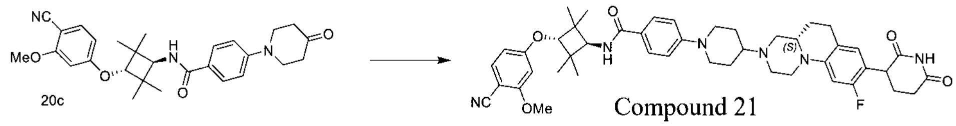

20c

Compound 21

Starting from compounds 20c and 2-D and following the synthetic method of example 20, the trifluoroacetate of compound 21 was obtained.

$^1$H NMR (400 MHz, DMSO-$d_6$) δ 10.76 (s, 1H), 7.79 - 7.70 (m, 2H), 7.64 (d, 1H), 7.48 (d, 1H), 7.01 - 6.91 (m, 2H), 6.78 (d, 1H), 6.67 - 6.49 (m, 3H), 4.27 (s, 1H), 4.05 (d, 1H), 3.97 - 3.85 (m, 5H), 3.81 (dd, 1H), 3.78 - 3.68 (m, 1H), 3.01 - 2.86 (m, 3H), 2.86 - 2.75 (m, 2H), 2.75 - 2.54 (m, 4H), 2.50 - 2.39 (m, 2H), 2.36 - 2.22 (m, 1H), 2.18 - 2.04 (m, 1H), 2.03 - 1.80 (m, 5H), 1.67 - 1.43 (m, 3H), 1.19 (d, 12H).
LCMS m/z = 777.4 [M+1] $^+$

**Example 22: Preparation of trifluoroacetate of compound 22**

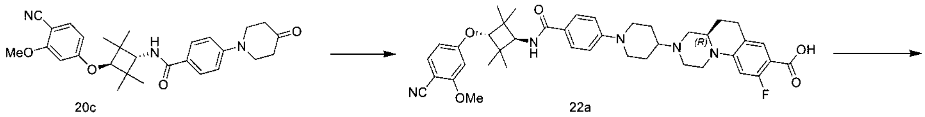

20c

22a

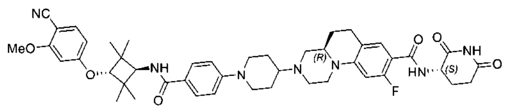

Compound 22

Step 1: Preparation of 22a

To a reaction flask were added 3-A (0.5 g, 1.43 mmol), trifluoroacetic acid (2 mL) and dichloromethane (6 mL), and the mixture was reacted at room temperature for 3 h. The reaction solution was concentrated under reduced pressure, and N,N-dimethylacetamide (5 mL) was added to the residue. 20c (0.68 g, 1.43 mmol), acetic acid (0.3 mL) and sodium triacetoxyborohydride (0.61 g, 2.88 mmol) were successively added, and the resulting mixture was reacted at room temperature for 16 h. Dichloromethane (15 mL) and a saturated aqueous sodium bicarbonate solution (15 mL) were added to the reaction solution. The aqueous phase was extracted with dichloromethane (15 mL×3), and the organic phases were combined, washed with a saturated aqueous sodium chloride solution (15 mL), dried over anhydrous sodium sulphate, and concentrated under reduced pressure. The crude product was separated and purified by silica gel column chromatography (dichloromethane : methanol (v/v) = 10 : 1) to obtain 22a (0.4 g, yield: 39%).
LCMS m/z = 710.3 [M+1] $^+$

Step 2: Preparation of trifluoroacetate of compound 22

To a reaction flask were added 22a (0.2 g, 0.28 mmol), EDCI (0.11 g, 0.57 mmol), HOBt (0.038 g, 0.28 mmol) and N-methylmorpholine (0.14 g, 1.38 mmol), followed by (S)-3-aminopiperidine-2,6-dione hydrochloride (0.092 g, 0.56 mmol) under nitrogen atmosphere, and the mixture was reacted at room temperature under nitrogen atmosphere for 16 h. Dichloromethane (15 mL) and purified water (15 mL) were added to the reaction solution. The aqueous phase was extracted with dichloromethane (15 mL×3), and the organic phases were combined, dried over anhydrous sodium sulphate, and concentrated under reduced pressure. The crude product was subjected to Pre-HPLC (instrument and preparative column: using Waters 2767 preparative liquid phase chromatographic instrument; preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 0.1% TFA)/acetonitrile. Gradient elution method: gradient elution with acetonitrile from 10% to 50% (elution

time: 15 min). Lyophilisation was performed to obtain the trifluoroacetate of compound 22 (50 mg).

$^1$H NMR (400 MHz, DMSO-$d_6$) δ 10.81 (s, 1H), 7.93 (t, 1H), 7.74 (d, 2H), 7.64 (d, 1H), 7.48 (d, 1H), 7.35 (d, 1H), 6.97 (d, 2H), 6.71 - 6.62 (m, 2H), 6.54 (dd, 1H), 4.78 - 4.61 (m, 1H), 4.27 (s, 1H), 4.05 (d, 1H), 3.98 - 3.78 (m, 6H), 3.13 - 3.02 (m, 1H), 3.02 - 2.91 (m, 2H), 2.87 - 2.62 (m, 6H), 2.57 - 2.41 (m, 2H), 2.36 - 2.21 (m, 1H), 2.17 - 1.80 (m, 6H), 1.66 - 1.44 (m, 3H), 1.19 (d, 12H).

LCMS m/z = 820.4 [M+1] $^+$

Following the synthetic method of other examples, the following compounds were obtained:

| Example No. | Structure | Starting material | LCMS / $^1$H NMR |
|---|---|---|---|
| Example 23 (trifluoroacetat e of compound 23) | 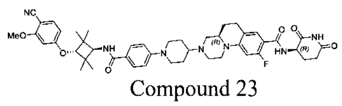 Compound 23 | 22a+ (R)-3-aminopiperidine -2,6-dione hydrochloride (following the synthetic method of example 22) | $^1$H NMR (400 MHz, DMSO-$d_6$) δ 10.81 (s, 1H), 7.98 - 7.89 (m, 1H), 7.78 - 7.70 (m, 2H), 7.68 - 7.61 (m, 1H), 7.49 (d, 1H), 7.35 (d, 1H), 6.97 (d, 2H), 6.72 - 6.61 (m, 2H), 6.54 (dd, 1H), 4.79 - 4.61 (m, 1H), 4.27 (s, 1H), 4.05 (d, 1H), 3.98 - 3.80 (m, 6H), 3.14 - 3.02 (m, 1H), 3.02 - 2.90 (m, 2H), 2.87 - 2.61 (m, 6H), 2.57 - 2.42 (m, 2H), 2.36 - 2.21 (m, 1H), 2.17 - 1.81 (m, 6H), 1.66 - 1.44 (m, 3H), 1.19 (d, 12H). LCMS m/z = 820.4 [M+1] $^+$ |
| Example 25 (trifluoroacetate of compound 25) | 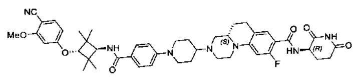 Compound 25 | 24a+(R)-3-aminopiperidine -2,6-dione hydrochloride (following the synthetic method of example 22) | $^1$H NMR (400 MHz, DMSO-$d_6$) δ 10.81 (s, 1H), 7.98 - 7.86 (m, 1H), 7.77 - 7.70 (m, 2H), 7.69 - 7.61 (m, 1H), 7.49 (d, 1H), 7.35 (d, 1H), 6.97 (d, 2H), 6.73 - 6.61 (m, 2H), 6.54 (dd, 1H), 4.79 - 4.61 (m, 1H), 4.27 (s, 1H), 4.05 (d, 1H), 3.98 - 3.80 (m, 6H), 3.14 - 3.03 (m, 1H), 3.02 - 2.91 (m, 2H), 2.87 - 2.61 (m, 6H), 2.58 - 2.42 (m, 2H), 2.36 - 2.21 (m, 1H), 2.17 - 1.81 (m, 6H), 1.66 - 1.43 (m, 3H), 1.19 (d, 12H). LCMS m/z = 820.4 [M+1] $^+$ |

**Example 24: Preparation of trifluoroacetate of compound 24**

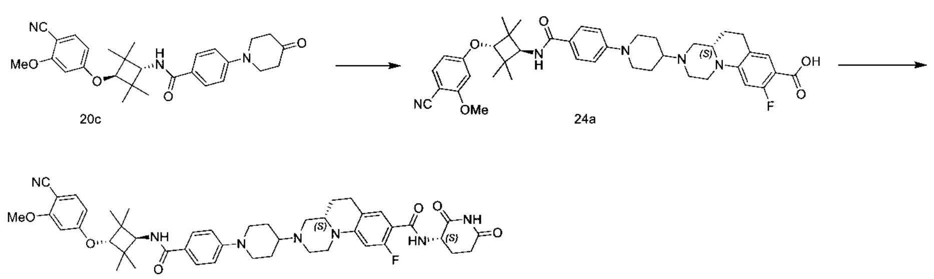
20c
24a

Compound 24

Starting from compounds 20c and 4-A and following the synthetic method of example 22, the trifluoroacetate of compound 24 was obtained.

$^1$H NMR (400 MHz, DMSO-$d_6$) δ 10.81 (s, 1H), 7.98 - 7.88 (m, 1H), 7.78 - 7.70 (m, 2H), 7.68 - 7.61 (m, 1H), 7.49 (d,

1H), 7.35 (d, 1H), 6.97 (d, 2H), 6.73 - 6.61 (m, 2H), 6.54 (dd, 1H), 4.79 - 4.61 (m, 1H), 4.27 (s, 1H), 4.05 (d, 1H), 3.98 - 3.81 (m, 6H), 3.14 - 3.03 (m, 1H), 3.02 - 2.90 (m, 2H), 2.87 - 2.61 (m, 6H), 2.58 - 2.42 (m, 2H), 2.36 - 2.22 (m, 1H), 2.17 - 1.81 (m, 6H), 1.66 - 1.43 (m, 3H), 1.19 (d, 12H).
LCMS m/z = 820.4 [M+1] $^+$

**Example 26: Preparation of compound 26**

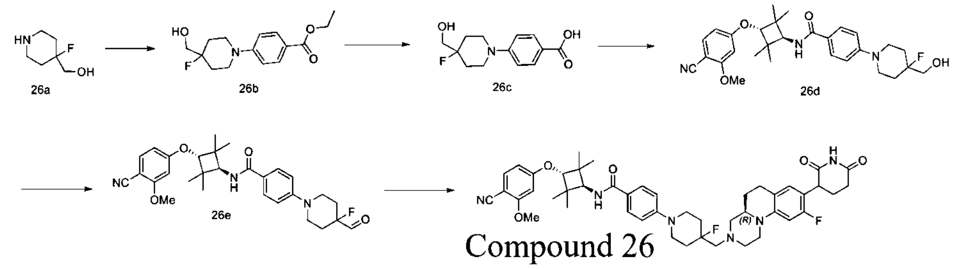

26a 26b 26c 26d 26e Compound 26

Step 1: Preparation of 26b

To a reaction flask were respectively added the hydrochloride of 26a (5.0 g, 29.48 mmol), ethyl 4-fluorobenzoate (4.96 g, 29.50 mmol), potassium carbonate (10.19 g, 73.7 mmol) and dimethyl sulphoxide (50 mL), and the mixture was reacted at 100°C for 16 h. The reaction system was cooled to room temperature, and water (100 mL) and ethyl acetate (100 mL) were added to the reaction solution. After phase separation, the aqueous phase was extracted with ethyl acetate (50 mL×3), and the organic phases were combined, washed with a saturated aqueous sodium chloride solution (50 mL), dried over anhydrous sodium sulphate, and filtered. The filtrate was concentrated under reduced pressure, and the crude product was separated and purified by silica gel column chromatography (petroleum ether/ethyl acetate (v/v) = 1 : 0-1 : 1) to obtain 26b (5.0 g, yield: 60%).

Step 2: Preparation of 26c

To a reaction flask were respectively added 26b (5.0 g, 17.77 mmol), potassium hydroxide (4 g, 71.43 mmol), ethanol (50 mL), tetrahydrofuran (50 mL) and water (50 mL), and the mixture was reacted at room temperature for 16 h. The reaction system was concentrated under reduced pressure, and adjusted to pH 2 with 2 mol/L hydrochloric acid. The aqueous phase was extracted with ethyl acetate (50 mL×3), and the organic phases were combined, washed with a saturated aqueous sodium chloride solution (50 mL), dried over anhydrous sodium sulphate, and filtered. The filtrate was concentrated under reduced pressure to obtain crude 26c (3.0 g).
LCMS m/z = 254.1 [M+1]$^+$

Step 3: Preparation of 26d

To a reaction flask were respectively added the above-mentioned crude 26c (0.82 g), HATU (2.46 g, 6.47 mmol), diisopropylethylamine (2.09 g, 16.17 mmol) and DMF (10 mL), and the mixture was stirred at room temperature for 0.5 h. trans-4-(3-Amino-2,2,4,4-tetramethylcyclobutoxy)-2-methoxybenzonitrile hydrochloride (1.0 g, 3.22 mmol) (see CN115175901 for the synthetic method) was then added, and the resulting mixture was reacted at room temperature for 2 h. Water (100 mL) and ethyl acetate (100 mL) were added to the reaction solution. After phase separation, the aqueous phase was extracted with ethyl acetate (50 mL×3), and the organic phases were combined, washed with a saturated aqueous sodium chloride solution (50 mL), dried over anhydrous sodium sulphate, and filtered. The filtrate was concentrated under reduced pressure, and the crude product was separated and purified by silica gel column chromatography (petroleum ether/ethyl acetate (v/v) = 1 : 0-0 : 1) to obtain 26d (1.5 g, yield: 91%).
LCMS m/z = 510.3 [M+1]$^+$

Step 4: Preparation of 26e

To a reaction flask were respectively added 26d (0.9 g, 1.77 mmol), Dess-Martin periodinane (0.9 g, 2.12 mmol) and dichloromethane (10 mL), and the mixture was reacted at room temperature for 4 h. The reaction solution was washed with a saturated aqueous sodium bicarbonate solution (10 mL), and the organic phase was dried over anhydrous sodium

sulphate, and concentrated under reduced pressure to obtain crude 26e (0.9 g).

Step 5: Preparation of compound 26

To a reaction flask were respectively added the above-mentioned crude 26e (0.25 g), the above-mentioned crude intermediate 1 (0.2 g), glacial acetic acid (0.2 mL) and DMAc (3 mL), and the mixture was reacted at room temperature for 1 h. Sodium triacetoxyborohydride (0.31 g, 1.46 mmol) was then added, and the resulting mixture was reacted at room temperature for 16 h. The reaction solution was subjected to Pre-HPLC (instrument and preparative column: using Waters 2767 preparative liquid phase chromatographic instrument; preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 10 mmol/L ammonium bicarbonate)/acetonitrile. Gradient elution method: gradient elution with acetonitrile from 10% to 50% (elution time: 15 min). Lyophilisation was performed to obtain compound 26 (91 mg, two-step yield from compound 26d: 23%).

$^1$H NMR (400 MHz, DMSO-$d_6$) δ 10.75 (s, 1H), 7.75 (d, 2H), 7.64 (d, 1H), 7.48 (d, 1H), 7.00 (d, 2H), 6.79 (d, 1H), 6.67 - 6.58 (m, 2H), 6.54 (dd, 1H), 4.27 (s, 1H), 4.05 (d, 1H), 3.91 (s, 3H), 3.85 - 3.77 (m, 1H), 3.75 - 3.59 (m, 3H), 3.19 - 3.07 (m, 2H), 3.04 - 2.89 (m, 3H), 2.80 - 2.64 (m, 3H), 2.63 - 2.52 (m, 4H), 2.31 - 2.21 (m, 1H), 2.18 - 2.05 (m, 1H), 2.04 - 1.88 (m, 4H), 1.88 - 1.68 (m, 3H), 1.64 - 1.51 (m, 1H), 1.18 (d, 12H) .
LCMS m/z = 809.4 [M+1]$^+$

**Example 28: Preparation of compound 28**

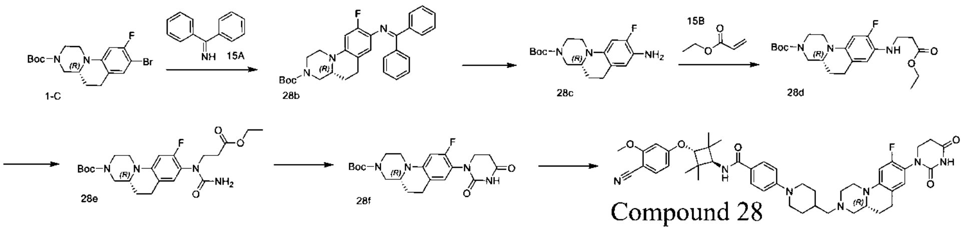


Starting from compounds 1-C and 15A and following the synthetic method of example 15, compound 28 was obtained by neutral preparative chromatography (ammonium bicarbonate in water (10 mmol/L)/acetonitrile) and lyophilisation.

$^1$H NMR (400 MHz, DMSO-$d_6$) δ 10.33 (s, 1H), 7.74 (d, 2H), 7.64 (d, 1H), 7.47 (d, 1H), 7.00 - 6.90 (m, 3H), 6.72 (d, 1H), 6.64 (d, 1H), 6.54 (dd, 1H), 4.27 (s, 1H), 4.05 (d, 1H), 3.91 (s, 3H), 3.89 - 3.81 (m, 2H), 3.79 - 3.71 (m, 1H), 3.60 (t, 2H), 3.04 - 2.95 (m, 1H), 2.94 - 2.85 (m, 2H), 2.84 - 2.57 (m, 7H), 2.19 (d, 2H), 2.11 - 2.00 (m, 1H), 1.94 - 1.85 (m, 1H), 1.85 - 1.71 (m, 4H), 1.67 - 1.54 (m, 1H), 1.28 - 1.10 (m, 14H).
LCMS m/z = 792.6 [M+1]$^+$

**Example 29: Preparation of compound 29**

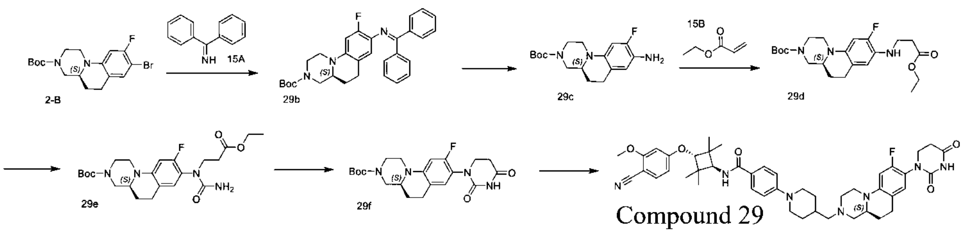


Starting from compounds 2-B and 15A and following the synthetic method of example 15, compound 29 was obtained by neutral preparative chromatography (ammonium bicarbonate in water (10 mmol/L)/acetonitrile) and lyophilisation.

$^1$H NMR (400 MHz, DMSO-$d_6$) δ 10.33 (s, 1H), 7.77 - 7.70 (m, 2H), 7.64 (d, 1H), 7.47 (d, 1H), 7.00 - 6.91 (m, 3H), 6.76 - 6.68 (m, 1H), 6.64 (d, 1H), 6.54 (dd, 1H), 4.27 (s, 1H), 4.05 (d, 1H), 3.91 (s, 3H), 3.91 - 3.81 (m, 2H), 3.79 - 3.71 (m,

1H), 3.60 (t, 2H), 3.06 - 2.94 (m, 1H), 2.94 - 2.85 (m, 2H), 2.84 - 2.55 (m, 7H), 2.19 (d, 2H), 2.11 - 2.00 (m, 1H), 1.94 - 1.85 (m, 1H), 1.85 - 1.71 (m, 4H), 1.67 - 1.53 (m, 1H), 1.29 - 1.11 (m, 14H).

**Example 30: Preparation of compound 30**

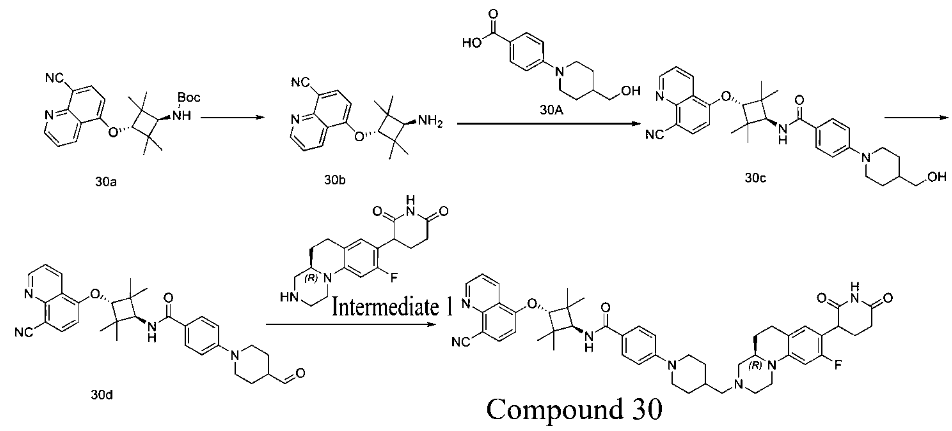


Step 1: Preparation of trifluoroacetate of 30b

30a (2.00 g, 5.06 mmol) (see US 2023135173 for the synthetic method) was dissolved in dichloromethane (20 mL), trifluoroacetic acid (10 mL) was added, and the mixture was reacted at room temperature for 3 h. The reaction system was concentrated under reduced pressure to obtain the trifluoroacetate of crude 30b (3.00 g).
LCMS m/z = 296.2 $[M+1]^+$

Step 2: Preparation of 30c

To a reaction flask were respectively added 30A (1.43 g, 6.07 mmol) (see CN115974840 for the synthetic method), HATU (2.87 g, 7.56 mmol), diisopropylethylamine (1.96 g, 15.18 mmol) and DMF (10 mL), and the mixture was stirred at room temperature for 0.5 h. The above-mentioned trifluoroacetate of crude 30b (3.00 g) was then added, and the resulting mixture was stirred at room temperature for 2 h. Water (100 mL) was added to the reaction solution, and the mixture was filtered. The filter cake was collected, and separated and purified by silica gel column chromatography (petroleum ether/ethyl acetate (v/v) = 1 : 0-0 : 1) to obtain 30c (1.50 g, yield: 58%).
LCMS m/z = 513.2 $[M+1]^+$

Step 3: Preparation of 30d

To a reaction flask were respectively added 30c (1.50 g, 2.93 mmol), Dess-Martin periodinane (2.49 g, 5.86 mmol) and dichloromethane (20 mL), and the mixture was reacted at room temperature for 30 min. The reaction system was filtered through a pad of diatomaceous earth, and the filtrate was concentrated under reduced pressure to obtain crude 30d (2.00 g).
LCMS m/z = 511.3 $[M+1]^+$

Step 4: Preparation of compound 30

To a reaction flask were respectively added the above-mentioned crude 30d (0.20 g), crude intermediate 1 (0.15 g), glacial acetic acid (0.2 mL) and DMAc (5 mL), and the mixture was reacted at room temperature for 1 h. Sodium triacetoxyborohydride (0.25 g, 1.17 mmol) was then added, and the resulting mixture was reacted at room temperature for 16 h. The reaction solution was subjected to Pre-HPLC (instrument and preparative column: using SHIMADZU LC-20AP preparative liquid chromatographic instrument, preparative column model: C18, 5 μm, inner diameter × length = 19 mm × 250 mm). Preparative method: a solution of the crude product in DMF was filtered through a 0.45 μm filter membrane to prepare a sample solution. Mobile phase system: water (containing 10 mmol/L ammonium bicarbonate)/acetonitrile. Gradient elution method: gradient elution with acetonitrile from 52% to 82% (elution time: 15 min). Lyophilisation was performed to obtain compound 30 (16.2 mg, two-step yield from compound 30c: 7%).

$^1$H NMR (400 MHz, DMSO-$d_6$) δ 10.76 (s, 1H), 9.13 - 9.08 (m, 1H), 8.74 (dd, 1H), 8.30 (d, 1H), 7.80 - 7.69 (m, 3H), 7.52 (d, 1H), 6.96 (d, 3H), 6.79 (d, 1H), 6.62 (d, 1H), 4.49 (s, 1H), 4.15 (d, 1H), 3.90 - 3.76 (m, 3H), 3.76 - 3.66 (m, 1H), 3.00 - 2.84 (m, 3H), 2.84 - 2.64 (m, 5H), 2.64 - 2.54 (m, 1H), 2.54 - 2.45 (m, 1H), 2.25 - 1.99 (m, 4H), 1.99 - 1.68 (m, 6H), 1.66 - 1.51 (m, 1H), 1.35 - 1.11 (m, 14H).
LCMS m/z = 812.4 [M+1]$^+$

Following the synthetic method of other examples, the following compounds were obtained:

| Example No. | Structure | Starting material | LCMS / $^1$H NMR |
|---|---|---|---|
| Example 27 (compound 27) | 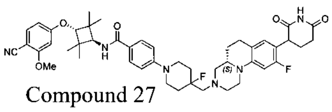 Compound 27 | 26e+ intermediate 2 (following the synthetic method of example 26) | $^1$H NMR (400 MHz, DMSO-$d_6$) δ 10.75 (s, 1H), 7.79 - 7.71 (m, 2H), 7.64 (d, 1H), 7.48 (d, 1H), 7.05 - 6.95 (m, 2H), 6.79 (d, 1H), 6.67 - 6.58 (m, 2H), 6.54 (dd, 1H), 4.27 (s, 1H), 4.05 (d, 1H), 3.91 (s, 3H), 3.85 - 3.77 (m, 1H), 3.75 - 3.58 (m, 3H), 3.19 - 3.06 (m, 2H), 3.04 - 2.88 (m, 3H), 2.80 - 2.64 (m, 3H), 2.63 - 2.51 (m, 4H), 2.31 - 2.21 (m, 1H), 2.18 - 2.05 (m, 1H), 2.04 - 1.88 (m, 4H), 1.88 - 1.68 (m, 3H), 1.64 - 1.50 (m, 1H), 1.18 (d, 12H). LCMS m/z = 809.4 [M+1]$^+$ |
| Example 31 (compound 31) | 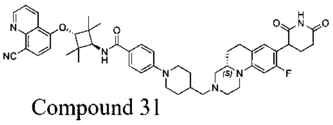 Compound 31 | 30d+ intermediate 2 (following the synthetic method of example 30) | $^1$H NMR (400 MHz, DMSO-$d_6$) δ 10.75 (s, 1H), 9.10 (dd, 1H), 8.74 (dd, 1H), 8.30 (d, 1H), 7.80 -7.70 (m, 3H), 7.52 (d, 1H), 6.96 (d, 3H), 6.79 (d, 1H), 6.62 (d, 1H), 4.49 (s, 1H), 4.16 (d, 1H), 3.90 - 3.76 (m, 3H), 3.76 - 3.66 (m, 1H), 3.02 - 2.84 (m, 3H), 2.84 - 2.65 (m, 5H), 2.64 - 2.54 (m, 1H), 2.54 - 2.45 (m, 1H), 2.24 - 1.99 (m, 4H), 1.99 - 1.68 (m, 6H), 1.67 - 1.51 (m, 1H), 1.38 - 1.10 (m, 14H). LCMS m/z = 812.4 [M+1]$^+$ |
| Example 32 (compound 32) | 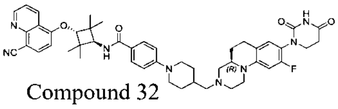 Compound 32 | 30d+28f (following the synthetic method of example 28) | $^1$H NMR (400 MHz, DMSO-$d_6$) δ 10.33 (s, 1H), 9.10 (dd, 1H), 8.74 (dd, 1H), 8.30 (d, 1H), 7.81 - 7.70 (m, 3H), 7.53 (d, 1H), 7.02 - 6.88 (m, 4H), 6.72 (d, 1H), 4.49 (s, 1H), 4.16 (d, 1H), 3.92 - 3.82 (m, 2H), 3.80 - 3.71 (m, 1H), 3.60 (t, 2H), 3.04 - 2.95 (m, 1H), 2.95 - 2.86 (m, 2H), 2.85 - 2.56 (m, 7H), 2.26 - 2.13 (m, 2H), 2.11 - 2.00 (m, 1H), 1.94 - 1.86 (m, 1H), 1.85 - 1.72 (m, 4H), 1.67 - 1.54 (m, 1H), 1.32 - 1.16 (m, 14H). LCMS m/z = 813.3 [M+1]$^+$ |
| Example 33 (trifluoroacetate of compound 33) | 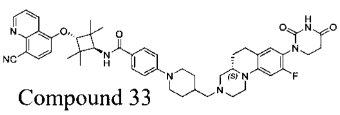 Compound 33 | 30d+29f (following the synthetic method of example 28) | $^1$H NMR (400 MHz, DMSO-$d_6$) δ 10.37 (s, 1H), 9.15 - 9.04 (m, 1H), 8.80 - 8.70 (m, 1H), 8.30 (d, 1H), 7.83 - 7.70 (m, 3H), 7.55 (d, 1H), 7.06 - 6.85 (m, 5H), 4.50 (s, 1H), 4.19 - 4.09 (m, 2H), 3.95 - 3.87 (m, 2H), 3.69 - 3.57 (m, 4H), 3.43 - 3.32 (m, 1H), 3.17 - 3.00 (m, 4H), 2.93 - 2.79 (m, 3H), 2.77 - 2.62 (m, 4H), 2.18 - 1.97 (m, 2H), 1.94 - 1.77 (m, 2H), 1.70 - 1.58 (m, 1H), 1.36 - 1.20 (m, 14H). LCMS m/z = 813.3 [M+1]$^+$ |

(continued)

| Example No. | Structure | Starting material | LCMS / $^1$H NMR |
|---|---|---|---|
| Example 34 (compound 34) | 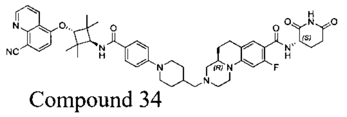 Compound 34 | 30d+5A (following the synthetic method of example 5) | $^1$H NMR (400 MHz, DMSO-$d_6$) δ 10.81 (s, 1H), 9.10 (dd, 1H), 8.74 (dd, 1H), 8.30 (d, 1H), 7.93 (t, 1H), 7.80 - 7.66 (m, 3H), 7.52 (d, 1H), 7.36 (d, 1H), 7.02 - 6.88 (m, 3H), 6.68 (d, 1H), 4.78 - 4.65 (m, 1H), 4.49 (s, 1H), 4.16 (d, 1H), 3.94 - 3.76 (m, 3H), 3.19 - 3.04 (m, 1H), 2.96 - 2.87 (m, 2H), 2.87 - 2.61 (m, 6H), 2.57 - 2.48 (m, 1H), 2.23 - 2.13 (m, 2H), 2.13 - 1.97 (m, 3H), 1.97 - 1.88 (m, 1H), 1.88 - 1.70 (m, 4H), 1.67 - 1.52 (m, 1H), 1.41 - 1.08 (m, 14H). LCMS m/z = 855.4 [M+1]$^+$ |
| Example 35 (compound 35) | 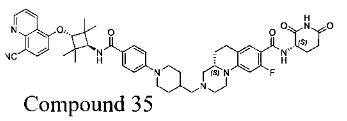 Compound 35 | 30d+7A (following the synthetic method of example 5) | $^1$H NMR (400 MHz, DMSO-$d_6$) δ 10.80 (s, 1H), 9.10 (dd, 1H), 8.74 (dd, 1H), 8.29 (d, 1H), 7.93 (t, 1H), 7.82 - 7.67 (m, 3H), 7.52 (d, 1H), 7.36 (d, 1H), 7.04 - 6.88 (m, 3H), 6.68 (d, 1H), 4.78 - 4.65 (m, 1H), 4.49 (s, 1H), 4.15 (d, 1H), 3.95 - 3.76 (m, 3H), 3.17 - 3.03 (m, 1H), 2.96 - 2.87 (m, 2H), 2.87 - 2.60 (m, 6H), 2.58 - 2.48 (m, 1H), 2.24 - 2.13 (m, 2H), 2.13 - 1.97 (m, 3H), 1.97 - 1.88 (m, 1H), 1.88 - 1.69 (m, 4H), 1.67 - 1.52 (m, 1H), 1.42 - 1.05 (m, 14H). LCMS m/z = 855.4 [M+1]$^+$ |

Example 36: Preparation of compound 36

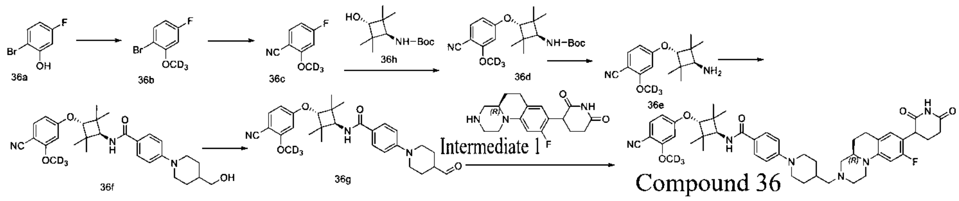


Step 1: Preparation of 36b

36a (10.00 g, 52.32 mmol) was dissolved in DMF (100 mL), potassium carbonate (14.46 g, 104.64 mmol) and deuterated iodomethane (9.10 g, 62.78 mmol) were added, and the mixture was reacted at room temperature for 19 h. The reaction solution was poured into water (200 mL), and the mixture was extracted with ethyl acetate (100 mL). The organic phase was dried over anhydrous sodium sulphate, and concentrated under reduced pressure, and the crude product was separated and purified by silica gel column chromatography (petroleum ether/ethyl acetate (v/v) = 10 : 1-2 : 1) to obtain crude 36b (11.5 g).

Step 2: Preparation of 36c

The above-mentioned crude 36b (11.5 g) was dissolved in NMP (100 mL), cuprous cyanide (11.30 g, 126.17 mmol) was added, and the mixture was reacted at 180°C for 19 h. The reaction solution was cooled to room temperature, concentrated aqueous ammonia (10 mL) and water (200 mL) were added, and the mixture was extracted with ethyl acetate (100 mL). The organic phase was dried over anhydrous sodium sulphate, and concentrated under reduced pressure, and the crude product was separated and purified by silica gel column chromatography (petroleum ether/ethyl acetate (v/v) = 10 : 1-2 : 1) to obtain 36c (7.5 g, two-step yield from compound 36a: 93%).

Step 3: Preparation of 36d

36h (8.37 g, 34.38 mmol) was dissolved in THF (50 mL), 60% sodium hydride (1.65 g) was added at 0°C, and the mixture was reacted at 0°C for 30 min. 36c (5.30 g, 34.38 mmol) was then added, and the resulting mixture was reacted at room temperature for 19 h. The reaction solution was poured into water (100 mL), and the mixture was extracted with ethyl acetate (100 mL). The organic phase was dried over anhydrous sodium sulphate, and concentrated under reduced pressure, and the crude product was separated and purified by silica gel column chromatography (petroleum ether/ethyl acetate (v/v) = 10 : 1-2 : 1) to obtain 36d (2.5 g, yield: 19%).
LCMS m/z = 378.3 $[M+1]^+$

Step 4: Preparation of hydrochloride of 36e

36d (2.5 g, 6.62 mmol) was dissolved in ethyl acetate (5 mL), 4 mol/L solution of hydrogen chloride in ethyl acetate (10 mL) was added, and the mixture was reacted at room temperature for 5 h. The reaction system was filtered, and the filter cake was collected, and dried under reduced pressure to obtain the hydrochloride of crude 36e (3.5 g).
LCMS m/z = 278.2 $[M+1]^+$

Step 5: Preparation of 36f

To a reaction flask were respectively added 30A (1.35 g, 5.74 mmol) (see CN115974840 for the synthetic method), HATU (2.73 g, 7.17 mmol), diisopropylethylamine (1.85 g, 14.34 mmol) and DMF (10 mL), and the mixture was stirred at room temperature for 0.5 h. The above-mentioned hydrochloride of crude 36e (2.5 g) was then added, and the resulting mixture was stirred at room temperature for 2 h. Water (100 mL) was added to the reaction solution, and the mixture was filtered. The filter cake was collected, and separated and purified by silica gel column chromatography (petroleum ether/ethyl acetate (v/v) = 1 : 0-0 : 1) to obtain 36f (1.6 g, two-step yield from compound 36d: 68%).
LCMS m/z = 495.3 $[M+1]^+$

Step 6: Preparation of 36g

To a reaction flask were respectively added 36f (99 mg, 0.20 mmol), Dess-Martin periodinane (0.17 g, 0.40 mmol) and dichloromethane (3 mL), and the mixture was reacted at room temperature for 30 min. To the reaction system was added a saturated aqueous sodium bicarbonate solution (10 mL), and the mixture was extracted with dichloromethane (30 mL). The organic phase was dried over anhydrous sodium sulphate, and filtered, and the filtrate was concentrated under reduced pressure to obtain crude 36g (110 mg).

Step 7: Preparation of compound 36

To a reaction flask were respectively added the above-mentioned crude 36g (0.11 g), crude intermediate 1 (0.12 g), glacial acetic acid (0.1 mL) and DMAc (5 mL), and the mixture was reacted at room temperature for 1 h. Sodium triacetoxyborohydride (0.13 g, 0.6 mmol) was then added, and the resulting mixture was reacted at room temperature for 16 h. The reaction solution was subjected to Pre-HPLC (instrument and preparative column: using SHIMADZU LC-20AP preparative liquid phase chromatographic instrument, preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 10 mmol/L $NH_4HCO_3$)/acetonitrile. Gradient elution method: gradient elution with acetonitrile from 53% to 83% (elution time: 15 min). Lyophilisation was performed to obtain compound 36 (46 mg, two-step yield from compound 36f: 29%).

$^1H$ NMR (400 MHz, $CDCl_3$) δ 7.89 (s, 1H), 7.68 (d, 2H), 7.45 (d, 1H), 6.91 (d, 2H), 6.78 - 6.65 (m, 1H), 6.54 - 6.43 (m, 2H), 6.40 (dd, 1H), 6.10 (d, 1H), 4.15 (d, 1H), 4.04 (s, 1H), 3.93 - 3.71 (m, 3H), 3.68 - 3.50 (m, 1H), 3.16 - 2.99 (m, 1H), 2.99 - 2.71 (m, 7H), 2.71 - 2.55 (m, 2H), 2.35 - 2.07 (m, 5H), 2.02 - 1.61 (m, 6H), 1.39 - 1.17 (m, 14H).
LCMS m/z = 794.7 $[M+1]^+$

Following the synthetic method of other examples, the following compounds were obtained:

| Example No. | Structure | Starting material | LCMS / $^1$H NMR |
|---|---|---|---|
| Example 37 (compound 37) | 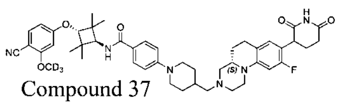 Compound 37 | 36g+intermediate 2 (following the synthetic method of example 36) | $^1$H NMR (400 MHz, DMSO-$d_6$) δ 10.76 (s, 1H), 7.73 (d, 2H), 7.64 (d, 1H), 7.47 (d, 1H), 6.95 (d, 2H), 6.79 (d, 1H), 6.67 - 6.57 (m, 2H), 6.54 (dd, 1H), 4.27 (s, 1H), 4.05 (d, 1H), 3.91 - 3.76 (m, 3H), 3.76 - 3.66 (m, 1H), 3.00 - 2.85 (m, 3H), 2.84 - 2.64 (m, 5H), 2.64 - 2.54 (m, 1H), 2.54 - 2.42 (m, 1H), 2.23 - 2.00 (m, 4H), 2.00 - 1.72 (m, 6H), 1.66 - 1.52 (m, 1H), 1.27 - 1.09 (m, 14H).<br>LCMS m/z = 794.7 [M+1]$^+$ |
| Example 38 (compound 38) | 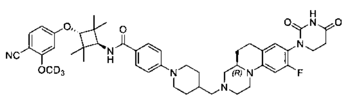 Compound 38 | 36g+28f (following the synthetic method of example 15) | $^1$H NMR (400 MHz, DMSO-$d_6$) δ 10.33 (s, 1H), 7.73 (d, 2H), 7.64 (d, 1H), 7.47 (d, 1H), 7.00 - 6.88 (m, 3H), 6.72 (d, 1H), 6.65 - 6.61 (m, 1H), 6.53 (dd, 1H), 4.27 (s, 1H), 4.05 (d, 1H), 3.91 - 3.80 (m, 2H), 3.80 - 3.69 (m, 1H), 3.59 (t, 2H), 3.04 - 2.95 (m, 1H), 2.94 - 2.85 (m, 2H), 2.84 - 2.64 (m, 6H), 2.64 - 2.56 (m, 1H), 2.23 - 2.13 (m, 2H), 2.10 - 1.99 (m, 1H), 1.94 - 1.71 (m, 5H), 1.67 - 1.53 (m, 1H), 1.28 - 1.09 (m, 14H).<br>LCMS m/z = 795.7 [M+1]$^+$ |
| Example 39 (compound 39) | 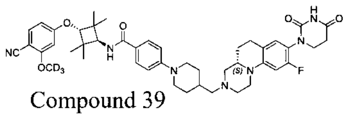 Compound 39 | 36g+29f (following the synthetic method of example 15) | $^1$H NMR (400 MHz, DMSO-$d_6$) δ 10.33 (s, 1H), 7.78 - 7.69 (m, 2H), 7.64 (d, 1H), 7.47 (d, 1H), 7.00 - 6.88 (m, 3H), 6.72 (d, 1H), 6.66 - 6.60 (m, 1H), 6.54 (dd, 1H), 4.27 (s, 1H), 4.05 (d, 1H), 3.91 - 3.80 (m, 2H), 3.79 - 3.70 (m, 1H), 3.59 (t, 2H), 3.04 - 2.95 (m, 1H), 2.94 - 2.85 (m, 2H), 2.84 - 2.56 (m, 7H), 2.24 - 2.12 (m, 2H), 2.10 - 1.98 (m, 1H), 1.94 - 1.85 (m, 1H), 1.85 - 1.70 (m, 4H), 1.67 - 1.52 (m, 1H), 1.29 - 1.10 (m, 14H).<br>LCMS m/z = 795.7 [M+1]$^+$ |
| Example 41 (compound 41) | 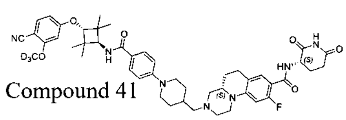 Compound 41 | 36g+7A (following the synthetic method of example 11) | $^1$H NMR (400 MHz, DMSO-$d_6$) δ 10.82 (s, 1H), 7.94 (t, 1H), 7.79 - 7.69 (m, 2H), 7.64 (d, 1H), 7.47 (d, 1H), 7.36 (d, 1H), 7.02 - 6.91 (m, 2H), 6.73 - 6.59 (m, 2H), 6.54 (dd, 1H), 4.79 - 4.65 (m, 1H), 4.27 (s, 1H), 4.05 (d, 1H), 3.93 - 3.78 (m, 3H), 3.16 - 3.05 (m, 1H), 2.98 - 2.61 (m, 8H), 2.58 - 2.44 (m, 1H), 2.25 - 1.88 (m, 6H), 1.87 - 1.70 (m, 4H), 1.67 - 1.52 (m, 1H), 1.28 - 1.10 (m, 14H).<br>LCMS m/z = 419.3 [M/2 +1]$^+$ |

**Example 40: Preparation of compound 40**

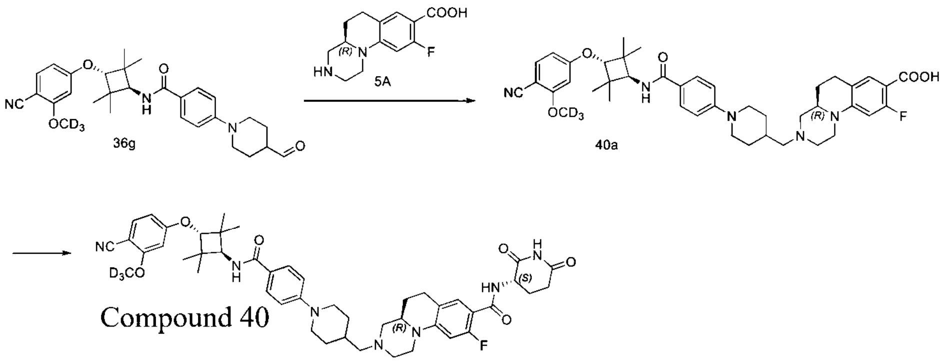
36g 5A 40a

Compound 40

Starting from compound 36g and the trifluoroacetate of 5A and following the synthetic method of example 11, compound 40 was obtained.

$^1$H NMR (400 MHz, DMSO-$d_6$) δ 10.82 (s, 1H), 7.94 (t, 1H), 7.78 - 7.68 (m, 2H), 7.64 (d, 1H), 7.47 (d, 1H), 7.36 (d, 1H), 6.95 (d, 2H), 6.74 - 6.59 (m, 2H), 6.54 (dd, 1H), 4.79 - 4.65 (m, 1H), 4.27 (s, 1H), 4.05 (d, 1H), 3.93 - 3.76 (m, 3H), 3.17 - 3.05 (m, 1H), 3.00 - 2.61 (m, 8H), 2.58 - 2.44 (m, 1H), 2.25 - 1.88 (m, 6H), 1.87 - 1.70 (m, 4H), 1.67 - 1.51 (m, 1H), 1.28 - 1.11 (m, 14H).

LCMS m/z = 419.3 [M/2 +1]$^+$

**Example 42: Preparation of compound 42**

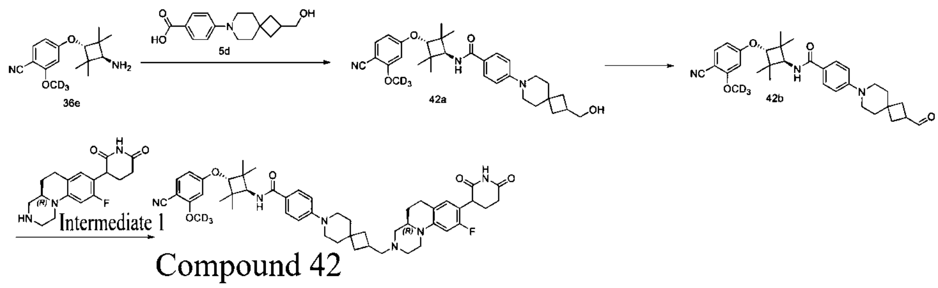
36e 5d 42a 42b Intermediate 1

Compound 42

Starting from the hydrochloride of compounds 36e and 5d and following the synthetic method of example 36, compound 42 was obtained.

$^1$H NMR (400 MHz, DMSO-d6) δ 10.75 (s, 1H), 7.72 (d, 2H), 7.64 (d, 1H), 7.47 (d, 1H), 6.95 (d, 2H), 6.78 (d, 1H), 6.65 - 6.57 (m, 2H), 6.56 - 6.50 (m, 1H), 4.26 (s, 1H), 4.04 (d, 1H), 3.81 (dd, 1H), 3.74 - 3.62 (m, 1H), 3.29 - 3.22 (m, 2H), 3.21 - 3.12 (m, 2H), 2.99 - 2.78 (m, 3H), 2.75 - 2.54 (m, 4H), 2.54 - 2.45 (m, 2H), 2.43 - 2.34 (m, 2H), 2.17 - 1.90 (m, 5H), 1.89 - 1.72 (m, 2H), 1.71 - 1.63 (m, 2H), 1.63 - 1.50 (m, 3H), 1.50 - 1.39 (m, 2H), 1.18 (d, 12H).

LCMS m/z = 834.7 [M+1]$^+$

Following the synthetic method of other examples, the following compounds were obtained:

| Example No. | Structure | Starting material | LCMS / $^1$H NMR |
|---|---|---|---|
| Example 43 (compound 43) | 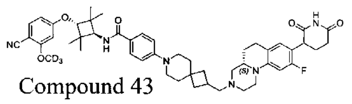 Compound 43 | 42b+intermediate 2 (following the synthetic method of example 36) | $^1$H NMR (400 MHz, DMSO-$d_6$) δ 10.75 (s, 1H), 7.72 (d, 2H), 7.64 (d, 1H), 7.47 (d, 1H), 6.95 (d, 2H), 6.78 (d, 1H), 6.66 - 6.56 (m, 2H), 6.56 - 6.50 (m, 1H), 4.26 (s, 1H), 4.04 (d, 1H), 3.85 - 3.75 (m, 1H), 3.74 - 3.62 (m, 1H), 3.30 - 3.21 (m, 2H), 3.21 - 3.11 (m, 2H), 2.99 - 2.76 (m, 3H), 2.75 - 2.54 (m, 4H), 2.54 - 2.45 (m, 2H), 2.44 - 2.34 (m, 2H), 2.18 - 1.90 (m, 5H), 1.89 - 1.72 (m, 2H), 1.71 - 1.63 (m, 2H), 1.63 - 1.50 (m, 3H), 1.50 - 1.39 (m, 2H), 1.18 (d, 12H). LCMS m/z = 834.7 [M+1]$^+$ |
| Example 44 (trifluoroacetate of compound 44) | 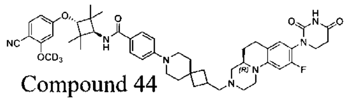 Compound 44 | 42b+28f (following the synthetic method of example 15) | $^1$H NMR (400 MHz, DMSO-$d_6$) δ 10.36 (s, 1H), 7.79 - 7.69 (m, 2H), 7.64 (d, 1H), 7.52 - 7.44 (m, 1H), 7.05 - 6.93 (m, 3H), 6.88 (d, 1H), 6.63 (d, 1H), 6.53 (dd, 1H), 4.27 (s, 1H), 4.15 - 4.08 (m, 1H), 4.07 - 4.03 (m, 1H), 3.60 (t, 2H), 3.56 - 3.44 (m, 2H), 3.35 - 3.15 (m, 7H), 3.14 - 3.00 (m, 2H), 2.91 - 2.81 (m, 1H), 2.80 - 2.61 (m, 5H), 2.14 - 1.95 (m, 3H), 1.76 - 1.52 (m, 7H), 1.18 (d, 12H). LCMS m/z = 835.7 [M+1]$^+$ |
| Example 45 (trifluoroacetate of compound 45) | 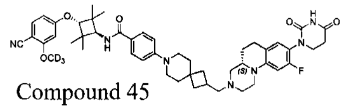 Compound 45 | 42b+29f (following the synthetic method of example 15) | $^1$H NMR (400 MHz, $CD_3OD$) δ 7.72 (d, 2H), 7.52 (d, 1H), 7.41 (d, 1H), 7.06 - 6.95 (m, 3H), 6.79 (d, 1H), 6.62 (d, 1H), 6.55 (dd, 1H), 4.26 (s, 1H), 4.16 - 4.03 (m, 2H), 3.72 (t, 2H), 3.66 - 3.57 (m, 1H), 3.56 - 3.49 (m, 1H), 3.38 - 3.21 (m, 7H), 3.19 - 3.08 (m, 2H), 2.98 - 2.87 (m, 1H), 2.87 - 2.72 (m, 5H), 2.26 - 2.16 (m, 2H), 2.12 - 1.99 (m, 1H), 1.88 - 1.64 (m, 7H), 1.26 (d, 12H). LCMS m/z = 835.7 [M+1]$^+$ |
| Example 46 (compound 46) | 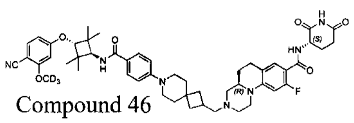 Compound 46 | 42b+trifluoroacetate of 5A (following the synthetic method of example 40) | $^1$H NMR (400 MHz, DMSO-$d_6$) δ 10.80 (s, 1H), 7.92 (t, 1H), 7.73 (d, 2H), 7.64 (d, 1H), 7.46 (d, 1H), 7.35 (d, 1H), 6.95 (d, 2H), 6.71 - 6.60 (m, 2H), 6.53 (dd, 1H), 4.77 - 4.65 (m, 1H), 4.27 (s, 1H), 4.04 (d, 1H), 3.88 - 3.77 (m, 1H), 3.32 - 3.22 (m, 2H), 3.22 - 3.13 (m, 2H), 3.13 - 3.02 (m, 1H), 2.90 - 2.64 (m, 6H), 2.56 - 2.45 (m, 2H), 2.43 - 2.36 (m, 2H), 2.17 - 1.86 (m, 6H), 1.83 - 1.73 (m, 1H), 1.71 - 1.63 (m, 2H), 1.63 - 1.50 (m, 3H), 1.50 - 1.41 (m, 2H), 1.18 (d, 12H). LCMS m/z = 877.7 [M+1]$^+$ |

(continued)

| Example No. | Structure | Starting material | LCMS / $^1$H NMR |
|---|---|---|---|
| Example 47 (compound 47) | 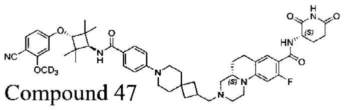 Compound 47 | 42b+7A (following the synthetic meth-od of example 40) | $^1$H NMR (400 MHz, DMSO-$d_6$) δ 10.80 (s, 1H), 7.92 (t, 1H), 7.73 (d, 2H), 7.64 (d, 1H), 7.46 (d, 1H), 7.35 (d, 1H), 6.95 (d, 2H), 6.70 - 6.60 (m, 2H), 6.53 (dd, 1H), 4.77 - 4.65 (m, 1H), 4.27 (s, 1H), 4.04 (d, 1H), 3.89 - 3.77 (m, 1H), 3.33 - 3.22 (m, 2H), 3.22 - 3.13 (m, 2H), 3.13 - 3.00 (m, 1H), 2.91 - 2.64 (m, 6H), 2.55 - 2.44 (m, 2H), 2.44 - 2.36 (m, 2H), 2.17 - 1.85 (m, 6H), 1.83 - 1.73 (m, 1H), 1.71 - 1.63 (m, 2H), 1.63 - 1.50 (m, 3H), 1.50 - 1.41 (m, 2H), 1.18 (d, 12H). LCMS m/z = 877.6 [M+1]$^+$ |

**Example 48: Preparation of compound 48**

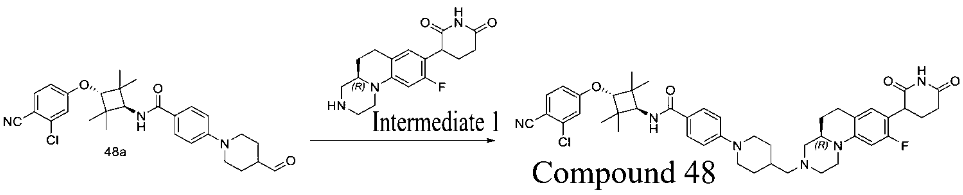


Starting from compound 48a (see WO 2021231927 for the synthetic method) and intermediate 1 and following the synthetic method of example 36, compound 48 was obtained.

$^1$H NMR (400 MHz, DMSO-$d_6$) δ 10.75 (s, 1H), 7.90 (d, 1H), 7.73 (d, 2H), 7.45 (d, 1H), 7.23 - 7.17 (m, 1H), 7.03 - 6.97 (m, 1H), 6.95 (d, 2H), 6.79 (d, 1H), 6.62 (d, 1H), 4.32 (s, 1H), 4.05 (d, 1H), 3.90 - 3.77 (m, 3H), 3.76 - 3.67 (m, 1H), 2.99 - 2.85 (m, 3H), 2.83 - 2.55 (m, 6H), 2.54 - 2.45 (m, 1H), 2.21 - 2.14 (m, 2H), 2.14 - 2.02 (m, 2H), 1.98 - 1.73 (m, 6H), 1.65 - 1.53 (m, 1H), 1.26 - 1.08 (m, 14H).
LCMS m/z = 795.3 [M+1]$^+$

Following the synthetic method of other examples, the following compounds were obtained:

| Example No. | Structure | Starting material | LCMS / $^1$H NMR |
|---|---|---|---|
| Example 49 (compound 49) | 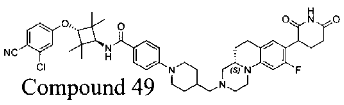 Compound 49 | 48a+intermediate 2 (following the synthetic method of example 36) | $^1$H NMR (400 MHz, DMSO-$d_6$) δ 10.75 (s, 1H), 7.90 (d, 1H), 7.73 (d, 2H), 7.45 (d, 1H), 7.24 - 7.16 (m, 1H), 7.03 - 6.96 (m, 1H), 6.95 (d, 2H), 6.79 (d, 1H), 6.62 (d, 1H), 4.32 (s, 1H), 4.05 (d, 1H), 3.90 - 3.76 (m, 3H), 3.76 - 3.66(m, 1H), 2.99 - 2.85 (m, 3H), 2.84 - 2.54 (m, 6H), 2.54 - 2.45 (m, 1H), 2.22 - 2.14 (m, 2H), 2.14 - 2.01 (m, 2H), 1.98 - 1.72 (m, 6H), 1.66 - 1.52 (m, 1H), 1.26 - 1.06 (m, 14H). LCMS m/z = 795.3 [M+1]$^+$ |

(continued)

| Example No. | Structure | Starting material | LCMS / $^1$H NMR |
|---|---|---|---|
| Example 50 (compound 50) | 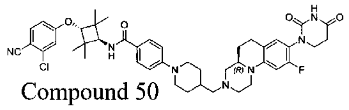 Compound 50 | 48a+28f (following the synthetic method of example 15) | $^1$H NMR (400 MHz, DMSO-$d_6$) δ 10.31 (s, 1H), 7.90 (d, 1H), 7.77 - 7.68 (m, 2H), 7.45 (d, 1H), 7.25 - 7.15 (m, 1H), 7.05 - 6.89 (m, 4H), 6.72 (d, 1H), 4.32 (s, 1H), 4.05 (d, 1H), 3.90 - 3.80 (m, 2H), 3.79 - 3.68 (m, 1H), 3.59 (t, 2H), 3.05 - 2.94 (m, 1H), 2.94 - 2.84 (m, 2H), 2.84 - 2.64 (m, 6H), 2.64 - 2.53 (m, 1H), 2.23 - 2.13 (m, 2H), 2.10 - 2.01 (m, 1H), 1.93 - 1.72 (m, 5H), 1.66 - 1.52 (m, 1H), 1.25 - 1.11 (m, 14H). LCMS m/z = 796.3 [M+1]$^+$ |
| Example 51 (compound 51) | 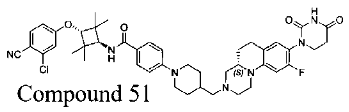 Compound 51 | 48a+29f (following the synthetic method of example 15) | $^1$H NMR (400 MHz, DMSO-$d_6$) δ 10.31 (s, 1H), 7.90 (d, 1H), 7.77 - 7.68 (m, 2H), 7.45 (d, 1H), 7.22 - 7.17 (m, 1H), 7.03 - 6.97 (m, 1H), 6.97 - 6.89 (m, 3H), 6.72 (d, 1H), 4.32 (s, 1H), 4.05 (d, 1H), 3.90 - 3.80 (m, 2H), 3.79 - 3.68 (m, 1H), 3.59 (t, 2H), 3.04 - 2.95 (m, 1H), 2.94 - 2.84 (m, 2H), 2.84 - 2.64 (m, 6H), 2.64 - 2.56 (m, 1H), 2.23 - 2.13 (m, 2H), 2.10 - 2.00 (m, 1H), 1.93 - 1.73 (m, 5H), 1.66 - 1.52 (m, 1H), 1.24 - 1.11 (m, 14H). LCMS m/z = 796.3 [M+1]$^+$ |
| Example 52 (compound 52) | 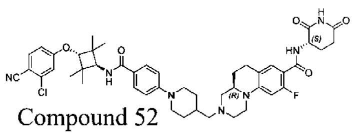 Compound 52 | 48a+trifluoroacetate of 5A (following the synthetic method of example 40) | $^1$H NMR (400 MHz, DMSO-$d_6$) δ 10.81 (s, 1H), 7.97 - 7.86 (m, 2H), 7.73 (d, 2H), 7.45 (d, 1H), 7.36 (d, 1H), 7.20 (d, 1H), 7.04 - 6.91 (m, 3H), 6.68 (d, 1H), 4.78 - 4.66 (m, 1H), 4.32 (s, 1H), 4.05 (d, 1H), 3.93 - 3.77 (m, 3H), 3.17 - 3.05 (m, 1H), 2.97 - 2.86 (m, 2H), 2.86 - 2.61 (m, 6H), 2.57 - 2.47 (m, 1H), 2.25 - 2.14 (m, 2H), 2.14 - 1.87 (m, 4H), 1.86 - 1.71 (m, 4H), 1.66 - 1.53 (m, 1H), 1.27 - 1.08 (m, 14H). LCMS m/z = 838.5 [M+1]$^+$ |
| Example 53 (compound 53) | 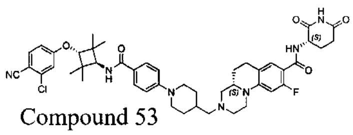 Compound 53 | 48a+7A (following the synthetic method of example 40) | $^1$H NMR (400 MHz, DMSO-$d_6$) δ 10.81 (s, 1H), 7.97 - 7.86 (m, 2H), 7.73 (d, 2H), 7.45 (d, 1H), 7.36 (d, 1H), 7.20 (d, 1H), 7.05 - 6.90 (m, 3H), 6.68 (d, 1H), 4.78 - 4.65 (m, 1H), 4.32 (s, 1H), 4.05 (d, 1H), 3.94 - 3.75 (m, 3H), 3.17 - 3.05 (m, 1H), 2.98 -2.61 (m, 8H), 2.57 - 2.47 (m, 1H), 2.26 - 2.14 (m, 2H), 2.14 - 1.86 (m, 4H), 1.86 - 1.70 (m, 4H), 1.67 - 1.52 (m, 1H), 1.27 - 1.07 (m, 14H). LCMS m/z = 838.6 [M+1]$^+$ |

**Example 54: Preparation of compound 54**

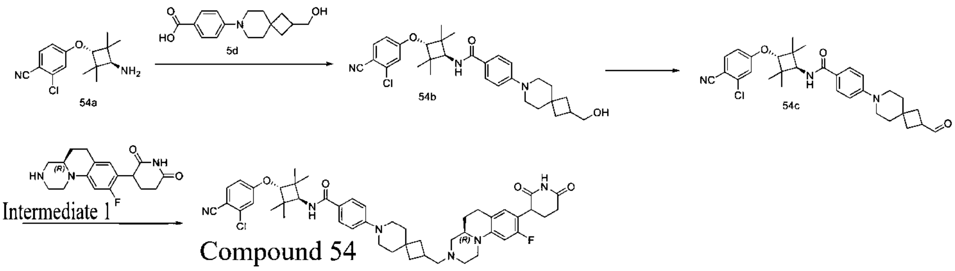


Compound 54

Starting from the hydrochloride of compound 54a (see WO 2021231927 for the synthetic method) and 5d and following the synthetic method of example 42, compound 54 was obtained.

$^1$H NMR (400 MHz, DMSO-$d_6$) δ 10.74 (s, 1H), 7.90 (d, 1H), 7.72 (d, 2H), 7.45 (d, 1H), 7.20 (d, 1H), 7.04 - 6.90 (m, 3H), 6.78 (d, 1H), 6.60 (d, 1H), 4.32 (s, 1H), 4.04 (d, 1H), 3.81 (dd, 1H), 3.74 - 3.62 (m, 1H), 3.28 - 3.23 (m, 2H), 3.20 - 3.15 (m, 2H), 2.97 - 2.79 (m, 3H), 2.74 - 2.56 (m, 4H), 2.55 - 2.51 (m, 2H), 2.42 - 2.36 (m, 2H), 2.14 - 2.03 (m, 2H), 2.00 - 1.90 (m, 3H), 1.89 - 1.81 (m, 1H), 1.81 - 1.73 (m, 1H), 1.71 - 1.63 (m, 2H), 1.62 - 1.50 (m, 3H), 1.50 - 1.40 (m, 2H), 1.17 (d, 12H). LCMS m/z = 835.4 $[M+1]^+$

Following the synthetic method of other examples, the following compounds were obtained:

| Example No. | Structure | Starting material | LCMS / $^1$H NMR |
|---|---|---|---|
| Example 55 (compound 55) | 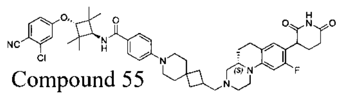 Compound 55 | 54c+intermediate 2 (following the synthetic method of example 54) | $^1$H NMR (400 MHz, DMSO-$d_6$) δ 10.74 (s, 1H), 7.90 (d, 1H), 7.76 - 7.67 (m, 2H), 7.45 (d, 1H), 7.20 (d, 1H), 7.04 - 6.91 (m, 3H), 6.78 (d, 1H), 6.60 (d, 1H), 4.32 (s, 1H), 4.04 (d, 1H), 3.81 (dd, 1H), 3.74 - 3.61 (m, 1H), 3.29 - 3.23 (m, 2H), 3.21 - 3.15 (m, 2H), 2.97 - 2.79 (m, 3H), 2.74 - 2.55 (m, 4H), 2.55 - 2.50 (m, 2H), 2.42 - 2.36 (m, 2H), 2.14 - 2.02 (m, 2H), 2.00 - 1.90 (m, 3H), 1.89 - 1.81 (m, 1H), 1.81 - 1.72 (m, 1H), 1.71 - 1.62 (m, 2H), 1.62 - 1.50 (m, 3H), 1.50 - 1.40 (m, 2H), 1.17 (d, 12H). LCMS m/z = 418.1 $[M/2 +1]^+$ |
| Example 56 (compound 56) | 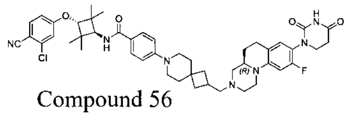 Compound 56 | 54c+28f (following the synthetic method of example 15) | $^1$H NMR (400 MHz, DMSO-$d_6$) δ 10.32 (s, 1H), 7.90 (d, 1H), 7.77 - 7.67 (m, 2H), 7.46 (d, 1H), 7.23 - 7.16 (m, 1H), 7.04 - 6.87 (m, 4H), 6.70 (d, 1H), 4.31 (s, 1H), 4.04 (d, 1H), 3.79 - 3.65 (m, 1H), 3.59 (t, 2H), 3.29 - 3.23 (m, 2H), 3.21 - 3.13 (m, 2H), 3.01 - 2.90 (m, 1H), 2.90 - 2.79 (m, 2H), 2.79 - 2.51 (m, 6H), 2.43 - 2.35 (m, 2H), 2.10 - 1.72 (m, 5H), 1.72 - 1.39 (m, 7H), 1.17 (d, 12H). LCMS m/z = 836.4 $[M+1]^+$ |
| Example 57 (compound 57) | 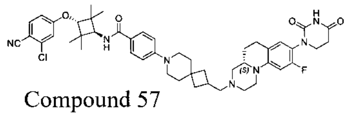 Compound 57 | 54c+29f (following the synthetic method of example 15) | $^1$H NMR (400 MHz, DMSO-$d_6$) δ 10.32 (s, 1H), 7.90 (d, 1H), 7.78 - 7.65 (m, 2H), 7.46 (d, 1H), 7.25 - 7.13 (m, 1H), 7.05 - 6.86 (m, 4H), 6.70 (d, 1H), 4.31 (s, 1H), 4.04 (d, 1H), 3.79 - 3.63 (m, 1H), 3.59 (t, 2H), 3.29 - 3.22 (m, 2H), 3.21 - 3.13 (m, 2H), 3.02 - 2.90 (m, 1H), 2.90 - 2.79 (m, 2H), 2.79 - 2.51 (m, 6H), 2.45 - 2.33 (m, 2H), 2.12 - 1.72 (m, 5H), 1.72 - 1.37 (m, 7H), 1.17 (d, 12H). LCMS m/z = 836.3 $[M+1]^+$ |

(continued)

| Example No. | Structure | Starting material | LCMS / $^1$H NMR |
|---|---|---|---|
| Example 58 (compound 58) | 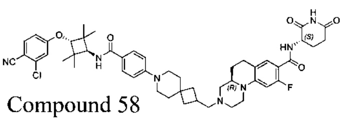 Compound 58 | 54c+trifluoroacetate of 5A (following the synthetic method of example 40) | $^1$H NMR (400 MHz, DMSO-$d_6$) δ 10.82 (s, 1H), 7.98 - 7.87 (m, 2H), 7.76 - 7.68 (m, 2H), 7.46 (d, 1H), 7.35 (d, 1H), 7.20 (d, 1H), 7.04 - 6.90 (m, 3H), 6.67 (d, 1H), 4.78 - 4.66 (m, 1H), 4.32 (s, 1H), 4.05 (d, 1H), 3.91 - 3.75 (m, 1H), 3.28 - 3.23 (m, 2H), 3.22 - 3.14 (m, 2H), 3.12 - 3.04 (m, 1H), 2.90 - 2.61 (m, 6H), 2.58 - 2.52 (m, 2H), 2.44 - 2.35 (m, 2H), 2.17 - 1.85 (m, 6H), 1.83 - 1.73 (m, 1H), 1.72 - 1.63 (m, 2H), 1.63 - 1.50 (m, 3H), 1.50 - 1.40 (m, 2H), 1.17 (d, 12H). LCMS m/z = 439.8 [M/2 +1]$^+$ |
| Example 59 (compound 59) | 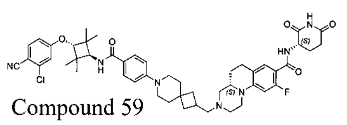 Compound 59 | 54c+7A (following the synthetic method of example 40) | $^1$H NMR (400 MHz, DMSO-$d_6$) δ 10.81 (s, 1H), 7.98 - 7.86 (m, 2H), 7.72 (d, 2H), 7.46 (d, 1H), 7.35 (d, 1H), 7.24 - 7.16 (m, 1H), 7.04 - 6.88 (m, 3H), 6.67 (d, 1H), 4.77 - 4.63 (m, 1H), 4.32 (s, 1H), 4.05 (d, 1H), 3.88 - 3.76 (m, 1H), 3.33 - 3.22 (m, 2H), 3.22 - 3.14 (m, 2H), 3.13 - 3.03 (m, 1H), 2.92 - 2.61 (m, 6H), 2.57 - 2.46 (m, 2H), 2.44 - 2.37 (m, 2H), 2.18 - 1.84 (m, 6H), 1.84 - 1.73 (m, 1H), 1.72 - 1.63 (m, 2H), 1.63 - 1.50 (m, 3H), 1.50 - 1.40 (m, 2H), 1.17 (d, 12H). LCMS m/z = 439.8 [M/2 +1]$^+$ |

**Example 60: Preparation of trifluoroacetate of compound 60**

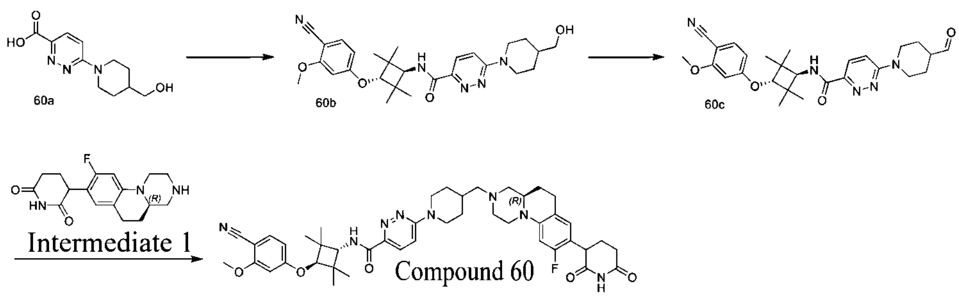


60a 60b 60c Compound 60

Step 1: Preparation of 60b

60a (0.51 g, 2.15 mmol) (see WO 2023066350 for the synthetic method) was dissolved in DMF (15 mL), trans-4-(3-amino-2,2,4,4-tetramethylcyclobutoxy)-2-methoxybenzonitrile hydrochloride (1.00 g, 3.22 mmol) (see CN 115175901 for the synthetic method), N,N-diisopropylethylamine (1.39 g, 10.75 mmol) and HATU (0.98 g, 2.58 mmol) were respectively added under nitrogen atmosphere, and the mixture was reacted at room temperature for 16 h. Ethyl acetate (30 mL) and water (30 mL) were added to the reaction solution. After phase separation, the aqueous phase was extracted with ethyl acetate (15 mL×3), and the organic phases were combined, washed with a saturated aqueous sodium chloride solution (15 mL), dried over anhydrous sodium sulphate, and concentrated under reduced pressure. The crude product was separated and purified by silica gel column chromatography (petroleum ether : ethyl acetate (v/v) = 1 : 2) to obtain 60b (0.8 g, yield: 75%).

LCMS m/z = 494.2 [M+1] +

Step 2: Preparation of 60c

60b (0.8 g, 1.62 mmol) was dissolved in dichloromethane (15 mL), Dess-Martin periodinane (0.82 g, 1.94 mmol) was added, and the mixture was reacted at room temperature for 2 h. The reaction solution was concentrated under reduced pressure, and the crude product was separated and purified by silica gel column chromatography (petroleum ether : ethyl acetate (v/v) = 1 : 2) to obtain 60c (0.6 g, yield: 75%).
LCMS m/z = 492.4 [M+1]+

Step 3: Preparation of trifluoroacetate of compound 60

Starting from compound 60c and intermediate 1 and following the synthetic method of example 1, the trifluoroacetate of compound 60 was obtained.

$^1$H NMR (400 MHz, DMSO-$d_6$) δ 10.78 (s, 1H), 8.23 (d, 1H), 7.86 (d, 1H), 7.64 (d, 1H), 7.42 (d, 1H), 6.88 (d, 1H), 6.80 (d, 1H), 6.67 (d, 1H), 6.57 (dd, 1H), 4.60 - 4.48 (m, 2H), 4.40 (s, 1H), 4.14 - 4.05 (m, 1H), 4.04 - 3.98 (m, 1H), 3.92 (s, 3H), 3.89 - 3.82 (m, 1H), 3.70 - 3.57 (m, 2H), 3.39 - 3.30 (m, 1H), 3.19 - 2.98 (m, 6H), 2.93 - 2.82 (m, 1H), 2.81 - 2.62 (m, 3H), 2.56 - 2.52 (m, 1H), 2.31 - 2.21 (m, 1H), 2.20 - 2.06 (m, 1H), 2.06 - 1.79 (m, 4H), 1.70 - 1.56 (m, 1H), 1.34 - 1.14 (m, 14H).
LCMS m/z = 793.8 [M+1]+

**Example 62: Preparation of trifluoroacetate of compound 62**

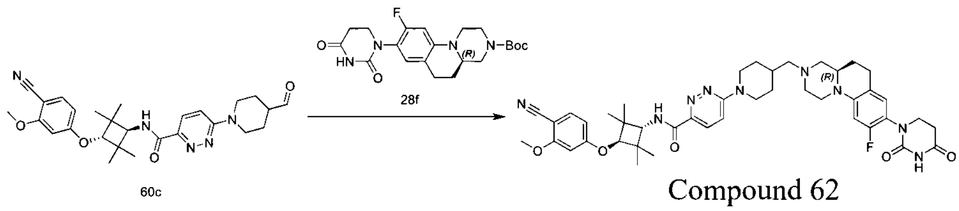


60c Compound 62

Starting from compounds 60c and 28f and following the synthetic method of example 15, the trifluoroacetate of compound 62 was obtained by acidic preparative chromatography (water (containing 0.1% TFA)/acetonitrile) and lyophilisation.

$^1$H NMR (400 MHz, DMSO-$d_6$) δ 10.36 (s, 1H), 8.23 (d, 1H), 7.86 (d, 1H), 7.64 (d, 1H), 7.42 (d, 1H), 7.03 (d, 1H), 6.96 - 6.86 (m, 1H), 6.67 (d, 1H), 6.57 (dd, 1H), 4.61 - 4.47 (m, 2H), 4.40 (s, 1H), 4.14 - 4.08 (m, 1H), 4.04 - 3.98 (m, 1H), 3.91 (s, 3H), 3.68 - 3.58 (m, 4H), 3.43 - 3.33 (m, 1H), 3.19 - 3.00 (m, 6H), 2.95 - 2.82 (m, 1H), 2.81 - 2.61 (m, 4H), 2.31 - 2.17 (m, 1H), 2.08 - 1.97 (m, 1H), 1.95 - 1.80 (m, 2H), 1.72 - 1.57 (m, 1H), 1.32 - 1.12 (m, 14H).
LCMS m/z = 794.6 [M +1]+

**Example 64: Preparation of trifluoroacetate of compound 64**

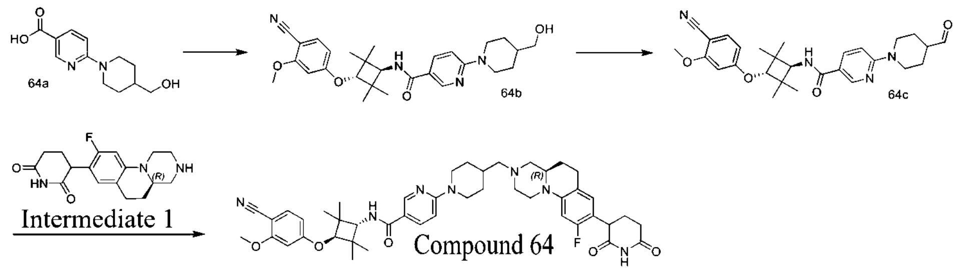


64a 64b 64c Compound 64

Step 1: Preparation of 64b

64a (1.12 g, 4.74 mmol) (see WO 2021249534 A1 for the synthetic method) was dissolved in DMF (30 mL),

trans-4-(3-amino-2,2,4,4-tetramethylcyclobutoxy)-2-methoxybenzonitrile hydrochloride (2.21 g, 7.11 mmol) (see CN 115175901 for the synthetic method), N,N-diisopropylethylamine (3.06 g, 23.70 mmol) and HATU (2.16 g, 5.69 mmol) were respectively added under nitrogen atmosphere, and the mixture was reacted at room temperature for 16 h. Ethyl acetate (50 mL) and water (50 mL) were added to the reaction solution. After phase separation, the aqueous phase was extracted with ethyl acetate (15 mL×3), and the organic phases were combined, washed with a saturated aqueous sodium chloride solution (15 mL), dried over anhydrous sodium sulphate, and concentrated under reduced pressure. The crude product was separated and purified by silica gel column chromatography (petroleum ether : ethyl acetate (v/v) = 1 : 2) to obtain 64b (1.5 g, yield: 64%).
LCMS m/z = 493.2 [M+1] $^+$

Step 2: Preparation of 64c

64b (1.5 g, 3.04 mmol) was dissolved in dichloromethane (30 mL), Dess-Martin periodinane (1.55 g, 3.65 mmol) was added, and the mixture was reacted at room temperature for 2 h. The reaction solution was concentrated under reduced pressure, and the crude product was separated and purified by silica gel column chromatography (petroleum ether : ethyl acetate (v/v) = 1 : 2) to obtain 64c (1.4 g, yield: 93%).
LCMS m/z = 491.2 [M+1] $^+$

Step 3: Preparation of trifluoroacetate of compound 64

Starting from compound 64c and intermediate 1 and following the synthetic method of example 1, the trifluoroacetate of compound 64 was obtained.

$^1$H NMR (400 MHz, DMSO-$d_6$) δ 10.78 (s, 1H), 8.63 (d, 1H), 8.00 (dd, 1H), 7.68 - 7.58 (m, 2H), 6.97 - 6.91 (m, 1H), 6.88 (d, 1H), 6.80 (d, 1H), 6.64 (d, 1H), 6.54 (dd, 1H), 4.48 - 4.37 (m, 2H), 4.26 (s, 1H), 4.13 - 4.02 (m, 2H), 3.91 (s, 3H), 3.89 - 3.82 (m, 1H), 3.68 - 3.57 (m, 2H), 3.40 - 3.30 (m, 1H), 3.19 - 2.81 (m, 7H), 2.81 - 2.58 (m, 3H), 2.57 - 2.45 (m, 1H), 2.27 - 2.07 (m, 2H), 2.05 - 1.79 (m, 4H), 1.70 - 1.57 (m, 1H), 1.27 - 1.10 (m, 14H).
LCMS m/z = 396.8 [M/2 +1]$^+$

Following the synthetic method of other examples, the following compounds were obtained:

| Example No. | Structure | Starting material | LCMS / $^1$H NMR |
|---|---|---|---|
| Example 61 (trifluoroa cetate of compound 61) | 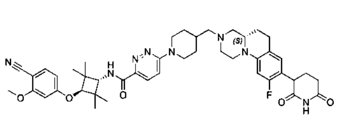 Compound 61 | 60c+intermediate 2 (following the synthetic method of example 1) | $^1$H NMR (400 MHz, DMSO-$d_6$) δ 10.81 - 10.74 (m, 1H), 8.23 (d, 1H), 7.86 (d, 1H), 7.64 (d, 1H), 7.42 (d, 1H), 6.88 (d, 1H), 6.80 (d, 1H), 6.67 (d, 1H), 6.57 (dd, 1H), 4.61 - 4.48 (m, 2H), 4.40 (s, 1H), 4.16 - 3.96 (m, 2H), 3.95 - 3.80 (m, 4H), 3.71 - 3.57 (m, 2H), 3.41 - 3.29 (m, 1H), 3.19 - 2.99 (m, 6H), 2.87 (d, 1H), 2.81 - 2.60 (m, 3H), 2.58 - 2.51 (m, 1H), 2.31 - 2.06 (m, 2H), 2.06 - 1.78 (m, 4H), 1.70 - 1.56 (m, 1H), 1.32 - 1.14 (m, 14H).<br>LCMS m/z = 793.8 [M +1]$^+$ |
| Example 63 (trifluoroa cetate of compound 63) | 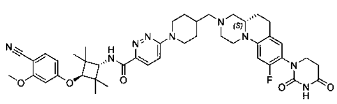 Compound 63 | 60c+29f (following the synthetic method of example 15) | $^1$H NMR (400 MHz, DMSO-$d_6$) δ 10.36 (s, 1H), 8.23 (d, 1H), 7.86 (d, 1H), 7.64 (d, 1H), 7.42 (d, 1H), 7.03 (d, 1H), 6.95 - 6.85 (m, 1H), 6.67 (d, 1H), 6.57 (dd, 1H), 4.61 - 4.48 (m, 2H), 4.40 (s, 1H), 4.16 - 4.07 (m, 1H), 4.04 - 3.98 (m, 1H), 3.91 (s, 3H), 3.71 - 3.55 (m, 4H), 3.45 - 3.32 (m, 1H), 3.22 - 2.97 (m, 6H), 2.95 - 2.82 (m, 1H), 2.81 - 2.60 (m, 4H), 2.32 - 2.18 (m, 1H), 2.08 - 1.98 (m, 1H), 1.96 - 1.81 (m, 2H), 1.72 - 1.55 (m, 1H), 1.32 - 1.14 (m, 14H).<br>LCMS m/z = 794.6 [M +1]+ |

(continued)

| Example No. | Structure | Starting material | LCMS / $^1$H NMR |
|---|---|---|---|
| Example 65 (trifluor-oa cetate of compound 65) | 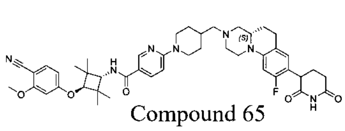 Compound 65 | 64c+intermediat e 2 (following the synthetic method of example 1) | $^1$H NMR (400 MHz, DMSO-$d_6$) δ 10.78 (s, 1H), 8.63 (d, 1H), 8.00 (dd, 1H), 7.68 - 7.57 (m, 2H), 6.99 - 6.90 (m, 1H), 6.88 (d, 1H), 6.80 (d, 1H), 6.64 (d, 1H), 6.54 (dd, 1H), 4.50 - 4.36 (m, 2H), 4.26 (s, 1H), 4.15 - 4.01 (m, 2H), 3.91 (s, 3H), 3.89 - 3.80 (m, 1H), 3.69 - 3.56 (m, 2H), 3.42 - 3.30 (m, 1H), 3.21 - 2.81 (m, 7H), 2.81 - 2.57 (m, 3H), 2.57 - 2.43 (m, 1H), 2.27 - 2.06 (m, 2H), 2.05 - 1.78 (m, 4H), 1.70 - 1.56 (m, 1H), 1.27 - 1.10 (m, 14H). LCMS m/z = 396.8 [M/2 +1]$^+$ |
| Example 66 (trifluoroa cetate of compound 66) | 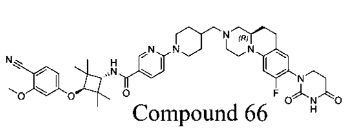 Compound 66 | 64c+28f (follow-ing the synthetic method of exam-ple 15) | $^1$H NMR (400 MHz, $CD_3OD$) δ 8.54 (d, 1H), 8.12 (dd, 1H), 7.52 (d, 1H), 7.10 (d, 1H), 7.04 (d, 1H), 6.85 - 6.77 (m, 1H), 6.63 (d, 1H), 6.55 (dd, 1H), 4.52 - 4.41 (m, 2H), 4.26 (s, 1H), 4.19 - 4.04 (m, 2H), 3.93 (s, 3H), 3.81 - 3.61 (m, 4H), 3.46 - 3.35 (m, 1H), 3.27 - 3.09 (m, 6H), 3.06 - 2.92 (m, 1H), 2.91 - 2.70 (m, 4H), 2.41 - 2.25 (m, 1H), 2.15 - 2.05 (m, 1H), 2.05 - 1.91 (m, 2H), 1.85 - 1.71 (m, 1H), 1.51 - 1.36 (m, 2H), 1.26 (d, 12H). LCMS m/z = 397.3 [M/2+1]$^+$ |
| Example 67 (trifluoroa cetate of compound 67) | 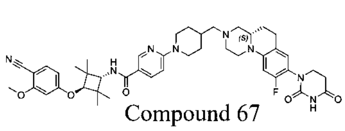 Compound 67 | 64c+29f (follow-ing the synthetic method of exam-ple 15) | $^1$H NMR (400 MHz, DMSO-$d_6$) δ 10.36 (s, 1H), 8.63 (d, 1H), 7.99 (dd, 1H), 7.68 - 7.56 (m, 2H), 7.02 (d, 1H), 6.97 - 6.84 (m, 2H), 6.64 (d, 1H), 6.54 (dd, 1H), 4.52 - 4.35 (m, 2H), 4.26 (s, 1H), 4.17 - 4.02 (m, 2H), 3.91 (s, 3H), 3.71 - 3.51 (m, 4H), 3.47 - 3.30 (m, 1H), 3.18 - 3.03 (m, 4H), 3.03 - 2.92 (m, 2H), 2.92 - 2.81 (m, 1H), 2.81 - 2.60 (m, 4H), 2.31 - 2.10 (m, 1H), 2.09 - 1.96 (m, 1H), 1.94 - 1.73 (m, 2H), 1.72 - 1.56 (m, 1H), 1.29 - 1.08 (m, 14H). LCMS m/z = 397.3 [M/2+1]$^+$ |
| Example 68 (trifluoroa cetate of compound 68) | 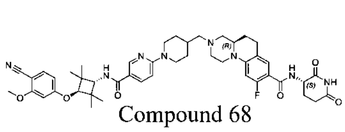 Compound 68 | 64c+trifluoroacet ate of 5A (following the synthetic method of exam-ple 5) | $^1$H NMR (400 MHz, DMSO-$d_6$) δ 10.83 (s, 1H), 8.63 (d, 1H), 8.09 - 7.95 (m, 2H), 7.69 - 7.58 (m, 2H), 7.43 (d, 1H), 6.94 (d, 1H), 6.86 (d, 1H), 6.64 (d, 1H), 6.54 (dd, 1H), 4.78 - 4.67 (m, 1H), 4.50 - 4.39 (m, 2H), 4.26 (s, 1H), 4.24 - 4.14 (m, 1H), 4.10 - 4.03 (m, 1H), 3.91 (s, 3H), 3.71 - 3.58 (m, 2H), 3.55 - 3.45 (m, 1H), 3.26 - 3.15 (m, 1H), 3.15 - 3.03 (m, 3H), 3.02 - 2.85 (m, 3H), 2.83 - 2.70 (m, 3H), 2.58 - 2.49 (m, 1H), 2.25 - 1.95 (m, 4H), 1.94 - 1.76 (m, 2H), 1.71 - 1.57 (m, 1H), 1.32 - 1.07 (m, 14H). LCMS m/z = 835.8 [M+1]$^+$ |

(continued)

| Example No. | Structure | Starting material | LCMS / $^1$H NMR |
|---|---|---|---|
| Example 69 (trifluor-oa cetate of compound 69) | 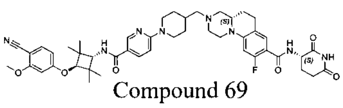 Compound 69 | 64c+7A (following the synthetic method of example 5) | $^1$H NMR (400 MHz, DMSO-$d_6$) δ 10.83 (s, 1H), 8.68 - 8.53 (m, 1H), 8.09 - 7.92 (m, 2H), 7.69 - 7.53 (m, 2H), 7.43 (d, 1H), 6.99 - 6.79 (m, 2H), 6.69 - 6.59 (m, 1H), 6.54 (dd, 1H), 4.80 - 4.67 (m, 1H), 4.50 - 4.36 (m, 2H), 4.30 - 4.11 (m, 2H), 4.10 - 4.00 (m, 1H), 3.91 (s, 3H), 3.71 - 3.55 (m, 2H), 3.55 - 3.39 (m, 1H), 3.26 - 3.15 (m, 1H), 3.15 - 3.03 (m, 3H), 3.02 - 2.85 (m, 3H), 2.83 - 2.70 (m, 3H), 2.58 - 2.49 (m, 1H), 2.28 - 1.95 (m, 4H), 1.94 - 1.73 (m, 2H), 1.71 - 1.57 (m, 1H), 1.32 - 1.07 (m, 14H). LCMS m/z = 835.5 [M+1]$^+$ |
| Example 70 (trifluoroa cetate of compound 70) | 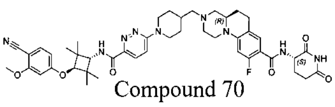 Compound 70 | 60c+trifluoroacetate of 5A (following the synthetic method of example 5) | $^1$H NMR (400 MHz, DMSO-$d_6$) δ 10.83 (s, 1H), 8.24 (d, 1H), 8.09 - 8.00 (m, 1H), 7.86 (d, 1H), 7.64 (d, 1H), 7.48 - 7.37 (m, 2H), 6.87 (d, 1H), 6.67 (d, 1H), 6.57 (dd, 1H), 4.80 - 4.67 (m, 1H), 4.61 - 4.47 (m, 2H), 4.40 (s, 1H), 4.25 - 4.15 (m, 1H), 4.01 (d, 1H), 3.92 (s, 3H), 3.76 - 3.41 (m, 3H), 3.26 - 3.16 (m, 1H), 3.16 - 3.01 (m, 5H), 2.95 - 2.84 (m, 1H), 2.83 - 2.67 (m, 3H), 2.59 - 2.51 (m, 1H), 2.33 - 2.19 (m, 1H), 2.18 - 1.96 (m, 3H), 1.96 - 1.81 (m, 2H), 1.72 - 1.58 (m, 1H), 1.33 - 1.12 (m, 14H). LCMS m/z = 836.6 [M+1]$^+$ |
| Example 71 (trifluoroa cetate of compound 71) | 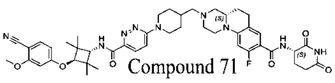 Compound 71 | 60c+7A (following the synthetic method of example 5) | $^1$H NMR (400 MHz, DMSO-$d_6$) δ 10.82 (s, 1H), 8.23 (d, 1H), 8.09 - 7.98 (m, 1H), 7.86 (d, 1H), 7.64 (d, 1H), 7.50 - 7.36 (m, 2H), 6.86 (d, 1H), 6.67 (d, 1H), 6.57 (dd, 1H), 4.79 - 4.67 (m, 1H), 4.61 - 4.47 (m, 2H), 4.40 (s, 1H), 4.27 - 4.15 (m, 1H), 4.01 (d, 1H), 3.92 (s, 3H), 3.76 - 3.41 (m, 3H), 3.26 - 3.16 (m, 1H), 3.16 - 3.00 (m, 5H), 2.97 - 2.84 (m, 1H), 2.83 - 2.67 (m, 3H), 2.59 - 2.51 (m, 1H), 2.33 - 2.19 (m, 1H), 2.18 - 1.96 (m, 3H), 1.96 - 1.80 (m, 2H), 1.72 - 1.56 (m, 1H), 1.34 - 1.12 (m, 14H). LCMS m/z = 836.6 [M+1]$^+$ |

**Example 72: Preparation of compound 72**

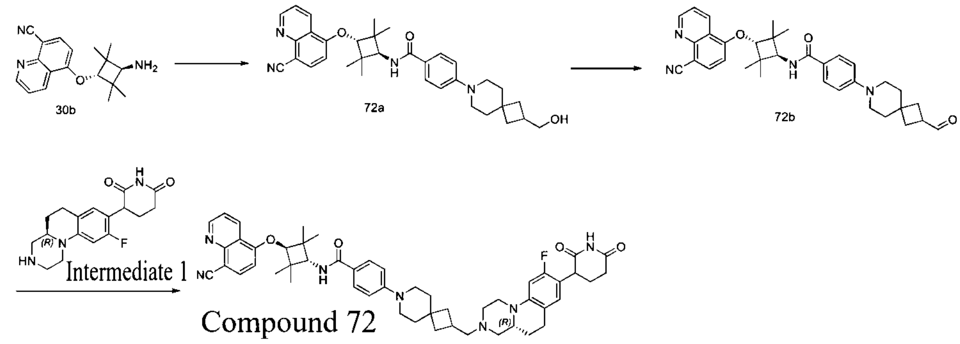

30b
72a
72b
Compound 72

Step 1: Preparation of 72a

To a reaction flask were respectively added 5d (1.00 g, 3.63 mmol), HATU (2.07 g, 5.45 mmol), diisopropylethylamine (1.41 g, 10.89 mmol) and DMF (8 mL), and the mixture was stirred at room temperature for 0.5 h. The above-mentioned trifluoroacetate of crude 30b (1.0 g) was then added, and the resulting mixture was stirred at room temperature for 2 h. Water (100 mL) was added to the reaction solution, and the mixture was filtered. The filter cake was separated and purified by silica gel column chromatography (petroleum ether/ethyl acetate (v/v) = 1 : 0-0 : 1) to obtain 72a (0.97 g, yield: 48%).
LCMS m/z = 553.4 $[M+1]^+$

Step 2: Preparation of 72b

To a reaction flask were respectively added 72a (0.97 g, 1.76 mmol), Dess-Martin periodinane (1.49 g, 3.52 mmol) and dichloromethane (20 mL), and the mixture was reacted at room temperature for 30 min. The reaction system was filtered through a pad of diatomaceous earth, and the filtrate was concentrated under reduced pressure to obtain crude 72b (1.50 g).
LCMS m/z = 551.3 $[M+1]^+$

Step 3: Preparation of compound 72

To a reaction flask were respectively added the above-mentioned crude 72b (0.10 g), the above-mentioned crude intermediate 1 (0.15 g), glacial acetic acid (0.2 mL) and DMAc (5 mL), and the mixture was reacted at room temperature for 1 h. Sodium triacetoxyborohydride (0.25 g, 1.17 mmol) was then added, and the resulting mixture was reacted at room temperature for 16 h. The reaction solution was subjected to Pre-HPLC (instrument and preparative column: using SHIMADZU LC-20AP preparative liquid phase chromatographic instrument; preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 10 mmol/L $NH_4HCO_3$)/acetonitrile. Gradient elution method: gradient elution with acetonitrile from 53% to 83% (elution time: 15 min), lyophilisation was performed to obtain compound 72 (27.0 mg, two-step yield from compound 72a: 27%).

$^1H$ NMR (400 MHz, DMSO-$d_6$) δ 10.76 (s, 1H), 9.10 (dd, 1H), 8.74 (dd, 1H), 8.30 (d, 1H), 7.80 - 7.68 (m, 3H), 7.54 (d, 1H), 7.01 - 6.90 (m, 3H), 6.78 (d, 1H), 6.61 (d, 1H), 4.49 (s, 1H), 4.15 (d, 1H), 3.81 (dd, 1H), 3.74 - 3.63 (m, 1H), 3.30 - 3.24 (m, 4H), 3.22 - 3.14 (m, 2H), 2.98 - 2.78 (m, 3H), 2.78 - 2.46 (m, 5H), 2.44 - 2.35 (m, 2H), 2.19 - 1.72 (m, 7H), 1.72 - 1.63 (m, 2H), 1.63 - 1.51 (m, 2H), 1.50 - 1.41 (m, 2H), 1.26 (d, 12H).
LCMS m/z = 426.8 $[M/2 +1]^+$

**Example 78: Preparation of compound 78**

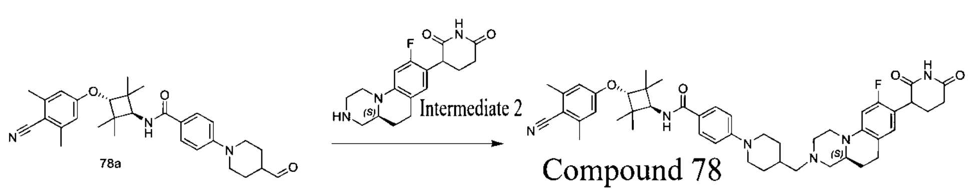

78a
Compound 78

Starting from compound 78a (see WO 2021127443 for the synthetic method) and intermediate 2 and following the synthetic method of example 1, compound 78 was obtained by neutral preparative chromatography (ammonium bicarbonate in water (10 mmol/L)/acetonitrile) and lyophilisation.

$^1$H NMR (400 MHz, DMSO-$d_6$) δ 10.75 (s, 1H), 7.73 (d, 2H), 7.44 (d, 1H), 6.95 (d, 2H), 6.79 (d, 1H), 6.73 (s, 2H), 6.62 (d, 1H), 4.22 (s, 1H), 4.03 (d, 1H), 3.90 - 3.76 (m, 3H), 3.76 - 3.67 (m, 1H), 3.00 - 2.85 (m, 3H), 2.84 - 2.64 (m, 5H), 2.64 - 2.54 (m, 1H), 2.54 - 2.46 (m, 1H), 2.43 (s, 6H), 2.25 - 2.00 (m, 4H), 1.99 - 1.70 (m, 6H), 1.67 - 1.52 (m, 1H), 1.26 - 1.07 (m, 14H).

LCMS m/z = 789.4 [M+1]$^+$

| Example No. | Structure | Starting material | LCMS / $^1$H NMR |
|---|---|---|---|
| Example 73 (compound 73) | 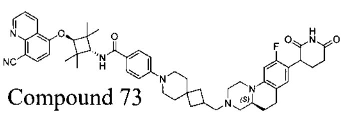 Compound 73 | 72b+intermediate 2 (following the synthetic method of example 72) | $^1$H NMR (400 MHz, DMSO-$d_6$) δ 10.76 (s, 1H), 9.10 (dd, 1H), 8.74 (dd, 1H), 8.30 (d, 1H), 7.81 - 7.66 (m, 3H), 7.54 (d, 1H), 7.03 - 6.91 (m, 3H), 6.78 (d, 1H), 6.61 (d, 1H), 4.49 (s, 1H), 4.15 (d, 1H), 3.81 (dd, 1H), 3.75 - 3.62 (m, 1H), 3.32 - 3.22 (m, 4H), 3.22 - 3.12 (m, 2H), 2.99 - 2.78 (m, 3H), 2.78 - 2.45 (m, 5H), 2.44 - 2.33 (m, 2H), 2.22 - 1.72 (m, 7H), 1.72 - 1.63 (m, 2H), 1.63 - 1.50 (m, 2H), 1.50 - 1.40 (m, 2H), 1.26 (d, 12H).<br>LCMS m/z = 852.4 [M+1]$^+$ |
| Example 74 (compound 74) | 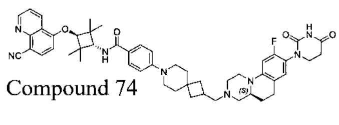 Compound 74 | 72b+29f (following the synthetic method of example 15) | $^1$H NMR (400 MHz, DMSO-$d_6$) δ 10.32 (s, 1H), 9.10 (dd, 1H), 8.74 (dd, 1H), 8.30 (d, 1H), 7.80 - 7.70 (m, 3H), 7.53 (d, 1H), 7.01 - 6.86 (m, 4H), 6.71 (d, 1H), 4.49 (s, 1H), 4.15 (d, 1H), 3.78 - 3.65 (m, 1H), 3.59 (t, 2H), 3.30 - 3.23 (m, 2H), 3.22 - 3.14 (m, 2H), 3.01 - 2.91 (m, 1H), 2.90 - 2.79 (m, 2H), 2.79 - 2.55 (m, 5H), 2.55 - 2.45 (m, 1H), 2.44 - 2.34 (m, 2H), 2.11 - 1.92 (m, 3H), 1.92 - 1.83 (m, 1H), 1.83 - 1.73 (m, 1H), 1.72 - 1.64 (m, 2H), 1.64 - 1.51 (m, 3H), 1.51 - 1.41 (m, 2H), 1.26 (d, 12H).<br>LCMS m/z = 853.4 [M+1]$^+$ |
| Example 75 (compound 75) | 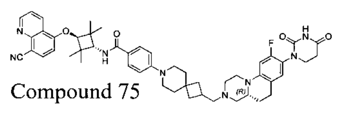 Compound 75 | 72b+28f (following the synthetic method of example 15) | $^1$H NMR (400 MHz, DMSO-$d_6$) δ 10.32 (s, 1H), 9.13 - 9.05 (m, 1H), 8.78 - 8.69 (m, 1H), 8.30 (d, 1H), 7.80 - 7.67 (m, 3H), 7.53 (d, 1H), 7.01 - 6.85 (m, 4H), 6.71 (d, 1H), 4.49 (s, 1H), 4.15 (d, 1H), 3.78 - 3.65 (m, 1H), 3.59 (t, 2H), 3.35 - 3.22 (m, 2H), 3.22 - 3.13 (m, 2H), 3.01 - 2.91 (m, 1H), 2.90 - 2.79 (m, 2H), 2.79 - 2.55 (m, 5H), 2.55 - 2.45 (m, 1H), 2.44 - 2.33 (m, 2H), 2.13 - 1.92 (m, 3H), 1.92 - 1.83 (m, 1H), 1.83 - 1.72 (m, 1H), 1.72 - 1.64 (m, 2H), 1.64 - 1.51 (m, 3H), 1.51 - 1.40 (m, 2H), 1.26 (d, 12H).<br>LCMS m/z = 853.5 [M+1]$^+$ |

(continued)

| Example No. | Structure | Starting material | LCMS / $^1$H NMR |
|---|---|---|---|
| Example 76 (compound 76) | 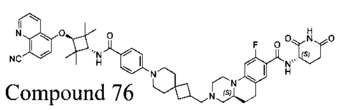 Compound 76 | 72b+7A (following the synthetic method of example 5) | $^1$H NMR (400 MHz, DMSO-$d_6$) $\delta$ 10.82 (s, 1H), 9.10 (dd, 1H), 8.74 (dd, 1H), 8.30 (d, 1H), 7.93 (t, 1H), 7.80 - 7.69 (m, 3H), 7.53 (d, 1H), 7.35 (d, 1H), 7.01 - 6.91 (m, 3H), 6.67 (d, 1H), 4.77 - 4.66 (m, 1H), 4.49 (s, 1H), 4.15 (d, 1H), 3.89 - 3.77 (m, 1H), 3.32 - 3.23 (m, 2H), 3.22 - 3.15 (m, 2H), 3.13 - 3.03 (m, 1H), 2.91 - 2.64 (m, 6H), 2.57 - 2.48 (m, 2H), 2.44 - 2.36 (m, 2H), 2.17 - 1.86 (m, 6H), 1.84 - 1.73 (m, 1H), 1.71 - 1.64 (m, 2H), 1.63 - 1.51 (m, 3H), 1.51 - 1.41 (m, 2H), 1.26 (d, 12H). LCMS m/z = 895.4 [M+1]$^+$ |
| Example 77 (compound 77) | 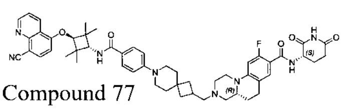 Compound 77 | 72b+trifluoroacetate of 5A (following the synthetic method of example 5) | $^1$H NMR (400 MHz, DMSO-$d_6$) $\delta$ 11.06 - 10.44 (m, 1H), 9.10 (dd, 1H), 8.74 (dd, 1H), 8.30 (d, 1H), 7.93 (t, 1H), 7.81 - 7.69 (m, 3H), 7.52 (d, 1H), 7.36 (d, 1H), 7.01 - 6.91 (m, 3H), 6.67 (d, 1H), 4.73 - 4.60 (m, 1H), 4.49 (s, 1H), 4.19 - 4.11 (m, 1H), 3.89 - 3.77 (m, 1H), 3.35 - 3.23 (m, 2H), 3.22 - 3.14 (m, 2H), 3.13 - 3.03 (m, 1H), 2.91 - 2.64 (m, 6H), 2.57 - 2.46 (m, 2H), 2.44 - 2.36 (m, 2H), 2.17 - 1.86 (m, 6H), 1.84 - 1.73 (m, 1H), 1.71 - 1.64 (m, 2H), 1.63 - 1.51 (m, 3H), 1.51 - 1.41 (m, 2H), 1.26 (d, 12H). LCMS m/z = 895.4 [M+1]$^+$ |
| Example 79 (compound 79) | 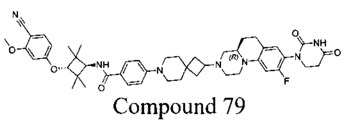 Compound 79 | 16f+28f (following the synthetic method of example 15) | $^1$H NMR (400 MHz, DMSO-$d_6$) $\delta$ 10.32 (s, 1H), 7.73 (d, 2H), 7.64 (d, 1H), 7.46 (d, 1H), 7.00 - 6.88 (m, 3H), 6.71 (d, 1H), 6.64 (d, 1H), 6.54 (dd, 1H), 4.30 - 4.22 (m, 1H), 4.05 (d, 1H), 3.91 (s, 3H), 3.81 - 3.71 (m, 1H), 3.59 (t, 2H), 3.29 - 3.24 (m, 2H), 3.23 - 3.15 (m, 2H), 3.02 - 2.92 (m, 1H), 2.91 - 2.80 (m, 2H), 2.79 - 2.54 (m, 6H), 2.06 - 1.95 (m, 2H), 1.94 - 1.80 (m, 2H), 1.70 - 1.51 (m, 8H), 1.18 (d, 12H). LCMS m/z = 818.3 [M+1]$^+$ |
| Example 80 (compound 80) | 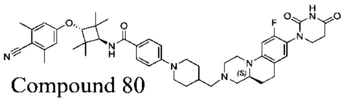 Compound 80 | 78a+29f (following the synthetic method of example 15) | $^1$H NMR (400 MHz, DMSO-$d_6$) $\delta$ 10.32 (s, 1H), 7.78 - 7.68 (m, 2H), 7.44 (d, 1H), 6.99 - 6.88 (m, 3H), 6.76 - 6.66 (m, 3H), 4.22 (s, 1H), 4.03 (d, 1H), 3.89 - 3.80 (m, 2H), 3.79 - 3.71 (m, 1H), 3.60 (t, 2H), 3.04 - 2.95 (m, 1H), 2.94 - 2.86 (m, 2H), 2.83 - 2.55 (m, 7H), 2.43 (s, 6H), 2.24 - 2.12 (m, 2H), 2.11 - 1.99 (m, 1H), 1.95 - 1.71 (m, 5H), 1.67 - 1.53 (m, 1H), 1.25 - 1.07 (m, 14H). LCMS m/z = 790.4 [M+1]$^+$ |

(continued)

| Example No. | Structure | Starting material | LCMS / $^1$H NMR |
|---|---|---|---|
| Example 81 (compound 81) | 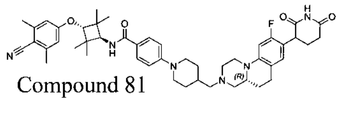 Compound 81 | 78a+intermediate 1 (following the synthetic method of example 1) | $^1$H NMR (400 MHz, DMSO-$d_6$) $\delta$ 10.76 (s, 1H), 7.77 - 7.70 (m, 2H), 7.45 (d, 1H), 6.99 - 6.91 (m, 2H), 6.79 (d, 1H), 6.73 (s, 2H), 6.62 (d, 1H), 4.22 (s, 1H), 4.03 (d, 1H), 3.89 - 3.77 (m, 3H), 3.77 - 3.68 (m, 1H), 3.01 - 2.85 (m, 3H), 2.84 - 2.55 (m, 6H), 2.54 - 2.51 (m, 1H), 2.43 (s, 6H), 2.22 - 2.00 (m, 4H), 1.99 - 1.71 (m, 6H), 1.66 - 1.51 (m, 1H), 1.30 - 1.07 (m, 14H). LCMS m/z = 789.6 [M+1]$^+$ |
| Example 82 (compound 82) | 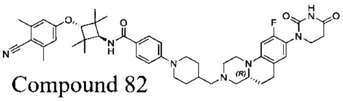 Compound 82 | 78a+28f (following the synthetic method of example 15) | $^1$H NMR (400 MHz, DMSO-$d_6$) $\delta$ 10.33 (s, 1H), 7.78 - 7.67 (m, 2H), 7.45 (d, 1H), 6.99 - 6.88 (m, 3H), 6.76 - 6.66 (m, 3H), 4.22 (s, 1H), 4.03 (d, 1H), 3.89 - 3.79 (m, 2H), 3.79 - 3.70 (m, 1H), 3.60 (t, 2H), 3.04 - 2.94 (m, 1H), 2.94 - 2.85 (m, 2H), 2.83 - 2.54 (m, 7H), 2.43 (s, 6H), 2.24 - 2.11 (m, 2H), 2.11 - 1.98 (m, 1H), 1.96 - 1.71 (m, 5H), 1.67 - 1.52 (m, 1H), 1.27 - 1.05 (m, 14H). LCMS m/z = 790.5 [M+1]$^+$ |

**Example 83: Preparation of compound 83**

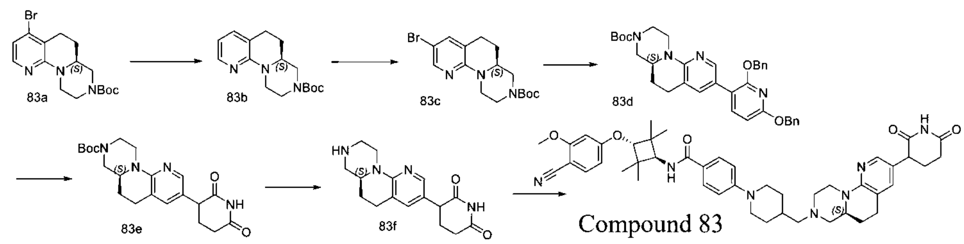

Step 1: Preparation of 83b

83a (3.3 g, 8.96 mmol) (see Tetrahedron Letters, 2018, 59, 2030-2033 for the synthetic method) was dissolved in methanol (20 mL), 10% palladium on carbon (2.38 g) was added, and the mixture was purged three times with hydrogen and reacted at 45°C under hydrogen atmosphere for 20 h. The reaction system was cooled to room temperature, and filtered. The filtrate was collected, and concentrated under reduced pressure to obtain crude 83b (2.55 g).
LCMS m/z = 290.2 [M+1]$^+$

Step 2: Preparation of 83c

The above-mentioned crude 83b (2.55 g) was dissolved in acetonitrile (20 ml), NBS (1.54 g, 8.64 mmol) was added, and the mixture was stirred at room temperature for 1 h. The reaction system was concentrated under reduced pressure, and the crude product was separated and purified by silica gel column chromatography (ethyl acetate : petroleum ether (v/v) = 0 : 1-1 : 9) to afford 83c (3.1 g, yield: 98%).

Step 3: Preparation of 83d

To a reaction flask were respectively added 83c (1.00 g, 2.72 mmol), 2,6-bisbenzyloxypyridine-3-boronic acid pinacol ester (1.70 g, 4.08 mmol), [1,1'-bis(diphenylphosphino)ferrocene]palladium dichloride-dichloromethane complex

(0.22 g, 0.27 mmol) and caesium carbonate (1.77 g, 5.44 mmol), and the mixture was purged three times with nitrogen. 1,4-Dioxane (30 mL) and water (3 mL) were added, and the mixture was reacted at 100°C under nitrogen atmosphere for 20 h. The reaction system was cooled to room temperature, and water (50 mL) and ethyl acetate (50 mL) were added. After phase separation, the aqueous phase was extracted with ethyl acetate (50 mL), and the organic phases were combined, washed with a saturated aqueous sodium chloride solution (20 mL), dried over anhydrous sodium sulphate, and filtered. The filtrate was concentrated under reduced pressure, and the crude product was separated and purified by silica gel column chromatography (petroleum ether/ethyl acetate (v/v) = 1 : 0-9 : 1) to obtain 83d (1.10 g, yield: 70%).
LCMS m/z = 579.3 $[M+1]^+$

Step 4: Preparation of 83e

83d (1.10g, 1.90 mmol) was dissolved in tetrahydrofuran (20 mL), 10% palladium on carbon (1.0 g) was added, and the mixture was purged three times with hydrogen and reacted at 45°C under hydrogen atmosphere for 20 h. The reaction system was cooled to room temperature, and filtered through a pad of diatomaceous earth. The filtrate was collected, and the filter cake was washed with tetrahydrofuran (30 mL), and dried under reduced pressure to obtain 83e (0.70 g, yield: 92%).
LCMS m/z = 401.2 $[M+1]^+$

Step 5: Preparation of 83f

83e (200 mg, 0.50 mmol) was dissolved in dichloromethane (2 mL), trifluoroacetic acid (2 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude 83f (150 mg).
LCMS m/z = 301.3 $[M+1]^+$

Step 6: Preparation of compound 83

Starting from crude 83f and 1a and following the synthetic method of example 1, compound 83 was obtained by neutral preparative chromatography (ammonium bicarbonate in water (10 mmol/L)/acetonitrile) and lyophilisation.

$^1H$ NMR (400 MHz, DMSO-$d_6$) δ 10.76 (s, 1H), 7.80 - 7.69 (m, 3H), 7.64 (d, 1H), 7.46 (d, 1H), 7.11 - 7.04 (m, 1H), 7.00 - 6.90 (m, 2H), 6.64 (d, 1H), 6.54 (dd, 1H), 4.65 - 4.49 (m, 1H), 4.27 (s, 1H), 4.05 (d, 1H), 3.93 - 3.79 (m, 5H), 3.68 (dd, 1H), 3.27 - 3.17 (m, 1H), 2.99 - 2.86 (m, 2H), 2.85 - 2.56 (m, 6H), 2.56 - 2.50 (m, 1H), 2.25 - 2.08 (m, 3H), 2.02 - 1.87 (m, 3H), 1.87 - 1.70 (m, 4H), 1.66 - 1.52 (m, 1H), 1.29 - 1.08 (m, 14H).
LCMS m/z = 774.5 $[M+1]^+$

**Example 85: Preparation of compound 85**

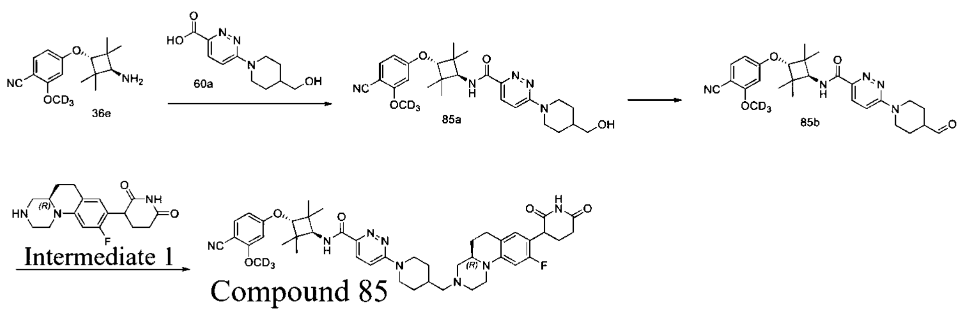


Starting from the hydrochloride of compound 36e and compound 60a and following the synthetic method of example 60, compound 85 was obtained by neutral preparative chromatography (ammonium bicarbonate in water (10 mmol/L)/acetonitrile) and lyophilisation.

$^1H$ NMR (400 MHz, DMSO-$d_6$) δ 10.76 (s, 1H), 8.24 (d, 1H), 7.82 (d, 1H), 7.64 (d, 1H), 7.36 (d, 1H), 6.79 (d, 1H), 6.69 - 6.52 (m, 3H), 4.55 - 4.44 (m, 2H), 4.40 (s, 1H), 4.00 (d, 1H), 3.81 (dd, 1H), 3.76 - 3.67 (m, 1H), 3.09 - 2.84 (m, 5H), 2.79 - 2.54 (m, 4H), 2.54 - 2.44 (m, 1H), 2.24 - 2.00 (m, 4H), 1.99 - 1.70 (m, 6H), 1.66 - 1.52 (m, 1H), 1.28 - 1.05 (m, 14H).
LCMS m/z = 796.6 $[M+1]^+$

Following the synthetic method of other examples, the following compounds were obtained:

| Example No. | Structure | Starting material | LCMS / $^1$H NMR |
|---|---|---|---|
| Example 84 (trifluoroacetate of compound 84) | Compound 84 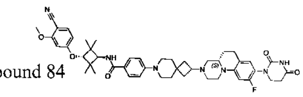 | 16f+29f (following the synthetic method of example 15) | $^1$H NMR (400 MHz, DMSO-$d_6$) δ 10.35 (s, 1H), 7.75 (d, 2H), 7.64 (d, 1H), 7.48 (d, 1H), 7.05 - 6.95 (m, 3H), 6.91 (d, 1H), 6.64 (d, 1H), 6.54 (dd, 1H), 4.28 (s, 1H), 4.22 - 4.11 (m, 1H), 4.08 - 4.01 (m, 1H), 3.91 (s, 3H), 3.85 - 3.72 (m, 1H), 3.61 (t, 2H), 3.54 - 3.43 (m, 2H), 3.35 - 3.17 (m, 5H), 3.08 - 2.97 (m, 1H), 2.96 - 2.83 (m, 1H), 2.81 - 2.60 (m, 5H), 2.30 - 2.17 (m, 2H), 2.12 - 1.99 (m, 3H), 1.74 - 1.54 (m, 5H), 1.19 (d, 12H). LCMS m/z = 818.7 [M+1]$^+$ |
| Example 86 (compound 86) | 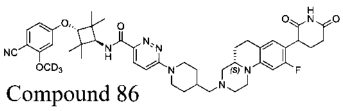 Compound 86 | 85b+intermediate 2 (following the synthetic method of example 60) | $^1$H NMR (400 MHz, DMSO-$d_6$) δ 10.75 (s, 1H), 8.23 (d, 1H), 7.82 (d, 1H), 7.63 (d, 1H), 7.35 (d, 1H), 6.79 (d, 1H), 6.69 - 6.53 (m, 3H), 4.56 - 4.44 (m, 2H), 4.40 (s, 1H), 4.00 (d, 1H), 3.81 (dd, 1H), 3.76 - 3.66 (m, 1H), 3.09 - 2.81 (m, 5H), 2.79 - 2.54 (m, 4H), 2.54 - 2.44 (m, 1H), 2.23 - 2.00 (m, 4H), 1.99 - 1.68 (m, 6H), 1.65 - 1.50 (m, 1H), 1.29 - 1.04 (m, 14H). LCMS m/z = 796.6 [M+1]$^+$ |
| Example 87 (compound 87) | 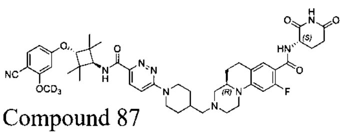 Compound 87 | 85b+trifluoroacetate of 5A (following the synthetic method of example 11) | $^1$H NMR (400 MHz, DMSO-$d_6$) δ 10.81 (s, 1H), 8.24 (d, 1H), 7.94 (t, 1H), 7.82 (d, 1H), 7.64 (d, 1H), 7.42 - 7.31 (m, 2H), 6.74 - 6.64 (m, 2H), 6.57 (dd, 1H), 4.78 - 4.65 (m, 1H), 4.58 - 4.45 (m, 2H), 4.40 (s, 1H), 4.00 (d, 1H), 3.94 - 3.78 (m, 1H), 3.18 - 2.99 (m, 3H), 2.98 - 2.60 (m, 6H), 2.58 - 2.47 (m, 1H), 2.27 - 2.15 (m, 2H), 2.14 - 1.72 (m, 8H), 1.68 - 1.52 (m, 1H), 1.29 - 1.03 (m, 14H). LCMS m/z = 839.6 [M +1]$^+$ |
| Example 88 (compound 88) | 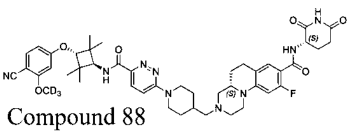 Compound 88 | 85b+7A (following the synthetic method of example 11) | $^1$H NMR (400 MHz, DMSO-$d_6$) δ 10.81 (s, 1H), 8.24 (d, 1H), 7.94 (t, 1H), 7.82 (d, 1H), 7.64 (d, 1H), 7.42 - 7.29 (m, 2H), 6.74 - 6.63 (m, 2H), 6.57 (dd, 1H), 4.78 - 4.66 (m, 1H), 4.58 - 4.44 (m, 2H), 4.40 (s, 1H), 4.00 (d, 1H), 3.93 - 3.79 (m, 1H), 3.19 - 2.98 (m, 3H), 2.98 - 2.60 (m, 6H), 2.58 - 2.47 (m, 1H), 2.28 - 2.15 (m, 2H), 2.14 - 1.72 (m, 8H), 1.68 - 1.53 (m, 1H), 1.29 - 1.02 (m, 14H). LCMS m/z = 839.6 [M +1]$^+$ |

**Example 89: Preparation of compound 89**

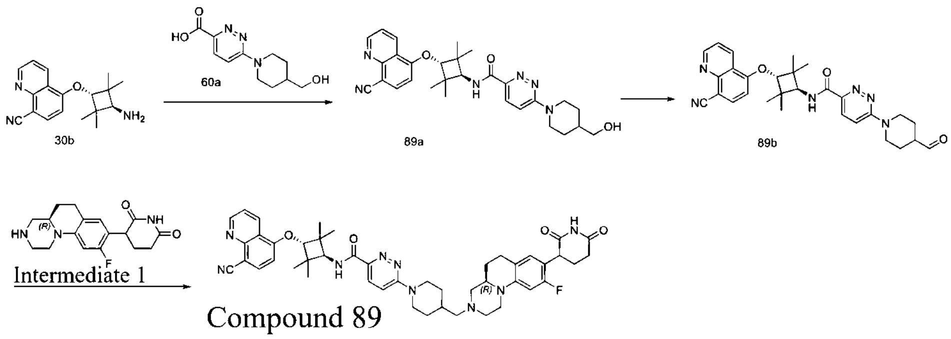

30b 89a 89b Compound 89

Starting from the trifluoroacetate of compound 30b and compound 60a and following the synthetic method of example 60, compound 89 was obtained by neutral preparative chromatography (water (containing 10 mmol/L ammonium bicarbonate)/acetonitrile) and lyophilisation.
$^1$H NMR (400 MHz, DMSO-$d_6$) δ 10.75 (s, 1H), 9.10 (d, 1H), 8.76 (d, 1H), 8.33 - 8.24 (m, 2H), 7.83 (d, 1H), 7.79 - 7.72 (m, 1H), 7.36 (d, 1H), 7.01 (d, 1H), 6.79 (d, 1H), 6.62 (d, 1H), 4.61 (s, 1H), 4.56 - 4.44 (m, 2H), 4.12 (d, 1H), 3.85 - 3.77 (m, 1H), 3.76 - 3.69 (m, 1H), 3.10 - 2.85 (m, 5H), 2.78 - 2.64 (m, 3H), 2.64 - 2.45 (m, 2H), 2.23 - 2.15 (m, 2H), 2.14 - 2.01 (m, 2H), 1.99 - 1.72 (m, 6H), 1.67 - 1.53 (m, 1H), 1.28 (d, 12H), 1.20 - 1.07 (m, 2H).
LCMS m/z = 814.6 [M+1]$^+$

**Example 93: Preparation of compound 93**

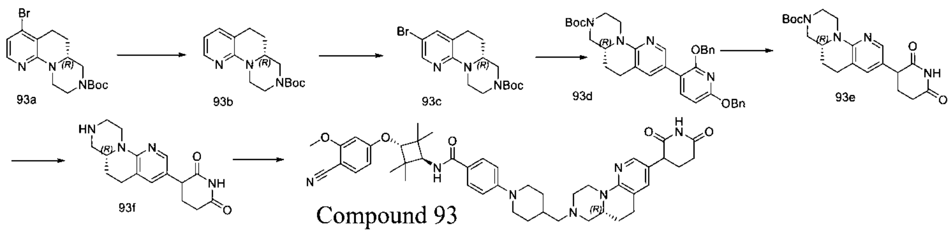
93a 93b 93c 93d 93e 93f Compound 93

Starting from compound 93a (see Tetrahedron Letters, 2018, 59, 2030-2033 for the synthetic method) and following the synthetic method of example 83, compound 93 was obtained.
$^1$H NMR (400 MHz, DMSO-$d_6$) δ 10.76 (s, 1H), 7.80 - 7.67 (m, 3H), 7.64 (d, 1H), 7.46 (d, 1H), 7.11 - 7.03 (m, 1H), 6.95 (d, 2H), 6.64 (d, 1H), 6.54 (dd, 1H), 4.66 - 4.49 (m, 1H), 4.27 (s, 1H), 4.05 (d, 1H), 3.93 - 3.78 (m, 5H), 3.68 (dd, 1H), 3.29 - 3.15 (m, 1H), 2.99 - 2.87 (m, 2H), 2.86 - 2.56 (m, 6H), 2.56 - 2.45 (m, 1H), 2.25 - 2.06 (m, 3H), 2.02 - 1.87 (m, 3H), 1.87 - 1.69 (m, 4H), 1.66 - 1.52 (m, 1H), 1.29 - 1.08 (m, 14H).
Following the synthetic method of other examples, the following compounds were obtained:

| Example No. | Structure | Starting material | LCMS / $^1$H NMR |
|---|---|---|---|
| Example 90 (compound 90) | 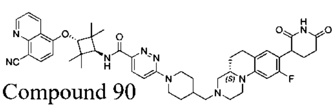 Compound 90 | 89b+intermediate 2 (following the synthetic method of example 60) | $^1$H NMR (400 MHz, DMSO-$d_6$) δ 10.75 (s, 1H), 9.14 - 9.07 (m, 1H), 8.76 (d, 1H), 8.34 - 8.23 (m, 2H), 7.83 (d, 1H), 7.79 - 7.70 (m, 1H), 7.36 (d, 1H), 7.01 (d, 1H), 6.79 (d, 1H), 6.62 (d, 1H), 4.61 (s, 1H), 4.56 - 4.44 (m, 2H), 4.12 (d, 1H), 3.85 - 3.77 (m, 1H), 3.76 - 3.69 (m, 1H), 3.13 - 2.84 (m, 5H), 2.79 - 2.64 (m, 3H), 2.64 - 2.45 (m, 2H), 2.23 -2.01 (m, 4H), 1.99 - 1.71 (m, 6H), 1.67 - 1.53 (m, 1H), 1.28 (d, 12H), 1.20 - 1.05 (m, 2H). LCMS m/z = 814.6 [M+1]$^+$ |

(continued)

| Example No. | Structure | Starting material | LCMS / $^1$H NMR |
|---|---|---|---|
| Example 91 (compound 91) | 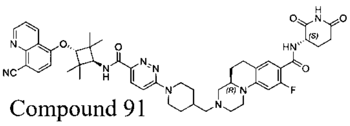 Compound 91 | 89b+trifluoroacetate of 5A (following the synthetic method of example 11) | $^1$H NMR (400 MHz, DMSO-$d_6$) δ 10.81 (s, 1H), 9.15 - 9.06 (m, 1H), 8.76 (d, 1H), 8.33 - 8.24 (m, 2H), 7.93 (t, 1H), 7.83 (d, 1H), 7.76 (dd, 1H), 7.42 - 7.31 (m, 2H), 7.01 (d, 1H), 6.68 (d, 1H), 4.78 - 4.66 (m, 1H), 4.61 (s, 1H), 4.57 - 4.42 (m, 2H), 4.12 (d, 1H), 3.91 - 3.81 (m, 1H), 3.18 - 2.99 (m, 3H), 2.98 - 2.61 (m, 6H), 2.59 - 2.47 (m, 1H), 2.27 - 2.15 (m, 2H), 2.14 - 1.72 (m, 8H), 1.67 - 1.52 (m, 1H), 1.28 (d, 12H), 1.21 - 1.07 (m, 2H). LCMS m/z = 857.4 [M +1]$^+$ |
| Example 92 (compound 92) | 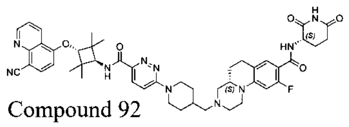 Compound 92 | 89b+7A (following the synthetic method of example 11) | $^1$H NMR (400 MHz, DMSO-$d_6$) δ 10.81 (s, 1H), 9.14 - 9.06 (m, 1H), 8.76 (d, 1H), 8.33 - 8.24 (m, 2H), 7.93 (t, 1H), 7.83 (d, 1H), 7.79 - 7.72 (m, 1H), 7.42 - 7.30 (m, 2H), 7.01 (d, 1H), 6.68 (d, 1H), 4.78 - 4.66 (m, 1H), 4.61 (s, 1H), 4.57 - 4.43 (m, 2H), 4.12 (d, 1H), 3.91 - 3.79 (m, 1H), 3.17 - 2.99 (m, 3H), 2.98 - 2.60 (m, 6H), 2.58 - 2.47 (m, 1H), 2.27 - 2.15 (m, 2H), 2.12 - 1.73 (m, 8H), 1.67 - 1.52 (m, 1H), 1.28 (d, 12H), 1.21 - 1.07 (m, 2H). LCMS m/z = 857.4 [M +1]$^+$ |
| Example 94 (compound 94) | 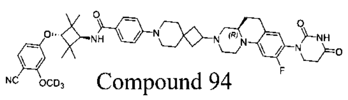 Compound 94 | hydrochloride of 36e +16d+28f (following the synthetic methods of examples 15 and 16) | $^1$H NMR (400 MHz, DMSO-$d_6$) δ 10.32 (s, 1H), 7.73 (d, 2H), 7.64 (d, 1H), 7.47 (d, 1H), 7.01 - 6.88 (m, 3H), 6.72 (d, 1H), 6.66 - 6.61 (m, 1H), 6.53 (dd, 1H), 4.27 (s, 1H), 4.05 (d, 1H), 3.82 - 3.72 (m, 1H), 3.63 - 3.55 (m, 2H), 3.30 - 3.24 (m, 2H), 3.23 - 3.15 (m, 2H), 3.02 - 2.92 (m, 1H), 2.92 - 2.80 (m, 2H), 2.79 - 2.55 (m, 6H), 2.06 - 1.95 (m, 2H), 1.94 - 1.81 (m, 2H), 1.71 - 1.51 (m, 8H), 1.18 (d, 12H). LCMS m/z = 821.8 [M+1]$^+$ |
| Example 95 (compound 95) | 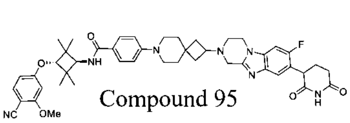 Compound 95 | 16f+intermediate 5 (following the synthetic method of example 20) | $^1$H NMR (400 MHz, DMSO-$d_6$) δ 10.83 (s, 1H), 7.73 (d, 2H), 7.64 (d, 1H), 7.51 - 7.44 (m, 2H), 7.38 (d, 1H), 6.96 (d, 2H), 6.64 (d, 1H), 6.54 (dd, 1H), 4.27 (s, 1H), 4.14 - 4.01 (m, 4H), 3.91 (s, 3H), 3.69 (s, 2H), 3.34 - 3.26 (m, 2H), 3.25 - 3.16 (m, 2H), 3.12 - 3.03 (m, 1H), 2.94 - 2.82 (m, 2H), 2.82 - 2.70 (m, 1H), 2.60 - 2.53 (m, 1H), 2.36 - 2.20 (m, 1H), 2.18 - 1.97 (m, 3H), 1.77 - 1.64 (m, 4H), 1.64 - 1.53 (m, 2H), 1.18 (d, 12H). LCMS m/z = 802.4 [M+1]$^+$ |

(continued)

| Example No. | Structure | Starting material | LCMS / $^1$H NMR |
|---|---|---|---|
| Example 96 (compound 96) | 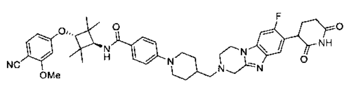 Compound 96 | 1a+intermediate 5 (following the synthetic method of example 1) | $^1$H NMR (400 MHz, DMSO-$d_6$) δ 10.83 (s, 1H), 7.73 (d, 2H), 7.64 (d, 1H), 7.50 - 7.43 (m, 2H), 7.39 (d, 1H), 6.96 (d, 2H), 6.64 (d, 1H), 6.54 (dd, 1H), 4.27 (s, 1H), 4.15 - 3.99 (m, 4H), 3.95 - 3.75 (m, 7H), 3.04 - 2.95 (m, 2H), 2.90 - 2.68 (m, 3H), 2.60 - 2.42 (m, 3H), 2.35 - 2.24 (m, 1H), 2.08 - 1.98 (m, 1H), 1.96 - 1.77 (m, 3H), 1.29 - 1.11 (m, 14H). LCMS m/z = 776.6 [M+1]$^+$ |

**Example 97: Preparation of trifluoroacetate of compound 97**

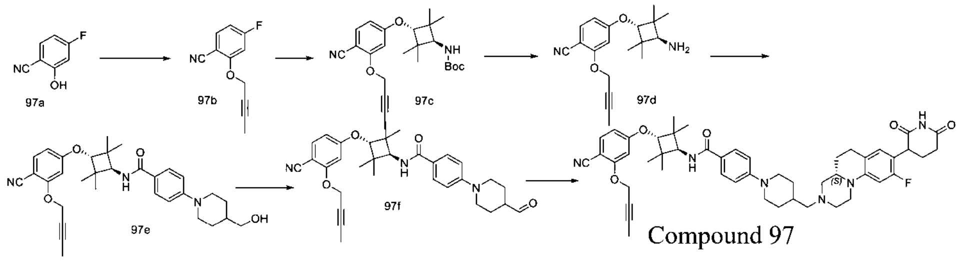

97a 97b 97c 97d 97e 97f Compound 97

Step 1: Preparation of 97b

To a reaction flask were respectively added 97a (5.0 g, 36.47 mmol), 1-bromo-2-butyne (9.7 g, 72.94 mmol), potassium carbonate (10.08 g, 72.94 mmol), potassium iodide (0.61 g, 3.67 mmol) and acetone (100 mL), and the mixture was reacted at room temperature for 12 h. To the reaction system was added water (200 mL), and the mixture was extracted with ethyl acetate (100 mL×3). The organic phases were combined, dried over anhydrous sodium sulphate, and concentrated under reduced pressure. The crude product was separated and purified by silica gel column chromatography (petroleum ether/ethyl acetate (v/v) = 1 : 0-1 : 1) to obtain 97b (5.0 g, yield: 72%).

Starting from compounds 97b and 36h and intermediate 2, and following the synthetic method of example 36, the trifluoroacetate of compound 97 was obtained by acidic preparative chromatography (water (containing 0.1% TFA)/acetonitrile) and lyophilisation.

$^1$H NMR (400 MHz, DMSO-$d_6$) δ 10.78 (s, 1H), 7.76 (d, 2H), 7.64 (d, 1H), 7.46 (d, 1H), 6.99 (d, 2H), 6.88 (d, 1H), 6.80 (d, 1H), 6.69 (d, 1H), 6.60 (dd, 1H), 5.00 - 4.92 (m, 2H), 4.25 (s, 1H), 4.10 - 4.03 (m, 2H), 3.95 - 3.78 (m, 3H), 3.68 - 3.57 (m, 2H), 3.41 - 3.28 (m, 1H), 3.21 - 2.97 (m, 4H), 2.93 - 2.77 (m, 3H), 2.77 - 2.60 (m, 3H), 2.58 - 2.48 (m, 1H), 2.20 - 2.06 (m, 2H), 2.05 - 1.91 (m, 2H), 1.91 - 1.76 (m, 5H), 1.71 - 1.56 (m, 1H), 1.38 - 1.09 (m, 14H).
LCMS m/z = 829.6 [M+1]$^+$

Following the synthetic method of other examples, the following compounds were obtained:

| Example No. | Structure | Starting material | LCMS / $^1$H NMR |
|---|---|---|---|
| Example 98 (trifluoroace tate of compound 98) | 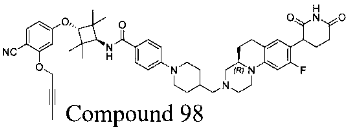 Compound 98 | 97f+intermediate 1 (following the synthetic method of example 97) | $^1$H NMR (400 MHz, DMSO-$d_6$) δ 10.78 (s, 1H), 7.76 (d, 2H), 7.64 (d, 1H), 7.47 (d, 1H), 6.99 (d, 2H), 6.88 (d, 1H), 6.80 (d, 1H), 6.69 (d, 1H), 6.60 (dd, 1H), 5.00 - 4.93 (m, 2H), 4.26 (s, 1H), 4.12 - 4.02 (m, 2H), 3.95 - 3.82 (m, 3H), 3.69 - 3.57 (m, 2H), 3.42 - 3.27 (m, 1H), 3.21 - 2.96 (m, 4H), 2.93 - 2.77 (m, 3H), 2.77 - 2.60 (m, 3H), 2.59 - 2.48 (m, 1H), 2.21 - 2.06 (m, 2H), 2.05 - 1.91 (m, 2H), 1.91 - 1.75 (m, 5H), 1.71 - 1.56 (m, 1H), 1.38 - 1.09 (m, 14H). LCMS m/z = 829.3 [M+1]$^+$ |
| Example 99 (trifluoroace tate of compound 99) | 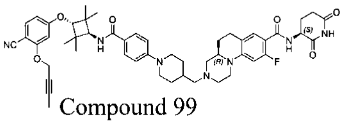 Compound 99 | 97f (following the synthetic method of example 5) | $^1$H NMR (400 MHz, DMSO-$d_6$) δ 10.83 (s, 1H), 8.04 (t, 1H), 7.76 (d, 2H), 7.65 (d, 1H), 7.53 - 7.38 (m, 2H), 6.99 (d, 2H), 6.86 (d, 1H), 6.69 (d, 1H), 6.60 (dd, 1H), 5.04 - 4.88 (m, 2H), 4.80 - 4.66 (m, 1H), 4.30 - 4.14 (m, 2H), 4.10 - 4.02 (m, 1H), 3.96 - 3.83 (m, 2H), 3.73 - 3.56 (m, 2H), 3.56 - 3.41 (m, 1H), 3.28 - 2.97 (m, 4H), 2.96 - 2.65 (m, 6H), 2.58 - 2.51 (m, 1H), 2.20 - 1.95 (m, 4H), 1.95 - 1.76 (m, 5H), 1.72 - 1.57 (m, 1H), 1.38 - 1.08 (m, 14H). LCMS m/z = 872.6 [M+1]$^+$ |
| Example 100 (trifluoroace tate of compound 100) | 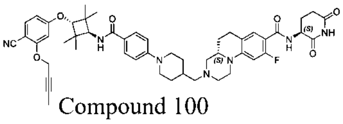 Compound 100 | 97f+4-A (following the synthetic method of example 5) | $^1$H NMR (400 MHz, DMSO-$d_6$) δ 10.82 (s, 1H), 8.08 - 7.98 (m, 1H), 7.76 (d, 2H), 7.64 (d, 1H), 7.52 - 7.37 (m, 2H), 6.99 (d, 2H), 6.86 (d, 1H), 6.69 (d, 1H), 6.60 (dd, 1H), 4.97 (d, 2H), 4.80 - 4.67 (m, 1H), 4.31 - 4.14 (m, 2H), 4.10 - 4.04 (m, 1H), 3.94 - 3.86 (m, 2H), 3.70 - 3.58 (m, 2H), 3.54 - 3.41 (m, 1H), 3.27 - 2.98 (m, 4H), 2.97 - 2.66 (m, 6H), 2.59 - 2.47 (m, 1H), 2.21 - 1.95 (m, 4H), 1.95 - 1.75 (m, 5H), 1.72 - 1.53 (m, 1H), 1.38 - 1.07 (m, 14H). LCMS m/z = 872.6 [M+1]$^+$ |

**Example 101: Preparation of trifluoroacetate of compound 101**

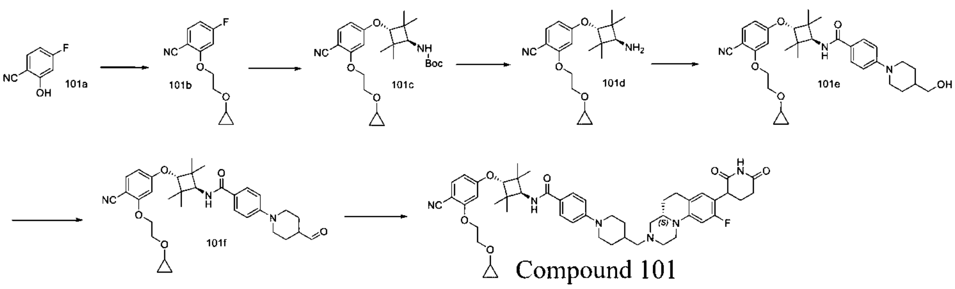
101a
101b
101c
101d
101e
101f
Compound 101

Step 1: Preparation of 101b

101a (10 g, 72.93 mmol), 2-cyclopropoxyethyl 4-methylbenzenesulphonate (22.62 g, 88.25 mmol) and caesium carbonate (35.64 g, 109.38 mmol) were dissolved in DMF (100 mL), and the mixture was reacted at 90°C for 16 h. The reaction system was cooled to room temperature and added to water (300 mL), and the mixture was extracted with ethyl acetate (200 mL×3). The organic phases were combined, dried over anhydrous sodium sulphate, and concentrated under reduced pressure, and the crude product was separated and purified by silica gel column chromatography (petroleum ether : ethyl acetate (v/v) = 1 : 0-1 : 1) to obtain 101b (10 g, yield: 62%).

Starting from compounds 101b and 36h and intermediate 2, and following the synthetic method of example 36, the trifluoroacetate of compound 101 was obtained by acidic preparative chromatography (water (containing 0.1% TFA)/acetonitrile) and lyophilisation.

$^1H$ NMR (400 MHz, DMSO-$d_6$) δ 10.79 (s, 1H), 7.76 (d, 2H), 7.64 (d, 1H), 7.48 (d, 1H), 6.99 (d, 2H), 6.88 (d, 1H), 6.80 (d, 1H), 6.65 (d, 1H), 6.55 (dd, 1H), 4.29 - 4.20 (m, 3H), 4.13 - 3.99 (m, 2H), 3.95 - 3.74 (m, 5H), 3.70 - 3.56 (m, 2H), 3.46 - 3.38 (m, 1H), 3.38 - 3.28 (m, 1H), 3.24 - 2.98 (m, 4H), 2.93 - 2.59 (m, 6H), 2.56 - 2.46 (m, 1H), 2.21 - 2.05 (m, 2H), 2.05 - 1.75 (m, 4H), 1.70 - 1.55 (m, 1H), 1.37 - 1.08 (m, 14H), 0.55 - 0.38 (m, 4H).
LCMS m/z = 861.6 $[M+1]^+$

**Example 105: Preparation of compound 105**

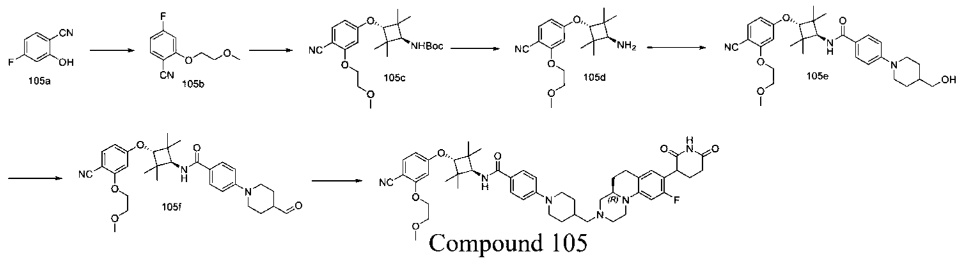

Compound 105

Step 1: Preparation of 105b

To a reaction flask were respectively added 105a(1.6 g, 11.67 mmol), 1-bromo-2-methoxyethane (3.80 g, 15.18 mmol), potassium carbonate (4.84 g, 35.02 mmol), potassium iodide (0.19 g, 1.14 mmol) and DMF (15 mL), and the mixture was reacted at room temperature for 12 h. To the reaction system was added water (100 mL), and the mixture was extracted with ethyl acetate (100 mL×3). The organic phases were combined, dried over anhydrous sodium sulphate, and concentrated under reduced pressure. The crude product was separated and purified by silica gel column chromatography (petroleum ether/ethyl acetate (v/v) = 1 : 0-3 : 1) to obtain 105b (1.1 g, yield: 48%).
LCMS m/z = 196.1 $[M+1]^+$

Starting from compounds 105b and 36h and following the synthetic method of example 36, compound 105 was obtained.

$^1H$ NMR (400 MHz, DMSO-$d_6$) δ 10.76 (s, 1H), 7.73 (d, 2H), 7.64 (d, 1H), 7.46 (d, 1H), 6.95 (d, 2H), 6.79 (d, 1H), 6.70 - 6.58 (m, 2H), 6.54 (dd, 1H), 4.30 - 4.21 (m, 3H), 4.04 (d, 1H), 3.90 - 3.77 (m, 3H), 3.76 - 3.66 (m, 3H), 3.34 (s, 3H), 3.00 - 2.85 (m, 3H), 2.83 - 2.64 (m, 5H), 2.64 - 2.54 (m, 1H), 2.54 - 2.46 (m, 1H), 2.23 - 1.99 (m, 4H), 1.99 - 1.72 (m, 6H), 1.66 - 1.52 (m, 1H), 1.27 - 1.09 (m, 14H).
LCMS m/z = 835.3 $[M+1]^+$

Following the synthetic method of other examples, the following compounds were obtained:

| Example No. | Structure | Starting material | LCMS / $^1$H NMR |
|---|---|---|---|
| Example 102 (trifluoroacetate of compound 102) | 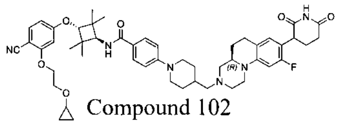 Compound 102 | 101f+intermediate 1 (following the synthetic method of example 101) | $^1$H NMR (400 MHz, DMSO-$d_6$) δ 10.78 (s, 1H), 7.76 (d, 2H), 7.64 (d, 1H), 7.48 (d, 1H), 6.99 (d, 2H), 6.88 (d, 1H), 6.80 (d, 1H), 6.65 (d, 1H), 6.55 (dd, 1H), 4.31 - 4.20 (m, 3H), 4.13 - 4.01 (m, 2H), 3.95 - 3.72 (m, 5H), 3.70 - 3.56 (m, 2H), 3.46 - 3.38 (m, 1H), 3.38 - 3.27 (m, 1H), 3.24 - 2.98 (m, 4H), 2.93 - 2.59 (m, 6H), 2.57 - 2.47 (m, 1H), 2.21 - 2.05 (m, 2H), 2.05 - 1.75 (m, 4H), 1.71 - 1.55 (m, 1H), 1.39 - 1.08 (m, 14H), 0.56 - 0.41 (m, 4H). LCMS m/z = 861.3 [M+1]$^+$ |
| Example 103 (trifluoroacetate of compound 103) | 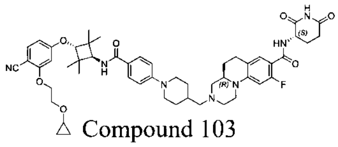 Compound 103 | 101f (following the synthetic method of example 5) | $^1$H NMR (400 MHz, DMSO-$d_6$) δ 10.82 (s, 1H), 8.08 - 8.00 (m, 1H), 7.76 (d, 2H), 7.64 (d, 1H), 7.53 - 7.38 (m, 2H), 6.99 (d, 2H), 6.86 (d, 1H), 6.65 (d, 1H), 6.55 (dd, 1H), 4.78 - 4.67 (m, 1H), 4.30 - 4.15 (m, 4H), 4.09 - 4.02 (m, 1H), 3.95 - 3.85 (m, 2H), 3.84 - 3.76 (m, 2H), 3.72 - 3.57 (m, 2H), 3.55 - 3.45 (m, 1H), 3.45 - 3.38 (m, 1H), 3.31 - 2.96 (m, 4H), 2.96 - 2.64 (m, 6H), 2.59 - 2.52 (m, 1H), 2.20 - 1.94 (m, 4H), 1.94 - 1.76 (m, 2H), 1.72 - 1.57 (m, 1H), 1.38 - 1.08 (m, 14H), 0.57 - 0.38 (m, 4H). LCMS m/z = 904.6 [M+1]$^+$ |
| Example 104 (trifluoroacetate of compound 104) | 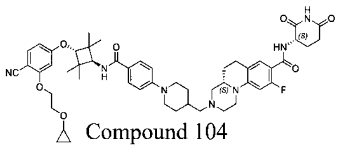 Compound 104 | 101f+4-A (following the synthetic method of example 5) | $^1$H NMR (400 MHz, DMSO-$d_6$) δ 10.82 (s, 1H), 8.08 - 8.00 (m, 1H), 7.76 (d, 2H), 7.64 (d, 1H), 7.53 - 7.38 (m, 2H), 7.06 - 6.91 (m, 2H), 6.86 (d, 1H), 6.65 (d, 1H), 6.60 - 6.49 (m, 1H), 4.80 - 4.66 (m, 1H), 4.34 - 4.12 (m, 4H), 4.09 - 4.02 (m, 1H), 3.95 - 3.75 (m, 4H), 3.72 - 3.57 (m, 2H), 3.55 - 3.45 (m, 1H), 3.45 - 3.38 (m, 1H), 3.31 - 2.96 (m, 4H), 2.97 - 2.64 (m, 6H), 2.59 - 2.52 (m, 1H), 2.21- 1.94 (m, 4H), 1.94 - 1.77 (m, 2H), 1.71 - 1.57 (m, 1H), 1.40 - 1.06 (m, 14H), 0.56 - 0.38 (m, 4H). LCMS m/z = 904.6 [M+1]$^+$ |
| Example 106 (compound 106) | 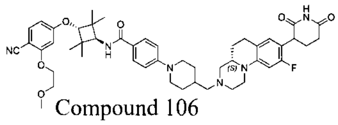 Compound 106 | 105f+intermediate 2 (following the synthetic method of example 105) | $^1$H NMR (400 MHz, DMSO-$d_6$) δ 10.76 (s, 1H), 7.73 (d, 2H), 7.64 (d, 1H), 7.46 (d, 1H), 6.95 (d, 2H), 6.79 (d, 1H), 6.71 - 6.58 (m, 2H), 6.54 (dd, 1H), 4.30 - 4.19 (m, 3H), 4.04 (d, 1H), 3.91 - 3.77 (m, 3H), 3.76 - 3.64 (m, 3H), 3.34 (s, 3H), 3.00 - 2.84 (m, 3H), 2.83 - 2.64 (m, 5H), 2.64 - 2.54 (m, 1H), 2.54 - 2.45 (m, 1H), 2.24 - 1.99 (m, 4H), 1.99 - 1.71 (m, 6H), 1.67 - 1.51 (m, 1H), 1.28 - 1.09 (m, 14H). LCMS m/z = 835.6 [M+1]$^+$ |

(continued)

| Example No. | Structure | Starting material | LCMS / $^1$H NMR |
|---|---|---|---|
| Example 107 (compound 107) | 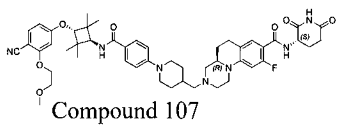 Compound 107 | 105f (following the synthetic method of example 5) | $^1$H NMR (400 MHz, DMSO-$d_6$) δ 10.82 (s, 1H), 7.94 (t, 1H), 7.73 (d, 2H), 7.64 (d, 1H), 7.46 (d, 1H), 7.36 (d, 1H), 6.95 (d, 2H), 6.74 - 6.61 (m, 2H), 6.54 (dd, 1H), 4.77 - 4.66 (m, 1H), 4.31 - 4.21 (m, 3H), 4.04 (d, 1H), 3.92 - 3.78 (m, 3H), 3.75 - 3.67 (m, 2H), 3.34 (s, 3H), 3.17 - 3.05 (m, 1H), 2.97 - 2.61 (m, 8H), 2.56 - 2.48 (m, 1H), 2.25 - 1.88 (m, 6H), 1.88 - 1.69 (m, 4H), 1.67 - 1.50 (m, 1H), 1.29 - 1.07 (m, 14H). LCMS m/z = 878.6 [M+1]$^+$ |
| Example 108 (compound 108) | 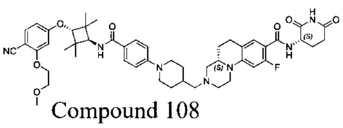 Compound 108 | 105f+4-A (following the synthetic method of example 5) | $^1$H NMR (400 MHz, DMSO-$d_6$) δ 10.82 (s, 1H), 7.94 (t, 1H), 7.73 (d, 2H), 7.64 (d, 1H), 7.46 (d, 1H), 7.36 (d, 1H), 6.95 (d, 2H), 6.74 - 6.62 (m, 2H), 6.54 (dd, 1H), 4.78 - 4.67 (m, 1H), 4.32 - 4.21 (m, 3H), 4.04 (d, 1H), 3.92 - 3.77 (m, 3H), 3.76 - 3.65 (m, 2H), 3.34 (s, 3H), 3.17 - 3.04 (m, 1H), 2.98 - 2.60 (m, 8H), 2.55 - 2.48 (m, 1H), 2.25 - 1.87 (m, 6H), 1.87 - 1.69 (m, 4H), 1.68 - 1.51 (m, 1H), 1.29 - 1.07 (m, 14H). LCMS m/z = 878.6 [M+1]$^+$ |

**Example 109:** Preparation of compound 109

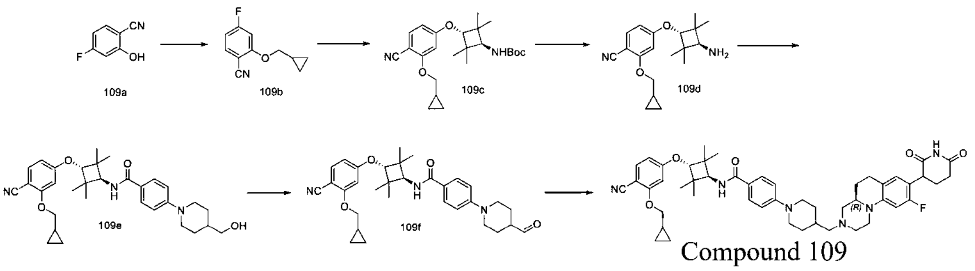

109a 109b 109c 109d 109e 109f Compound 109

Step 1: Preparation of 109b

To a reaction flask were respectively added 109a (5.0 g, 36.47 mmol), (bromomethyl)cyclopropane (6.4 g, 47.41 mmol), potassium carbonate (15.12 g, 109.41 mmol), potassium iodide (0.61 g, 3.67 mmol) and DMF (50 mL), and the mixture was reacted at room temperature for 12 h. To the reaction system was added water (200 mL), and the mixture was extracted with ethyl acetate (100 mL×3). The organic phases were combined, dried over anhydrous sodium sulphate, and concentrated under reduced pressure. The crude product was separated and purified by silica gel column chromatography (petroleum ether/ethyl acetate (v/v) = 1 : 0-3 : 1) to obtain 109b (6.2 g, yield: 89%).
LCMS m/z = 192.1 [M+1]$^+$

Starting from compounds 109b and 36h and following the synthetic method of example 36, compound 109 was obtained.

$^1$H NMR (400 MHz, DMSO-$d_6$) δ 10.76 (s, 1H), 7.73 (d, 2H), 7.63 (d, 1H), 7.46 (d, 1H), 6.95 (d, 2H), 6.79 (d, 1H), 6.66 - 6.58 (m, 2H), 6.52 (dd, 1H), 4.25 (s, 1H), 4.04 (d, 1H), 3.98 (d, 2H), 3.89 - 3.77 (m, 3H), 3.76 - 3.67 (m, 1H), 3.01 - 2.84 (m, 3H), 2.83 - 2.64 (m, 5H), 2.64 - 2.52 (m, 2H), 2.24 - 1.98 (m, 4H), 1.98 - 1.71 (m, 6H), 1.66 - 1.51 (m, 1H), 1.32 - 1.06 (m, 15H), 0.64 - 0.57 (m, 2H), 0.40 - 0.34 (m, 2H).

LCMS m/z = 831.5 $[M+1]^+$

**Example 113: Preparation of trifluoroacetate of compound 113**

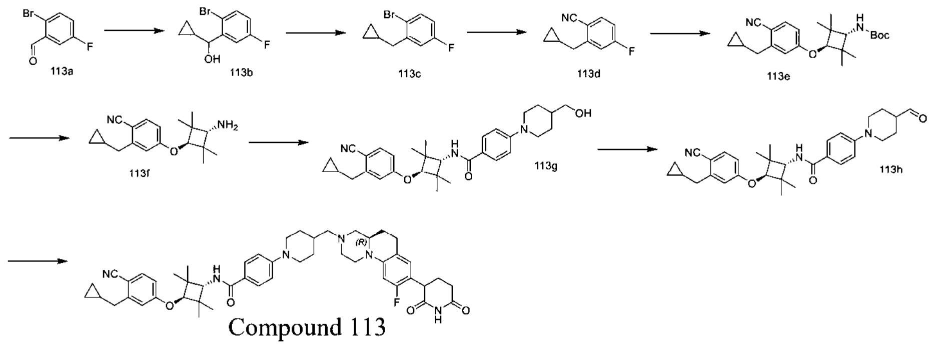

Step 1: Preparation of 113b

113a (5 g, 24.63 mmol) was dissolved in tetrahydrofuran (100 mL), and the mixture was cooled to -78°C under nitrogen atmosphere. 1 mol/L solution of cyclopropyl magnesium bromide in tetrahydrofuran (24.63 mL) was added dropwise, and the mixture was slowly warmed to room temperature and reacted for 3 h. A saturated aqueous ammonium chloride solution (100 mL) was added to the reaction solution, and the mixture was extracted with ethyl acetate (100 mL×3). The organic phases were combined, washed with a saturated aqueous sodium chloride solution (100 mL), dried over anhydrous sodium sulphate, and concentrated under reduced pressure. The crude product was separated and purified by silica gel column chromatography (petroleum ether : ethyl acetate (v/v) = 2 : 1) to obtain crude 113b (3.3 g).

Step 2: Preparation of 113c

The above-mentioned crude 113b (4.5 g) was dissolved in dichloromethane (20 mL), triethylsilane (3.20 g, 27.52 mmol) and trifluoroacetic acid (12.56 g, 110.16 mmol) were added at 0°C, and the mixture was reacted at 40°C for 2 h. The reaction solution was cooled to room temperature, and the crude product was separated and purified by silica gel column chromatography (petroleum ether : ethyl acetate (v/v) = 5 : 1) to obtain 113c (4.0 g, two-step yield from compound 113a: 52%).

Step 3: Preparation of 113d

113c (4.0 g, 17.46 mmol) was added to DMF (40 mL), followed by zinc cyanide (3.08 g, 26.23 mmol) and tetrakis(triphenylphosphine)palladium (2.02 g, 1.75 mmol) under nitrogen atmosphere, and the mixture was reacted at 110°C for 2 h. The reaction solution was cooled to room temperature, and water (60 mL) and ethyl acetate (60 mL) were added. After phase separation, the aqueous phase was extracted with ethyl acetate (50 mL×3), and the organic phases were combined, washed with a saturated aqueous sodium chloride solution (100 mL), dried over anhydrous sodium sulphate, and filtered. The filtrate was concentrated under reduced pressure, and the crude product was separated and purified by silica gel column chromatography (petroleum ether : ethyl acetate (v/v) = 5 : 1) to obtain 113d (2.2 g, yield: 72%).
LCMS m/z = 176.1 $[M+1]^+$

Starting from compounds 113d and 36h and following the synthetic method of example 36, the trifluoroacetate of compound 113 was obtained by acidic preparative chromatography (water (containing 0.1% TFA)/acetonitrile) and lyophilisation.

$^1$H NMR (400 MHz, DMSO-$d_6$) δ 10.79 (s, 1H), 7.81 - 7.65 (m, 3H), 7.50 (d, 1H), 7.04 - 6.93 (m, 3H), 6.91 - 6.83 (m, 2H), 6.80 (d, 1H), 4.25 (s, 1H), 4.12 - 4.00 (m, 2H), 3.96 - 3.79 (m, 3H), 3.68 - 3.55 (m, 2H), 3.41 - 3.27 (m, 1H), 3.19 - 2.98 (m, 4H), 2.93 - 2.59 (m, 8H), 2.57 - 2.46 (m, 1H), 2.21 - 2.05 (m, 2H), 2.05 - 1.75 (m, 4H), 1.70 - 1.54 (m, 1H), 1.38 - 1.09 (m, 14H), 1.09 - 0.96 (m, 1H), 0.57 - 0.47 (m, 2H), 0.31 - 0.24 (m, 2H).
LCMS m/z = 408.4 $[M/2+1]^+$

Following the synthetic method of other examples, the following compounds were obtained:

| Example No. | Structure | Starting material | LCMS / $^1$H NMR |
|---|---|---|---|
| Example 110 (compound 110) | 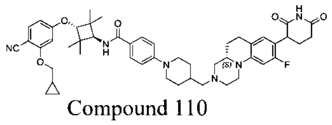 Compound 110 | 109f+intermediate 2 (following the synthetic method of example 109) | $^1$H NMR (400 MHz, DMSO-$d_6$) δ 10.76 (s, 1H), 7.73 (d, 2H), 7.63 (d, 1H), 7.46 (d, 1H), 6.95 (d, 2H), 6.79 (d, 1H), 6.67 - 6.58 (m, 2H), 6.52 (dd, 1H), 4.25 (s, 1H), 4.04 (d, 1H), 3.98 (d, 2H), 3.89 - 3.76 (m, 3H), 3.76 - 3.65 (m, 1H), 3.00 - 2.84 (m, 3H), 2.84 - 2.64 (m, 5H), 2.64 - 2.52 (m, 2H), 2.25 - 1.98 (m, 4H), 1.98 - 1.71 (m, 6H), 1.65 - 1.51 (m, 1H), 1.32 - 1.06 (m, 15H), 0.65 - 0.57 (m, 2H), 0.40 - 0.34 (m, 2H). LCMS m/z = 831.4 [M+1]$^+$ |
| Example 111 (compound 111) | 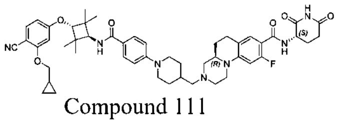 Compound 111 | 109f (following the synthetic method of example 5) | $^1$H NMR (400 MHz, DMSO-$d_6$) δ 10.81 (s, 1H), 7.94 (t, 1H), 7.73 (d, 2H), 7.63 (dd, 1H), 7.46 (d, 1H), 7.35 (d, 1H), 6.95 (d, 2H), 6.68 (d, 1H), 6.60 (s, 1H), 6.55 - 6.48 (m, 1H), 4.77 - 4.66 (m, 1H), 4.25 (s, 1H), 4.03 (d, 1H), 4.01 - 3.93 (m, 2H), 3.92 - 3.76 (m, 3H), 3.18 - 3.05 (m, 1H), 2.96 - 2.86 (m, 2H), 2.86 - 2.60 (m, 6H), 2.58 - 2.45 (m, 1H), 2.24 - 1.88 (m, 6H), 1.87 - 1.71 (m, 4H), 1.67 - 1.51 (m, 1H), 1.31 - 1.09 (m, 15H), 0.66 - 0.55 (m, 2H), 0.41 - 0.33 (m, 2H). LCMS m/z = 874.3 [M+1]$^+$ |
| Example 112 (compound 112) | 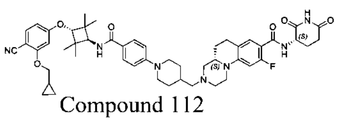 Compound 112 | 109f+4-A (following the synthetic method of example 5) | $^1$H NMR (400 MHz, DMSO-$d_6$) δ 10.81 (s, 1H), 7.94 (t, 1H), 7.77 - 7.68 (m, 2H), 7.63 (dd, 1H), 7.46 (d, 1H), 7.35 (d, 1H), 6.95 (d, 2H), 6.68 (d, 1H), 6.60 (s, 1H), 6.52 (dd, 1H), 4.77 - 4.67 (m, 1H), 4.25 (s, 1H), 4.03 (d, 1H), 3.98 (d, 2H), 3.92 - 3.79 (m, 3H), 3.16 - 3.06 (m, 1H), 2.97 - 2.86 (m, 2H), 2.86 - 2.61 (m, 6H), 2.59 - 2.45 (m, 1H), 2.24 - 1.87 (m, 6H), 1.87 - 1.70 (m, 4H), 1.67 - 1.51 (m, 1H), 1.31 - 1.09 (m, 15H), 0.66 - 0.54 (m, 2H), 0.42 - 0.32 (m, 2H). LCMS m/z = 874.6 [M+1]$^+$ |
| Example 114 (trifluoroacetate of compound 114) | 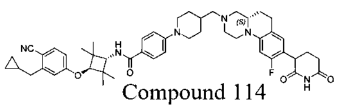 Compound 114 | 113h+intermediate 2 (following the synthetic method of example 113) | $^1$H NMR (400 MHz, DMSO-$d_6$) δ 10.79 (s, 1H), 7.82 - 7.67 (m, 3H), 7.50 (d, 1H), 7.05 - 6.92 (m, 3H), 6.91 - 6.82 (m, 2H), 6.80 (d, 1H), 4.25 (s, 1H), 4.13 - 4.01 (m, 2H), 3.98 - 3.76 (m, 3H), 3.69 - 3.55 (m, 2H), 3.43 - 3.27 (m, 1H), 3.19 - 2.95 (m, 4H), 2.95 - 2.59 (m, 8H), 2.59 - 2.46 (m, 1H), 2.21 - 2.05 (m, 2H), 2.05 - 1.75 (m, 4H), 1.73 - 1.54 (m, 1H), 1.39 - 1.09 (m, 14H), 1.09 - 0.95 (m, 1H), 0.58 - 0.47 (m, 2H), 0.32 - 0.24 (m, 2H). LCMS m/z = 408.4 [M/2+1]$^+$ |

(continued)

| Example No. | Structure | Starting material | LCMS / $^1$H NMR |
|---|---|---|---|
| Example 115 (trifluoroacetate of compound 115) | 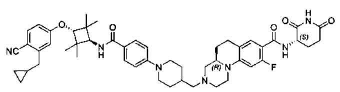 Compound 115 | 113h (following the synthetic method of example 5) | $^1$H NMR (400 MHz, DMSO-$d_6$) δ 10.82 (s, 1H), 8.08 - 7.99 (m, 1H), 7.77 (d, 2H), 7.71 (d, 1H), 7.49 (d, 1H), 7.43 (d, 1H), 7.04 - 6.94 (m, 3H), 6.91 - 6.81 (m, 2H), 4.77 - 4.68 (m, 1H), 4.31 - 4.14 (m, 2H), 4.10 - 4.01 (m, 1H), 3.98 - 3.83 (m, 2H), 3.71 - 3.58 (m, 2H), 3.55 - 3.41 (m, 1H), 3.28 - 2.99 (m, 4H), 2.95 - 2.64 (m, 8H), 2.59 - 2.52 (m, 1H), 2.20 - 1.94 (m, 4H), 1.94 - 1.76 (m, 2H), 1.71 - 1.58 (m, 1H), 1.39 - 1.09 (m, 14H), 1.08 - 0.97 (m, 1H), 0.56 - 0.48 (m, 2H), 0.34 - 0.24 (m, 2H). |
| Example 116 (trifluoroacetate of compound 116) | 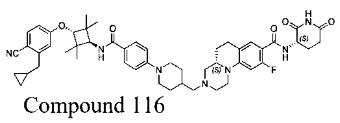 Compound 116 | 113h +4-A (following the synthetic method of example 5) | $^1$H NMR (400 MHz, DMSO-$d_6$) δ 10.82 (s, 1H), 8.07 - 7.99 (m, 1H), 7.77 (d, 2H), 7.71 (d, 1H), 7.49 (d, 1H), 7.43 (d, 1H), 7.04 - 6.95 (m, 3H), 6.90 - 6.81 (m, 2H), 4.78 - 4.68 (m, 1H), 4.30 - 4.14 (m, 2H), 4.10 - 4.02 (m, 1H), 3.97 - 3.83 (m, 2H), 3.71 - 3.58 (m, 2H), 3.54 - 3.41 (m, 1H), 3.28 - 2.99 (m, 4H), 2.94 - 2.64 (m, 8H), 2.59 - 2.52 (m, 1H), 2.20 - 1.95 (m, 4H), 1.94 - 1.76 (m, 2H), 1.71 - 1.58 (m, 1H), 1.39 - 1.09 (m, 14H), 1.09 - 0.97 (m, 1H), 0.57 - 0.48 (m, 2H), 0.32 - 0.24 (m, 2H). |

**Example 117: Preparation of compound 117**

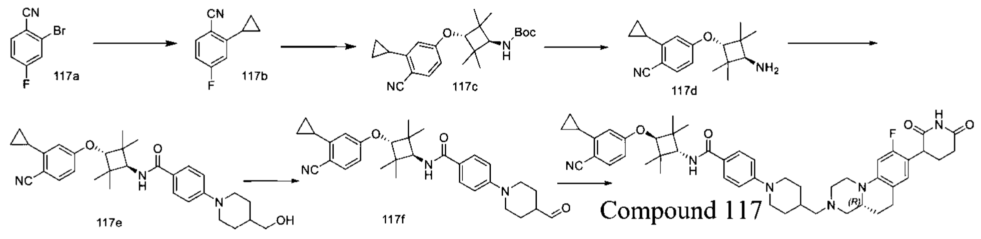
117a 117b 117c 117d 117e 117f Compound 117

Step 1: Preparation of 117b

To a reaction flask were respectively added 117a (5.00 g, 25.0 mmol), cyclopropyl boronic acid (2.79 g, 32.48 mmol), palladium acetate (0.56 g, 2.49 mmol), tricyclohexylphosphine (0.76 g, 2.71 mmol), potassium phosphate (15.92 g, 75.0 mmol) and toluene (100 mL), and the mixture was reacted at 100°C under nitrogen atmosphere for 3 h. The reaction system was cooled to room temperature, and filtered through a pad of diatomaceous earth. The filtrate was concentrated under reduced pressure, and the crude product was separated and purified by silica gel column chromatography (petroleum ether/ethyl acetate (v/v) = 1 : 0-9 : 1) to obtain 117b (3.8 g, yield: 94%).

Starting from compounds 117b and 36h and following the synthetic method of example 36, compound 117 was obtained.

$^1$H NMR (400 MHz, DMSO-$d_6$) δ 10.75 (s, 1H), 7.79 - 7.60 (m, 3H), 7.45 (d, 1H), 6.95 (d, 2H), 6.79 (dd, 2H), 6.62 (d, 1H), 6.45 (d, 1H), 4.24 (s, 1H), 4.03 (d, 1H), 3.93 - 3.76 (m, 3H), 3.76 - 3.64 (m, 1H), 3.00 - 2.84 (m, 3H), 2.84 -2.55 (m,

6H), 2.55 - 2.46 (m, 1H), 2.25 - 2.00 (m, 5H), 1.99 - 1.70 (m, 6H), 1.68 - 1.52 (m, 1H), 1.29 - 1.05 (m, 16H), 0.86 - 0.74 (m, 2H).
LCMS m/z = 801.4 [M+1]$^+$

**Example 119: Preparation of compound 119**

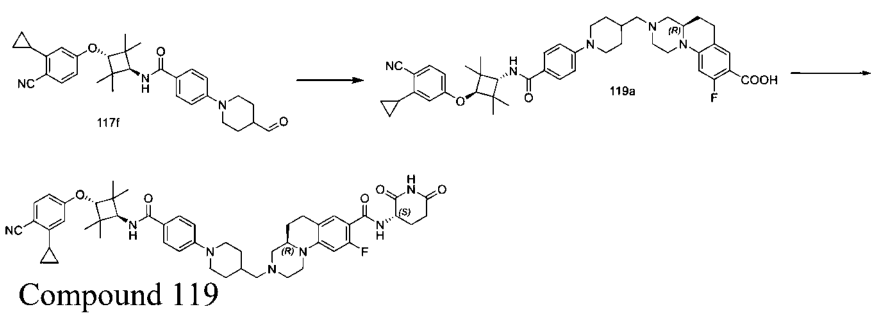

Compound 119

Starting from compound 117f and following the synthetic method of example 5, compound 119 was obtained by neutral preparative chromatography (ammonium bicarbonate in water (10 mmol/L)/acetonitrile) and lyophilisation.
$^1$H NMR (400 MHz, DMSO-d6) δ 10.82 (s, 1H), 7.94 (t, 1H), 7.79 - 7.63 (m, 3H), 7.46 (d, 1H), 7.36 (d, 1H), 6.95 (d, 2H), 6.79 (dd, 1H), 6.68 (d, 1H), 6.45 (d, 1H), 4.79 - 4.66 (m, 1H), 4.24 (s, 1H), 4.03 (d, 1H), 3.95 - 3.74 (m, 3H), 3.17 - 3.05 (m, 1H), 2.98 - 2.62 (m, 8H), 2.58 - 2.48 (m, 1H), 2.25 - 1.97 (m, 6H), 1.97 - 1.87 (m, 1H), 1.87 - 1.69 (m, 4H), 1.68 - 1.51 (m, 1H), 1.25 - 1.07 (m, 16H), 0.85 - 0.75 (m, 2H).
LCMS m/z = 844.5 [M+1]+
Following the synthetic method of other examples, the following compounds were obtained:

| Example No. | Structure | Starting material | LCMS / $^1$H NMR |
|---|---|---|---|
| Example 118 (compound 118) | 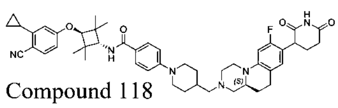 Compound 118 | 117f +intermediate 2 (following the synthetic method of example 117) | $^1$H NMR (400 MHz, DMSO-$d_6$) δ 10.75 (s, 1H), 7.78 - 7.61 (m, 3H), 7.45 (d, 1H), 6.95 (d, 2H), 6.79 (dd, 2H), 6.62 (d, 1H), 6.45 (d, 1H), 4.24 (s, 1H), 4.03 (d, 1H), 3.91 - 3.77 (m, 3H), 3.76 - 3.66 (m, 1H), 3.00 - 2.84 (m, 3H), 2.84 - 2.64 (m, 5H), 2.64 - 2.55 (m, 1H), 2.54 - 2.46 (m, 1H), 2.24 - 2.00 (m, 5H), 1.99 - 1.71 (m, 6H), 1.66 - 1.52 (m, 1H), 1.27 - 1.05 (m, 16H), 0.84 - 0.76 (m, 2H). LCMS m/z = 801.4 [M+1]+ |
| Example 120 (compound 120) | 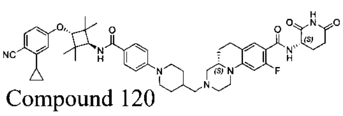 Compound 120 | 117f +4-A (following the synthetic method of example 119) | $^1$H NMR (400 MHz, DMSO-$d_6$) δ 10.81 (s, 1H), 7.93 (t, 1H), 7.78 - 7.63 (m, 3H), 7.45 (d, 1H), 7.36 (d, 1H), 6.95 (d, 2H), 6.79 (dd, 1H), 6.68 (d, 1H), 6.45 (d, 1H), 4.79 - 4.67 (m, 1H), 4.24 (s, 1H), 4.03 (d, 1H), 3.92 - 3.72 (m, 3H), 3.17 - 3.06 (m, 1H), 2.98 - 2.61 (m, 8H), 2.58 - 2.47 (m, 1H), 2.25 - 1.97 (m, 6H), 1.97 - 1.87 (m, 1H), 1.87 - 1.68 (m, 4H), 1.68 - 1.51 (m, 1H), 1.25 - 1.07 (m, 16H), 0.85 - 0.76 (m, 2H). LCMS m/z = 844.4 [M+1]+ |

(continued)

| Example No. | Structure | Starting material | LCMS / $^1$H NMR |
|---|---|---|---|
| Example 121 (trifluoroacetate of compound 121) | 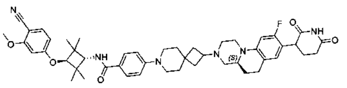 Compound 121 | 16f +intermediate 2 (following the synthetic method of example 1) | $^1$H NMR (400 MHz, $CD_3OD$) δ 7.81 - 7.70 (m, 2H), 7.52 (d, 1H), 7.12 - 7.01 (m, 2H), 6.90 (d, 1H), 6.70 (d, 1H), 6.62 (d, 1H), 6.55 (dd, 1H), 4.26 (s, 1H), 4.17 - 4.05 (m, 2H), 3.93 (s, 3H), 3.87 (dd, 1H), 3.83 - 3.71 (m, 1H), 3.67 - 3.50 (m, 2H), 3.42 - 3.35 (m, 2H), 3.34 - 3.22 (m, 3H), 3.13 - 2.94 (m, 2H), 2.89 - 2.59 (m, 5H), 2.48 - 2.35 (m, 2H), 2.30 - 1.99 (m, 5H), 1.93 - 1.68 (m, 5H), 1.26 (d, 12H). LCMS m/z = 817.6 [M+1]+ |
| Example 122 (trifluoroacetate of compound 122) | 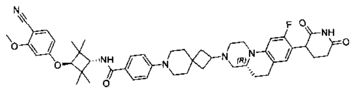 Compound 122 | 16f +intermediate 1 (following the synthetic method of example 1) | $^1$H NMR (400 MHz, $CD_3OD$) δ 7.79 - 7.70 (m, 2H), 7.52 (d, 1H), 7.11 - 7.01 (m, 2H), 6.90 (d, 1H), 6.71 (d, 1H), 6.62 (d, 1H), 6.55 (dd, 1H), 4.26 (s, 1H), 4.17 - 4.07 (m, 2H), 3.93 (s, 3H), 3.87 (dd, 1H), 3.83 - 3.71 (m, 1H), 3.67 - 3.50 (m, 2H), 3.42 - 3.35 (m, 2H), 3.33 - 3.24 (m, 3H), 3.12 - 2.95 (m, 2H), 2.89 - 2.59 (m, 5H), 2.48 - 2.35 (m, 2H), 2.30 - 2.17 (m, 1H), 2.17 - 2.04 (m, 4H), 1.90 - 1.69 (m, 5H), 1.26 (d, 12H). LCMS m/z = 817.6 [M+1]+ |

**Example 127: Preparation of compound 127**

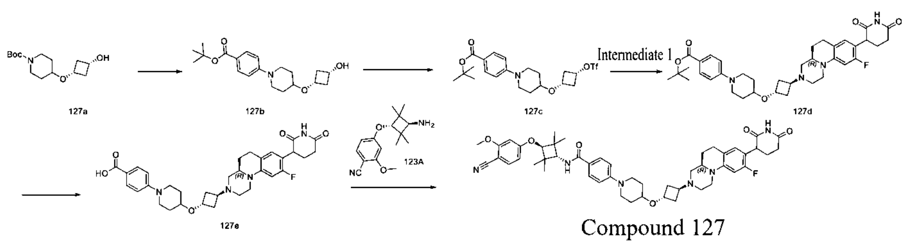

Compound 127

Step 1: Preparation of 127b

127a (4.98 g, 18.35 mmol) (see CN112912376 for the synthetic method) was dissolved in dichloromethane (20 mL), trifluoroacetic acid (10 mL) was added, and the mixture was reacted at room temperature for 3 h. The reaction solution was concentrated under reduced pressure and dissloved in DMSO (50 mL), DIPEA (11.86 g, 91.76 mmol) and tert-butyl 4-fluorobenzoate (3.6 g, 18.35 mmol) were respectively added, and the mixture was reacted at 100°C for 19 h. The reaction solution was cooled to room temperature, and water (100 mL) and ethyl acetate (100 mL) were added. After phase separation, the aqueous phase was extracted with ethyl acetate (80 mL×3), and the organic phases were combined, washed with a saturated aqueous sodium chloride solution (100 mL), dried over anhydrous sodium sulphate, and filtered. The filtrate was concentrated under reduced pressure, and the crude product was separated and purified by silica gel column chromatography (petroleum ether : ethyl acetate (v/v) = 5 : 1-1 : 1) to obtain 127b (2.75 g, yield: 43%).

Step 2: Preparation of 127c

127b (0.4 g, 1.15 mmol) was dissolved in dichloromethane (2 mL), and triethylamine (0.58 g, 5.73 mmol) was added. The system was cooled to -50°C, and trifluoromethanesulphonic anhydride (0.39 g, 1.38 mmol) was slowly added

dropwise, and the mixture was allowed to warm to room temperature and reacted for 3 h. The reaction solution was concentrated under reduced pressure to obtain crude 127c (1.0 g).

Step 3: Preparation of 127d

The above-mentioned crude 127c (1.0 g) was dissolved in acetonitrile (5 mL), DIPEA (0.59 g, 4.6 mmol) and the above-mentioned crude intermediate 1 (0.30 g) were respectively added, and the mixture was reacted at room temperature for 19 h. Water (10 mL) and ethyl acetate (20 mL) were added to the reaction solution. After phase separation, the aqueous phase was extracted with ethyl acetate (80 mL×3), and the organic phases were combined, washed with a saturated aqueous sodium chloride solution (50 mL), dried over anhydrous sodium sulphate, and filtered. The filtrate was concentrated under reduced pressure, and the crude product was separated and purified by silica gel column chromatography (petroleum ether : ethyl acetate (v/v) = 5 : 1-2 : 1) to obtain 127d (0.45 g, two-step yield from compound 127b: 60%).

Step 4: Preparation of hydrochloride of 127e

127d (0.2 g, 0.31 mmol) was dissolved in dichloromethane (2 mL), 4 mol/L solution of hydrogen chloride in ethyl acetate (5 mL) was added, and the mixture was reacted at room temperature for 5 h. The reaction solution was concentrated under reduced pressure to obtain the hydrochloride of crude 127e (0.22 g).
LCMS m/z = 591.3 [M+1]+

Step 5: Preparation of compound 127

The above-mentioned hydrochloride of crude 127e (0.22 g) was dissolved in DMF (5 mL), DIPEA (0.2 g, 1.55 mmol), HATU (0.18 g, 0.47 mmol) and the hydrochloride of 123A (96 mg, 0.31 mmol) were respectively added, and the mixture was reacted at room temperature under nitrogen atmosphere for 16 h. Ethyl acetate (50 mL) and purified water (50 mL) were added to the reaction solution. The aqueous phase was extracted with ethyl acetate (25 mL×2), and the organic phases were combined, washed with a saturated aqueous sodium chloride solution (25 mL), dried over anhydrous sodium sulphate, and filtered. The filtrate was concentrated under reduced pressure, and the crude product was separated and purified by silica gel column chromatography (petroleum ether : ethyl acetate (v/v) = 1 : 2) to obtain compound 127 (110 mg, two-step yield from compound 127d: 42%).

$^1$H NMR (400 MHz, $CDCl_3$) δ 7.88 (s, 1H), 7.67 (d, 2H), 7.45 (d, 1H), 6.96 - 6.86 (m, 2H), 6.72 (d, 1H), 6.53 - 6.43 (m, 2H), 6.40 (dd, 1H), 6.10 (d, 1H), 4.33 - 4.20 (m, 1H), 4.18 - 4.10 (m, 1H), 4.04 (s, 1H), 3.91 (s, 3H), 3.84 - 3.72 (m, 1H), 3.71 - 3.56 (m, 3H), 3.56 - 3.46 (m, 1H), 3.12 - 2.71 (m, 9H), 2.70 - 2.59 (m, 2H), 2.32 - 2.07 (m, 6H), 2.03 - 1.84 (m, 4H), 1.79 - 1.62 (m, 4H), 1.24 (d, 12H).
LCMS m/z = 847.4 [M+1]+

Following the synthetic method of other examples, the following compounds were obtained:

| Example No. | Structure | Starting material | LCMS / $^1$H NMR |
|---|---|---|---|
| Example 128 (compound 128) | 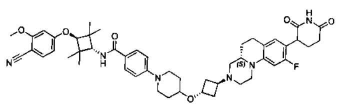 Compound 128 | 127c+intermediate 2 (following the synthetic method of example 127) | $^1$H NMR (400 MHz, $CDCl_3$) δ 7.88 (s, 1H), 7.68 (d, 2H), 7.45 (d, 1H), 6.97 - 6.85 (m, 2H), 6.72 (d, 1H), 6.53 - 6.42 (m, 2H), 6.40 (dd, 1H), 6.09 (d, 1H), 4.35 - 4.20 (m, 1H), 4.20 - 4.11 (m, 1H), 4.04 (s, 1H), 3.91 (s, 3H), 3.86 - 3.56 (m, 4H), 3.56 - 3.44 (m, 1H), 3.12 - 2.70 (m, 9H), 2.70 - 2.58 (m, 2H), 2.33 - 2.06 (m, 6H), 2.06 - 1.83 (m, 4H), 1.79 - 1.61 (m, 4H), 1.24 (d, 12H). LCMS m/z = 847.4 [M+1]+ |

(continued)

| Example No. | Structure | Starting material | LCMS / $^1$H NMR |
|---|---|---|---|
| Example 129 (compound 129) | 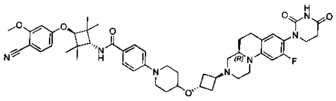 Compound 129 | 127c+28f (following the synthetic methods of examples 127 and 15) | $^1$H NMR (400 MHz, DMSO-$d_6$) δ 10.32 (s, 1H), 7.77 - 7.69 (m, 2H), 7.64 (d, 1H), 7.49 (d, 1H), 7.00 - 6.89 (m, 3H), 6.72 (d, 1H), 6.67 - 6.60 (m, 1H), 6.56 - 6.50 (m, 1H), 4.27 (s, 1H), 4.22 - 4.13 (m, 1H), 4.05 (d, 1H), 3.91 (s, 3H), 3.81 - 3.72 (m, 1H), 3.70 - 3.55 (m, 4H), 3.54 - 3.45 (m, 1H), 3.04 - 2.87 (m, 5H), 2.82 - 2.56 (m, 6H), 2.26 - 2.14 (m, 2H), 2.04 - 1.94 (m, 2H), 1.94 - 1.78 (m, 4H), 1.65 - 1.41 (m, 4H), 1.18 (d, 12H). LCMS m/z = 848.4 [M+1]$^+$ |
| Example 130 (compound 130) | 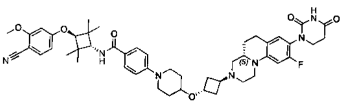 Compound 130 | 127c+29f (following the synthetic methods of examples 127 and 15) | $^1$H NMR (400 MHz, DMSO-$d_6$) δ 10.32 (s, 1H), 7.77 - 7.68 (m, 2H), 7.64 (d, 1H), 7.49 (d, 1H), 7.01 - 6.88 (m, 3H), 6.72 (d, 1H), 6.69 - 6.60 (m, 1H), 6.57 - 6.50 (m, 1H), 4.27 (s, 1H), 4.24 - 4.12 (m, 1H), 4.05 (d, 1H), 3.91 (s, 3H), 3.82 - 3.70 (m, 1H), 3.70 - 3.55 (m, 4H), 3.54 - 3.45 (m, 1H), 3.05 - 2.85 (m, 5H), 2.82 - 2.56 (m, 6H), 2.27 - 2.14 (m, 2H), 2.05 - 1.94 (m, 2H), 1.94 - 1.78 (m, 4H), 1.67 - 1.40 (m, 4H), 1.18 (d, 12H). LCMS m/z = 848.4 [M+1]$^+$ |

**Biological test examples**

**1. Detection of AR degradation in VCap cells**

VCap is a human prostate cancer cell line purchased from ATCC and cultured under the following conditions: DMEM + 10% FBS + 1% penicillin-streptomycin solution. The cells were cultured in a 37°C, 5% $CO_2$ incubator. The cells were seeded in a 6-well plate, with $5 \times 10^5$ cells/well. After seeding, compounds at various concentrations were added, and the plate was incubated for 24 hours in the 37°C, 5% $CO_2$ incubator. Following incubation, the cells were harvested and RIPA lysis buffer (beyotime, Cat. P0013B) was added. The cells were lysed on ice for 15 minutes and centrifuged at 12,000 rpm at 4°C for 10 minutes. The supernatant protein samples were collected and the protein was quantified using the BCA kit (Beyotime, Cat. P0009). Then the protein was diluted to 0.25 mg/mL. The expressions of AR (CST, Cat.5153S) and the internal reference β-actin (CST, Cat. 3700S) were detected using a fully automated western blot quantitative analyser (Proteinsimple). Using the "Compass for SW" software, the relative peak area of AR was calculated when the internal reference area was 10,000. The proportion of AR relative to the solvent control group at various drug concentrations was calculated according to Equation (1), where $AR_{treat}$ represents the relative peak area of the compound-treated group, and $AR_{solvent}$ represents the relative peak area of the solvent control group. The processed data obtained from Equation (1) were analysed using GraphPad Prism 8.3.0 software, and the $DC_{50}$ value was calculated using a four-parameter nonlinear regression model. The degradation rate at a specified concentration was determined as 100-AR%.

$$AR\% = AR_{treat}/AR_{solvent} \times 100\% \quad \text{Equation 1}$$

The results of $DC_{50}$ values for the degradation of AR protein in VCaP cells are shown in Table 1.

Table 1 $DC_{50}$ values for degradation of AR protein in VCaP cells by compounds of the present invention

| Serial No. | Compound No. | $DC_{50}$ (nM) | Serial No. | Compound No. | $DC_{50}$ (nM) |
|---|---|---|---|---|---|
| 1 | Trifluoroacetate of compound 1 | <100 | 29 | Compound 37 | <100 |

(continued)

| Serial No. | Compound No. | $DC_{50}$ (nM) | Serial No. | Compound No. | $DC_{50}$ (nM) |
|---|---|---|---|---|---|
| 2 | Chiral isomer 1 of compound 1 | <100 | 30 | Compound 38 | <100 |
| 3 | Chiral isomer 2 of compound 1 | <100 | 31 | Compound 39 | <100 |
| 4 | Trifluoroacetate of compound 2 | <100 | 32 | Compound 40 | <100 |
| 5 | Trifluoroacetate of compound 3 | <100 | 33 | Compound 42 | <100 |
| 6 | Trifluoroacetate of compound 4 | <100 | 34 | Compound 43 | <100 |
| 7 | Trifluoroacetate of compound 5 | <100 | 35 | Trifluoroacetate of compound 44 | <100 |
| 8 | Trifluoroacetate of compound 7 | <100 | 36 | Compound 48 | <100 |
| 9 | Trifluoroacetate of compound 9 | <100 | 37 | Compound 49 | <100 |
| 10 | Trifluoroacetate of compound 10 | <100 | 38 | Compound 50 | <100 |
| 11 | Compound 11 | <100 | 39 | Compound 51 | <100 |
| 12 | Compound 13 | <100 | 40 | Compound 54 | <100 |
| 13 | Compound 14 | <100 | 41 | Compound 55 | <100 |
| 14 | Compound 15 | <100 | 42 | Compound 56 | <100 |
| 15 | Trifluoroacetate of compound 18 | <100 | 43 | Compound 58 | <100 |
| 16 | Trifluoroacetate of compound 20 | <100 | 44 | Trifluoroacetate of compound 60 | <100 |
| 17 | Trifluoroacetate of compound 21 | <100 | 45 | Trifluoroacetate of compound 61 | <100 |
| 18 | Trifluoroacetate of compound 22 | <100 | 46 | Trifluoroacetate of compound 62 | <100 |
| 19 | Trifluoroacetate of compound 24 | <100 | 47 | Trifluoroacetate of compound 63 | <100 |
| 20 | Trifluoroacetate of compound 25 | <100 | 48 | Compound 72 | <100 |
| 21 | Compound 28 | <100 | 49 | Compound 73 | <100 |
| 22 | Compound 29 | <100 | 50 | Compound 78 | <100 |
| 23 | Compound 30 | <100 | 51 | Compound 79 | <100 |
| 24 | Compound 31 | <100 | 52 | Compound 80 | <100 |
| 25 | Compound 32 | <100 | 53 | Compound 82 | <100 |
| 26 | Trifluoroacetate of compound 33 | <100 | 54 | Compound 83 | <100 |
| 27 | Compound 34 | <100 | 55 | Trifluoroacetate of compound 84 | <100 |
| 28 | Compound 35 | <100 | | | |

Table 2 Degradation rate of AR protein in VCaP cells by compounds of the present invention at concentration of 30 nM

| Serial No. | Compound No. | Degradation rate % |
|---|---|---|
| 1 | Compound 93 | >70% |
| 2 | Trifluoroacetate of compound 99 | >70% |
| 4 | Compound 117 | >70% |
| 5 | Compound 118 | >70% |
| 6 | Compound 119 | >70% |
| 7 | Trifluoroacetate of compound 121 | >70% |
| Conclusion: The compounds of the present invention, such as the example compounds, have good AR degradation activity. | | |

## 2. Pharmacokinetic test in rats

**Experimental objective:** In this experiment, a single dose of the test compound was administered to SD rats via intravenous administration and intragastric gavage, and the concentration of the test compound in plasma of the rats was measured to evaluate the pharmacokinetic profile of the test compound in the rats.

**Experimental animals:** Male SD rats, 200-220 g, 6 rats/compound, purchased from Chengdu Ddossy Experimental Animals Co., Ltd.

**Experimental method:** On the day of the experiment, 6 SD rats were randomly grouped according to their body weights. The animals were fasted with water available for 12 to 14 h one day before the administration, and were fed 4 h after the administration.

Table 3 Administration information of pharmacokinetic test in rats

| Group | Number | Administration information | | | | | | |
|---|---|---|---|---|---|---|---|---|
| | Male | Test compound | Administration dosage* (mg/kg) | Administration concentration (mg/mL) | Administration volume (mL/kg) | Collected sample | Mode of administration | Vehicle |
| G1 | 3 | Compound of the present invention | 5 | 0.5 | 10 | Plasma | Oral administration (intragastric gavage) | 5% DMSO+5 % Solutol+9 0% (20% SBE-β-CD) |
| G2 | 3 | Compound of the present invention | 2 | 0.4 | 5 | Plasma | Intravenous injection | 5% DMA + 5% Solutol + 90% Saline |

*Dosage is calculated based on a free base.

**Sampling:** Before and after the administration, 0.1 mL of blood was taken from the orbits under isoflurane anaesthesia, and placed in an EDTAK2 centrifuge tube. Centrifugation was carried out at 5000 rpm at 4°C for 10 min, and the plasma was collected.

The plasma collection time points for IV&PO groups were 0, 5 min, 15 min, 30 min, 1 h, 2 h, 4 h, 7 h, 24 h, and 48 h.

Before analysis and detection, all samples were stored at -60°C. The samples were analysed quantitatively by LC-MS/MS.

Table 4 Pharmacokinetic parameters of compounds of the present invention in plasma of rats

| Test compound | Mode of administration | $AUC_{0-t}$ (hr*ng/mL) | F (%) |
|---|---|---|---|
| Control compound### | i.g. (5 mg/kg) | 2867±338 | 26.9±3.2 |
| Compound 1### | i.g. (5 mg/kg) | 5494±1011 | 46.5±8.6 |
| Compound 2# | i.g. (5 mg/kg) | 6009±889 | 61.4+9.1 |
| Compound 31## | i.g. (5 mg/kg) | 5427±1362 | 49.1+12 |
| Trifluoroacetate of compound 60### | i.g. (5 mg/kg) | 6605±349 | 57.5±3.0 |
| Trifluoroacetate of compound 61### | i.g. (5 mg/kg) | 5463+1776 | 43.5+14 |
| Compound 79## | i.g. (5 mg/kg) | 6968+2007 | - |

# Vehicle for oral administration is 5% DMSO+ 5% Solutol+30% PEG-400+60% (20% SBE-β-CD)
## Vehicle for oral administration is 5% DMSO+5% Solutol+10% PEG400+80% (20% SBE-β-CD)
### Vehicle for oral administration is 5% DMSO+5% Solutol+90% (20% SBE-β-CD)
Conclusion: The compounds of the present invention, such as the example compounds, have good oral absorption in rats.

**3. VCaP cell proliferation inhibition experiment**

Prostate cancer cells VCaP were purchased from ATCC, the cell medium was 1640+10% FBS, and the cells were cultured in a 37°C, 5% $CO_2$ incubator. Prior to the experiment, cells maintained in normal medium were passaged into phenol red-free RPMI 1640 containing 10% charcoal-stripped FBS and cultured for 3 days. On day 4, the cells were digested using phenol red-free digestion solution (Trypsin LE), and the digestion was stopped with phenol red-free RPMI 1640 medium containing 1% charcoal-stripped FBS+0.5% PS+0.1 nM R1881. After centrifugation and resuspension, viable cell counting was performed using a Vi-Cell XR cell counter. The cell suspension was adjusted to an appropriate concentration with phenol red-free RPMI 1640 medium containing 1% charcoal-stripped FBS+0.5% PS+0.1 nM R1881. Then, 180 μl of the cell suspension was added per well to a 96-well cell culture plate at a cell density of 7,500 cells/well. At the time of seeding, T0 wells were also prepared. The following day, R1881 at a final concentration of 0.1 nM and compounds at various concentrations were added. The plate was placed back into the incubator and cultured for an additional 7 days. At the time of compound addition, the $T_0$ plate was measured using the CellTiter-Glo (CTG) kit (Promega, Cat. No. G7572) and recorded as $RLU_0$. Following incubation, 75 μl of pre-thawed and room temperature-equilibrated CellTiter-Glo solution was added to each well. The plate was mixed uniformly for 2 minutes using a microplate shaker, and maintained at room temperature for 10 minutes, and then the luminescence signal value was measured using an Envision 2104 plate reader. The results were processed according to Equation (2). The cell growth rates at various compound concentrations were calculated in Excel, and the $GI_{50}$ values (the compound concentrations required to inhibit proliferation by 50%) were calculated using GraphPad software, where RLU compound represents the readout of the compound-treated group, and RLU control represents the average value of the solvent control group.

$$\text{Growth \%} = (RLU_{\text{compound}} - RLU_0)/(RLU_{\text{control}} - RLU_0) \times 100\% \quad \text{Equation (2)}$$

The results of $GI_{50}$ values for the inhibition of VCaP cell proliferation are shown in Table 5.

Table 5 $GI_{50}$ values for inhibition of VCaP cell proliferation by compounds of the present invention

| Serial No. | Compound No. | $GI_{50}$ (nM) | Serial No. | Compound No. | $GI_{50}$ (nM) |
|---|---|---|---|---|---|
| 1 | Control compound | >2000 | 15 | Trifluoroacetate of compound 65 | <300 |
| 2 | Compound 1 | <300 | 16 | Trifluoroacetate of compound 66 | <300 |

(continued)

| Serial No. | Compound No. | $GI_{50}$ (nM) | Serial No. | Compound No. | $GI_{50}$ (nM) |
|---|---|---|---|---|---|
| 3 | Compound 11 | <300 | 17 | Trifluoroacetate of compound 67 | <300 |
| 4 | Trifluoroacetate of compound 33 | <500 | 18 | Trifluoroacetate of compound 68 | <300 |
| 5 | Compound 36 | <300 | 19 | Trifluoroacetate of compound 69 | <300 |
| 6 | Compound 37 | <300 | 20 | Trifluoroacetate of compound 70 | <500 |
| 7 | Compound 40 | <300 | 21 | Trifluoroacetate of compound 71 | <500 |
| 8 | Compound 41 | <300 | 22 | Compound 79 | <300 |
| 9 | Compound 52 | <300 | 23 | Compound 85 | <300 |
| 10 | Compound 53 | <300 | 24 | Compound 86 | <300 |
| 11 | Compound 57 | <500 | 25 | Compound 87 | <500 |
| 12 | Compound 59 | <300 | 26 | Compound 88 | <500 |
| 13 | Trifluoroacetate of compound 60 | <300 | 27 | Compound 94 | <300 |
| 14 | Trifluoroacetate of compound 61 | <300 | | | |
| Conclusion: The compounds of the present invention, such as the example compounds, have good inhibitory activity against VCaP cell proliferation. | | | | | |

**4. Caco2 permeability test**

In the experiment, a monolayer of Caco-2 cells was used, and incubated in triplicate in a 96-well Transwell plate. A transport buffer solution (HBSS, 10 mM HEPES, pH 7.4 ± 0.05) containing the compound of the present invention (2 μM) or the control compound digoxin (10 μM), nadolol (2 μM) or metoprolol (2 μM) was added to the administration end hole on the apical side or basal side. A DMSO-containing transport buffer solution was added to the corresponding receiving end hole. After incubation for 2 hours at 37°C ± 1°C, the cell plate was removed, and an appropriate amount of samples was taken from both the apical side and basal side and transferred to a new 96-well plate. Subsequently, acetonitrile containing an internal standard was added to precipitate the protein. The samples were analysed using LC MS/MS, and the concentrations of the compound of the present invention and the control compound were determined. Concentration data were used to calculate apparent permeability coefficients for transport from the apical side to the basal side, and from the basal side to the apical side of the cell monolayer, and thus to calculate the efflux ratio. The integrity of the cell monolayer after 2 hours of incubation was evaluated by leakage of Lucifer Yellow.

Conclusion: The compounds of the present invention, such as the example compounds, have good Caco2 permeability.

**5. Pharmacokinetic test in mice**

**Experimental objective:** In this experiment, a single dose of the test compound was administered to ICR mice via intravenous administration and intragastric gavage, and the concentration of the test compound in plasma of the mice was measured to evaluate the pharmacokinetic profile of the test compound in the mice.

**Experimental animals:** Male ICR mice, 6 mice/compound, purchased from Chengdu Ddossy Experimental Animals Co., Ltd.

**Experimental method:** On the day of the experiment, 6 SD mice were randomly grouped according to their body weights. The animals were fasted with water available for 12 to 14 h one day before the administration, and were fed 4 h after the administration.

Table 6 Administration information of pharmacokinetic test in mice

| Group | Number | Administration information | | | | | | |
|---|---|---|---|---|---|---|---|---|
| | Male | Test compound | Administration dosage* (mg/kg) | Administration concentration (mg/mL) | Administration volume (mL/kg) | Collected sample | Mode of administration | Vehicle |
| G1 | 3 | Compound of the present invention | 5 | 0.5 | 10 | Plasma | Oral administration (intragastric gavage) | Vehicle 1: 5% DMSO+5% Solutol+30% PEG400+60% (20% SBE-CD) |
| | | | | | | | | Vehicle 2: 5% DMSO+5% |
| | | | | | | | | Solutol+10% PEG400+80% (20% SBE-CD) |
| | | | | | | | | Vehicle 3: 5% DMSO+5% Solutol+90% (20% SBE-β-CD) |
| G2 | 3 | Compound of the present invention | 2 | 0.4 | 5 | Plasma | Intravenous injection | 5% DMA+5% HS+90% NS |

*Dosage is calculated based on a free base.

**Sampling:** Before and after the administration, 0.15 mL of blood was taken from the orbits under isoflurane anaesthesia, and placed in an EDTAK2 centrifuge tube. Centrifugation was carried out at 5000 rpm at 4°C for 10 min, and the plasma was collected.

The plasma collection time points for PO groups were 0, 5 min, 15 min, 30 min, 1 h, 2 h, 4 h, 7 h, 24 h, and 48 h.

The plasma collection time points for IV groups were 0, 5 min, 15 min, 30 min, 1 h, 2 h, 4 h, 7 h, 24 h, and 48 h.

Before analysis and detection, all samples were stored at -60°C. The samples were analysed quantitatively by LC-MS/MS.

Table 7 Pharmacokinetic parameters of compounds of the present invention in plasma of mice

| Test compound | Mode of administration | Vehicle | $AUC_{0-t}$ (hr*ng/mL) | F (%) |
|---|---|---|---|---|
| Control compound | i.g. (5 mg/kg) | Vehicle 1 | 20727±1076 | 63.4±3.3% |
| Compound 13 | i.g. (5 mg/kg) | Vehicle 1 | 33521±1683 | - |
| Compound 30 | i.g. (5 mg/kg) | Vehicle 3 | 33000±9331 | - |
| Compound 32 | i.g. (5 mg/kg) | Vehicle 3 | 30874±6079 | - |
| Trifluoroacetate of compound 60 | i.g. (5 mg/kg) | Vehicle 3 | 41602±4140 | 84.2±8.4% |
| Trifluoroacetate of compound 61 | i.g. (5 mg/kg) | Vehicle 3 | 28537±3461 | 98.8±12% |
| Trifluoroacetate of compound 66 | i.g. (5 mg/kg) | Vehicle 3 | 30449±9308 | - |
| Conclusion: The compounds of the present invention, such as the example compounds, have good oral absorption in mice. | | | | |

## 6. Pharmacokinetic test in beagle dogs

**Experimental animals:** Male beagle dogs, about 8 to 10 kg, 6 beagle dogs/compound, purchased from Beijing Marshall Biotechnology Co., Ltd.

**Experimental method:** On the day of the experiment, 6 beagle dogs were randomly grouped according to their body weights. The animals were fasted with water available for 14 to 18 h one day before the administration, and were fed 4 h after the administration.

Table 8 Administration information of pharmacokinetic test in beagle dogs

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Compound of the present invention | 0.5 | 0.5 | 1 | Plasma | Intravenous administration |
| G2 | 3 | Compound of the present invention | 2 | 0.4 | 5 | Plasma | Oral administration (intragastric gavage) |
| Note: Vehicle for intravenous administration: 5% DMA+5% Solutol+90% NS;<br>vehicle for oral administration (intragastric gavage): 5% DMSO+5% Solutol+10% PEG400+80% (20% SBE-CD);<br>*Dosage is calculated based on a free base. | | | | | | | |

Before and after the administration, 1 ml of blood was taken from the jugular veins or limb veins, and placed in an EDTAK2 centrifuge tube. Centrifugation was carried out at 5000 rpm at 4°C for 10 min, and the plasma was collected. The blood collection time points for the intravenous administration group and intragastric gavage group were 0, 5 min, 15 min, 30 min, 1 h, 2 h, 4 h, 6 h, 8 h, 10 h, 12 h, 24 h and 48 h. Before analysis and detection, all samples were stored at -80°C. The samples were analysed quantitatively by LC-MS/MS.

Conclusion: The compounds of the present invention, such as the example compounds, have good oral

absorption in beagle dogs.

### 7. Pharmacokinetic test in monkeys

**Experimental animals:** Male cynomolgus monkeys, 3-5 kg, 3-6 years old, 4 monkeys/compound, purchased from Suzhou Xishan Biotechnology Inc.

**Experimental method:** On the day of the experiment, 4 monkeys were randomly grouped according to their body weights. The animals were fasted with water available for 14 to 18 h one day before the administration, and were fed 4 h after the administration.

Table 9 Administration information of pharmacokinetic test in monkeys

| Group | Number | Administration information | | | | | |
|---|---|---|---|---|---|---|---|
| | Male | Test compound | Administration dosage (mg/kg) | Administration concentration (mg/mL) | Administ ration volume (mL/kg) | Collected sample | Mode of administration |
| G1 | 2 | Compound of the present invention | 0.5 | 0.5 | 1 | Plasma | Intravenous administration |
| G2 | 2 | Compound of the present invention | 2 | 0.4 | 5 | Plasma | Oral administration (intragastric gavage) |
| Note: Vehicle for intravenous administration: 5% DMA+5% Solutol+90% NS;<br>vehicle for oral administration (intragastric gavage): 5% DMSO+5% Solutol+10% PEG400+80% (20% SBE-CD);<br>*Dosage is calculated based on a free base. | | | | | | | |

Before and after the administration, 1.0 mL of blood was taken from the jugular veins, and placed in an EDTAK2 centrifuge tube. Centrifugation was carried out at 5000 rpm at 4°C for 10 min, and the plasma was collected. The blood collection time points for the intravenous administration group and intragastric gavage group were 0, 5 min, 15 min, 30 min, 1 h, 2 h, 4 h, 6 h, 8 h, 10 h, 12 h, 24 h and 48 h. Before analysis and detection, all samples were stored at -60°C. The samples were analysed quantitatively by LC-MS/MS.

Conclusion: The compounds of the present invention, such as the example compounds, have good oral absorption in monkeys.

### 8. hERG potassium ion channel test

**Experimental platform:** Electrophysiological manual patch-clamp system

**Cell line:** Chinese hamster ovary (CHO) cell lines stably expressing hERG potassium ion channel

**Experimental method:** In Chinese Hamster Ovary (CHO) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarised from -80 mV to +20 mV for 2 s, then repolarised to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition\%} = [1 - (I / Io)] \times 100\%$$

where Inhibition% represents the percentage of inhibition of hERG potassium current by the compound, and *I* and *I*o represent the amplitude of hERG potassium current after and before the administration, respectively.

Compound $IC_{50}$ was calculated using GraphPad Prism 5 software by fitting according to the following equation:

$$Y = \text{Bottom} + (\text{Top-Bottom})/(1+10\text{^}((\text{LogIC}_{50}\text{-X})\text{*HillSlope}))$$

where X represents the Log value of the tested concentration of the test sample, Y represents the inhibition percentage at the corresponding concentration, and Bottom and Top represent the minimum and maximum inhibition percentage, respectively.

Conclusion: The compounds of the present invention, such as the example compounds, exhibit no significant inhibitory effects on hERG potassium channel current. Specifically, the trifluoroacetates of compounds 1, 2, 3, and 4, as well as compounds 11, 28, 30, 31, 34, 35, 73, 79, and 118, all exhibit $IC_{50}$ values greater than 40 μM in their inhibitory activity against hERG potassium channel current.

### 9. Liver microsomal stability test

In this experiment, liver microsomes of five species, including human, dog, rat, and mouse, were used as *in vitro* models to evaluate the metabolic stability of the test compound.

At 37°C, 1 μM test compound was co-incubated with microsomal protein and coenzyme NADPH. At given time points of the reaction (5 min, 10 min, 20 min, 30 min and 60 min), the reaction was terminated by adding ice-cold acetonitrile containing an internal standard. The LC-MS/MS method was used to measure the concentration of the test compound in the sample. $T_{1/2}$ was calculated using the natural logarithm (In) of the residual rate of the drug in the incubation system and the incubation time. In addition, the intrinsic clearance in liver microsomes $CL_{int(mic)}$ and the intrinsic clearance in liver $CL_{int(Liver)}$ were further calculated.

Conclusion: The compounds of the present invention, such as the example compounds, have good stability in liver microsomes.

### 10. CYP450 enzyme inhibition test

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of various concentrations, and reduced nicotinamide adenine dinucleotide phosphate (NADPH) was added to initiate the reaction. After the reaction was completed, the sample was treated and liquid chromatography-tandem mass spectrometry (LC-MS/MS) was used to quantitatively detect metabolites produced by specific substrates, changes in CYP enzyme activity were determined, and $IC_{50}$ value was calculated to evaluate the inhibitory potential of the test compound on each CYP enzyme subtype.

Conclusion: The compounds of the present invention, such as the example compounds, do not exhibit significant inhibitory effects on the five isozymes of CYP.

### 11. Human CD34+ hematopoietic stem cell proliferation inhibition experiment

Human CD34+ Hematopoietic stem cells (TPCS, Cat.hmPB34-P-2CW) are CD34-positive stem cells obtained from human PBMCs through immunomagnetic bead sorting. The culture conditions are as follows: DPBS (Gibco, Cat.14190-144) +StemSpan SFEM II (STEMCELL, Cat.9655) +1X StemSpan CD34+ Expansion Supplement (STEMCELL, Cat.2691). First, 40 nL of DMSO, the positive reference compound Talazoparib, or the test compounds were respectively added to a 384-well plate (Corning, Cat. 3764). Then, a cell suspension was added at 400 cells per well in 40 μL of medium, resulting in final concentrations of 0.1% DMSO, 3 μM Talazoparib, or various concentrations of the compounds in each well. The plate was centrifuged at room temperature at 1000 rpm for 1 minute, followed by incubation for 7 days in a 37°C, 5% $CO_2$ incubator. Following incubation, 20 μL of CellTiter-Glo Reagent (Promega, Cat. G7573) was directly added to each well, and the plate was centrifuged at room temperature at 1000 rpm for 1 minute, followed by incubation for 20 minutes in the dark. Following incubation, the chemiluminescent signal (CFU) was read and recorded using an Envision multimode microplate reader (PerkinElmer, Cat. 2104). The cell proliferation inhibition rate at various compound concentrations was calculated according to Equation (3), where $CFU_{high}$ control represents the average signal value of the DMSO group, $CFU_{low}$ control represents the average signal value of the Talazoparib group, and $CFU_{compound}$

represents the average signal value of the compound-treated group. The processed data obtained from Equation (3) were analysed using XLfit or GraphPad Prism software, and a four-parameter curve fitting model was applied to calculate the compound concentration corresponding to a 50% inhibition rate, i.e., the $IC_{50}$ value.

$$\text{Inhibiton\%} = (CFU_{\text{high control}}\text{-}CFU_{\text{compound}}) / (CFU_{\text{high control}}\text{-}CFU_{\text{low control}}) \times 100\%$$

(Equation 3)

Conclusion: The compounds of the present invention, such as the example compounds, do not exhibit significant inhibitory effects on the proliferation of CD34+ hematopoietic stem cells. Specifically, the trifluoroacetate of compound 7, compound 11, and compound 31, all exhibit $IC_{50}$ values greater than 10,000 nM in their inhibitory activity against the proliferation of CD34+ hematopoietic stem cells.

**12. AR degradation activity studies in MDA-PCA-2B cells**

Human prostate cancer cells MDA-PCA-2B were cultured in a complete medium consisting of F-12K+20% FBS+25 ng/ml cholera toxin+10 ng/ml mouse Epidermal Growth Factor+0.005 mM phosphoethanolamine+100 pg/ml hydrocortisone+45 nM sodium selenite+0.005 mg/ml insulin, in a 37°C, 5% $CO_2$ incubator. Cells in the exponential growth phase were collected. The cell suspension was adjusted to the appropriate concentration using hormone-deprived medium (containing 10% charcoal-stripped serum) and seeded into a 12-well plate at a cell density of $5 \times 10^5$ cells per well. After seeding, the cells were cultured for 72 h in the incubator, after which the medium was replaced with the experimental medium (containing 1% charcoal-stripped serum) and various concentrations of compounds were added, and further incubated for 24 hours in the 37°C, 5% $CO_2$ incubator. Following incubation, the cells were washed with pre-cooled PBS, followed by lysis on ice for 15 minutes with complete cell lysis buffer containing Protease/Phosphatase Inhibitor Cocktail (cell lysis buffer information: CST, Cat. 9803, diluted to 1X before use; Protease/Phosphatase Inhibitor Cocktail (100X, information: Cat. 5872); subsequently, the Cocktail was diluted 100-fold with the 1X cell lysis buffer to obtain the complete cell lysis buffer). The cells were then scraped into new, pre-chilled EP tubes using a cell scraper and centrifuged at 13,500 rpm at 4°C for 20 minutes. The supernatant protein samples were collected and the protein was quantified using the BCA kit (Thermofisher, Cat. 23225). Then the protein was diluted to 2 mg/mL. The prepared samples were loaded at 5 μL (10 μg) onto a 4%-12% precast gel. The expression of Androgen Receptor (D6F11) XP® Rabbit mAb (CST, Cat. 5153S) and the internal reference β-Actin (CST, Cat. 3700S) was detected by the conventional Western blot method. Secondary antibodies used were HRP-conjugated Anti-rabbit IgG Antibody (CST, Cat. 7074V) and Anti-mouse IgG Antibody (CST, Cat. 7076V). The expression levels of AR and the internal reference were calculated using the protein expression quantification analysis software "Image Studio". Then, the degradation rate (% Degradation) of the compound at various concentrations was calculated according to Equation (4), where $R_{compound}$ represents the relative expression level of AR in the compound-treated groups at various concentrations, and $AVER_{DMSO}$ represents the average relative expression level of AR in the DMSO control group. Subsequently, GraphPad Prism software was used to generate the inhibition curve and calculate the $DC_{50}$.

$$\%\ \text{Degradation} = (AVE_R_{DMSO}\text{-}R_{compound}) / AVE_R_{DMSO} \times 100\%\ \text{Equation (4)}$$

Conclusion: The compounds of the present invention, such as the example compounds, exhibit good degradation activity against AR proteins in MDA-PCA-2B cells.

**13. MDA-PCA-2B cell proliferation inhibition experiment**

Human prostate cancer cells MDA-PCA-2B were purchased from ATCC, and cultured in a complete medium consisting of F-12K+20% FBS+25 ng/ml cholera toxin+10 ng/ml mouse Epidermal Growth Factor+0.005 mM phosphoethanolamine +100 pg/ml hydrocortisone + 45 nM sodium selenite + 0.005 mg/ml human recombinant insulin+1% PS, in a 37°C, 5% $CO_2$ incubator. At the beginning of the experiment, the cells were digested using 0.25% trypsin digestion solution containing phenol red (Trypsin-EDTA (1x), phenol red). The digestion was terminated with complete medium, followed by centrifugation. The cells were resuspended in experimental medium (DMEM/F-12, without phenol red + 1% charcoal-stripped FBS + 0.5% PS + 25 ng/ml cholera toxin + 10 ng/ml mouse Epidermal Growth Factor + 0.005 mM phosphoethanolamine + 100 pg/ml hydrocortisone + 45 nM sodium selenite + 0.005 mg/ml human recombinant insulin). Viable cell counting was performed using a cell counter, after which the cell suspension was adjusted to an appropriate concentration. 100 μl of the cell suspension was seeded per well into a 96-well cell culture plate at a cell density of 30,000 cells/well, followed by overnight incubation. On the following day, the diluted 2x working solution (containing the test samples at various concentrations and the exogenous androgen R1881, with a total volume of 100 μL) was added to

achieve a final total volume of 200 μL and a final R1881 concentration of 0.2 nM. The plate was placed in an incubator and incubated for another 7 days. Concurrently, T0 wells were set up on the day of compound treatment to measure the Day 0 cell viability using the CellCounting-lite 2.0 assay kit (Vazyme, DD1101-03), and the reading was recorded as RLU0. Following incubation, 100 μL of supernatant was removed from each well, and then 60 μl of pre-thawed and room temperature-equilibrated CellCounting-lite 2.0 solution was added to each well. The plate was mixed uniformly for 2 minutes using a microplate shaker, and maintained at room temperature for 30 minutes, and then the luminescence signal value was measured using a BMG multimode microplate reader.

The results were processed according to Equation (5). The inhibition rates at various compound concentrations were calculated in Excel, and the $GI_{50}$ values (the compound concentrations required to achieve 50% inhibition) were calculated using GraphPad software, where RLU compound represents the readout of the compound-treated group, and RLU control represents the average value of the solvent control group.

$$\text{inhibition \%} = 1\text{- (RLU compound-RLU0)/ (RLU control- RLU0)} \times 100\%$$

Equation (5)

Conclusion: The compounds of the present invention, such as the example compounds, exhibit good inhibitory activity against the proliferation of MDA-PCA-2B cells.

**14. LNCaP $AR^{F877L}$ cell proliferation inhibition experiment**

Prostate cancer cells LNCaP $AR^{F877L}$ were constructed in WuXi AppTec, the cell medium was RPMI1640+10% FBS, and the cells were cultured in a 37°C, 5% $CO_2$ incubator. Prior to the experiment, cells maintained in normal medium were passaged into phenol red-free RPMI 1640 containing 10% charcoal-stripped FBS and cultured for 3 days. On day 4, the cells were digested using phenol red-free digestion solution (Trypsin LE), and the digestion was stopped with phenol red-free RPMI 1640 medium containing 1% charcoal-stripped FBS+0.5% PS+0.1 nM R1881. After centrifugation and resuspension, viable cell counting was performed using a Vi-Cell XR cell counter. The cell suspension was adjusted to an appropriate concentration with phenol red-free RPMI 1640 medium containing 1% charcoal-stripped FBS+0.5% PS+0.1 nM R1881. Then, 180 μl of cell suspension was added per well to a 96-well cell culture plate at a density of 2500 cells/well. At the time of seeding, T0 wells were also prepared. The following day, R1881 at a final concentration of 0.1 nM and compounds at various concentrations were added. The plate was placed back into the incubator and cultured for an additional 7 days. At the time of compound addition, the $T_0$ plate was measured using the CellTiter-Glo (CTG) kit (Promega, Cat. No. G7572) and recorded as $RLU_0$. Following incubation, 75 μl of pre-thawed and room temperature-equilibrated CellTiter-Glo solution was added to each well. The plate was mixed uniformly for 2 minutes using a microplate shaker, and maintained at room temperature for 10 minutes, and then the luminescence signal value (RLU) was measured using an Envision 2104 plate reader. The results were processed according to Equation (6). The inhibition rates (Inhibition %) at various compound concentrations were calculated in Excel, and the $GI_{50}$ values (the compound concentrations required to achieve 50% inhibition) were calculated using GraphPad software, where $RLU_{compound}$ represents the readout of the compound-treated group, and RLU control represents the average value of the solvent control group.

$$\text{Inhibition \%} = 1\text{- }(RLU_{compound}-RLU_0)/(RLU_{control}-RLU_0) \times 100\% \quad \text{Equation (6)}$$

Conclusion: The compounds of the present invention, such as the example compounds, exhibit good inhibitory activity against the proliferation of LNCaP $\mathbf{AR^{F877L}}$ cells.

**15. AR degradation activity studies in LNCaP $AR^{F877L}$ $_{cells}$**

Prostate cancer cells LNCaP $\mathbf{AR^{F877L}}$ $_{were}$ constructed in WuXi AppTec, and cultured in a complete medium consisting of RPMI 1640+10% FBS+1% PS in a 37°C, 5% $CO_2$ incubator. Prior to the experiment, cells maintained in normal medium were passaged into phenol red-free RPMI 1640 containing 10% charcoal-stripped FBS and cultured for 3 days. On day 4, the cells were digested using phenol red-free digestion solution (Trypsin LE), and the cell suspension was adjusted to the appropriate concentration using hormone-deprived medium (containing 1% charcoal-stripped serum) and seeded into a 24-well plate at a cell density of 50,000 cells per well. After seeding, the cells were cultured for 24 h in the incubator, after which various concentrations of compounds were added, and further incubated for 24 hours in the 37°C, 5% $CO_2$ incubator. Following incubation, the cells were digested using phenol red-free digestion solution (Trypsin LE), and the digestion was stopped with phenol red-free RPMI 1640 medium containing 1% charcoal-stripped FBS+0.5% PS. The cells were collected into 1.5 mL centrifuge tubes, centrifuged, and resuspended. After washing twice with PBS, 20 μL of complete cell lysis buffer containing Protease/Phosphatase Inhibitor Cocktail (cell lysis buffer RIPA information: Sigma,

Cat. R0278; complete™ Mini protease inhibitor cocktail information: Roche, Cat. 04693124001; the complete cell lysis buffer was prepared by mixing RIPA and Complete™ Mini protease inhibitor cocktail according to the instructions.), pre-cooled with an ice bath, was added to each tube. The cells were lysed on ice for 30 minutes, followed by centrifugation at 12,000 × g and 4°C for 10 minutes. The supernatant protein samples were collected, SDS-PAGE protein loading buffer (5X) was added, and the samples were heated at a constant temperature of 100°C for 10 minutes. The prepared samples were loaded at 15 μL onto a 4%-12% precast gel. The expression of Androgen Receptor (D6F11) XP® Rabbit mAb (CST, Cat. 5153S) and the internal reference β-Actin (CST, Cat. 3700S) was detected by the conventional Western blot method. Secondary antibodies used were Anti-rabbit IgG (H+L) (DyLight™ 800 4X PEG Conjugate) (CST, Cat. 5151) and Anti-mouse IgG (H+L) (DyLight™ 680 Conjugate) (CST, Cat. 5470). The expression levels of AR and the internal reference were calculated using the protein expression quantification analysis software "Image Studio". Then, the degradation rate (% Degradation) of the compound at various concentrations was calculated according to Equation (7), where $R_{compound}$ represents the relative expression level of AR in the compound-treated groups at various concentrations, and $AVE_R_{DMSO}$ represents the average relative expression level of AR in the DMSO control group. Subsequently, GraphPad Prism software was used to generate the inhibition curve and calculate the $DC_{50}$.

$$\% \text{ Degradation}=(AVE_R_{DMSO}-R_{compound}) / AVE_R_{DMSO} \times 100\% \quad \text{Equation (7)}$$

Conclusion: The compounds of the present invention, such as the example compounds, exhibit good degradation activity against AR proteins in LNCaP **AR$^{F877L}$** cells.

The structure of the control compound is as follows:

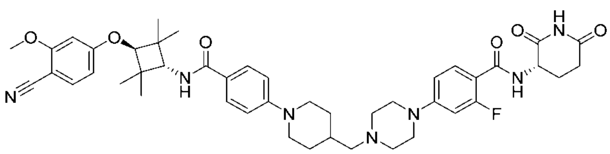

## Claims

**1.** A compound or a stereoisomer, tautomer, deuterated substance, solvate, prodrug, metabolite, pharmaceutically acceptable salt or co-crystal thereof, **characterised in that** the compound is a compound represented by general formula (I),

B-L-K (I);

L is -Ak1-Cy1-Ak2-Cy2-Ak3-Cy3-Ak4-Cy4-Ak5-;

Ak1, Ak2, Ak3, Ak4, and Ak5 are each independently selected from $-(CH_2)_q-$, $-(CH_2)_q-O-$, $-O-(CH_2)_q-$, $-(CH_2)_q-S-$, $-S-(CH_2)_q-$, $-(CH_2)_q-NR^L-$, $-NR^L-(CH_2)_q-$, - $(CH_2)_q-NR^LC(=O)-$, $-(CH_2)_q-C(=O)NR^L-$, -C(=O)-, $-C(=O)-(CH_2)_q-NR^L-$, $-(C{\equiv}C)_q-$ or a bond, wherein the $-CH_2-$ is optionally substituted with 1 to 2 substituents selected from deuterium, halogen, =O, OH, CN, $C_{1-4}$ alkyl or $C_{3-6}$ cycloalkyl;

q is selected from 0, 1, 2 or 3;

each $R^L$ is independently selected from H or $C_{1-4}$ alkyl;

each Cy1, Cy2, Cy3 or Cy4 is independently selected from a bond or one of the following groups optionally substituted with 1 to 4 $R^{L2}$: 4- to 7-membered mono-heterocyclyl, 4- to 12-membered fused heterocyclyl, 5- to 13-membered spiroheterocyclyl, 7- to 12-membered bridged heterocyclyl, $C_{3-7}$ monocycloalkyl, $C_{4-7}$ monocycloalkenyl, $C_{4-12}$ fused cycloalkyl, $C_{5-13}$ spirocycloalkyl, $C_{5-12}$ bridged cycloalkyl, 5- to 10-membered heteroaryl or $C_{6-10}$ aryl;

B is

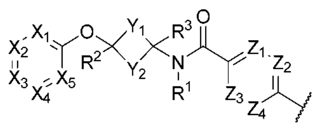;

$X_1$ is selected from N or $CR^{x1}$; $X_2$ is selected from N or $CR^{x2}$; $X_3$ is selected from N or $CR^{x3}$; $X_4$ is selected from N or $CR^{x4}$; $X_5$ is selected from N or $CR^{x5}$;
at most two of $X_1$, $X_2$, $X_3$, $X_4$, and $X_5$ are N;
$Z_1$ is selected from N or $CR^{z1}$; $Z_2$ is selected from N or $CR^{z2}$; $Z_3$ is selected from N or $CR^{z3}$; $Z_4$ is selected from N or $CR^{z4}$;
at most three of $Z_1$, $Z_2$, $Z_3$, and $Z_4$ are N;
$Y_1$ and $Y_2$ are each independently selected from $-CR^{y1}R^{y2}-$, $-(CR^{y1}R^{y2})_2-$, and - $(CR^{y1}R^{y2})_3-$;
$R^1$ is selected from H, deuterium, $C_{1-4}$ alkyl or $C_{3-6}$ cycloalkyl, wherein the alkyl or cycloalkyl is optionally substituted with 1 to 4 substituents selected from deuterium, halogen, OH, $NH_2$, CN, $C_{1-4}$ alkyl, and $C_{1-4}$ alkoxy;
$R^{x1}$, $R^{x2}$, $R^{x3}$, $R^{x4}$, $R^{x5}$, $R^{z1}$, $R^{z2}$, $R^{z3}$, and $R^{z4}$ are each independently selected from H, deuterium, halogen, OH, $NH_2$, CN, $NO_2$, COOH, $CONH_2$, $NHC_{1-4}$ alkyl, $N(C_{1-4}$ alkyl$)_2$, $C_{1-4}$ alkyl, $C_{2-4}$ alkenyl, $C_{2-4}$ alkynyl, $-OC_{1-4}$ alkyl, $-SC_{1-4}$ alkyl, $-C_{0-4}$ alkylene-$C_{3-6}$ carbocyclyl, $-C_{0-4}$ alkylene-4- to 6-membered heterocyclyl, 5- to 6-membered heteroaryl, $-O-C_{3-6}$ carbocyclyl, and -O-4- to 6-membered heterocyclyl, wherein the alkylene, alkyl, alkenyl, alkynyl, carbocyclyl, heterocyclyl, and heteroaryl are optionally substituted with 1 to 4 $R^s$;
or $R^{x1}$, $R^{x2}$, $R^{x3}$, and $R^{x4}$ are each independently selected from $-S(=O)_2NH_2$ and $-S(=O)_2C_{1-4}$ alkyl;
or $R^{x1}$ and $R^{x2}$, $R^{x2}$ and $R^{x3}$, $R^{x3}$ and $R^{x4}$, $R^{x4}$ and $R^{x5}$, $R^1$ and $R^{z3}$, and $R^1$ and $R^{z1}$ are directly connected to form $C_{4-6}$ carbocyclyl or 4- to 7-membered heterocyclyl, wherein the carbocyclyl or heterocyclyl is optionally substituted with 1 to 4 $R^5$;
$R^2$, $R^3$, $R^{y1}$, and $R^{y2}$ are each independently selected from H, deuterium, halogen, OH, $NH_2$, CN, $NO_2$, COOH, $CONH_2$, $C_{1-4}$ alkyl, $C_{2-4}$ alkenyl, $C_{2-4}$ alkynyl, $-OC_{1-4}$ alkyl, and $-SC_{1-4}$ alkyl, wherein the alkyl, alkenyl, and alkynyl are optionally substituted with 1 to 4 substituents selected from deuterium, halogen, OH, $NH_2$, CN, $C_{1-4}$ alkyl, and $C_{1-4}$ alkoxy;
K is

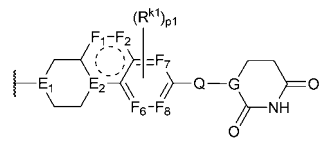

;

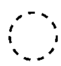

represents that the ring is an aromatic ring or a non-aromatic ring;
$F_1$ is selected from N, NH, CH, $CH_2$, $CHR^{k1}$, $NR^{k1}$, $CR^{k1}$, C(=O), and $C(R^{k1})_2$;
$F_2$ is selected from a bond, O, N, NH, CH, $CH_2$, $CHR^{k1}$, $NR^{k1}$, $CR^{k1}$ or $C(R^{k1})_2$;
$F_6$, $F_7$, and $F_8$ are each independently selected from N, C, CH or $CR^{k1}$, and at most two of $F_6$, $F_7$, and $F_8$ are N;
G is selected from CH or N;
$E_1$ is selected from N or CH;
$E_2$ is selected from C, N or CH;
each Q is independently selected from a bond, -O-, -S-, $-CH_2-$, $-NR^q-$, -C(=O)-, $-NR^qC(=O)-$, and $-C(=O)NR^q-$;
Q and G cannot be directly bonded to form an N-N or N-O bond;
each $R^q$ is independently selected from H or $C_{1-4}$ alkyl;
each $R^{k1}$ is independently selected from deuterium, halogen, OH, $NH_2$, CN, COOH, $CONH_2$, $C_{1-4}$ alkyl, $C_{2-4}$ alkenyl, $C_{2-4}$ alkynyl, $C_{1-4}$ alkoxy, $C_{3-6}$ cycloalkyl, and 4- to 6-membered heterocycloalkyl, wherein the alkyl, alkenyl, alkynyl, alkoxy, cycloalkyl, and heterocycloalkyl are optionally substituted with 1 to 4 $R^s$;
$R^{L2}$ and $R^s$ are each independently selected from deuterium, halogen, OH, CN, =O, $CF_3$, $SF_5$, $NO_2$, $NH_2$, $NHC_{1-4}$ alkyl, $N(C_{1-4}$ alkyl$)_2$, COOH, $CONH_2$, $C_{1-4}$ alkyl, $C_{2-4}$ alkenyl, $C_{2-4}$ alkynyl, $C_{1-4}$ alkoxy, $-S-C_{1-4}$ alkyl, $-C_{0-4}$ alkylene-$C_{3-6}$ cycloalkyl, and $-C_{0-4}$ alkylene-4- to 6-membered heterocyclyl, wherein the alkyl, alkylene, alkoxy, alkenyl, alkynyl, and cycloalkyl are optionally substituted with 1 to 4 substituents selected from deuterium, F, Cl, Br, I, OH, CN, $C_{1-4}$ alkyl, and $C_{1-4}$ alkoxy;
each pl is independently selected from 0, 1 or 2;
or each $R^s$ is independently $-O-C_{3-6}$ cycloalkyl.

**2.** The compound or the stereoisomer, tautomer, deuterated substance, solvate, prodrug, metabolite, pharmaceutically acceptable salt or co-crystal thereof according to claim 1, **characterised in that**

$R^{x1}$, $R^{x2}$, $R^{x3}$, $R^{x4}$, $R^{x5}$, $R^{z1}$, $R^{z2}$, $R^{z3}$, and $R^{z4}$ are each independently selected from H, deuterium, halogen, OH, $NH_2$, CN, $NO_2$, COOH, $CONH_2$, $NHC_{1-4}$ alkyl, $N(C_{1-4}$ alkyl$)_2$, $C_{1-4}$ alkyl, $C_{2-4}$ alkenyl, $C_{2-4}$ alkynyl, $-OC_{1-4}$ alkyl, $-SC_{1-4}$ alkyl, $-C_{0-2}$ alkylene-$C_{3-6}$ cycloalkyl, $-C_{0-2}$ alkylene-4- to 6-membered heterocyclyl, 5- to 6-membered heteroaryl, $-O-C_{3-6}$ cycloalkyl, and -O-4- to 6-membered heterocyclyl, wherein the alkylene, alkyl, alkenyl, alkynyl, cycloalkyl, heterocyclyl, and heteroaryl are optionally substituted with 1 to 4 $R^s$;

or $R^{x1}$, $R^{x2}$, $R^{x3}$, and $R^{x4}$ are each independently selected from $-S(=O)_2NH_2$ and $-S(=O)_2C_{1-4}$ alkyl;

or $R^{x1}$ and $R^{x2}$, $R^{x2}$ and $R^{x3}$, $R^{x3}$ and $R^{x4}$, and $R^{x4}$ and $R^{x5}$ are directly connected to form $C_{4-6}$ carbocyclyl, 5- to 6-membered heteroaryl or 5- to 7-membered heterocyclyl, wherein the carbocyclyl, heteroaryl or heterocyclyl is optionally substituted with 1 to 4 $R^5$;

or $R^1$ and $R^{z3}$, and $R^1$ and $R^{z1}$ are directly connected to form 5- to 7-membered heterocyclyl, wherein the heterocyclyl is optionally substituted with 1 to 4 $R^5$;

$R^L$ is selected from H, methyl or ethyl;

each $R^q$ is independently selected from H, methyl, ethyl or isopropyl;

Cy1, Cy2, Cy3, and Cy4 are each independently selected from a bond or one of the following groups optionally substituted with 1 to 4 $R^{L2}$: phenyl, pyridyl, pyrimidyl, pyrazinyl, pyridazinyl, pyrazolyl, thiazolyl, oxazolyl, triazolyl,

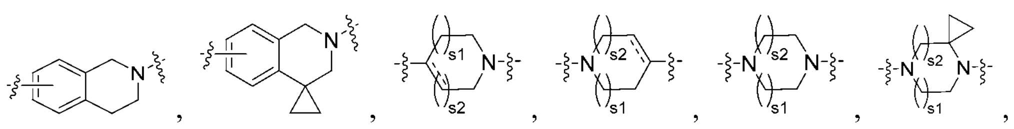

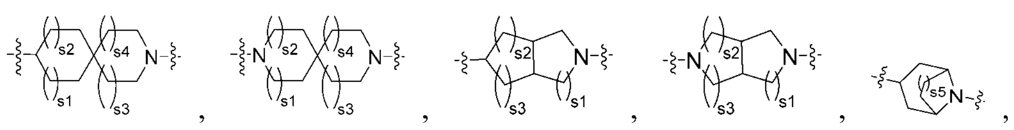

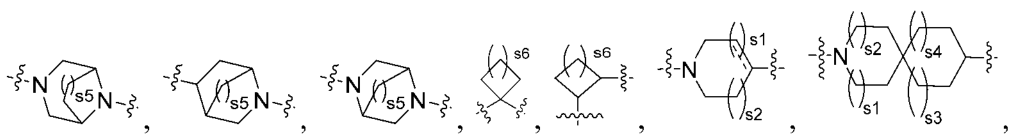

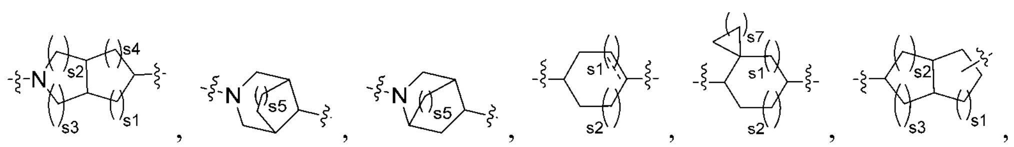

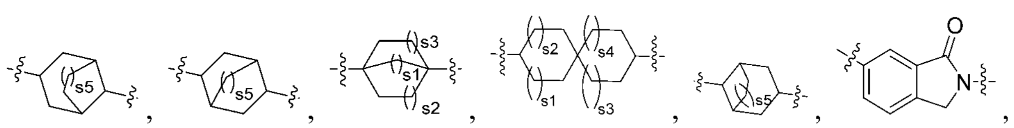

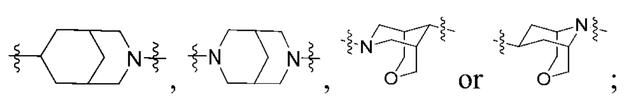

s1, s3 and s5 are each independently selected from 0, 1 or 2;

s2 and s4 are each independently selected from 0 or 1;

s6 is selected from 0, 1, 2 or 3;

s7 is selected from 1, 2 or 3;

$R^{L2}$ and $R^s$ are each independently selected from deuterium, halogen, OH, CN, =O, $CF_3$, $SF_5$, $NO_2$, $NH_2$, $NHC_{1-4}$ alkyl, $N(C_{1-4}$ alkyl$)_2$, COOH, $CONH_2$, $C_{1-4}$ alkyl, $C_{2-4}$ alkenyl, $C_{2-4}$ alkynyl, $C_{1-4}$ alkoxy, $-S-C_{1-4}$ alkyl, $-C_{0-2}$ alkylene-$C_{3-6}$ cycloalkyl, and $-C_{0-2}$ alkylene-4- to 6-membered heterocyclyl, wherein the alkyl, alkylene, alkoxy, alkenyl, alkynyl, and cycloalkyl are optionally substituted with 1 to 4 substituents selected from deuterium, F, Cl, Br, I, OH, CN, $C_{1-4}$ alkyl, and $C_{1-4}$ alkoxy;

or each $R^s$ is independently $-O-C_{3-6}$ cycloalkyl.

**3.** The compound or the stereoisomer, tautomer, deuterated substance, solvate, prodrug, metabolite, pharmaceutically acceptable salt or co-crystal thereof according to claim 2, **characterised in that**

$X_3$ is $CR^{x3}$;
$Y_1$ and $Y_2$ are each independently selected from $-CR^{y1}R^{y2}-$ and $-(CR^{y1}R^{y2})_2-$;
$R^1$ is selected from H, deuterium, methyl, ethyl, isopropyl, cyclopropyl, and cyclobutyl, wherein the methyl, ethyl, isopropyl, cyclopropyl, and cyclobutyl are optionally substituted with 1 to 4 substituents selected from deuterium, F, Cl, Br, I, OH, $NH_2$, CN, $C_{1-4}$ alkyl, and $C_{1-4}$ alkoxy;
$R^{x1}$, $R^{x2}$, $R^{x3}$, $R^{x4}$, $R^{x5}$, $R^{z1}$, $R^{z2}$, $R^{z3}$, and $R^{z4}$ are each independently selected from H, deuterium, F, Cl, Br, I, OH, $NH_2$, CN, $NO_2$, COOH, $CONH_2$, $N(CH_3)_2$, $NHCH_3$, or one of the following groups optionally substituted with 1 to 4 $R^s$: methyl, ethyl, vinyl, ethynyl, methoxy, ethoxy, methylthio, cyclopropyl, cyclobutyl, cyclopentyl, $-CH_2$-cyclopropyl, $-CH_2$-cyclobutyl, -O-cyclopropyl, and pyrazolyl;
or $R^{x1}$, $R^{x2}$, $R^{x3}$, and $R^{x4}$ are each independently selected from $-S(=O)_2NH_2$, - $S(=O)_2$ methyl, and $-S(=O)_2$ ethyl;
or $R^{x1}$ and $R^{x2}$, $R^{x2}$ and $R^{x3}$, $R^{x3}$ and $R^{x4}$, and $R^{x4}$ and $R^{x5}$ are directly connected to form one of the following groups optionally substituted with 1 to 3 $R^s$: piperidyl, cyclohexyl, phenyl, pyridyl, pyrimidyl, pyridazinyl, and pyrazinyl;
or $R^{x1}$ and $R^{x2}$, $R^{x2}$ and $R^{x3}$, $R^{x3}$ and $R^{x4}$, and $R^{x4}$ and $R^{x5}$ are directly connected to form one of the following groups optionally substituted with 1 to 3 $R^s$: pyrrolidyl, oxolanyl, oxanyl, and 1,3-dioxolane;
or $R^1$ and $R^{z3}$, and $R^1$ and $R^{z1}$ are directly connected to form the following structure optionally substituted with 1 to 4 $R^s$:

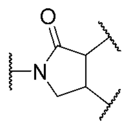
, or
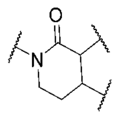
;

$R^2$, $R^3$, $R^{y1}$, and $R^{y2}$ are each independently selected from H, deuterium, F, Cl, Br, I, OH, $NH_2$, CN, $NO_2$, COOH, $CONH_2$, methyl, ethyl, vinyl, ethynyl, methoxy, ethoxy, and methylthio, wherein the methyl, ethyl, vinyl, ethynyl, methoxy, ethoxy, and methylthio are substituted with 1 to 4 substituents selected from deuterium, F, Cl, Br, I, OH, $NH_2$, CN, $C_{1-4}$ alkyl, and $C_{1-4}$ alkoxy;
Akl, Ak2, Ak3, Ak4, and Ak5 are each independently selected from a bond, - O-, -S-, $-OCH_2-$, $-CH_2O-$, $-OCH_2CH_2-$, $-CH_2CH_2O-$, -C=C-, $-CH(CH_3)-$, $-CH_2-$, - $C(CH_3)_2-$, $-CH_2CH_2-$, $-CH_2CH_2CH_2-$, $-N(CH_3)-$, -NH-, $-CH_2N(CH_3)-$, $-CH_2NH-$, - $NHCH_2-$, $-CH_2CH_2N(CH_3)-$, $-CH_2CH_2NH-$, $-NHCH_2CH_2-$, -C(=O)-, - $C(=O)CH_2NH-$, $-CH_2C(=O)NH-$, -C(=O)NH- or -NHC(=O)-;
K is selected from

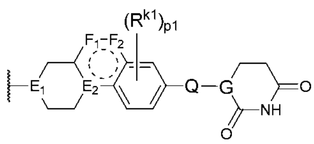
,
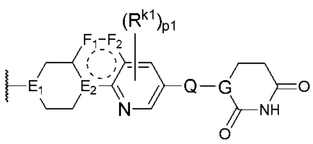
,

and

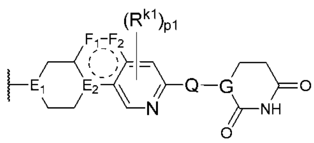
;

each Q is independently selected from a bond, $CH_2$, NH, $N(CH_3)$, O, S, C(=O), NHC(=O), C(=O)NH, $N(CH_3)C(=O)$, and $C(=O)N(CH_3)$;
each $R^{k1}$ is independently selected from deuterium, F, Cl, Br, I, OH, $NH_2$, CN, COOH, $CONH_2$, methyl, ethyl, isopropyl, methoxy, ethoxy, isopropoxy, and cyclopropyl, wherein the methyl, ethyl, isopropyl, methoxy, ethoxy, isopropoxy, and cyclopropyl are optionally substituted with 1 to 4 substituents selected from deuterium, F, Cl, Br, I, OH, and $NH_2$;
$R^{L2}$ and $R^s$ are each independently selected from deuterium, F, Cl, Br, I, OH, =O, $CF_3$, $SF_5$, CN, $NH_2$, $NO_2$, COOH, $CONH_2$, $N(CH_3)_2$, $NHCH_3$, methyl, ethyl, vinyl, ethynyl, methoxy, ethoxy, methylthio, cyclopropyl, cyclobutyl,

cyclopentyl, cyclohexyl, -$CH_2$-cyclopropyl, -$CH_2$-cyclobutyl, -$CH_2$-cyclopentyl, and -$CH_2$-cyclohexyl, wherein the methyl, ethyl, vinyl, ethynyl, methoxy, ethoxy, methylthio, cyclopropyl, cyclobutyl, cyclopentyl, and cyclohexyl are optionally substituted with 1 to 4 substituents selected from deuterium, F, Cl, Br, I, OH, CN, $C_{1-4}$ alkyl, and $C_{1-4}$ alkoxy;

or each $R^s$ is independently selected from propynyl and -O-cyclopropyl.

**4.** The compound or the stereoisomer, tautomer, deuterated substance, solvate, prodrug, metabolite, pharmaceutically acceptable salt or co-crystal thereof according to claim 3, **characterised in that**

B is selected from

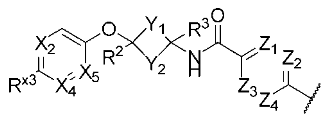, 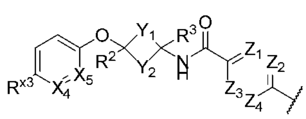,

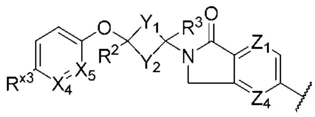, and 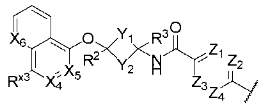;

or B is

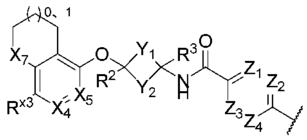;

$X_6$ is selected from N or CH;
$X_7$ is selected from NH or O;
at most two of $Z_1$, $Z_2$, $Z_3$, and $Z_4$ are N;
$R^2$ and $R^3$ are each independently selected from H, deuterium, F, Cl, and Br; $R^{y1}$ and $R^{y2}$ are each independently selected from H, deuterium, F, Cl, Br, I, OH, $NH_2$, CN, $NO_2$, COOH, $CONH_2$, methyl, ethyl, vinyl, ethynyl, methoxy, ethoxy, and methylthio, wherein the methyl, ethyl, vinyl, ethynyl, methoxy, ethoxy, and methylthio are substituted with 1 to 4 substituents selected from deuterium, F, Cl, Br, I, OH, $NH_2$, CN, methyl, ethyl, and methoxy;
Cy1, Cy2, Cy3, and Cy4 are each independently selected from a bond or one of the following optionally substituted groups:

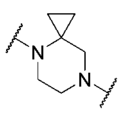, 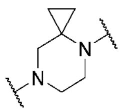, 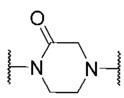,

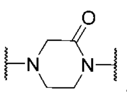, 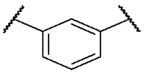, 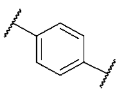, 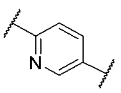, 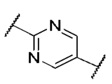, 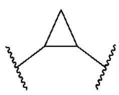, 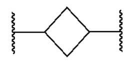,

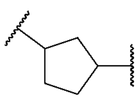, 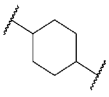, 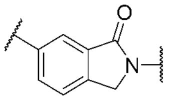, 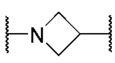, 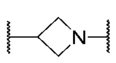, 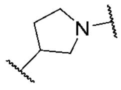, 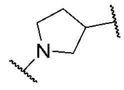,

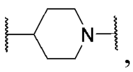, 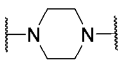, 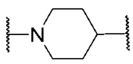, 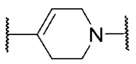, 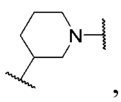, 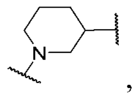, 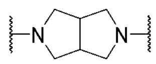,

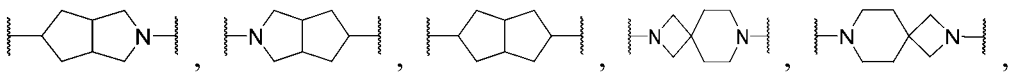

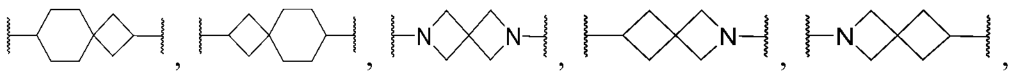

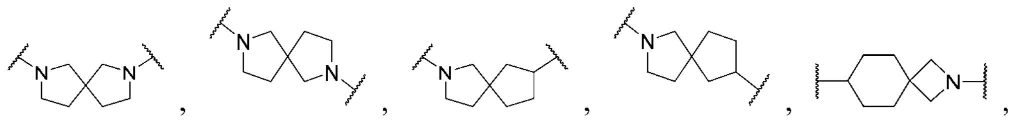

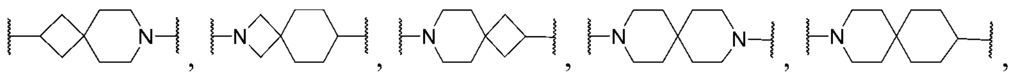

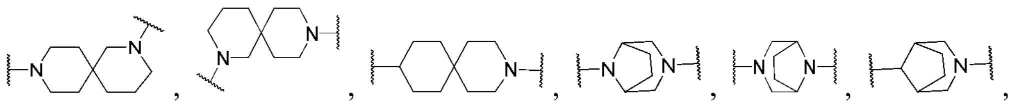

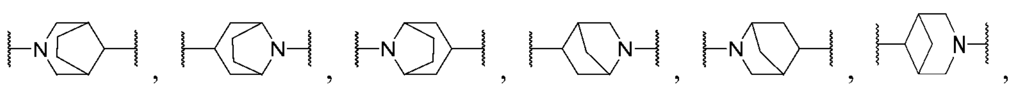

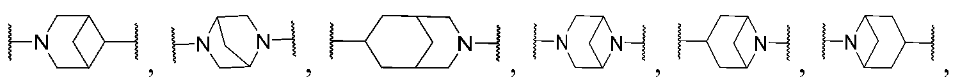

and

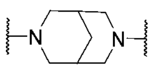,

which, when substituted, are substituted with 1 to 4 substituents selected from deuterium, F, $CF_3$, OH, =O, COOH, CN, $NH_2$, hydroxymethyl, methyl, methoxy, and cyclopropyl;
K is selected from

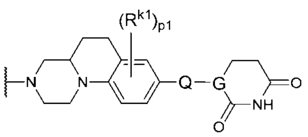, 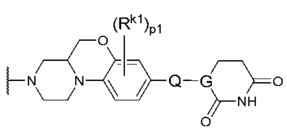,

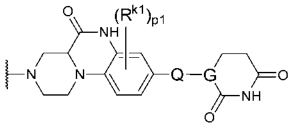, 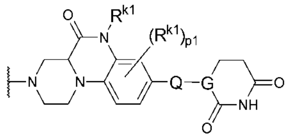, 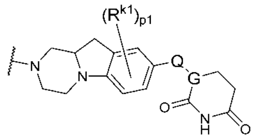,

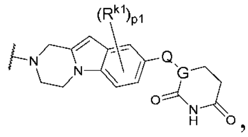, and 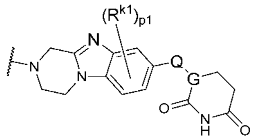;

or K is selected from

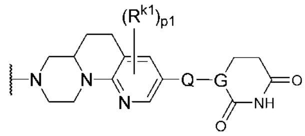 and 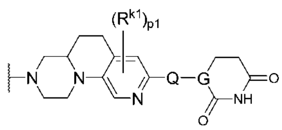 .

**5.** The compound or the stereoisomer, tautomer, deuterated substance, solvate, prodrug, metabolite, pharmaceutically acceptable salt or co-crystal thereof according to claim 4, **characterised in that**

B is selected from

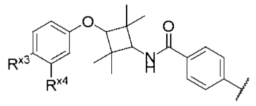 , 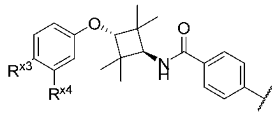 ,

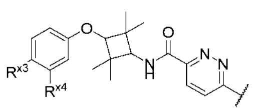 , 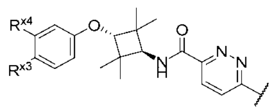 , 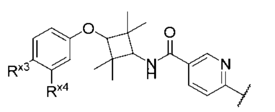 ,

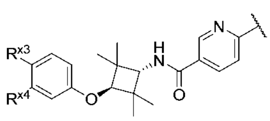 , 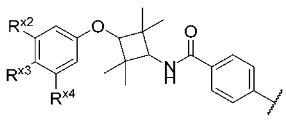 , 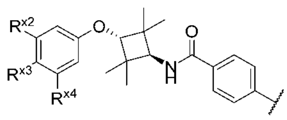 ,

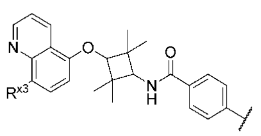 , 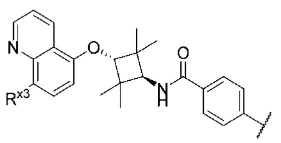 , 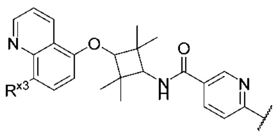 ,

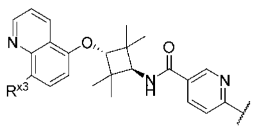 , 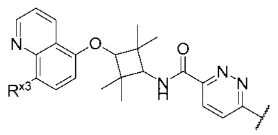 , 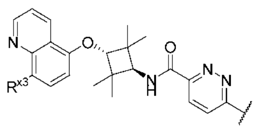 ,

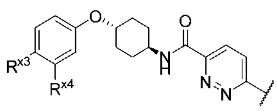 , 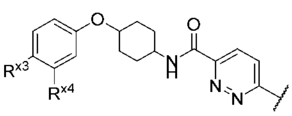 , 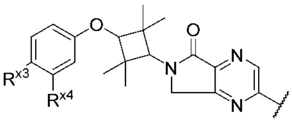 ,

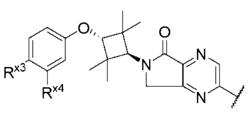 , 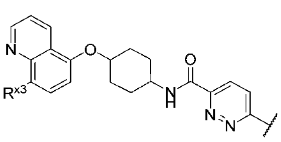 , and 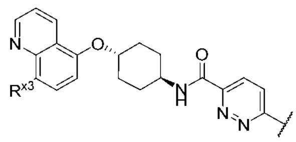 ;

or B is selected from

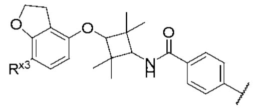 , 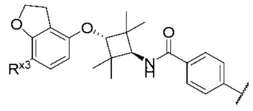 ,

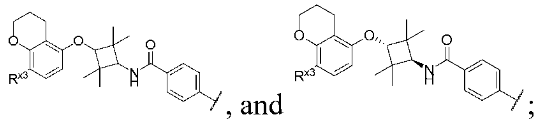

$R^{x2}$, $R^{x3}$, and $R^{x4}$ are each independently selected from H, deuterium, F, Cl, Br, I, OH, $NH_2$, CN, $NO_2$, COOH, $CONH_2$, $N(CH_3)_2$, $NHCH_3$, $CF_3$, $CHF_2$, $CH_2F$, $OCF_3$, $OCH_2F$, $OCD_3$, $CH_2OH$, methyl, ethyl, vinyl, ethynyl, methoxy, ethoxy, methylthio, cyclopropyl, -$CH_2$-cyclopropyl, and -O-cyclopropyl;

or $R^{x2}$ and $R^{x4}$ are each independently selected from -$S(=O)_2CH_3$, -O-$CH_2$-propynyl, -O-$CH_2$-cyclopropyl, -O-$CH_2CH_2$-$OCH_3$, -O-$CH_2CH_2$-O-cyclopropyl,

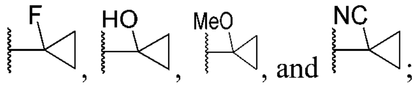

each Q is independently selected from a bond, NH, $N(CH_3)$, O, S, NHC(=O), C(=O)NH, $N(CH_3)C(=O)$, and $C(=O)N(CH_3)$;

L is selected from -Cy1-, -Cy1-Ak2-, -Cy1-$CH_2$-, -Ak1-Cy1-, -Cy1-Cy2-, - Cy1-$CH_2$-Cy2-, -Cy1-Cy2-Cy3-, -Cy1-$CH_2$-Cy2-Cy3-, and -Cy1-Cy2-$CH_2$-Cy3-;

or L is selected from -Cy1-Ak2-Cy2- and -Cy1-O-Cy2-;

Cy1, Cy2, and Cy3 are each independently selected from one of the following optionally substituted groups:

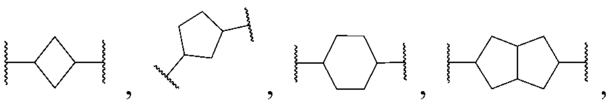

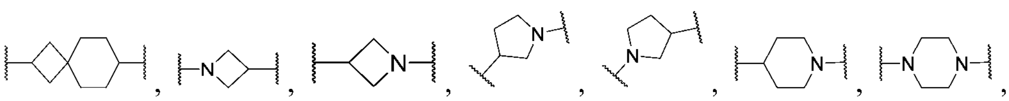

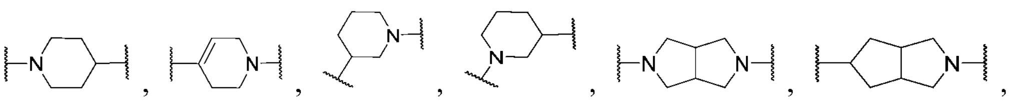

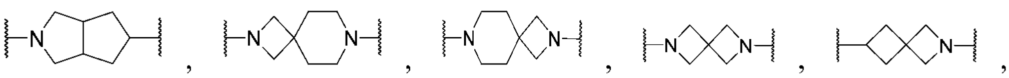

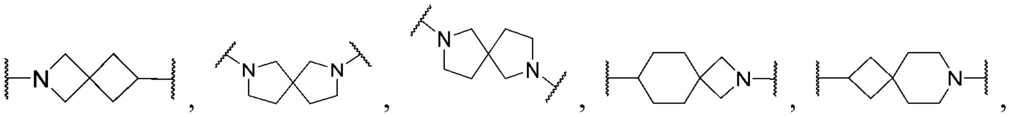

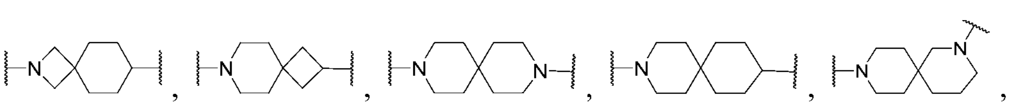

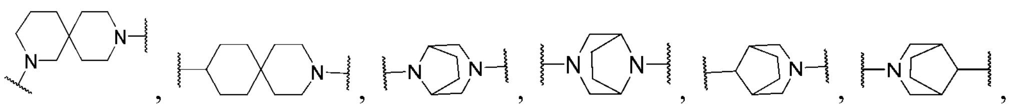

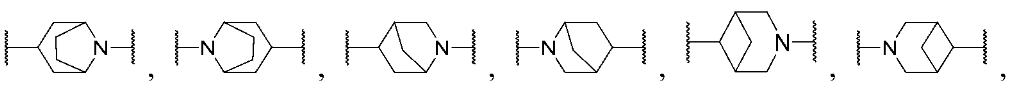

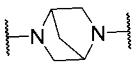, 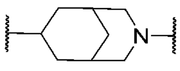, 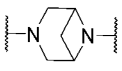, 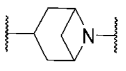, 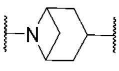, and 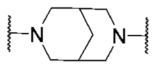,

which, when substituted, are substituted with 1 to 4 substituents selected from deuterium, F, $CF_3$, OH, =O, COOH, CN, $NH_2$, hydroxymethyl, methyl, methoxy, and cyclopropyl.

**6.** The compound or the stereoisomer, tautomer, deuterated substance, solvate, prodrug, metabolite, pharmaceutically acceptable salt or co-crystal thereof according to claim 5, **characterised in that**

K is selected from

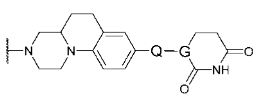, 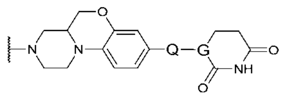,

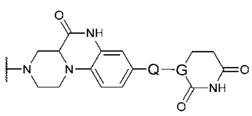, 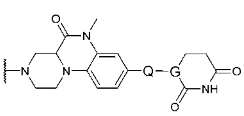, 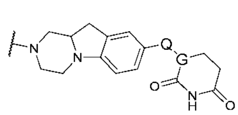, 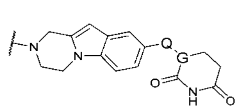,

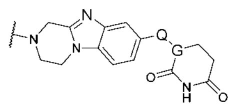, 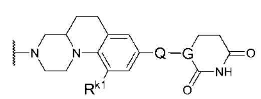, 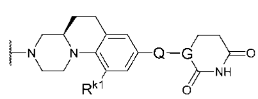,

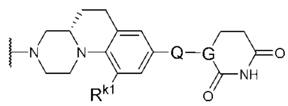, 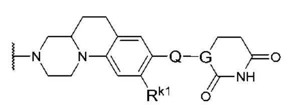, 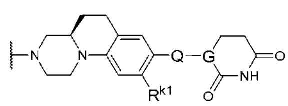,

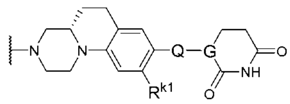, 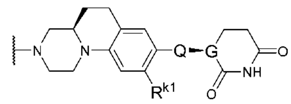, 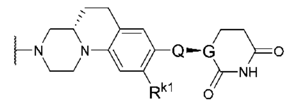,

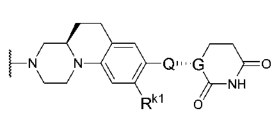, 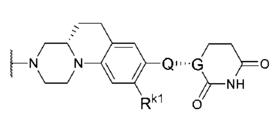, 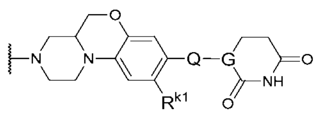,

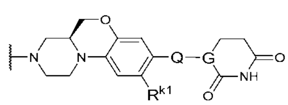, 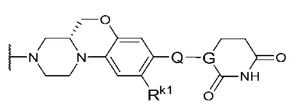, 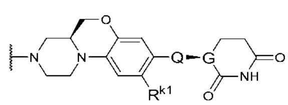,

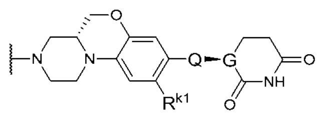, 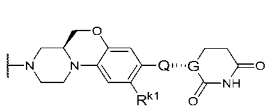, 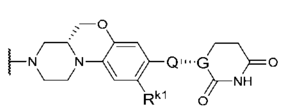,

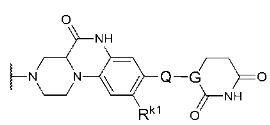, 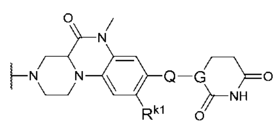, 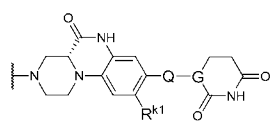,

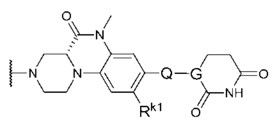, 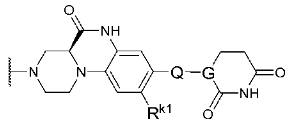, 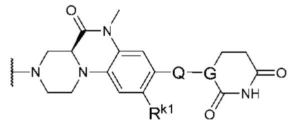,

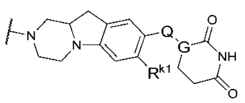, 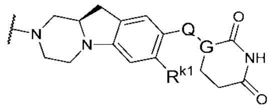, 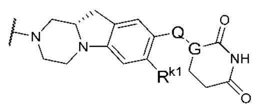,

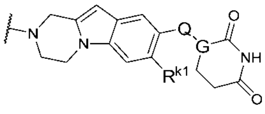, and 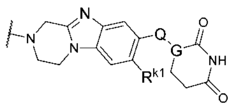;

or K is selected from

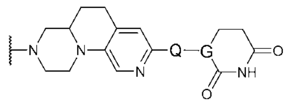, 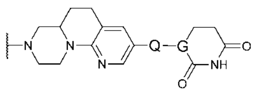,

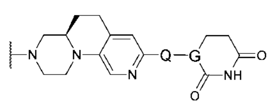, 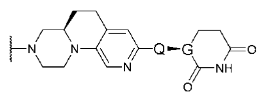, 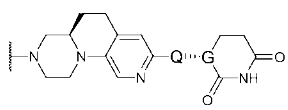,

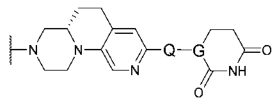, 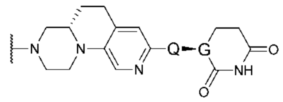, 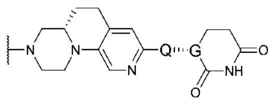,

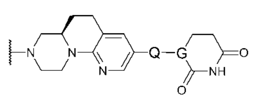, 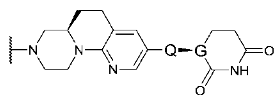, 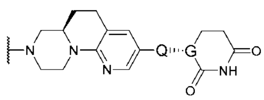,

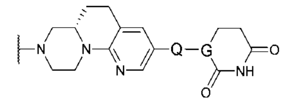, 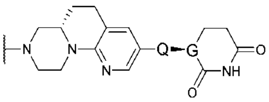, and 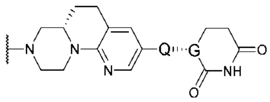;

each Q is independently selected from a bond, NH, and C(=O)NH;
each $R^{k1}$ is independently selected from deuterium, F, Cl, Br, I, OH, $NH_2$, CN, COOH, $CONH_2$, $CF_3$, $CHF_2$, $CH_2F$, $OCF_3$, $OCH_2F$, $CH_2OH$, methyl, ethyl, isopropyl, methoxy, ethoxy, isopropoxy, and cyclopropyl;
L is selected from one of the structural fragments shown in Table L-1.

7. The compound or the stereoisomer, tautomer, deuterated substance, solvate, prodrug, metabolite, pharmaceutically acceptable salt or co-crystal thereof according to claim 1, **characterised in that** the compound has a structure selected from one of the structures shown in Table E.

8. A pharmaceutical composition, **characterised by** comprising the compound or the stereoisomer, tautomer, deuterated substance, solvate, prodrug, metabolite, pharmaceutically acceptable salt or co-crystal thereof according to any one of claims 1-7, and a pharmaceutically acceptable carrier, wherein preferably, the pharmaceutical composition comprises 1-1500 mg of the compound or the stereoisomer, tautomer, deuterated substance, solvate, prodrug, metabolite, pharmaceutically acceptable salt or co-crystal thereof according to any one of claims 1-7.

9. Use of the compound or the stereoisomer, tautomer, deuterated substance, solvate, prodrug, metabolite, pharmaceutically acceptable salt or co-crystal thereof according to any one of claims 1-7, or the pharmaceutical composition according to claim 8 in the preparation of a medicament for the treatment of a disease related to AR activity or expression.

**10.** Use of the compound or the stereoisomer, tautomer, deuterated substance, solvate, prodrug, metabolite, pharmaceutically acceptable salt or co-crystal thereof according to any one of claims 1-7, or the pharmaceutical composition according to claim 8 in the preparation of a medicament for the treatment of a disease related to the inhibition or degradation of AR.

**11.** The use according to claim 10, **characterised in that** the disease is cancer, preferably prostate cancer.

**12.** A method for treating a disease in a mammal, **characterised by** comprising administering to a subject a therapeutically effective amount of the compound or the stereoisomer, tautomer, deuterated substance, solvate, prodrug, metabolite, pharmaceutically acceptable salt or co-crystal thereof according to any one of claims 1-7, or the pharmaceutical composition according to claim 8, wherein the therapeutically effective amount is preferably 1-1500 mg, and the disease is preferably cancer, more preferably prostate cancer.

**INTERNATIONAL SEARCH REPORT**

International application No.
**PCT/CN2024/112785**

**A. CLASSIFICATION OF SUBJECT MATTER**

C07D 471/04(2006.01)i; C07D 471/12(2006.01)i; A61K 31/4985(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C07D; A61K; A61P

Documentation searched other than minimum documentation to the extent that such documents are included in the fields searched

Electronic data base consulted during the international search (name of data base and, where practicable, search terms used)

CNTXT, ENTXTC, DWPI, CNKI, (CAPLUS, REGISTRY) STN: 雄激素受体, 靶向蛋白降解, androgen receptor, PROTAC, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| Y | WO 2021231927 A1 (THE REGENTS OF THE UNIVERSITY OF MICHIGAN) 18 November 2021 (2021-11-18)<br>claims 1-49, abstract, and description, pages 36-82, table 1 | 1-12 |
| Y | WO 2022262782 A1 (MEDSHINE DISCOVERY INC.) 22 December 2022 (2022-12-22)<br>claims 1-11 | 1-12 |
| Y | CN 114163444 A (JIANGSU HENGRUI MEDICINE CO., LTD. et al.) 11 March 2022 (2022-03-11)<br>claims 1-27 | 1-12 |
| Y | WO 2021127443 A1 (ARVINAS OPERATIONS, INC.) 24 June 2021 (2021-06-24)<br>claims 1-69 | 1-12 |
| Y | US 2021087171 A1 (ACCUTAR BIOTECHNOLOGY INC.) 25 March 2021 (2021-03-25)<br>claims 84-117 | 1-12 |
| Y | CN 116496318 A (SICHUAN HAISCO PHARMACEUTICAL CO., LTD.) 28 July 2023 (2023-07-28)<br>claims 1-12 | 1-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 October 2024** | **11 November 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/112785**

**C. DOCUMENTS CONSIDERED TO BE RELEVANT**

| Category* | Citation of document, with indication, where appropriate, of the relevant passages | Relevant to claim No. |
|---|---|---|
| A | CN 114641337 A (THE REGENTS OF THE UNIVERSITY OF MICHIGAN) 17 June 2022 (2022-06-17)<br>claims 1-77, and description, embodiment 12 | 1-12 |
| A | WO 2022069520 A1 (ASTRAZENECA AB) 07 April 2022 (2022-04-07)<br>claims 1-71 | 1-12 |

Form PCT/ISA/210 (second sheet) (July 2022)

**INTERNATIONAL SEARCH REPORT**

International application No.
**PCT/CN2024/112785**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12**
because they relate to subject matter not required to be searched by this Authority, namely:
Claim 12 relates to a method for treating diseases. The search is performed on the basis of the use of the compound for preparing a drug for treating corresponding diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/112785**

| Patent document cited in search report | | | Publication date (day/month/year) | Patent family member(s) | | | Publication date (day/month/year) |
|---|---|---|---|---|---|---|---|
| WO | 2021231927 | A1 | 18 November 2021 | US | 2023357249 | A1 | 09 November 2023 |
| WO | 2022262782 | A1 | 22 December 2022 | CN | 117580575 | A | 20 February 2024 |
| CN | 114163444 | A | 11 March 2022 | CN | 114163444 | B | 14 July 2023 |
| WO | 2021127443 | A1 | 24 June 2021 | CA | 3165168 | A1 | 24 June 2021 |
| | | | | US | 2021196710 | A1 | 01 July 2021 |
| | | | | TW | 202136240 | A | 01 October 2021 |
| | | | | AU | 2020405129 | A1 | 23 June 2022 |
| | | | | KR | 20220119094 | A | 26 August 2022 |
| | | | | CN | 115175901 | A | 11 October 2022 |
| | | | | EP | 4077309 | A1 | 26 October 2022 |
| | | | | JP | 2023507570 | W | 24 February 2023 |
| | | | | US | 11883393 | B2 | 30 January 2024 |
| | | | | CN | 115175901 | B | 22 March 2024 |
| | | | | US | 2024131023 | A1 | 25 April 2024 |
| | | | | CN | 118059104 | A | 24 May 2024 |
| US | 2021087171 | A1 | 25 March 2021 | US | 11535606 | B2 | 27 December 2022 |
| | | | | WO | 2021061644 | A1 | 01 April 2021 |
| CN | 116496318 | A | 28 July 2023 | | None | | |
| CN | 114641337 | A | 17 June 2022 | CA | 3151824 | A1 | 04 March 2021 |
| | | | | WO | 2021041664 | A1 | 04 March 2021 |
| | | | | AU | 2020336381 | A1 | 03 March 2022 |
| | | | | EP | 4021580 | A1 | 06 July 2022 |
| | | | | US | 2022388978 | A1 | 08 December 2022 |
| | | | | JP | 2022545735 | A | 28 October 2022 |
| WO | 2022069520 | A1 | 07 April 2022 | CA | 3195695 | A1 | 07 April 2022 |
| | | | | KR | 20230079408 | A | 07 June 2023 |
| | | | | CN | 116249554 | A | 09 June 2023 |
| | | | | EP | 4221756 | A1 | 09 August 2023 |
| | | | | JP | 2023543299 | W | 13 October 2023 |
| | | | | US | 2023374007 | A1 | 23 November 2023 |
| | | | | IN | 202317029427 | A | 24 November 2023 |
| | | | | HK | 40098244 | A0 | 28 March 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022235945 A
- WO 2021127443 A
- CN 115175901
- US 2023135173 A
- CN 115974840
- WO 2021231927 A
- WO 2023066350 A
- WO 2021249534 A1
- CN 112912376


### Non-patent literature cited in the description


- *CHEMICAL ABSTRACTS*, 1122-58-3
- *CHEMICAL ABSTRACTS*, 128-08-5
- *CHEMICAL ABSTRACTS*, 148893-10-1
- *Bioorganic & Medicinal Chemistry Letters*, 2016, vol. 26, 5877-5882
- *Bioorg. Med. Chem. Lett.*, 2016, vol. 26, 5877-5882
- *Tetrahedron Letters*, 2018, vol. 59, 2030-2033